# EUROPEAN PATENT SPECIFICATION

(11) **EP 2 694 545 B1**
(45) Date of publication and mention of the grant of the patent: **24.08.2016**
(21) Application number: 12711200.1
(22) Date of filing: 30.03.2012
(51) Int. Cl.: C07K 16/18, C07K 16/22, C07K 16/46

(54) **BISPECIFIC BINDING MOLECULES BINDING TO DLL4 AND ANG2**
BISPEZIFISCHE, AN DII4 UND ANG2 BINDENDE BINDEMOLEKÜLE
MOLÉCULES DE LIAISON BISPÉCIFIQUES SE LIANT À DLL4 ET ANG2

(30) Priority: 01.04.2011 EP 11160920
(43) Date of publication of application: 12.02.2014
(73) Proprietor: Boehringer Ingelheim International GmbH, 55216 Ingelheim am Rhein (DE)
(72) Inventor: GSCHWIND, Andreas, 55216 Ingelheim am Rhein (DE); OTT, Rene Georg, 55216 Ingelheim am Rhein (DE); BOUCNEAU, Joachim, 9840 De Pinte (BE); BUYSE, Marie-Ange, 9820 Merelbeke (BE); DEPLA, Erik, 9070 Destelbergen (BE)
(74) Representative: Simon, Elke Anna Maria
(86) International application number: PCT/EP2012/055897
(87) International publication number: WO 2012/131076

(56) References cited:
- WO-A1-2010/032060
- WO-A1-2010/040508
- WO-A1-2011/014469
- WO-A2-2007/143689
- WO-A2-2008/076379
- WO-A2-2011/039368
- J. ZHANG ET AL: "Angiopoietin-1/Tie2 Signal Augments Basal Notch Signal Controlling Vascular Quiescence by Inducing Delta-Like 4 Expression through AKT-mediated Activation of -Catenin", JOURNAL OF BIOLOGICAL CHEMISTRY, vol. 286, no. 10, 11 March 2011 (2011-03-11), pages 8055-8066, XP055004080, ISSN: 0021-9258, DOI: 10.1074/jbc.M110.192641
- Minhong Yan ET AL: "Chronic DLL4 blockade induces vascular neoplasms", Nature, vol. 463, no. 7282, 11 February 2010 (2010-02-11), pages E6-E7, XP055200996, ISSN: 0028-0836, DOI: 10.1038/nature08751

## Description

### FIELD OF THE INVENTION

The invention relates to the field of human therapy, in particular cancer therapy and agents and compositions useful in such therapy.

### BACKGROUND OF THE INVENTION

When tumors reach a critical size of approximately 1 mm³ they become dependent on angiogenesis for maintaining blood supply with oxygen and nutritients to allow for further growth. Anti-angiogenesis therapies have become an important treatment option for several types of tumors. These therapies have focused on blocking the VEGF pathway (Ferrara et al., Nat Rev Drug Discov. 2004 May;3(5):391-400.) by neutralizing VEGF (Avastin) or its receptors (Sutent and Sorafinib). Recent studies in mice have shown, that Angiopoietin2 (Ang2), a ligand of the Tie2 receptor, controls vascular remodeling by enabling the functions of other angiogenic factors, such as VEGF. Ang2 is primarily expressed by endothelial cells, strongly induced by hypoxia and other angiogenic factors and has been demonstrated to regulate tumor vessel plasticity, allowing vessels to respond to VEGF and FGF2 (Augustin et al., Nat Rev Mol Cell Biol. 2009 Mar;10(3):165-77.). Consistent with this role, the deletion or inhibition of Ang2 results in reduced angiogenesis (Falcón et al., Am J Pathol. 2009 Nov;175(5):2159-70.). Elevated Ang2 serum concentrations have been reported for patients with colorectal cancer, NSCLC and melanoma (Goede et al., Br J Cancer. 2010 Oct 26;103(9):1407-14),(Park et al., Chest. 2007 Jul;132(1): 200-6.),(Helfrich et al., Clin Cancer Res. 2009 Feb 15;15(4):1384-92.). In CRC cancer Ang2 serum levels correlate with therapeutic response to anti-VEGF therapy.

The Ang-Tie system consists of 2 receptors (Tie1 and Tie2) and 3 ligands (Ang1, Ang2 and Ang4) (Augustin et al., Nat Rev Mol Cell Biol. 2009 Mar;10(3):165-77.). Tie2, Ang1 and Ang2 are the best studied members of this family, Tie1 is an orphan receptor and the role of Ang4 for vascular remodelling still needs to be defined. Ang2 and Ang1 mediate opposing functions upon Tie2 binding and activation. Ang2-mediated Tie2 activation results in endothelial cell activation, pericyte dissociation, vessel leakage and induction of vessel sprouting. In contrast to Ang2, Ang1 signaling maintains vessel integrity by recruitment of pericytes, thereby maintaining endothelial cell quiescence.

Angiopoietin 2 (Ang2) is a secreted, 66 kDa ligand for the Tie2 receptor tyrosine kinase (Augustin et al., Nat Rev Mol Cell Biol. 2009 Mar;10(3):165-77.). Ang2 consists of an N-terminal coiled-coil domain and a C-terminal fibrinogen-like domain, the latter is required for Tie2 interaction. Ang2 is primarily expressed by endothelial cells and strongly induced by hypoxia and other angiogenic factors, including VEGF. Tie2 is found on endothelial cells, haematopoietic stem cells and tumor cells. Ang2-Tie2 has been demonstrated to regulate tumor vessel plasticity, allowing vessels to respond to VEGF and FGF2.

In vitro Ang2 has been shown to act as a modest mitogen, chemoattractant and inducer of tube formation in human umbilical vein endothelial cells (HUVEC). Ang2 induces tyrosine phosphorylation of ectopically expressed Tie2 in fibroblasts and promotes downstream signaling events, such as phosphorylation of ERK-MAPK, AKT and FAK in HUVEC. An antagonistic role of Ang2 in Ang1-induced endothelial cell responses has been described.

Ang2 -deficiency has been shown to result in a profound lymphatic patterning defect in mice. Although the loss of Ang2 is dispensable for embryonic vascular development, Ang2 -deficient mice have persistent vascular defects in the retina and kidney. Together with the dynamic pattern of Ang2 expression at sites of angiogenesis (for example ovary), these findings indicate that Ang2 controls vascular remodeling by enabling the functions of other angiogenic factors, such as VEGF.

The Ang2-Tie2 system exerts crucial roles during the angiogenic switch and later stages of tumor angiogenesis. Ang2 expression is strongly up-regulated in the tumor-associated endothelium. Reduced growth of tumors has been observed when implanted into Ang2 -deficient mice, especially during early stages of tumor growth. Therapeutic blocking of Ang2 with Ang2 mAbs has shown broad efficacy in a variety of tumor xenograft models.

As summarized in US 2008/0014196, angiogenesis is implicated in the pathogenesis of a number of disorders, including solid tumors and metastasis.

In the case of tumor growth, angiogenesis appears to be crucial for the transition from hyperplasia to neoplasia, and for providing nourishment for the growth and metastasis of the tumor. Folkman et al., Nature 339 -58 (1989), which allows the tumor cells to acquire a growth advantage compared to the normal cells. Therefore, anti-angiogenesis therapies have become an important treatment option for several types of tumors. These therapies have focused on blocking the VEGF pathway (Ferrara et al., Nat Rev Drug Discov. 2004 May;3(5):391-400.

The Notch signaling pathway is important for cell-cell communication, which involves gene regulation mechanisms that control multiple cell differentiation processes during embryonic development and in adult organisms. Notch signaling is dysregulated in many cancers, e.g. in T-cell acute lymphoblastic leukemia and in solid tumors (Sharma et al. 2007, Cell Cycle 6 (8): 927-30; Shih et al., Cancer Res. 2007 Mar 1;67(5): 1879-82).

DII4 (or Delta like 4 or delta-like ligand 4) is a member of the Delta family of Notch ligands. The extracellular domain of DII4 is composed of an N-terminal domain, a Delta/Serrate/Lag-2 (DSL) domain, and a tandem of eight epidermal growth factor (EGF)-like repeats. Generally, the EGF domains are recognized as comprising amino acid residues 218-251 (EGF-1; domain 1), 252-282 (EGF-2; domain 2), 284-322 (EGF-3; domain 3), 324-360 (EGF-4; domain 4), and 362-400 (EGF-5; domain 5), with the DSL domain at about amino acid residues 173-217 and the N-terminal domain at about amino acid residues 27-172 of hDII4 (WO 2008/076379).

It has been reported that DII4 exhibits highly selective expression by vascular endothelium, in particular in arterial endothelium (Shutter et al. (2000) Genes Develop. 14: 1313-1318). Recent studies in mice have shown that DII4 is induced by VEGF and is a negative feedback regulator that restrains vascular sprouting and branching. Consistent with this role, the deletion or inhibition of DII4 results in excessive angiogenesis (Scehnet et al., Blood. 2007 Jun 1;109(11):4753-60). This unrestrained angiogenesis paradoxically decreases tumor growth due to the formation of non-productive vasculature, even in tumors resistant to anti-VEGF therapies (Thurston et al., Nat Rev Cancer. 2007 May;7(5):327-31;
WO 2007/070671; Noguera-Troise et al., Nature. 2006 Dec 21; 444(7122)). Furthermore, the combined inhibition of VEGF and DII4 is shown to provide superior anti-tumor activity compared to anti-VEGF alone in xenograft models of multiple tumor types (Noguera-Troise et al., Nature. 2006 Dec 21; 444(7122):1032-7; Ridgway et al., Nature. 2006 Dec 21;444(7122):1083-7).

Due to these results, DII4 is being considered a promising target for cancer therapy, and several biological compounds that target DII4 are in (pre-)clinical development have been described: REGN-421 (= SAR153192; Regeneron, Sanofi-Aventis; WO2008076379) and OPM-21M18 (OncoMed) (Hoey et al., Cell Stem Cell. 2009 Aug 7; 5(2):168-77), both fully human DII4 antibodies; YW152F (Genentech), a humanized DII4 antibody (Ridgway et al., Nature. 2006 Dec 21; 444(7122):1083-7); DII4-Fc (Regeneron, Sanofi-Aventis), a recombinant fusion protein composed of the extracellular region of DII4 and the Fc region of human IgG1 (Noguera-Troise et al., Nature. 2006 Dec 21;444(7122)).

However, according to a report published by Minhong Yan et al., Nature 2010; Vol. 463(7282):E6-7, inhibition of DLL4 mediated signaling in vivo may disrupt normal organ homeostasis and produce significant pathology in multiple organs, thereby causing significant side effects.

WO2011014469 discloses anti-Ang2 antibodies and the possibility of combining such antibody with an anti-DII4 antibody.

However, the state-of-the art monoclonal antibodies (MAbs) and fusion proteins have several shortcomings in view of their therapeutic application: To prevent their degradation, they must be stored at near freezing temperatures. Also, since they are quickly digested in the gut, they are not suited for oral administration. Another major restriction of MAbs for cancer therapy is poor transport, which results in low concentrations and a lack of targeting of all cells in a tumor.

It has been an object of the present invention to provide novel anti-angiogenic binding molecules for human therapy.

It has been a further object of the invention to provide methods for the prevention, treatment, alleviation and/or diagnosis of such diseases, disorders or conditions, involving the use and/or administration of such binding molecules and compositions comprising them. In particular, it is has been an object of the invention to provide such pharmacologically active binding molecules, compositions and/or methods that provide advantages compared to the agents, compositions and/or methods currently used and/or known in the art. These advantages include improved therapeutic and/or pharmacological properties and/or other advantageous properties, e.g. for manufacturing purposes, especially as compared to conventional antibodies as those described above, or fragments thereof.

### BRIEF SUMMARY OF THE INVENTION

According to a first aspect, there are provided binding molecules, as defined in claims 1 and 2.

In another aspect, the invention relates to nucleic acids encoding the binding molecules of the invention as well as host cells containing same.

The invention further relates to a product or composition containing or comprising at least one binding molecule of the invention and optionally one or more further components of such compositions.

It has been found that the Ang2-binding component of the binding molecules according to the present invention binds to Ang2 with a potency at least 5,000 times higher, preferably 10,000 times higher than to Ang1 or Ang4. This will largely avoid blocking activation of Ang1-mediated signalling, which would counter the intended anti-angiogenetic effect.

It has further been found that the DLL4-binding component of the binding molecules according to the present invention binds to DLL4-A with an affinity of at least 1,000 times higher than to DII1, Jagged1 and preferably also against Jagged2. Due to this selectivity unwanted side reactions can be avoided.

The binding molecules of the present invention are provided as linked VHH domains. Such molecules are significantly smaller than conventional antibodies and have thus the potential for penetrating into a tumor deeper than such conventional antibodies. This benefit is further accentuated by the specific sequences disclosed herein after being free of glycosylation sites.

Further, due to the bispecific nature (DII4- and Ang2-binding components in one molecule) the tumor penetration of both functionalities will be necessarily equal, which will ensure that the beneficial effects of the combined antagonism of DII4 and Ang2 will be provided within the whole depth of penetration of the tumor. This is an advantage over the combination of individual antagonists against these targets, since the depth of penetration of individual antagonists will always vary to some degree. Another advantage of the binding molecules of the present invention is their increased serum half-like due to a serum albumin binding component such as a serum albumin binding molecule as described herein.

These and other aspects, embodiments, advantages and applications of the invention will become clear from the further description hereinbelow.

### DEFINITIONS

Unless indicated or defined otherwise, all terms used have their usual meaning in the art, which will be clear to the skilled person. Reference is for example made to the standard handbooks, such as Sambrook et al, "Molecular Cloning: A Laboratory Manual" (2nd Ed.), Vols. 1-3, Cold Spring Harbor Laboratory Press (1989); Lewin, "Genes IV", Oxford University Press, New York, (1990), and Roitt et al., "Immunology" (2nd Ed.), Gower Medical Publishing, London, New York (1989), as well as to the general background art cited herein; Furthermore, unless indicated otherwise, all methods, steps, techniques and manipulations that are not specifically described in detail can be performed and have been performed in a manner known *per se,* as will be clear to the skilled person. Reference is for example again made to the standard handbooks, to the general background art referred to above and to the further references cited therein.

The term "bispecific binding molecule" refers to a molecule comprising at least one Ang2-binding molecule (or "Ang2-binding component") and at least one DII4-binding molecule (or "DII4-binding component"). A bispecific binding molecule may contain more than one Ang2-binding molecule and/or more than one DII4-binding molecule, i.e. in the case that the bispecific binding molecule contains a biparatopic (as defined below) Ang2-binding molecule and/or a biparatopic DII4-binding molecule, in the part of the molecule that binds to Ang2 or to DII4, i.e. in its "Ang2-binding component" (or anti-Ang2 component) or "DII4-binding component" (or anti-DII4 component), respectively. The word "bispecific" in this context is however not to be construed as to exclude further binding components with binding specificity to molecules other than DII4 and Ang2 from the bispecific binding molecule. Non-limiting examples of such further binding components are binding components binding to serum albumin.

Unless indicated otherwise, the terms "*immunoglobulin*" and "*immunoglobulin sequence" -* whether used herein to refer to a *heavy chain antibody* or to a *conventional 4-chain antibody -* are used as general terms to include both the full-size antibody, the individual chains thereof, as well as all parts, domains or fragments thereof (including but not limited to antigen-binding domains or fragments such as VHH domains or VH/VL domains, respectively). In addition, the term "sequence" as used herein (for example in terms like "immunoglobulin sequence", "antibody sequence", "(single) variable domain sequence", "VHH sequence" or "protein sequence"), should generally be understood to include both the relevant amino acid sequence as well as nucleic acid sequences or nucleotide sequences encoding the same, unless the context requires a more limited interpretation.

The term *"domain"* (of a polypeptide or protein) as used herein refers to a folded protein structure which has the ability to retain its tertiary structure independently of the rest of the protein. Generally, domains are responsible for discrete functional properties of proteins, and in many cases may be added, removed or transferred to other proteins without loss of function of the remainder of the protein and/or of the domain.

The term "*immunoglobulin domain"* as used herein refers to a globular region of an antibody chain (such as e.g. a chain of a conventional 4-chain antibody or of a heavy chain antibody), or to a polypeptide that essentially consists of such a globular region. Immunoglobulin domains are characterized in that they retain the immunoglobulin fold characteristic of antibody molecules, which consists of a 2-layer sandwich of about 7 antiparallel beta-strands arranged in two beta-sheets, optionally stabilized by a conserved disulphide bond. An immunoglobulin domain comprises (a) variable domain(s), i.e., one or more immunoglobulin variable domains.

The term "*immunoglobulin variable domain"* as used herein means an immunoglobulin domain essentially consisting of four "framework regions" which are referred to in the art and hereinbelow as "framework region 1" or "FR1"; as "framework region 2" or"FR2"; as "framework region 3" or "FR3"; and as "framework region 4" or "FR4", respectively; which framework regions are interrupted by three "complementarity determining regions" or "CDRs", which are referred to in the art and hereinbelow as "complementarity determining region 1 "or "CDR1"; as "complementarity determining region 2" or "CDR2"; and as "complementarity determining region 3" or "CDR3", respectively. Thus, the general structure or sequence of an immunoglobulin variable domain can be indicated as follows: FR1 - CDR1 - FR2 - CDR2 - FR3 - CDR3 - FR4. It is the immunoglobulin variable domain(s) that confer specificity to an antibody for the antigen by carrying the antigen-binding site. In the context of the present invention immunoglobulin single variable domains like VHHs and domain antibodies are preferred.

The term "*immunoglobulin single variable domain"* as used herein means an immunoglobulin variable domain which is capable of specifically binding to an epitope of the antigen without pairing with an additional variable immunoglobulin domain. One example of immunoglobulin single variable domains in the meaning of the present invention are *"domain antibodies",* such as the immunoglobulin single variable domains VH and VL (VH domains and VL domains). Another example of immunoglobulin single variable domains are *"VHH domains"* (or simply "VHHs") from camelids, as defined hereinafter.

In view of the above definition, the antigen-binding domain of a conventional 4-chain antibody (such as an IgG, IgM, IgA, IgD or IgE molecule; known in the art) or of a Fab fragment, a F(ab')2 fragment, an Fv fragment such as a disulphide linked Fv or a scFv fragment, or a diabody (all known in the art) derived from such conventional 4-chain antibody, would normally not be regarded as an immunoglobulin single variable domain, as, in these cases, binding to the respective epitope of an antigen would normally not occur by one (single) immunoglobulin domain but by a pair of (associating) immunoglobulin domains such as light and heavy chain variable domains, i.e. by a VH-VL pair of immunoglobulin domains, which jointly bind to an epitope of the respective antigen.

*"VHH domains",* also known as VHHs, V_{H}H domains, VHH antibody fragments, and VHH antibodies, have originally been described as the antigen binding immunoglobulin (variable) domain of "heavy chain antibodies" (i.e. of "antibodies devoid of light chains"; Hamers-Casterman C, Atarhouch T, Muyldermans S, Robinson G, Hamers C, Songa EB, Bendahman N, Hamers R.: "Naturally occurring antibodies devoid of light chains"; Nature 363, 446-448 (1993)). The term "VHH domain" has been chosen in order to distinguish these variable domains from the heavy chain variable domains that are present in conventional 4-chain antibodies (which are referred to herein as "V_{H} domains" or "VH domains") and from the light chain variable domains that are present in conventional 4-chain antibodies (which are referred to herein as "V_{L} domains" or "VL domains"). VHH domains can specifically bind to an epitope without an additional antigen binding domain (as opposed to VH or VL domains in a conventional 4-chain antibody, in which case the epitope is recognized by a VL domain together with a VH domain). VHH domains are small, robust and efficient antigen recognition units formed by a single immunoglobulin domain.

In the context of the present invention, the terms VHH domain, VHH, V_{H}H domain, VHH antibody fragment, VHH antibody, as well as "Nanobody®" and "Nanobody® domain" ("Nanobody" being a trademark of the company Ablynx N.V.; Ghent; Belgium) are used interchangeably and are representatives of immunoglobulin single variable domains (having the structure FR1-CDR1-FR2-CDR2-FR3-CDR3-FR4 and specifically binding to an epitope without requiring the presence of a second immunoglobulin variable domain), and which are distinguished from VH domains by the so-called "hallmark residues", as defined in e.g. WO2009/109635, Fig. 1.

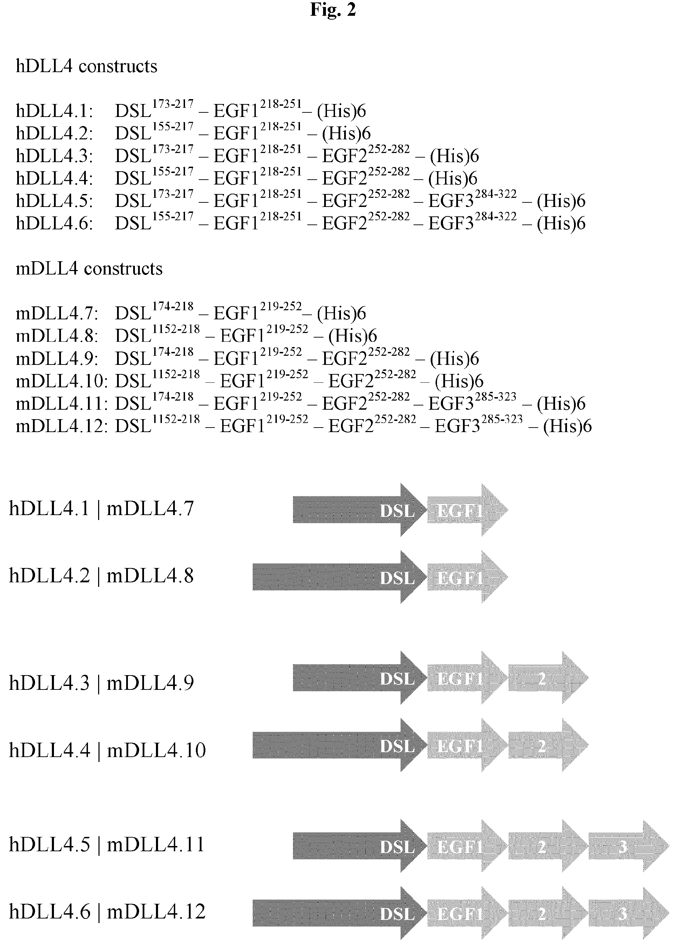

The amino acid residues of a immunoglobulin single variable domain, e.g. a VHH, are numbered according to the general numbering for V_{H} domains given by Kabat et al. ("Sequence of proteins of immunological interest", US Public Health Services, NIH Bethesda, MD, Publication No. 91), as applied to VHH domains from Camelids, as shown e.g. in Figure 2 of Riechmann and Muyldermans, J. Immunol. Methods 231, 25-38 (1999). According to this numbering,
- FR1 comprises the amino acid residues at positions 1-30,
- CDR1 comprises the amino acid residues at positions 31-35,
- FR2 comprises the amino acids at positions 36-49,
- CDR2 comprises the amino acid residues at positions 50-65,
- FR3 comprises the amino acid residues at positions 66-94,
- CDR3 comprises the amino acid residues at positions 95-102, and
- FR4 comprises the amino acid residues at positions 103-113.

However, it should be noted that - as is well known in the art for V_{H} domains and for VHH domains - the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, generally, the numbering according to Kabat may or may not correspond to the actual numbering of the amino acid residues in the actual sequence.

Alternative methods for numbering the amino acid residues of V_{H} domains, which methods can also be applied in an analogous manner to VHH domains, are known in the art. However, in the present description, claims and figures, the numbering according to Kabat and applied to VHH domains as described above will be followed, unless indicated otherwise.

The total number of amino acid residues in a VHH domain will usually be in the range of from 110 to 120, often between 112 and 115. It should however be noted that smaller and longer sequences may also be suitable for the purposes described herein.

Immunoglobulin single variable domains, e.g. VHHs and domain antibodies, according to the preferred embodiments of the invention, have a number of unique structural characteristics and functional properties which makes them highly advantageous for use in therapy as functional antigen-binding molecules. In particular, and without being limited thereto, VHH domains (which have been "designed" by nature to functionally bind to an antigen without pairing with a light chain variable domain) can function as single, relatively small, functional antigen-binding structural units.

Due to their unique properties, immunoglobulin single variable domains, as defined herein, like VHHs or VHs (or VLs) - either alone or as part of a larger polypeptide, e.g. a biparatopic molecule - offer a number of significant advantages:
- only a single domain is required to bind an antigen with high affinity and with high selectivity, so that there is no need to have two separate domains present, nor to assure that these two domains are present in the right spacial conformation and configuration (i.e. through the use of especially designed linkers, as with scFv's);
- immunoglobulin single variable domains can be expressed from a single nucleic acid molecule and do not require any post-translational modification (like glycosylation;
- immunoglobulin single variable domains can easily be engineered into multivalent and multispecific formats (as further discussed herein);
- immunoglobulin single variable domains have high specificity and affinity for their target, low inherent toxicity and can be administered via alternative routes than infusion or injection;
- immunoglobulin single variable domains are highly stable to heat, pH, proteases and other denaturing agents or conditions and, thus, may be prepared, stored or transported without the use of refrigeration equipments;
- immunoglobulin single variable domains are easy and relatively inexpensive to prepare, both on small scale and on a manufacturing scale. For example, immunoglobulin single variable domains can be produced using microbial fermentation (e.g. as further described below) and do not require the use of mammalian expression systems, as with for example conventional antibodies;
- immunoglobulin single variable domains are relatively small (approximately 15 kDa, or 10 times smaller than a conventional IgG) compared to conventional 4-chain antibodies and antigen-binding fragments thereof, and therefore show high(er) penetration into tissues (including but not limited to solid tumors and other dense tissues) and can be administered in higher doses than such conventional 4-chain antibodies and antigen-binding fragments thereof;
- VHHs have specific so-called "cavity-binding properties" (inter alia due to their extended CDR3 loop, compared to VH domains from 4-chain antibodies) and can therefore also access targets and epitopes not accessible to conventional 4-chain antibodies and antigen-binding fragments thereof;
- VHHs have the particular advantage that they are highly soluble and very stable and do not have a tendency to aggregate (as with the mouse-derived antigen-binding domains described by Ward et al., Nature 341: 544-546 (1989)).

The immunoglobulin single variable domains of the invention are not limited with respect to a specific biological source from which they have been obtained or to a specific method of preparation. For example, obtaining VHHs may include the following steps:
(1) isolating the VHH domain of a naturally occurring heavy chain antibody; or screening a library comprising heavy chain antibodies or VHHs and isolating VHHs therefrom;
(2) expressing a nucleic acid molecule encoding a VHH with the naturally occurring sequence;
(3) "humanizing" (as described herein) a VHH, optionally after affinity maturation, with a naturally occurring sequence or expressing a nucleic acid encoding such humanized VHH;
(4) "camelizing" (as described below) a immunoglobulin single variable heavy domain from a naturally occurring antibody from an animal species, in particular a species of mammal, such as from a human being, or expressing a nucleic acid molecule encoding such camelized domain;
(5) "camelizing" a VH, or expressing a nucleic acid molecule encoding such a camelized VH;
(6) using techniques for preparing synthetically or semi-synthetically proteins, polypeptides or other amino acid sequences;
(7) preparing a nucleic acid molecule encoding a VHH domain using techniques for nucleic acid synthesis, followed by expression of the nucleic acid thus obtained;
(8) subjecting heavy chain antibodies or VHHs to affinity maturation, to mutagenesis (e.g. random mutagenesis or site-directed mutagenesis) and/or any other technique(s) in order to increase the affinity and/or specificity of the VHH; and/or
(9) combinations or selections of the foregoing steps.

Suitable methods and techniques for performing the above-described steps are known in the art and will be clear to the skilled person. By way of example, methods of obtaining VHH domains binding to a specific antigen or epitope have been described in WO2006/040153 and WO2006/122786.

According to specific embodiments, the immunoglobulin single variable domains of the invention or present in the polypeptides of the invention are VHH domains with an amino acid sequence that essentially corresponds to the amino acid sequence of a naturally occurring VHH domain, but that has been "humanized" or "sequence-optimized" (optionally after affinity-maturation), i.e. by replacing one or more amino acid residues in the amino acid sequence of said naturally occurring VHH sequence by one or more of the amino acid residues that occur at the corresponding position(s) in a variable heavy domain of a conventional 4-chain antibody from a human being. This can be performed using methods known in the art, which can by routinely used by the skilled person.

A humanized VHH domain may contain one or more fully human framework region sequences, and, in an even more specific embodiment, may contain human framework region sequences derived from the human germline Vh3 sequences DP-29, DP-47, DP-51, or parts thereof, or be highly homologous thereto, optionally combined with JH sequences, such as JH5. Thus, a humanization protocol may comprise the replacement of any of the VHH residues with the corresponding framework 1, 2 and 3 (FRI, FR2 and FR3) residues of germline VH genes such as DP 47, DP 29 and DP 51) either alone or in combination. Suitable framework regions (FR) of the immunoglobulin single variable domains of the invention can be selected from those as set out e.g. in WO 2006/004678 and specifically, include the so-called "KERE" and "GLEW" classes. Examples are immunoglobulin single variable domains having the amino acid sequence G-L-E-W at about positions 44 to 47, and their respective humanized counterparts. A humanized VHH domain may contain one or more fully human framework region sequences.

By way of example, a humanizing substitution for VHHs belonging to the 103 P,R,S-group and/or the GLEW-group (as defined below) is 108Q to 108L. Methods for humanizing immunoglobulin single variable domains are known in the art.

Binding immunoglobulin single variable domains with improved properties in view of therapeutic application, e.g. enhanced affinity or decreased immunogenicity, may be obtained from individual binding molecules by techniques known in the art, such as affinity maturation (for example, starting from synthetic, random or naturally occurring immunoglobulin sequences), CDR grafting, humanizing, combining fragments derived from different immunoglobulin sequences, PCR assembly using overlapping primers, and similar techniques for engineering immunoglobulin sequences well known to the skilled person; or any suitable combination of any of the foregoing, also termed "sequence optimization", as described herein. Reference is, for example, made to standard handbooks, as well as to the further description and Examples.

If appropriate, a binding molecule with increased affinity may be obtained by affinity-maturation of another binding molecule, the latter representing, with respect to the affinity-matured molecule, the "parent" binding molecule.

Methods of obtaining VHHs that bind to a specific antigen or epitope have been described earlier, e.g. in WO2006/040153 and WO2006/122786. As also described therein in detail, VHH domains derived from camelids can be "humanized" (also termed "sequence-optimized" herein, "sequence-optimizing" may, in addition to humanization, encompass an additional modification of the sequence by one or more mutations that furnish the VHH with improved properties, such as the removal of potential post translational modification sites) by replacing one or more amino acid residues in the amino acid sequence of the original VHH sequence by one or more of the amino acid residues that occur at the corresponding position(s) in a VH domain from a conventional 4-chain antibody from a human being. A humanized VHH domain can contain one or more fully human framework region sequences, and, in an even more specific embodiment, can contain human framework region sequences derived from DP-29, DP-47, DP-51, or parts thereof, optionally combined with JH sequences, such as JH5.

*Domain antibodies,* also known as "Dab"s and "dAbs" (the terms "Domain Antibodies" and "dAbs" being used as trademarks by the GlaxoSmithKline group of companies) have been described in e.g. Ward, E.S., et al.: "Binding activities of a repertoire of single immunoglobulin variable domains secreted from Escherichia coli"; Nature 341: 544-546 (1989); Holt, L.J. et al.: "Domain antibodies: proteins for therapy"; TRENDS in Biotechnology 21(11): 484-490 (2003); and WO2003/002609.

Domain antibodies essentially correspond to the VH or VL domains of antibodies from non-camelid mammals, in particular human 4-chain antibodies. In order to bind an epitope as a single antigen binding domain, i.e. without being paired with a VL or VH domain, respectively, specific selection for such antigen binding properties is required, e.g. by using libraries of human single VH or VL domain sequences.

Domain antibodies have, like VHHs, a molecular weight of approximately 13 to approximately 16 kDa and, if derived from fully human sequences, do not require humanization for e.g. therapeutical use in humans. As in the case of VHH domains, they are well expressed also in prokaryotic expression systems, providing a significant reduction in overall manufacturing cost.

Furthermore, it will also be clear to the skilled person that it is possible to "graft" one or more of the CDR's mentioned above onto other "scaffolds", including but not limited to human scaffolds or non-immunoglobulin scaffolds. Suitable scaffolds and techniques for such CDR grafting are known in the art.

The terms "*epitope*" and *"antigenic determinant*"*,* which can be used interchangeably, refer to the part of a macromolecule, such as a polypeptide, that is recognized by antigen-binding molecules, such as conventional antibodies or the polypeptides of the invention, and more particularly by the antigen-binding site of said molecules. Epitopes define the minimum binding site for an immunoglobulin, and thus represent the target of specificity of an immunoglobulin.

A polypeptide (such as an immunoglobulin, an antibody, an immunoglobulin single variable domain of the invention, or generally an antigen-binding molecule or a fragment thereof) that can *"bind to"* or *"specifically bind to*"*,* that *"has affinity for*" and/or that *"has specificity for*" a certain epitope, antigen or protein (or for at least one part, fragment or epitope thereof) is said to be "*against*" or *"directed against"* said epitope, antigen or protein or is a *"binding"* molecule with respect to such epitope, antigen or protein. In this context, a DII4-binding component may also be referred to as "DII4-neutralizing".

Generally, the term "*specificity*" refers to the number of different types of antigens or epitopes to which a particular antigen-binding molecule or antigen-binding protein (such as an immunoglobulin single variable domain of the invention) molecule can bind. The specificity of an antigen-binding molecule can be determined based on its affinity and/or avidity. The affinity, represented by the equilibrium constant for the dissociation of an antigen with an antigen-binding protein (KD), is a measure for the binding strength between an epitope and an antigen-binding site on the antigen-binding protein: the lesser the value of the KD, the stronger the binding strength between an epitope and the antigen-binding molecule (alternatively, the affinity can also be expressed as the affinity constant (KA), which is 1/KD). As will be clear to the skilled person (for example on the basis of the further disclosure herein), affinity can be determined in a manner known per se, depending on the specific antigen of interest. Avidity is the measure of the strength of binding between an antigen-binding molecule (such as an immunoglobulin, an antibody, an immunoglobulin single variable domain or a polypeptides containing it and the pertinent antigen. Avidity is related to both the affinity between an epitope and its antigen binding site on the antigen-binding molecule and the number of pertinent binding sites present on the antigen-binding molecule.

The part of an antigen-binding molecule that recognizes the epitope is called a *paratope.*

Unless indicated otherwise, the term "DII4-binding molecule" or "Ang2-binding molecule" includes anti-DII4 or anti-Ang2 antibodies, anti-DII4 antibody or anti-Ang2 antibody fragments, "anti-DII4 antibody-like molecules" or "anti-Ang2 antibody-like molecules", as defined herein, and conjugates with any of these. Antibodies include, but are not limited to, monoclonal and chimerized monoclonal antibodies. The term "antibody" encompasses complete immunoglobulins, like monoclonal antibodies produced by recombinant expression in host cells, as well as antibody fragments or "antibody-like molecules", including single-chain antibodies and linear antibodies, so-called "SMIPs" ("Small Modular Immunopharmaceuticals"), as e.g described in WO 02/056910; Antibody-like molecules include immunoglobulin single variable domains, as defined herein. Other examples for antibody-like molecules are immunoglobulin super family antibodies (IgSF), or CDR-grafted molecules.

*"Ang2-binding molecule" or* "*DII4-binding molecule" respectively,* refers to both monovalent target-binding molecules (i.e. molecules that bind to one epitope of the respective target) as well as to bi- or multivalent binding molecules (i.e. binding molecules that bind to more than one epitope, e.g. "biparatopic" molecules as defined hereinbelow). Ang2(or DII4)-binding molecules containing more than one Ang2(or DII4)-binding immunoglobulin single variable domain are also termed "formatted" binding molecules, they may, within the target-binding component, in addition to the immunoglobulin single variable domains, comprise linkers and/or moieties with effector functions, e.g. half-life-extending moieties like albumin-binding immunoglobulin single variable domains, and/or a fusion partner like serum albumin and/or an attached polymer like PEG.

The term *"biparatopic Ang2(or DII4)-binding molecule"* or "*biparatopic immunoglobulin single variable domain"as* used herein shall mean a binding molecule comprising a first immunoglobulin single variable domain and a second immunoglobulin single variable domain as herein defined, wherein the two molecules bind to two non-overlapping epitopes of the respective antigen. The biparatopic binding molecules are composed of immunoglobulin single variable domains which have different specificities with respect to the epitope. The part of an antigen-binding molecule (such as an antibody or an immunoglobulin single variable domain of the invention) that recognizes the epitope is called a *paratope.*

A formatted binding molecule may, albeit less preferred, also comprise two identical immunoglobulin single variable domains or two different immunoglobulin single variable domains that recognize the same or overlapping epitopes or their respective antigen. In this case, with respect to VEGF, the two immunoglobulin single variable domains may bind to the same or an overlapping epitope in each of the two monomers that form the VEGF dimer.

Typically, the binding molecules of the invention will bind with a dissociation constant (K_{D}) of 10E-5 to 10E-14 moles/liter (M) or less, and preferably 10E-7 to 10E-14 moles/liter (M) or less, more preferably 10E-8 to 10E-14 moles/liter, and even more preferably 10E-11 to 10E-13, as measured e.g. in a Biacore or in a Kinexa assay), and/or with an association constant (K_{A}) of at least 10E7 ME-1, preferably at least 10E8 ME-1, more preferably at least 10E9 ME-1, such as at least 10E11 ME-1. Any K_{D} value greater than 10E-4 M is generally considered to indicate non-specific binding. Preferably, a polypeptide of the invention will bind to the desired antigen, i.e. VEGF or DII4, respectively, with a K_{D} less than 500 nM, preferably less than 200 nM, more preferably less than 10 nM, such as less than 500 pM. Specific binding of an antigen-binding protein to an antigen or epitope can be determined in any suitable manner known per se, including, for example, the assays described herein, Scatchard analysis and/or competitive binding assays, such as radioimmunoassays (RIA), enzyme immunoassays (EIA) and sandwich competition assays, and the different variants thereof known per se in the art.

Amino acid residues will be indicated according to the standard three-letter or one-letter amino acid code, as generally known and agreed upon in the art. When comparing two amino acid sequences, the term "*amino acid difference*" refers to insertions, deletions or substitutions of the indicated number of amino acid residues at a position of the reference sequence, compared to a second sequence. In case of substitution(s), such substitution(s) will preferably be conservative amino acid substitution(s), which means that an amino acid residue is replaced with another amino acid residue of similar chemical structure and which has little or essentially no influence on the function, activity or other biological properties of the polypeptide. Such conservative amino acid substitutions are well known in the art, for example from WO 98/49185, wherein conservative amino acid substitutions preferably are substitutions in which one amino acid within the following groups (i) - (v) is substituted by another amino acid residue within the same group: (i) small aliphatic, nonpolar or slightly polar residues: Ala, Ser, Thr, Pro and Gly; (ii) polar, negatively charged residues and their (uncharged) amides: Asp, Asn, Glu and Gin; (iii) polar, positively charged residues: His, Arg and Lys; (iv) large aliphatic, nonpolar residues: Met, Leu, Ile, Val and Cys; and (v) aromatic residues:
Phe, Tyr and Trp. Particularly preferred conservative amino acid substitutions are as follows: Ala into Gly or into Ser; Arg into Lys; Asn into Gin or into His; Asp into Glu;Cys into Ser; Gin into Asn; Glu into Asp; Gly into Ala or into Pro; His into Asn or into Gin; Ile into Leu or into Val; Leu into Ile or into Val; Lys into Arg, into Gin or into Glu; Met into Leu, into Tyr or into Ile; Phe into Met, into Leu or into Tyr; Ser into Thr; Thr into Ser;Trp into Tyr; Tyr into Trp or into Phe; Val into Ile or into Leu.

A polypeptide or nucleic acid molecule is considered to be "*(in) essentially isolated (form)" -* for example, when compared to its native biological source and/or the reaction medium or cultivation medium from which it has been obtained - when it has been separated from at least one other component with which it is usually associated in said source or medium, such as another protein/polypeptide, another nucleic acid, another biological component or macromolecule or at least one contaminant, impurity or minor component. In particular, a polypeptide or nucleic acid molecule is considered "essentially isolated" when it has been purified at least 2-fold, in particular at least 10-fold, more in particular at least 100-fold, and up to 1000-fold or more. A polypeptide or nucleic acid molecule that is "in essentially isolated form" is preferably essentially homogeneous, as determined using a suitable technique, such as a suitable chromatographical technique, such as polyacrylamide gel electrophoresis.

*"Sequence identity*" between two DII4-binding molecule sequences or between two Ang2-binding molecule sequences indicates the percentage of amino acids that are identical between the sequences. It may be calculated or determined as described in paragraph f) on pages 49 and 50 of WO 2008/020079. "Sequence similarity" indicates the percentage of amino acids that either are identical or that represent conservative amino acid substitutions.

Alternative methods for numbering the amino acid residues of V_{H} domains, which methods can also be applied in an analogous manner to VHH domains, are known in the art. However, in the present description, claims and figures, the numbering according to Kabat and applied to VHH domains as described above will be followed, unless indicated otherwise.

An "affinity-matured" DII4-binding molecule or Ang2-binding molecule, in particular a VHH or a domain antibody, has one or more alterations in one or more CDRs which result in an improved affinity for DII4 or Ang2, as compared to the respective parent DII4-binding molecule or Ang2-binding molecule. Afffinity-matured DII4-binding molecules or Ang2-binding molecules of the invention may be prepared by methods known in the art, for example, as described by Marks et al., 1992, Biotechnology 10:779-783, or Barbas, et al., 1994, Proc. Nat. Acad. Sci, USA 91: 3809-3813.; Shier et al., 1995, Gene 169:147-155; Yelton et al., 1995, Immunol. 155: 1994-2004; Jackson et al., 1995, J. Immunol. 154(7):3310-9; and Hawkins et al., 1992, J. Mol. Biol. 226(3): 889 896; KS Johnson and RE Hawkins, "Affinity maturation of antibodies using phage display", Oxford University Press 1996.

For the present invention, an "amino acid sequences of SEQ ID NO: x": includes, if not otherwise stated, an amino acid sequence that is 100% identical with the sequence shown in the respective SEQ ID NO: x;
a) amino acid sequences that have at least 80% amino acid identity with the sequence shown in the respective SEQ ID NO: x;
b) amino acid sequences that have 3, 2, or 1 amino acid differences with the sequence shown in the respective SEQ ID NO: x.

The terms "cancer" and "cancerous" refer to or describe the physiological condition in mammals that is typically characterized by unregulated cell growth/proliferation. Examples of cancer to be treated with a binding molecule of the invention, include but are not limited to carcinoma, lymphoma, blastoma, sarcoma, and leukemia. More particular examples of such cancers, as suggested for treatment with DII4 antagonists in US 2008/0014196, include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, gastric cancer, melanoma, and various types of head and neck cancer. Dysregulation of angiogenesis can lead to many disorders that can be treated by compositions and methods of the invention. These disorders include both non-neoplastic and neoplastic conditions. Neoplasties include but are not limited those described above.

Non-neoplastic disorders include, but are not limited to, as suggested for treatment with DII4 antagonists in US 2008/0014196, undesired or aberrant hypertrophy, arthritis, rheumatoid arthritis (RA), psoriasis, psoriatic plaques, sarcoidosis, atherosclerosis, atherosclerotic plaques, diabetic and other proliferative retinopathies including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, chronic inflammation, lung inflammation, acute lung injury/ ARDS, sepsis, primary pulmonary hypertension, malignant pulmonary effusions, cerebral edema (e.g., associated with acute stroke/ closed head injury/ trauma), synovial inflammation, pannus formation in RA, myositis ossificans, hypertropic bone formation, osteoarthritis (OA), refractory ascites, polycystic ovarian disease, endometriosis, 3^{rd} spacing of fluid diseases (pancreatitis, compartment syndrome, burns, bowel disease), uterine fibroids, premature labor, chronic inflammation such as IBD (Crohn's disease and ulcerative colitis), renal allograft rejection, inflammatory bowel disease, nephrotic syndrome, undesired or aberrant tissue mass growth (non-cancer), hemophilic joints, hypertrophic scars, inhibition of hair growth, Osier-Weber syndrome, pyogenic granuloma retrolental fibroplasias, scleroderma, trachoma, vascular adhesions, synovitis, dermatitis, preeclampsia, ascites, pericardial effusion (such as that associated with pericarditis), and pleural effusion.

### DETAILED DESCRIPTION OF THE INVENTION

In a first aspect, the present invention relates to a binding molecule according to claim 1 or claim 2.

Two binding molecules (two VHHs or domain antibodies or VHH and a domain antibody), or two binding components, may be linked to each other via an additional VHH or domain antibody, respectively (in such binding molecules, the two or more immunoglobulin single variable domains may be linked directly to said additional immunoglobulin single variable domain or via suitable linkers). Such an additional VHH or domain antibody may for example be a VHH or domain antibody that provides for an increased half-life. For example, the latter VHH or domain antibody may be one that is capable of binding to a (human) serum protein such as (human) serum albumin or (human) transferrin.

Alternatively, the two or more immunoglobulin single variable domains that bind to the respective target may be linked in series (either directly or via a suitable linker) and the additional VHH or domain antibody (which may provide for increased half-life) may be connected directly or via a linker to one of these two or more aforementioned immunoglobulin sequences.

Suitable linkers are described herein in connection with specific polypeptides of the invention and may - for example and without limitation - comprise an amino acid sequence, which amino acid sequence preferably has a length of 9 or more amino acids, more preferably at least 17 amino acids, such as about 20 to 40 amino acids. However, the upper limit is not critical but is chosen for reasons of convenience regarding e.g. biopharmaceutical production of such polypeptides.

The linker sequence may be a naturally occurring sequence or a non-naturally occurring sequence. If used for therapeutic purposes, the linker is preferably non-immunogenic in the subject to which the binding molecule of the invention is administered.

One useful group of linker sequences are linkers derived from the hinge region of heavy chain antibodies as described in WO 1996/34103 and WO 1994/04678.

Other examples are poly-alanine linker sequences such as Ala- Ala- Ala.

Further preferred examples of linker sequences are Gly/Ser linkers of different length such as (glyₓser_{y})_{z} linkers, including (gly₄ser)₃, (gly₄ser)₄, (gly₄ser), (gly₃ser), gly₃, and (gly₃ser₂)₃.

Some non-limiting examples of linkers are shown in Figures 40 and 48, e.g. the linkers
GGGGSGGGGSGGGGSGGGGSGGGGSGGGGSGGGGS (35GS; SEQ ID NO: 90);
GGGGSGGGS (9GS; SEQ ID NO: 91);
GGGGSGGGGSGGGGSGGGGSGGGGSGGGGSGGGGSGGGGS (40GS; SEQ ID NO: 92).

If a binding molecule is modified by the attachment of a polymer, for example of a polyethylene glycol PEG (polyethylene glycol) moiety, the linker sequence preferably includes an amino acid residue, such as a cysteine or a lysine, allowing such modification, e.g. PEGylation, in the linker region.

Examples of linkers useful for PEGylation are:
GGGGCGGGS ("GS9,C5", SEQ ID NO:93);
GGGGCGGGGSGGGGSGGGGSGGGGS ("GS25,C5, SEQ ID NO:94)
GGGSGGGGSGGGGCGGGGSGGGGSGGG ("GS27,C14", SEQ ID NO:95),
GGGGSGGGGSGGGGCGGGGSGGGGSGGGGSGGGGS ("GS35,C15", SEQ ID NO:96), and
GGGGCGGGGSGGGGSGGGGSGGGGSGGGGSGGGGS ("GS35,C5", SEQ ID NO:97).

Furthermore, the linker may also be a poly(ethylene glycol) moiety, as shown in e.g. WO 2004/081026.

In another embodiment, the immunoglobulin single variable domains are linked to each other via another moiety (optionally via one or two linkers), such as another polypeptide which, in a preferred but non-limiting embodiment, may be a further immunoglobulin single variable domain as described above. Such moiety may either be essentially inactive or may have a biological effect such as improving the desired properties of the polypeptide or may confer one or more additional desired properties to the polypeptide. For example, and without limitation, the moiety may improve the half-life of the protein or polypeptide, and/or may reduce its immunogenicity or improve any other desired property.

The binding molecule of the invention includes a moiety which extends the half-life of the polypeptide of the invention in serum or other body fluids of a patient. The term "half-life" is defined as the time it takes for the serum concentration of the (modified) polypeptide to reduce by 50%, *in vivo,* for example due to degradation of the polypeptide and/or clearance and/or sequestration by natural mechanisms.

The binding molecule of the invention comprises a moiety which binds to an antigen found in blood, i.e. a humanized albumin-binding VHH domain, i.e. the albumin binding VHH domain ALB8 which consists of the amino acid sequence shown in SEQ ID NO: 519.

The binding components have specificity for Ang2 or DII4.

Specific binding of a binding component to its antigen Ang2 or DII4 can be determined in any suitable manner known *per se,* including, for example, the assays described herein, Scatchard analysis and/or competitive binding assays, such as radioimmunoassays (RIA), enzyme immunoassays (EIA and ELISA) and sandwich competition assays, and the different variants thereof known per se in the art.

In another aspect, the invention relates to nucleic acid molecules that encode binding molecules of the invention. Such nucleic acid molecules will also be referred to herein as "nucleic acids of the invention" and may also be in the form of a genetic construct, as defined herein. A nucleic acid of the invention may be genomic DNA, cDNA or synthetic DNA (such as DNA with a codon usage that has been specifically adapted for expression in the intended host cell or host organism). According to one embodiment of the invention, the nucleic acid of the invention is in essentially isolated form, as defined hereabove.

The nucleic acid of the invention may also be in the form of, may be present in and/or may be part of a vector, such as for example a plasmid, cosmid or YAC. The vector may especially be an expression vector, i.e. a vector that can provide for expression of the binding molecule *in vitro* and/or *in vivo* (i.e. in a suitable host cell, host organism and/or expression system). Such expression vector generally comprises at least one nucleic acid of the invention that is operably linked to one or more suitable regulatory elements, such as promoter(s), enhancer(s), terminator(s), and the like. Such elements and their selection in view of expression of a specific sequence in a specific host are common knowledge of the skilled person. Specific examples of regulatory elements and other elements useful or necessary for expressing binding molecules of the invention, such as promoters, enhancers, terminators, integration factors, selection markers, leader sequences, reporter genes, and the like, are disclosed e.g. on pp. 131 to 133 of WO 2006/040153.

The nucleic acids of the invention may be prepared or obtained in a manner known *per se* (e.g. by automated DNA synthesis and/or recombinant DNA technology), based on the information on the amino acid sequences for the polypeptides of the invention given herein, and/or can be isolated from a suitable natural source.

In another aspect, the invention relates to host cells that express or that are capable of expressing one or more binding molecules of the invention; and/or that contain a nucleic acid of the invention. According to a particularly preferred embodiment, said host cells are bacterial cells; other useful cells are yeast cells, fungal cells or mammalian cells.

Suitable bacterial cells include cells from gram-negative bacterial strains such as strains of *Escherichia coli, Proteus,* and *Pseudomonas,* and gram-positive bacterial strains such as strains of *Bacillus, Streptomyces, Staphylococcus,* and *Lactococcus.* Suitable fungal cell include cells from species of *Trichoderma, Neurospora,* and *Aspergillus.* Suitable yeast cells include cells from species of *Saccharomyces* (for example *Saccharomyces cerevisiae), Schizosaccharomyces* (for example *Schizosaccharomyces pombe), Pichia* (for example *Pichia pastoris* and *Pichia methanolica*)*,* and *Hansenula.*

Suitable mammalian cells include for example CHO cells, BHK cells, HeLa cells, COS cells, and the like. However, amphibian cells, insect cells, plant cells, and any other cells used in the art for the expression of heterologous proteins can be used as well.

The invention further provides methods of manufacturing a binding molecule of the invention, such methods generally comprising the steps of:
- culturing host cells comprising a nucleic acid capable of encoding a binding molecule under conditions that allow expression of the binding molecule of the invention; and
- recovering or isolating the polypeptide expressed by the host cells from the culture; and
- optionally further purifying and/or modifying and/or formulating the binding molecule of the invention.

For production on an industrial scale, preferred host organisms include strains of *E. coli, Pichia pastoris,* and S. *cerevisiae* that are suitable for large scale expression, production and fermentation, and in particular for large scale pharmaceutical expression, production and fermentation.

The choice of the specific expression system depends in part on the requirement for certain post-translational modifications, more specifically glycosylation. The production of a binding molecule of the invention for which glycosylation is desired or required would necessitate the use of mammalian expression hosts that have the ability to glycosylate the expressed protein. In this respect, it will be clear to the skilled person that the glycosylation pattern obtained (i.e. the kind, number and position of residues attached) will depend on the cell or cell line that is used for the expression.

Binding molecules of the invention may be produced either in a cell as set out above intracellullarly (e.g. in the cytosol, in the periplasma or in inclusion bodies) and then isolated from the host cells and optionally further purified; or they can be produced extracellularly (e.g. in the medium in which the host cells are cultured) and then isolated from the culture medium and optionally further purified.

Methods and reagents used for the recombinant production of polypeptides, such as specific suitable expression vectors, transformation or transfection methods, selection markers, methods of induction of protein expression, culture conditions, and the like, are known in the art. Similarly, protein isolation and purification techniques useful in a method of manufacture of a polypeptide of the invention are well known to the skilled person.

In a further aspect, the invention relates to a peptide having an amino acid sequence of a CDR3 contained in an anti-DII4-VHH having an amino acid sequence selected from sequences shown in SEQ ID NOs: 1 to 166 and 458, SEQ ID NOs: 333 to 353, or SEQ ID NOs: 375 to 395, respectively, and a nucleic acid molecule encoding same.

These peptides correspond to CDR3s derived from the VHHs of the invention. They, in particular the nucleic acid molecules encoding them, are useful for CDR grafting in order to replace a CDR3 in an immunoglobulin chain, or for insertion into a non-immunoglobulin scaffold, e.g. a protease inhibitor, DNA-binding protein, cytochrome b562, a helix-bundle protein, a disulfide-bridged peptide, a lipocalin or an anticalin, thus conferring target-binding properties to such scaffold. The method of CDR-grafting is well known in the art and has been widely used, e.g. for humanizing antibodies (which usually comprises grafting the CDRs from a rodent antibody onto the Fv frameworks of a human antibody).

In order to obtain an immunoglobulin or a non-immunoglobulin scaffold containing a CDR3 as disclosed herein, the DNA encoding such molecule may be obtained according to standard methods of molecular biology, e.g. by gene synthesis, by oligonucleotide annealing or by means of overlapping PCR fragments, as e.g. described by Daugherty et al., 1991, Nucleic Acids Research, Vol. 19, 9, 2471-2476. A method for inserting a VHH CDR3 into a non-immunoglobulin scaffold has been described by Nicaise et al., 2004, Protein Science, 13, 1882-1891.

The invention further relates to a product or composition containing or comprising at least one binding molecule of the invention and optionally one or more further components of such compositions known *per se,* i.e. depending on the intended use of the composition.

For pharmaceutical use, a binding molecule of the invention or a polypeptide containing same may be formulated as a pharmaceutical preparation or composition comprising at least one binding molecule of the invention and at least one pharmaceutically acceptable carrier, diluent or excipient and/or adjuvant, and optionally one or more further pharmaceutically active polypeptides and/or compounds. By means of non-limiting examples, such a formulation may be in a form suitable for oral administration, for parenteral administration (such as by intravenous, intramuscular or subcutaneous injection or intravenous infusion), for topical administration, for administration by inhalation, by a skin patch, by an implant, by a suppository, etc. Such suitable administration forms - which may be solid, semi-solid or liquid, depending on the manner of administration - as well as methods and carriers for use in the preparation thereof, will be clear to the skilled person, and are further described herein.

Thus, in a further aspect, the invention relates to a pharmaceutical composition that contains at least one binding molecule, in particular one immunoglobulin single variable domain of the invention or a polypeptide containing same and at least one suitable carrier, diluent or excipient (i.e. suitable for pharmaceutical use), and optionally one or more further active substances.

The binding molecules of the invention may be formulated and administered in any suitable manner known per se: Reference, in particular for the immunoglobulin single variable domains, is for example made to WO 2004/041862, WO 2004/041863, WO 2004/041865, WO 2004/041867 and WO 2008/020079, as well as to the standard handbooks, such as Remington's Pharmaceutical Sciences, 18th Ed., Mack Publishing Company, USA (1990), Remington, the Science and Practice of Pharmacy, 21th Edition, Lippincott Williams and Wilkins (2005); or the Handbook of Therapeutic Antibodies (S. Dubel, Ed.), Wiley, Weinheim, 2007 (see for example pages 252-255).

For example, a binding molecule of the invention may be formulated and administered in any manner known per se for conventional antibodies and antibody fragments (including ScFv's and diabodies) and other pharmaceutically active proteins. Such formulations and methods for preparing the same will be clear to the skilled person, and for example include preparations suitable for parenteral administration (for example intravenous, intraperitoneal, subcutaneous, intramuscular, intraluminal, intra-arterial or intrathecal administration) or for topical (i.e. transdermal or intradermal) administration.

Preparations for parenteral administration may for example be sterile solutions, suspensions, dispersions or emulsions that are suitable for infusion or injection. Suitable carriers or diluents for such preparations for example include, without limitation, sterile water and pharmaceutically acceptable aqueous buffers and solutions such as physiological phosphate-buffered saline, Ringer's solutions, dextrose solution, and Hank's solution; water oils; glycerol; ethanol; glycols such as propylene glycol or as well as mineral oils, animal oils and vegetable oils, for example peanut oil, soybean oil, as well as suitable mixtures thereof. Usually, aqueous solutions or suspensions will be preferred.

Thus, the binding molecule of the invention may be systemically administered, e.g., orally, in combination with a pharmaceutically acceptable vehicle such as an inert diluent or an assimilable edible carrier. For oral therapeutic administration, the binding molecule of the invention may be combined with one or more excipients and used in the form of ingestible tablets, buccal tablets, troches, capsules, elixirs, suspensions, syrups, wafers, and the like. Such compositions and preparations should contain at least 0.1% of the DII4-binding molecule of the invention. Their percentage in the compositions and preparations may, of course, be varied and may conveniently be between about 2 to about 60% of the weight of a given unit dosage form. The amount of the binding molecule of the invention in such therapeutically useful compositions is such that an effective dosage level will be obtained.

The tablets, pills, capsules, and the like may also contain binders, excipients, disintegrating agents, lubricants and sweetening or flavouring agents, for example those mentioned on pages 143-144 of WO 08/020079. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the binding molecules of the invention, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the binding molecules of the invention may be incorporated into sustained-release preparations and devices.

Preparations and formulations for oral administration may also be provided with an enteric coating that will allow the constructs of the invention to resist the gastric environment and pass into the intestines. More generally, preparations and formulations for oral administration may be suitably formulated for delivery into any desired part of the gastrointestinal tract. In addition, suitable suppositories may be used for delivery into the gastrointestinal tract.

The binding molecules of the invention may also be administered intravenously or intraperitoneally by infusion or injection, as further described on pages 144 and 145 of WO 2008/020079.

For topical administration of the binding molecules of the invention, it will generally be desirable to administer them to the skin as compositions or formulations, in combination with a dermatologically acceptable carrier, which may be a solid or a liquid, as further described on page 145 of WO 2008/020079.

Generally, the concentration of the binding molecules of the invention in a liquid composition, such as a lotion, will be from about 0.1-25 wt-%, preferably from about 0.5-10 wt-%. The concentration in a semi-solid or solid composition such as a gel or a powder will be about 0.1-5 wt-%, preferably about 0.5-2.5 wt-%.

The amount of the binding molecules of the invention required for use in treatment will vary not only with the particular binding molecule selected, but also with the route of administration, the nature of the condition being treated and the age and condition of the patient and will be ultimately at the discretion of the attendant physician or clinician. Also, the dosage of the binding molecules of the invention varies depending on the target cell, tumor, tissue, graft, or organ. The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

An administration regimen may include long-term, daily treatment. By "long-term" is meant at least two weeks and preferably, several weeks, months, or years of duration. Necessary modifications in this dosage range may be determined by one of ordinary skill in the art using only routine experimentation given the teachings herein. See Remington's Pharmaceutical Sciences (Martin, E.W., ed. 4), Mack Publishing Co., Easton, PA. The dosage can also be adjusted by the individual physician in the event of any complication.

According to a further embodiment, the invention relates to the use of binding molecules of the invention for therapeutic purposes, such as
- for the prevention, treatment and/or alleviation of a disorder, disease or condition, especially in a human being, that is associated with DII4-mediated and/or Ang2-related effects on angiogenesis or that can be prevented, treated or alleviated by modulating the Notch signaling pathway and/or the Tie2 signalling pathway with a binding molecule according to the invention,
- in a method of treatment of a patient in need of such therapy, such method comprising administering, to a subject in need thereof, a pharmaceutically active amount of at least one binding molecule of the invention or a pharmaceutical composition containing same;
- for the preparation of a medicament for the prevention, treatment or alleviation of disorders, diseases or conditions associated with DII4-mediated and/or Ang2-mediated effects on angiogenesis;
- as an active ingredient in a pharmaceutical composition or medicament used for the above purposes.

According to a specific aspect, said disorder disorder, disease or condition is a cancer or cancerous disease, as defined herein.

According to another aspect, the disease is an eye disease associated with associated with DII4-mediated and/or Ang2-mediated effects on angiogenesis or which can be treated or alleviated by modulating the Notch signaling pathway and/or the Tie2 signalling pathway with a binding molecule.

Depending on the cancerous disease to be treated, a binding molecule of the invention may be used on its own or in combination with one or more additional therapeutic agents, in particular selected from chemotherapeutic agents like DNA damaging agents or therapeutically active compounds that inhibit angiogenesis, signal transduction pathways or mitotic checkpoints in cancer cells.

The additional therapeutic agent may be administered simultaneously with, optionally as a component of the same pharmaceutical preparation, or before or after administration of the binding molecule.

In certain embodiments, the additional therapeutic agent may be, without limitation, one or more inhibitors selected from the group of inhibitors of EGFR, VEGFR, HER2-neu, Her3, AuroraA, AuroraB, PLK and PI3 kinase, FGFR, PDGFR, Raf, Ras, KSP, PDK1, PTK2, IGF-R or IR.

Further examples of additional therapeutic agents are inhibitors of CDK, Akt, src/bcr abl, cKit, cMet/HGF, c-Myc, Flt3, HSP90, hedgehog antagonists, inhibitors of JAK/STAT, Mek, mTor, NFkappaB, the proteasome, Rho, an inhibitor of wnt signaling or an inhibitor of the ubiquitination pathway or another inhibitor of the Notch signaling pathway.

Examples for Aurora inhibitors are, without limitation, PHA-739358, AZD-1152, AT 9283, CYC-116, R-763, VX-680, VX-667, MLN-8045, PF-3814735.

An example for a PLK inhibitor is GSK-461364.

Examples for raf inhibitors are BAY-73-4506 (also a VEGFR inhibitor), PLX 4032, RAF-265 (also in addition a VEGFR inhibitor), sorafenib (also in addition a VEGFR inhibitor), and XL 281.

Examples for KSP inhibitors are ispinesib, ARRY-520, AZD-4877, CK-1122697, GSK 246053A, GSK-923295, MK-0731, and SB-743921.

Examples for a src and/or bcr-abl inhibitors are dasatinib, AZD-0530, bosutinib, XL 228 (also an IGF-1R inhibitor), nilotinib (also a PDGFR and cKit inhibitor), imatinib (also a cKit inhibitor), and NS-187.

An example for a PDK1 inhibitor is BX-517.

An example for a Rho inhibitor is BA-210.

Examples for PI3 kinase inhibitors are PX-866, BEZ-235 (also an mTor inhibitor), XL 418 (also an Akt inhibitor), XL-147, and XL 765 (also an mTor inhibitor).

Examples for inhibitors of cMet or HGF are XL-184 (also an inhibitor of VEGFR, cKit, Flt3), PF-2341066, MK-2461, XL-880 (also an inhibitor of VEGFR), MGCD-265 (also an inhibitor of VEGFR, Ron, Tie2), SU-11274, PHA-665752, AMG-102, and AV-299.

An example for a c-Myc inhibitor is CX-3543.

Examples for Flt3 inhibitors are AC-220 (also an inhibitor of cKit and PDGFR), KW 2449, lestaurtinib (also an inhibitor of VEGFR, PDGFR, PKC), TG-101348 (also an inhibitor of JAK2), XL-999 (also an inhibitor of cKit, FGFR, PDGFR and VEGFR), sunitinib (also an inhibitor of PDGFR, VEGFR and cKit), and tandutinib (also an inhibitor of PDGFR, and cKit).

Examples for HSP90 inhibitors are tanespimycin, alvespimycin, IPI-504 and CNF 2024.

Examples for JAK/STAT inhibitors are CYT-997 (also interacting with tubulin), TG 101348 (also an inhibitor of Flt3), and XL-019.

Examples for Mek inhibitors are ARRY-142886, PD-325901, AZD-8330, and XL 518.

Examples for mTor inhibitors are temsirolimus, AP-23573 (which also acts as a VEGF inhibitor), everolimus (a VEGF inhibitor in addition). XL-765 (also a PI3 kinase inhibitor), and BEZ-235 (also a PI3 kinase inhibitor).

Examples for Akt inhibitors are perifosine, GSK-690693, RX-0201, and triciribine.

Examples for cKit inhibitors are AB-1010, OSI-930 (also acts as a VEGFR inhibitor), AC-220 (also an inhibitor of Flt3 and PDGFR), tandutinib (also an inhibitor of Flt3 and PDGFR), axitinib (also an inhibitor of VEGFR and PDGFR), XL-999 (also an inhibitor of Flt3, PDGFR, VEGFR, FGFR), sunitinib (also an inhibitor of Flt3, PDGFR, VEGFR), and XL-820 (also acts as a VEGFR- and PDGFR inhibitor), imatinib (also a bcr-abl inhibitor), nilotinib (also an inhibitor of bcr-abl and PDGFR).

Examples for hedgehog antagonists are IPI-609 and CUR-61414.

Examples for CDK inhibitors are seliciclib, AT-7519, P-276, ZK-CDK (also inhibiting VEGFR2 and PDGFR), PD-332991, R-547, SNS-032, PHA-690509, and AG 024322.

Examples for proteasome inhibitors are bortezomib, carfilzomib, and NPI-0052 (also an inhibitor of NFkappaB).

An example for an NFkappaB pathway inhibitor is NPI-0052.

An example for an ubiquitination pathway inhibitor is HBX-41108.

In preferred embodiments, the additional therapeutic agent is an anti-angiogenic agent.

Examples for anti-angiogenic agents are inhibitors of the FGFR, PDGFR and VEGFR or the respective ligands (e.g VEGF inhibitors like pegaptanib or the anti-VEGF antibody bevacizumab), and thalidomides, such agents being selected from, without limitation, bevacizumab, motesanib, CDP-791, SU-14813, telatinib, KRN-951, ZK-CDK (also an inhibitor of CDK), ABT-869, BMS-690514, RAF-265, IMC-KDR, IMC-18F1, IMiDs (immunomodulatory drugs), thalidomide derivative CC-4047, lenalidomide, ENMD 0995, IMC-D11, Ki 23057, brivanib, cediranib, XL-999 (also an inhibitor of cKit and Flt3), 1 B3, CP 868596, IMC 3G3, R-1530 (also an inhibitor of Flt3), sunitinib (also an inhibitor of cKit and Flt3), axitinib (also an inhibitor of cKit), lestaurtinib (also an inhibitor of Flt3 and PKC), vatalanib, tandutinib (also an inhibitor of Flt3 and cKit), pazopanib, GW 786034, PF-337210, IMC-1121 B, AVE-0005, AG-13736, E-7080, CHIR 258, sorafenib tosylate (also an inhibitor of Raf), RAF-265 (also an inhibitor of Raf), vandetanib, CP-547632, OSI-930, AEE-788 (also an inhibitor of EGFR and Her2), BAY-57-9352 (also an inhibitor of Raf), BAY-73-4506 (also an inhibitor of Raf), XL 880 (also an inhibitor of cMet), XL-647 (also an inhibitor of EGFR and EphB4), XL 820 (also an inhibitor of cKit), and nilotinib (also an inhibitor of cKit and brc-abl).

The additional therapeutic agent may also be selected from EGFR inhibitors, it may be a small molecule EGFR inhibitor or an anti-EGFR antibody. Examples for anti-EGFR antibodies, without limitation, are cetuximab, panitumumab, matuzumab; an example for a small molecule EGFR inhibitor is gefitinib. Another example for an EGFR modulator is the EGF fusion toxin.

Among the EGFR and Her2 inhibitors useful for combination with the binding molecule of the invention are lapatinib, gefitinib, erlotinib, cetuximab, trastuzumab, nimotuzumab, zalutumumab, vandetanib (also an inhibitor of VEGFR), pertuzumab, XL-647, HKI-272, BMS-599626 ARRY-334543, AV 412, mAB-806, BMS-690514, JNJ-26483327, AEE-788 (also an inhibitor of VEGFR), ARRY-333786, IMC-11F8, Zemab.

Other agents that may be advantageously combined in a therapy with the binding molecule of the invention are tositumumab and ibritumomab tiuxetan (two radiolabelled anti-CD20 antibodies), alemtuzumab (an anti-CD52 antibody), denosumab, (an osteoclast differentiation factor ligand inhibitor), galiximab (a CD80 antagonist), ofatumumab (a CD20 inhibitor), zanolimumab (a CD4 antagonist), SGN40 (a CD40 ligand receptor modulator), rituximab (a CD20 inhibitor) or mapatumumab (a TRAIL-1 receptor agonist).

Other chemotherapeutic drugs that may be used in combination with the binding molecule s of the present invention are selected from, but not limited to hormones, hormonal analogues and antihormonals (e.g. tamoxifen, toremifene, raloxifene, fulvestrant, megestrol acetate, flutamide, nilutamide, bicalutamide, cyproterone acetate, finasteride, buserelin acetate, fludrocortisone, fluoxymesterone, medroxyprogesterone, octreotide, arzoxifene, pasireotide, vapreotide), aromatase inhibitors (e.g. anastrozole, letrozole, liarozole, exemestane, atamestane, formestane), LHRH agonists and antagonists (e.g. goserelin acetate, leuprolide, abarelix, cetrorelix, deslorelin, histrelin, triptorelin), antimetabolites (e.g. antifolates like methotrexate, pemetrexed, pyrimidine analogues like 5 fluorouracil, capecitabine, decitabine, nelarabine, and gemcitabine, purine and adenosine analogues such as mercaptopurine thioguanine, cladribine and pentostatin, cytarabine, fludarabine); antitumor antibiotics (e.g. anthracyclines like doxorubicin, daunorubicin, epirubicin and idarubicin, mitomycin-C, bleomycin dactinomycin, plicamycin, mitoxantrone, pixantrone, streptozocin); platinum derivatives (e.g. cisplatin, oxaliplatin, carboplatin, lobaplatin, satraplatin); alkylating agents (e.g. estramustine, meclorethamine, melphalan, chlorambucil, busulphan, dacarbazine, cyclophosphamide, ifosfamide, hydroxyurea, temozolomide, nitrosoureas such as carmustine and lomustine, thiotepa); antimitotic agents (e.g. vinca alkaloids like vinblastine, vindesine, vinorelbine, vinflunine and vincristine; and taxanes like paclitaxel, docetaxel and their formulations, larotaxel; simotaxel, and epothilones like ixabepilone, patupilone, ZK-EPO); topoisomerase inhibitors (e.g. epipodophyllotoxins like etoposide and etopophos, teniposide, amsacrine, topotecan, irinotecan) and miscellaneous chemotherapeutics such as amifostine, anagrelide, interferone alpha, procarbazine, mitotane, and porfimer, bexarotene, celecoxib.

Particularly preferred combination partners of the binding molecules of the present invention are VEGF antagonists, like bevacizumab (Avastin^{®}), Vargatef^{®}, Sorafenib and Sunitinib.

The efficacy of binding molecules of the invention or polypeptides containing them, and of compositions comprising the same, can be tested using any suitable *in vitro* assay, cell- based assay, *in vivo* assay and/or animal model known per se, or any combination thereof, depending on the specific disease or disorder of interest. Suitable assays and animal models will be clear to the skilled person, and for example include the assays described herein and used in the Examples below, e.g. a proliferation assay.

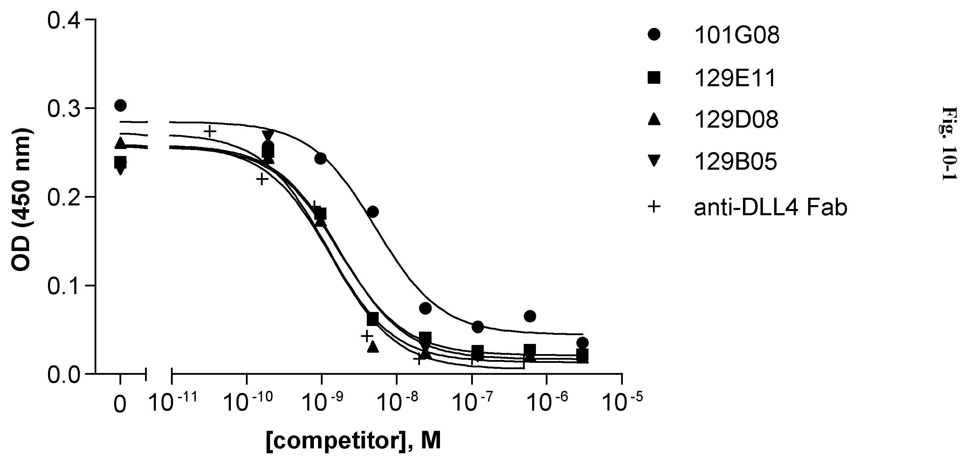

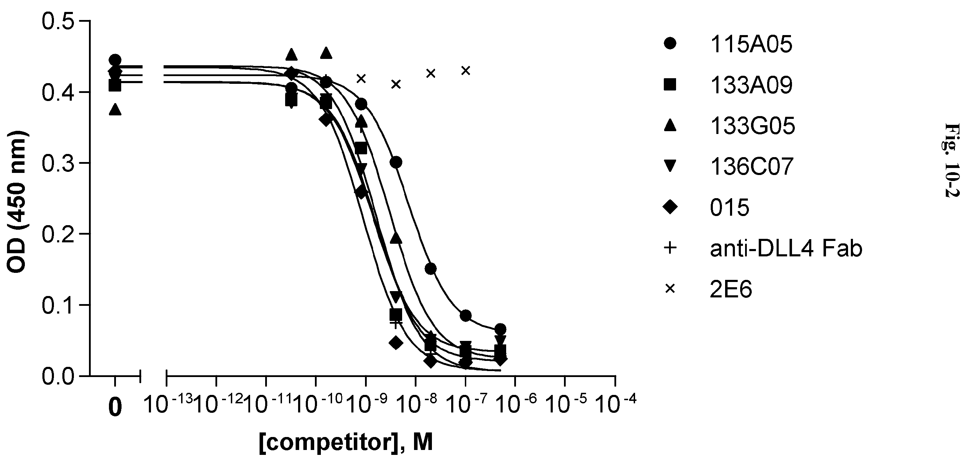

The data obtained in the experiments of the invention confirm that DII4-binding components of the invention have properties that are superior to those of DII4-binding molecules of the prior art, as can e.g. be taken from the ELISA data of Figure 10, showing that affinity-matured VHHs block hDLL4/hNotch1-Fc interaction in a complete manner, as well as the IC₅₀ (nM) values for affinity matured VHHs in hDLL4/hNotch1-Fc competition ELISA; and the affinity K_{D} (nM) of purified affinity matured VHHs on recombinant human DLL4 and mouse DLL4. This indicates that DII4-binding components of the invention are promising candidates to have therapeutic efficacy in diseases and disorders associated with DII4-mediated effects on angiogenesis, such as cancer.

### Brief description of the Figures:

- **Figure 1:**: Amino acid sequence alignment of human, rhesus and cynomolgus DLL4.
- **Figure 2:**: Human and mouse DLL4 deletion mutants (amino acid domain boundaries in superscript).
- **Figure 3:**: Purified VHHs blocking hDLL4/hNotch1-Fc interaction (ELISA).
- **Figure 4:**: Purified VHHs blocking hDLL4/hNotch1-Fc interaction (AlphaScreen).
- **Figure 5:**: Purified VHHs blocking CHO-hDLL4/hNotch1-Fc and CHO-mDLL4/hNotch1-Fc interaction (FMAT).
- **Figure 6:**: Purified VHHs blocking DLL4 mediated Notch1 cleavage (reporter).
- **Figure 7:**: Binding of purified VHHs to recombinant human and mouse DLL4 (ELISA).
- **Figure 8:**: Binding of purified VHHs to recombinant human DLL1 and human Jagged-1 (ELISA).
- **Figure 9:**: Binding of purified VHHs to human/mouse/cynomolgus DLL4 (FACS).
- **Figure 10:**: Affinity matured VHHs blocking hDLL4/hNotch1-Fc interaction (ELISA).
- **Figure 11:**: Purified affinity matured VHHs blocking CHO-hDLL4/hNotch1-Fc and CHO-mDLL4/hNotch1-Fc interaction (FMAT).
- **Figure 12:**: Binding of purified VHHs to human/mouse DLL4 (ELISA).
- **Figure 13:**: Binding of purified affinity matured VHHs to recombinant human DLL1 and human Jagged-1 (ELISA).

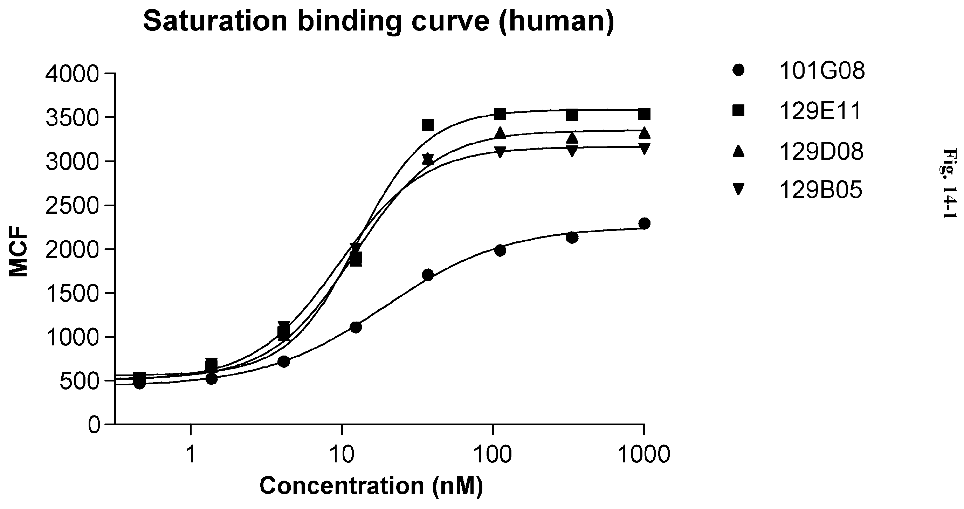

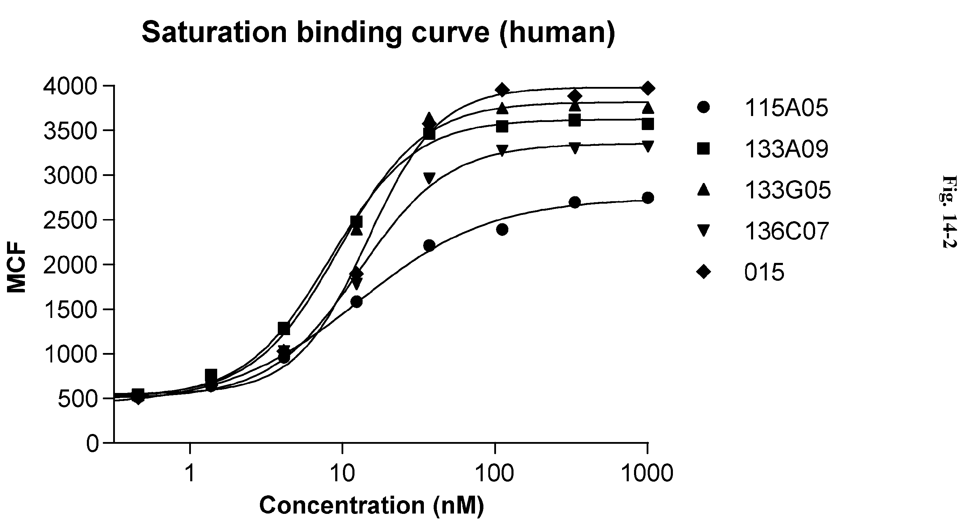

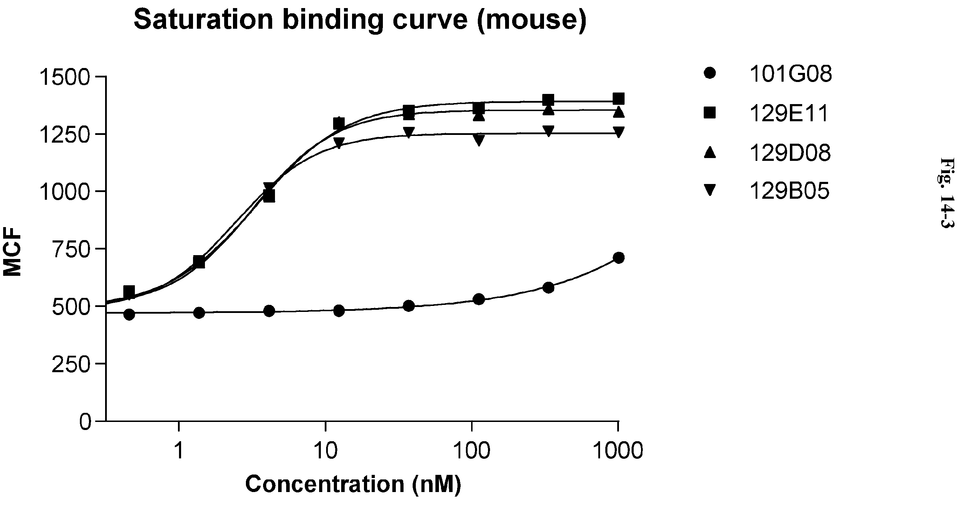

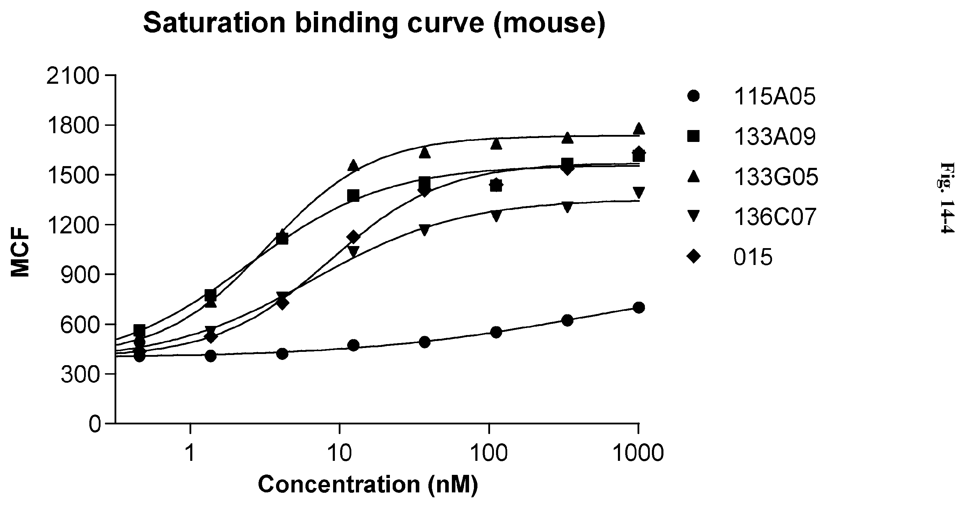

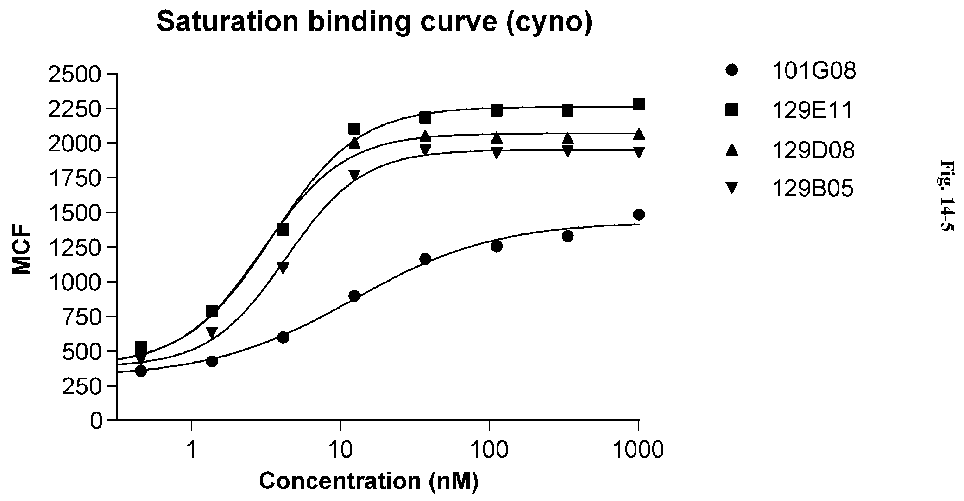

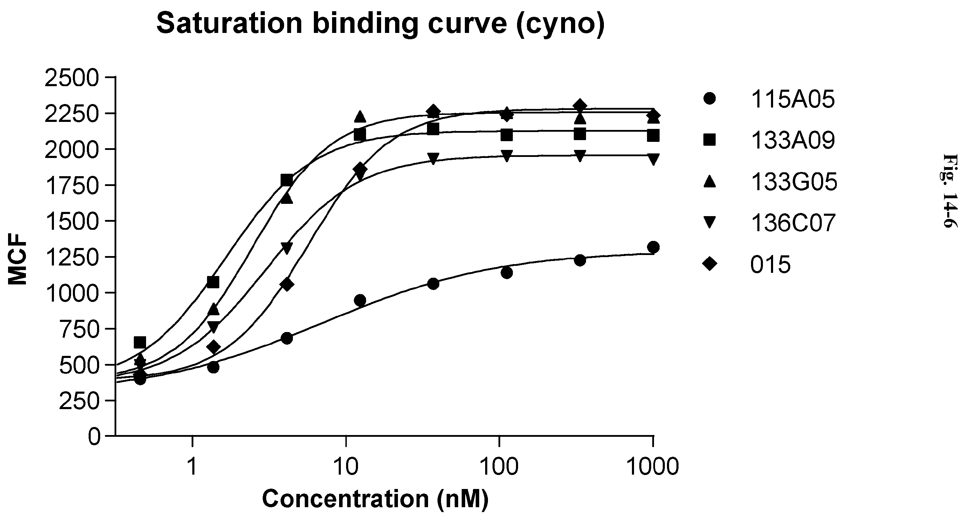

- **Figure 14:**: Binding of purified VHHs to human/mouse/cynomolgus DLL4 (FACS).
- **Figure 15:**: Evaluation of VHH effects on DII4-mediated inhibition of HUVEC proliferation.
- **Figure 16:**: Description cycle 1 DLL4xAng2 VHHs
- **Figure 17:**: Purified cycle 1 DLL4xAng2 VHHs blocking hDLL4-hNotch1 interaction (ELISA)
- **Figure 18:**: Purified cycle 1 DLL4xAng2 VHHs blocking CHO-hDLL4/Notch1 (44-1) and CHO-mDLL4/Notch1 (44-2) interaction (FMAT)
- **Figure 19:**: Purified cycle 1 DLL4xAng2 VHHs binding to human, mouse and cynomolgus DLL4 overexpressing CHO cells (FACS)
- **Figure 20:**: Purified cycle 1 DLL4xAng2 VHHs binding to human, mouse and rat DLL4 (ELISA)
- **Figure 21:**: Purified cycle 1 DLL4xAng2 VHHs binding to human DLL1 and Jagged-1 (ELISA)
- **Figure 22:**: Purified cycle 1 DLL4xAng2 VHHs blocking hAng2-hTie2 (48-1), mAng2-mTie2 (48-2) and cAng2/cTie2 (48-3) interaction (ELISA)
- **Figure 23:**: Description cycle 2 DLL4xAng2 bispecific VHHs
- **Figure 24:**: Purified cycle 2 DLL4xAng2 VHHs blocking hDLL4-hNotch1 interaction (ELISA)
- **Figure 25:**: Purified cycle 2 DLL4xAng2 VHHs blocking CHO-hDLL4/Notch1 (51-1) and CHO-mDLL4/Notch1 (51-2) interaction (FMAT)
- **Figure 26:**: Purified cycle 2 DLL4xAng2 VHHs blocking hDLL4 mediated Notch1 activation (reporter gene assay)
- **Figure 27:**: Purified cycle 2 DLL4xAng2 VHHs binding to human, mouse and cynomolgus DLL4 overexpressing CHO cells (FACS)
- **Figure 28:**: Purified cycle 2 DLL4xAng2 VHHs binding to human, mouse and rat DLL4 (ELISA)
- **Figure 29:**: Purified cycle 2 DLL4xAng2 VHHs binding to human DLL1 and Jagged-1 (ELISA)
- **Figure 30:**: Purified cycle 2 DLL4xAng2 VHHs blocking hAng2-hTie2 (56-1), mAng2-mTie2 (56-2) and cAng2/cTie2 (56-3) interaction (ELISA)
- **Figure 31:**: Purified cycle 2 DLL4xAng2 VHHs blocking hAng1-hTie2 interaction (ELISA)
- **Figure 32 :**: Purified cycle 2 DLL4xAng2 VHHs blocking hAng2 mediated HUVEC survival.

### Materials and methods

### a) Generation CHO and HEK293 cell lines overexpressing human, mouse and cynomolgus DII4

The cDNAs encoding human (SEQ ID NO: 417; NM_019074.2) and mouse DII4 (NM_019454.3) are amplified from a Human Adult Normal Tissue Heart cDNA library (BioChain, Hayward, CA, USA) and a Mouse Heart Tissue cDNA library (isolated from C57/BI6 strain), respectively, using oligonucleotides designed in the 5' and 3' UTR of the corresponding sequence (see Table 1; SEQ ID NO:421 to 426). Amplicons are cloned into the mammalian expression vector pCDNA3.1 (+)-neo (Invitrogen, Carlsbad, CA, USA).

**Table 1: Oligonucleotide sequences used for amplification of DLL4 gene full length orthologues.**

| **Human DLL4** | **Mouse DLL4** | **Cynomolgus DLL4** |
|---|---|---|
| >Fwd_hDLL4 | >Fwd_mDLL4 | >Fwd_cDLL4 |
| | | |
| >Rev_hDLL4 | >Rev_mDLL4 | >Rev_cDLL4 |
| | | |

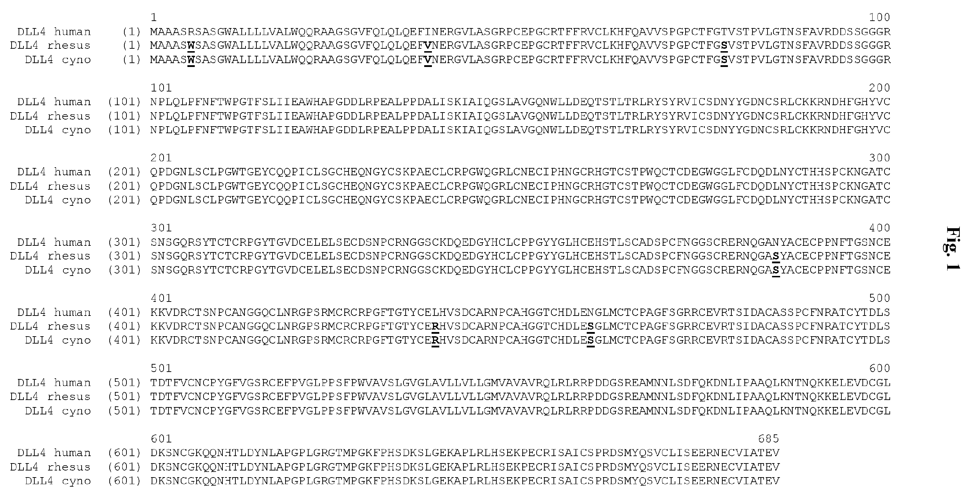

Cynomolgus DII4 cDNA is amplified from a Cynomolgus Normal Tissue Heart cDNA library (BioChain, Hayward, CA, USA), using primers designed on the 5' and 3' UTR of the DII4 encoding sequence of the closely related species rhesus (Macaca mulatta DII4, SEQ ID NO:418; XM_001099250.1) (see Table 1). The final amplicon is cloned in the mammalian expression vector pCDNA3.1 (+)-neo (Invitrogen, Carlsbad, CA, USA). The amino acid sequence of cynomolgus DII4 was shown to be 100% identical to rhesus, and 99% identical to human (see Figure 1; differences from the human sequence are indicated as bold-underlined).

To establish Chinese Hamster Ovary (CHO) cells overexpressing human DII4, mouse DII4 or cynomolgus DII4, parental CHO cells are electroporated with pCDNA3.1 (+)-neo-hDII4, pcDNA3.1(+)-neo-mDII4 or pcDNA3.1(+)-neo-cDII4, respectively. Human Embyonic Kidney (HEK293) cells overexpressing human DII4 and mouse DII4 are generated by lipid-mediated transfection with Fugene (Roche) of pCDNA3.1 (+)-neo-hDII4 or mDII4 plasmids, respectively, in the HEK293 parental cell line. For all conditions, transfectants are selected by adding 1 mg/mL geneticin (Invitrogen, Carlsbad, CA, USA).

### b) Generation of monoclonal anti-DII4 IgG and Fab fragment

In US 2008/0014196 (Genentech) a human/mouse cross-reactive DII4 mAb is described that was used by Ridgway *et al.* (2006) to show additive effects of VEGF mAb and DII4 mAb on tumor growth in a number of xenograft models. This anti-DII4 mAb and its corresponding Fab are purified to assess the properties of this antibody (fragment) in biochemical/cellular assays and xenograft models and for specific elutions during phage selections. The published variable heavy and light chain sequences of DII4 mAb are cloned into a hlgG2aK framework, transiently expressed in HEK293 cells and purified from supernatants using protein A chromatography. Purified DII4 mAb shows binding to human DII4 and mouse DII4 in ELISA and FACS (using CHO-mDII4 and CHO-hDII4 cells), sub-nanomolar affinities to both growth factor orthologues in Biacore.

The corresponding DII4 Fab fragment is constructed via gene assembly based on back-translation and codon optimization for expression in E. *coli* using Leto's Gene Optimization software (www.entechelon.com). Oligonucleotide primers for the assembly of the variable light chain (V_{L}), variable heavy chain (V_{H}), constant light chain (C_{L}) and constant domain 1 of the heavy chain (C_{H1}) are designed and an assembly PCR is performed. The cDNA seqments encoding V_{L}+C_{L} and V_{H}+C_{H1} are cloned into a pUC119-derived vector, which contains the LacZ promotor, a resistance gene for kanamycin, a multiple cloning site and a hybrid gIII-pelB leader sequence, using the restriction sites *Sfil* and *Ascl* and the restriction sites *Kpnl* and *Notl,* respectively. In frame with the Fab coding sequence, the expression vector encodes a C-terminal HA and His6-tag. The Fab fragment is expressed in *E. coli* as His6-tagged protein and subsequently purified from the culture medium by immobilized metal affinity chromatography (IMAC) and size exclusion chromatography (SEC). Relevant amino acid sequences of the variable heavy and variable light chain are depicted (SEQ ID NO: 1 and SEQ ID NO: 2; respectively, of US 2008/0014196); the amino acid sequences of the complete heavy and light chain are shown in SEQ ID NOs: 419 and 420, respectively.

### c) Generation of DII4 mutants for epitope mapping

To identify the region in the extracellular domain (ECD) of DII4 that comprises the epitope recognized by the anti-DII4 VHHs, progressive deletion mutants of the DII4 ECD are generated. The mammalian expression vector pSecTag2/Hygro (Invitrogen, Carlsbad, CA, USA) comprising a CMV promotor upstream of polynucleotides encoding a nested series of deletion fragments of the DII4 ECD fused to a polyHis-tag are generated using standard recombinant DNA technology (see Figure 2; amino acid domain boundaries in superscript).). These recombinant proteins are expressed in transiently transfected HEK293 cells using the Freestyle 293 Expression System (Invitrogen, Carlsbad, CA, USA) from which conditioned medium is collected and purified via IMAC. Only DII4 mutants lacking the EGF2-like domain showed impaired binding to the humanized human/mouse cross-reactive anti-DII4 mAb described above (immobilized via a capturing anti-human IgG coated Biacore sensor chip). This IgG is known to have a specific binding epitope in this DII4 domain (patent application Genentech, US 2008/0014196A1).

### d) Generation of DII4 reporter assay plasmids

*A* reporter assay is developed based on the γ-secretase mediated cleavage of Notch1 and nuclear translocation of the intracellular domain of Notch1 (NICD) upon stimulation with DII4, essentially as described (Struhl and Adachi, Cell. 1998 May 15; 93(4):649-60). Gal4/VP16 coding sequences are inserted into the NICD-coding sequence. The potent hybrid transcriptional activator GAL4-VP16, which consists of a DNA binding fragment of yeast GAL4 fused to a Herpes simplex viral transcriptional activator domain VP16, is inserted carboxy-terminal to the transmembrane domain of Notch1. Cleavage of this construct by γ-secretase results in the release of the Gal4/VP16 NICD fusion protein which will translocate to the nucleus where it will bind to and transcriptionally activate a co-transfected luciferase reporter plasmid, containing a strong GAL4-UAS promoter sequence (Struhl, G. and Adachi, A., Cell, vol. 93, 649-660, 1998). The human Notch1-Gal4/VP16 expression cassette is cloned in pcDNA3.1 (+)-neo (Invitrogen, Carlsbad, CA, USA). The pGL4.31 [Luc2P/Gal4UAS/Hygro] vector (Promega, Madison, WI, USA) is used as luciferase reporter plasmid.

The following Examples demonstrate how the inventors arrived at the invention. An example for a polypeptide of the invention is shown in Example 8 / DLLANGBII00018.

### Example 1

### Immunization with DII4 from different species induces a humoral immune response in llama

### 1.1. Immunizations

After approval of the Ethical Committee of the faculty of Veterinary Medicine (University Ghent, Belgium), 4 llamas (designated No. 208, 209, 230, 231) are immunized with 6 intramuscular injections (100 or 50 µg/dose at weekly intervals) of recombinant human DII4 (R&D Systems, Minneapolis, MN, US). The DII4 antigen is formulated in Stimune (Cedi Diagnostics BV, Lelystad, The Netherlands). Three additional llamas (designated No. 127b, 260, 261) are immunized according to standard protocols with 4 subcutaneous injections of alternating human DII4 and mouse DII4 overexpressing CHO cells which are established as described above. Cells are re-suspended in D-PBS and kept on ice prior to injection. Furthermore, three additional llamas (designated No. 282, 283, 284) are immunized according to standard protocols with 4 intramuscular injections (100 or 50 µg/dose at biweekly intervals) of alternating recombinant human DII4 and mouse DII4 (R&D Systems, Minneapolis, MN, US). The first injection at day 0 with human DII4 is formulated in Complete Freund's Adjuvant (Difco, Detroit, MI, USA), while the subsequent injections with human and mouse DII4 are formulated in Incomplete Freund's Adjuvant (Difco, Detroit, MI, USA).

### 1.2. Evaluation of induced immune responses in llama

To evaluate the induction of an immune responses in the animals against human DII4 by ELISA, sera are collected from llamas 208, 209, 230 and 231 at day 0 (pre-immune), day 21 and day 43 (time of peripheral blood lymphocyte [PBL] collection), from llamas 127b, 260 and 261 at day 0 and day 51, and from llamas 282, 283 and 284 at day 0, day 28 and day 50. In short, 2 µg/mL of recombinant human DII4 or mouse DII4 (R&D Systems, Minneapolis, MN, USA) are immobilized overnight at 4°C in a 96-well MaxiSorp plate (Nunc, Wiesbaden, Germany). Wells are blocked with a casein solution (1%). After addition of serum dilutions, specifically bound immunoglobulins are detected using a horseradish peroxidase (HRP)-conjugated goat anti-llama immunoglobulin (Bethyl Laboratories Inc., Montgomery, TX, USA) and a subsequent enzymatic reaction in the presence of the substrate TMB (3,3',5,5'-tetramentylbenzidine) (Pierce, Rockford, IL, USA), showing that a significant antibody-dependend immune response against DII4 is induced. The antibody response is mounted both by conventional and heavy-chain only antibody expressing B-cell repertoires since specifically bound immunoglobulins can be detected with antibodies specifically recognizing the conventional llama IgG1 antibodies or the heavy chain only llama IgG2 or IgG3 antibodies (Table 2-A). In all llamas injected with mouse DII4, an antibody response is mounted by conventional and heavy chain only antibody expressing B-cells specifically against mouse DII4. Additionally, serum titers of cell immunized animals are confirmed by FACS analysis on human and mouse DII4 overexpressing HEK293 cells (Table 2-B). The DII4 serum titer responses for each llama are depicted in Table 2.

**Table 2: Antibody mediated specific serum response against DLL4.**

| **A) ELISA** (recombinant protein solid phase coated) | | | | | | | | | |
|---|---|---|---|---|---|---|---|---|---|
| | | **Recombinant human DLL4** | | | | **Recombinant mouse DLL4** | | | |
| **Llama** | **Immunogen** | **Total IgG** | **IgG1** | **IgG2** | **IgG3** | **Total IgG** | **IgG1** | **IgG2** | **IgG3** |
| | | | | | | | | | |
| | | | | | | | | | |
| 208 | rec. human DLL4 | + | + | +/- | +/- | ND | ND | ND | ND |
| 209 | rec. human DLL4 | + | + | +/- | +/- | ND | ND | ND | ND |
| 230 | rec. human DLL4 | ++ | ++ | +/- | +/- | ND | ND | ND | ND |
| 231 | rec. human DLL4 | ++ | ++ | ++ | ++ | ND | ND | ND | ND |
| | | | | | | | | | |
| 127b | CHO-hDLL4 + CHO-mDLL4 | ++ | ++ | +/- | +/- | + | ++ | +/- | +/- |
| 260 | CHO-hDLL4 + CHO-mDLL4 | ++ | ++ | + | + | ++ | ++ | + | ++ |
| 261 | CHO-hDLL4 + CHO-mDLL4 | ++ | ++ | +/- | +/- | + | + | +/- | +/- |
| | | | | | | | | | |
| 282 | rec. human DLL4 + mouse DLL4 | ++ | ++ | ++ | ++ | ++ | ++ | + | + |
| 283 | rec. human DLL4 + mouse DLL4 | ++ | ++ | ++ | ++ | ++ | ++ | ++ | ++ |
| 284 | rec. human DLL4 + mouse DLL4 | + | + | + | + | + | ++ | + | ++ |

| **B) FACS** (natively expressed protein on HEK293 cells) | | | | | | | | | |
|---|---|---|---|---|---|---|---|---|---|
| | | **human DLL4** | | | | **mouse DLL4** | | | |
| **Llama** | **Immunogen** | **Total IgG** | **IgG1** | **IgG2** | **IgG3** | **Total IgG** | **IgG1** | **IgG2** | **IgG3** |
| | | | | | | | | | |
| | | | | | | | | | |
| 208 | rec. human DLL4 | ND | ND | ND | ND | ND | ND | ND | ND |
| 209 | rec. human DLL4 | ND | ND | ND | ND | ND | ND | ND | ND |
| 230 | rec. human DLL4 | ND | ND | ND | ND | ND | ND | ND | ND |
| 231 | rec. human DLL4 | ND | ND | ND | ND | ND | ND | ND | ND |
| | | | | | | | | | |
| 127b | CHO-hDLL4 + CHO-mDLL4 | + | ND | ND | ND | + | ND | ND | ND |
| 260 | CHO-hDLL4 + CHO-mDLL4 | ++ | ND | ND | ND | ++ | ND | ND | ND |
| 261 | CHO-hDLL4 + CHO-mDLL4 | + | ND | ND | ND | + | ND | ND | ND |
| | | | | | | | | | |
| 282 | rec. human DLL4 + mouse DLL4 | ND | ND | ND | ND | ND | ND | ND | ND |
| 283 | rec. human DLL4 + mouse DLL4 | ND | ND | ND | ND | ND | ND | ND | ND |
| 284 | rec. human DLL4 + mouse DLL4 | ND | ND | ND | ND | ND | ND | ND | ND |

| | | | | | | | | | |
|---|---|---|---|---|---|---|---|---|---|
| ND: not determined | | | | | | | | | |

### Example 2

### Cloning of the heavy-chain only anti-DII4 antibody fragment repertoires and preparation of phage

Following the final immunogen injection, immune tissues as the source of B-cells that produce the heavy-chain antibodies are collected from the immunized llamas. Typically, two 150-ml blood samples, collected 4 and 8 days after the last antigen injection, and one lymph node biopsy, collected 4 days after the last antigen injection are collected per animal. From the blood samples, peripheral blood mononuclear cells (PBMCs) are prepared using Ficoll-Hypaque according to the manufacturer's instructions (Amersham Biosciences, Piscataway, NJ, USA). From the PBMCs and the lymph node biopsy, total RNA is extracted, which is used as starting material for RT-PCR to amplify the VHH encoding DNA segments, as described in WO 05/044858. For each immunized llama, a library is constructed by pooling the total RNA isolated from all collected immune tissues of that animal. In short, the PCR-amplified VHH repertoire is cloned via specific restriction sites into a vector designed to facilitate phage display of the VHH library. The vector is derived from pUC119 and contains the LacZ promoter, a M13 phage gIII protein coding sequence, a resistance gene for ampicillin or carbenicillin, a multiple cloning site and a hybrid gIII-pelB leader sequence (pAX050). In frame with the VHH coding sequence, the vector encodes a C-terminal c-myc tag and a His6 tag. Phage are prepared according to standard protocols and stored after filter sterilization at 4°C for further use.

### Example 3

### Selection of DII4 specific VHHs via phage display

VHH repertoires obtained from all llamas and cloned as phage library are used in different selection strategies, applying a multiplicity of selection conditions. Variables include i) the DII4 protein format (C-terminally His-tagged recombinantly expressed extracellular domain of human DII4 (Met1-Pro524) and mouse DII4 (Met1-Pro525) (R&D Systems, Minneapolis, MN, USA), or full length human DII4 and mouse DII4 present on DII4-overexpressing CHO or HEK293 cells, ii) the antigen presentation method (plates directly coated with DII4 or Neutravidin plates coated with DII4 via a biotin-tag; solution phase: incubation in solution followed by capturing on Neutravidin-coated plates), iii) the antigen concentration and iv) different elution methods (non-specific via trypsin or specfic via cognate receptor Notch1/Fc chimera or anti-DII4 IgG/Fab). All selections are done in Maxisorp 96-well plates (Nunc, Wiesbaden, Germany).

Selections are performed as follows: DII4 antigen preparations for solid and solution phase selection formats are presented as described above at multiple concentrations. After 2h incubation with the phage libraries followed by extensive washing, bound phage are eluted with trypsin (1 mg/mL) for 30 minutes. In case trypsin is used for phage elution, the protease activity is immediately neutralized applying 0.8 mM protease inhibitor ABSF. As control, selections w/o antigen are performed in parallel. Phage outputs that show enrichment over background (non-antigen control) are used to infect E. coli. Infected E. coli cells are either used to prepare phage for the next selection round (phage rescue) or plated on agar plates (LB+amp+glucose^{2%} for analysis of individual VHH clones. In order to screen a selection output for specific binders, single colonies are picked from the agar plates and grown in 1 mL 96-deep-well plates. LacZ-controlled VHH expression is induced by adding IPTG (0.1-1 mM final) in the absence of glucose. Periplasmic extracts (in a volume of ~ 80 uL) are prepared according to standard protocols

### Example 4

### Screening of periplasmic extracts in DII4-Notch1 AlphaScreen and FMAT competition assay

Periplasmic extracts are screened in a human DII4/human Notch1 AlphaScreen assay to assess the blocking capacity of the expressed VHHs. Human DII4 is biotinylated using biotin (Sigma, St Louis, MO, USA) and biotinamidohexanoic acid 3-sulfo-N-hydroxysuccinimide ester sodium salt (Sigma, St Louis, MO, USA). Notch1/Fc chimera (R&D Systems, Minneapolis, MN, USA) is captured using an anti-Fc VHH which is coupled to acceptor beads according to the manufacturer's instructions (Perkin Elmer, Waltham, MA, US). To evaluate the neutralizing capacity of the VHHs, dilution series of the periplasmic extracts are pre-incubated with biotinylated human DII4. To this mixture, the acceptor beads and the streptavidin donor beads are added and further incubated for 1 hour at room temperature. Fluorescence is measured by reading plates on the Envision Multilabel Plate reader (Perkin Elmer, Waltham, MA, USA) using an excitation wavelength of 680 nm and an emission wavelength of 520 nm. Decrease in fluorescence signal indicates that the binding of biotinylated human DII4 to the human Notch1/Fc receptor is blocked by the VHH expressed in the periplasmic extract.

Alternatively, CHO-hDII4 and CHO-mDII4 cells are used in a human Notch1/Fc FMAT (Fluorometric Microvolume Assay Technology) competition assay. Recombinant human Notch1/Fc chimera (R&D Systems, Minneapolis, MN, USA) is randomly labeled with Alexa-647 (Invitrogen, Carlsbad, CA, USA). In brief, 5 µL periplasmic material is added to 100 pM or 175 pM labeled human Notch1/Fc together with 7,500 CHO-hDII4 or CHO-mDII4 overexpressing cells, respectively, and readout is performed after 2 hours of incubation. To set the no-competition baseline, at least 30 replicates of cells with human Notch1/Fc~Alexa647 are included and the percentage of inhibition is calculated from this baseline. All calculations are based on the FL1_total signal which comprises the average of the fluorescence per well times the number of counts per well.

From this screening, inhibiting VHHs are selected and sequenced. Sequence analysis revealed 167 unique VHHs belonging to 40 different B-cell lineages. The total number of variants found for each B-cell lineage is depicted in Table 3. An overview of periplasmic screening data is given in Table 4. The amino acid sequences of all obtained unique VHHs are shown in the Sequence Listing (SEQ ID NO:167 - 332 and 459) and in Table 5 (CDRs and framework regions are indicated).

**Table 3: Selection parameters used for the identification of DLL4 specific VHH B-cell lineages.**

| **B-cell lineage** | **VHH ID** | **# variants** | **library** | **selection format** | **phage elution** | **selection rounds** |
|---|---|---|---|---|---|---|
| 1 | DLLBII8A09 | 31 | 231 | rhDLL4 (3 nM) | trypsin | 1 |
| 2 | DLLBII5B11 | 1 | 231 | rhDLL4 (3 nM) | trypsin | 1 |
| 3 | DLLBII7B5 | 21 | 231 | RI: biot-rhDLL4 (3 nM) | trypsin | 2 |
| | | | | RII: biot-rhDLL4 (0.03 nM) | | |
| 4 | DLLBII6B11 | 13 | 231 | biot-rhDLL4 (3 M) | trypsin | 1 |
| 5 | DLLBII8C11 | 5 | 231 | RI: biot-rhDLL4 (3 nM) | trypsin | 2 |
| | | | | RII: biot-rhDLL4 (3 nM) | | |
| 6 | DLLBII19D10 | 1 | 231 | biot-rhDLL4 (3 nM) | trypsin | 1 |
| 7 | DLLBII33C5 | 2 | 231 | CHO-hDLL4 (2E6/mL) | trypsin | 1 |
| 8 | DLLBII28B6 | 2 | 231 | rmDLL4 (0.5 ug/mL) | trypsin | 1 |
| 9 | DLLBII17G10 | 1 | 231 | biot-rhDLL4 (3 nM) | trypsin | 1 |
| 10 | DLLBII1 C1 | 8 | 231 | biot-rhDLL4 (3 nM) | trypsin | 1 |
| 11 | DLLBII19F4 | 1 | 231 | biot-rhDLL4 (3 nM) | trypsin | 1 |
| 12 | DLLBII17F10 | 1 | 231 | biot-rhDLL4 (3 nM) | trypsin | 1 |
| 13 | DLLBII17B3 | 5 | 231 | biot-rhDLL4 (3 nM) | trypsin | 1 |
| 14 | DLLBII19F12 | 2 | 231 | biot-rhDLL4 (3 nM) | trypsin | 1 |
| 15 | DLLBII42B7 | 1 | 231 | RI: biot-rhDLL4 (3 nM) | rhNotch1/Fc | 2 |
| | | | | RII: biot-rhDLL4 (3 nM) | | |
| 16 | DLLBII47D1 | 1 | 230 | RI: biot-rhDLL4 (3 nM) | rhNotch1/Fc | 2 |
| | | | | RII: biot-rhDLL4 (3 nM) | | |
| 17 | DLLBII56A09 | 15 | 230 | RI: CHO-mDLL4 (2E6/mL) | rhNotch1/Fc | 2 |
| | | | | RII: CHO-mDLL4 (2E6/mL) | | |
| 18 | DLLBII95F2 | 5 | 230 | RI: CHO-mDLL4 (2E6/mL) | trypsin | 2 |
| | | | | RII: CHO-mDLL4 (2E6/mL) | | |
| 19 | DLLBII96C3 | 20 | 230 | RI: CHO-mDLL4 (2E6/mL) | trypsin | 2 |
| | | | | RII: CHO-mDLL4 (2E6/mL) | | |
| 20 | DLLBII104G1 | 1 | 230 | RI: CHO-mDLL4 (2E6/mL) | rh Notch1/Fc (RI-RII) trypsin (RIII) | 3 |
| | | | | RII: CHO-mDLL4 (2E6/mL) | | |
| | | | | RIII: biot-rhDLL4 (+rhDLL4) | | |
| 21 | DLLBII102F8 | 3 | 230 | RI: CHO-mDLL4 (2E6/mL) | rhNotch1/Fc (RI-RII) trypsin (RIII) | 3 |
| | | | | RII: CHO-mDLL4 (2E6/mL) | | |
| | | | | RIII: biot-rhDLL4 (0.01 nM) | | |
| 22 | DLLBII112A3 | 1 | 209 | RI: CHO-mDLL4 (2E6/mL) | trypsin | 2 |
| | | | | RII: CHO-mDLL4 (2E6/mL) | | |
| 23 | DLLBII102G4 | 2 | 230 | RI: CHO-mDLL4 (2E6/mL) | rhNotch1/Fc (RI-RII) trypsin (RIII) | 3 |
| | | | | RII: CHO-mDLL4 (2E6/mL) | | |
| | | | | RIII: biot-rhDLL4 (0.01 nM) | | |
| 24 | DLLBII101G8 | 1 | 230 | RI: CHO-mDLL4 (2E6/mL) | rhNotch1/Fc (RI-RII) trypsin (RIII) | 3 |
| | | | | RII: CHO-mDLL4 (2E6/mL) | | |
| | | | | RIII: biot-rhDLL4 (0.1 nM) | | |
| 25 | DLLBII112A4 | 1 | 209 | RI: CHO-mDLL4 (2E6/mL) | trypsin | 2 |
| | | | | RII: CHO-mDLL4 (2E6/mL) | | |
| 26 | DLLBII101H9 | 1 | 230 | RI: CHO-mDLL4 (2E6/mL) | rhNotch1/Fc (RI-RII) trypsin (RIII) | 3 |
| | | | | RII: CHO-mDLL4 (2E6/mL) | | |
| | | | | RIII: biot-rhDLL4 (0.1 nM) | | |
| 27 | DLLBII101H5 | 1 | 230 | RI: CHO-mDLL4 (2E6/mL) | rhNotch1/Fc (RI-RII) trypsin (RIII) | 3 |
| | | | | RII: CHO-mDLL4 (2E6/mL) | | |
| | | | | RIII: biot-rhDLL4 (1 nM) | | |
| 28 | DLLBII112E7 | 1 | 209 | RI: CHO-mDLL4 (2E6/mL) | trypsin | 2 |
| | | | | RII: CHO-mDLL4 (2E6/mL) | | |
| 29 | DLLBII101F1 | 1 | 230 | RI: CHO-mDLL4 (2E6/mL) | rhNotch1/Fc (RI-RII) trypsin (RIII) | 3 |
| | | | | RII: CHO-mDLL4 (2E6/mL) | | |
| | | | | RIII: biot-rhDLL4 (1 nM) | | |
| 30 | DLLBII104A3 | 1 | 230 | RI: CHO-mDLL4 (2E6/mL) | rhNotch1/Fc (RI-RII) trypsin (RIII) | 3 |
| | | | | RII: CHO-mDLL4 (2E6/mL) | | |
| | | | | RIII: biot-rhDLL4 (1 nM) + rhDLL4 | | |
| 31 | DLLBII104C4 | 1 | 230 | RI: CHO-mDLL4 (2E6/mL) | rhNotch1/Fc (RI-RII) trypsin (RIII) | 3 |
| | | | | RII: CHO-mDLL4 (2E6/mL) | | |
| | | | | RIII: biot-rhDLL4 (1 nM) + rhDLL4 | | |
| 32 | DLLBII104B5 | 1 | 230 | RI: CHO-mDLL4 (2E6/mL) | rhNotch1/Fc (RI-RII) trypsin (RIII) | 3 |
| | | | | RII: CHO-mDLL4 (2E6/mL) | | |
| | | | | RIII: biot-rhDLL4 (1 nM) + rhDLL4 | | |
| 33 | DLLBII107C3 | 1 | 208 | RI: CHO-mDLL4 (2E6/mL) | rhNotch1/Fc | 2 |
| | | | | RII: CHO-mDLL4 (2E6/mL) | | |
| 34 | DLLBII58A11 | 4 | 260 | RI: biot-rhDLL4 (3 nM) | rhNotch1/Fc | 2 |
| | | | | RII: biot-rmDLL4 (3 nM) | | |
| 35 | DLLBII61F5 | 1 | 260 | RI: HEK293H-hDLL4 (2E6/mL) | trypsin | 2 |
| | | | | RII: HEK293H-hDLL4 (2E6/mL) | | |
| 36 | DLLBII61F7 | 1 | 260 | RI: HEK293H-hDLL4 (2E6/mL) | trypsin | 2 |
| | | | | RII: HEK293H-hDLL4 (2E6/mL) | | |
| 37 | DLLBII62C11 | 1 | 260 | RI: HEK293H-hDLL4 (2E6/mL) | trypsin | 2 |
| | | | | RII: HEK293H-mDLL4 (2E6/mL) | | |
| 38 | DLLBII115A5 | 1 | 230 | RI: CHO-mDLL4 (2E6/mL) | rhNotch1/Fc (RI-RII) trypsin (RIII) trypsin (RIV) | 4 |
| | | | | RII: CHO-mDLL4 (2E6/mL) | | |
| | | | | RIII: biot-rhDLL4 (1 nM) | | |
| | | | | RIV:CHO-mDLL4 (2E6/mL) | | |
| 39 | DLLBII83 G1 | 4 | 284 | RI: CHO-mDLL4 (2E6/mL) | DLL4 IgG | 2 |
| | | | | RI: CHO-hDLL4 (2E6/mL) | | |
| 40 | DLLBII80E8 | 1 | 283 | RI: CHO-hDLL4 (2E6/mL) | DLL4 IgG | 2 |
| | | | | RI: CHO-hDLL4 (2E6/mL) | | |

**Table 4: Screening of periplasmic extracts containing expressed anti-DLL4 VHH**

| **B-cell lineage** | **Representative VHH ID** | **# unique sequences** | **ELISA** | **Alpha Screen** | **FMAT** | **FMAT** | **Biacore (a)** |
|---|---|---|---|---|---|---|---|
| | | | hDLL4% inhibition | hDLL4% inhibition | hDLL4% inhibit tion | mDLL4% inhibittion | k_{d} (s⁻¹) |
| 1 | DLLBII8A09 | 31 | 96 | - | - | - | (*1.2^{E-03}*-*2.4^{E-04}*) |
| 2 | DLLBII5B11 | 1 | 98 | - | - | - | - |
| 3 | DLLBII7B05 | 21 | 84 | - | - | - | (*2.4^{E-04}*) |
| 4 | DLLBII6B11 | 13 | 98 | - | - | - | (*9.4^{E-04}*-*3.7^{E-04}*) |
| 5 | DLLBII8C11 | 5 | 57 | - | - | - | (*7.3^{E-04}*-*6.0^{E-04}*) |
| 6 | DLLBII19D10 | 1 | 98 | 85 | - | - | 1.3^{E-03} |
| 7 | DLLBII33C05 | 2 | 86 | 75 | - | - | 9.2^{E-04} (*2.1^{E-03}*) |
| 8 | DLLBII28B06 | 2 | 23 | 54 | - | - | 7.5^{E-03} (*1.6^{E-04}*) |
| 9 | DLLBII17G10 | 1 | 93 | 82 | - | - | 1.5^{E-03} |
| 10 | DLLBII17C01 | 8 | 82 | 84 | - | - | 5.6^{E-04} (*5.6^{E-04}*-*5.3^{E-04}*) |
| 11 | DLLBII19F04 | 1 | 98 | 95 | - | - | 1.1^{E-03} |
| 12 | DLLBII17F10 | 1 | 98 | 88 | - | - | 1.1^{E-03} / 3.1^{E-04(b)} |
| 13 | DLLBII17B03 | 5 | 76 | 77 | - | - | 1.2^{E-03} / 2.2^{E-04(b)} |
| 14 | DLLBII19F12 | 2 | 98 | 98 | - | - | 4.9^{E-04} (*1.0^{E-03}*) |
| 15 | DLLBII42B07 | 1 | - | - | - | - | - |
| 16 | DLLBII47D01 | 1 | - | - | 87 | - | - |
| 17 | DLLBII56A09 | 15 | - | - | - | - | 1.1 ^{E-03} (*9.5^{E-03}*-*1.1^{E-03}*) |
| 18 | DLLBII95F02 | 5 | - | - | 81 | 71 | 6.7^{E-04} |
| 19 | DLLBII96C03 | 20 | - | - | 75 | 83 | - |
| 20 | DLLBII104G01 | 1 | - | - | 94 | 86 | 1.2^{E-03} (*1.4^{E-03}*-*9.4^{E-04}*) |
| 21 | DLLBII102F08 | 3 | - | - | 85 | 75 | - |
| 22 | DLLBII112A03 | 1 | - | - | 72 | 97 | - |
| 23 | DLLBII102G04 | 2 | - | - | 86 | 82 | - |
| 24 | DLLBII101G08 | 1 | - | - | 91 | 92 | 2.1^{E-03} |
| 25 | DLLBII112A04 | 1 | - | - | 75 | 90 | - |
| 26 | DLLBII101H09 | 1 | - | - | 87 | 75 | - |
| 27 | DLLBII101H05 | 1 | - | - | 85 | 83 | - |
| 28 | DLLBII112E07 | 1 | - | - | 80 | 85 | - |
| 29 | DLLBII101F01 | 1 | - | - | 85 | 78 | 2.0^{E-02} |
| 30 | DLLBII104A03 | 1 | - | - | 86 | 83 | - |
| 31 | DLLBII104C04 | 1 | - | - | 87 | 83 | 1.0^{E-03} |
| 32 | DLLBII104B05 | 1 | - | - | 86 | 78 | - |
| 33 | DLLBII107C03 | 1 | - | - | 75 | 80 | - |
| 34 | DLLBII58A11 | 4 | - | - | 95 | 73 | 1.6^{E-03} (*1.7^{E-03}*-*1.6^{E-03}*) |
| 35 | DLLBII61F05 | 1 | - | - | 74 | 76 | - |
| 36 | DLLBII61F07 | 1 | - | - | 79 | 77 | - |
| 37 | DLLBII62C11 | 1 | - | - | 74 | 71 | - |
| 38 | DLLBII115A05 | 1 | - | - | 74 | 84 | 3.1^{E-03} |
| 39 | DLLBII83G01 | 4 | - | - | 87 | 93 | 4.1^{E-04} |
| 40 | DLLBII80E08 | 1 | - | - | 71 | 82 | - |

| | | | | | | | |
|---|---|---|---|---|---|---|---|
| ^{(a)} if multiple unique variants within a B-cell lineage are identified, the range (max-min) in off-rate or the off-rate of a lineage member is given between brackets in italics). ^{(b)} heterogeneous fit: fast and slow off-rate determined. | | | | | | | |

**Table 5 Framework and CDR Sequences of anti-DLL4 VHH**

| VHH ID | | | | | | | |
|---|---|---|---|---|---|---|---|
| **SEQ ID NO** | **Framework 1** | **CDR 1** | **Framework 2** | **CDR 2** | **Framework 3** | **CDR 3** | **Framework 4** |
| DLLBII05B06 | | | | | | | |
| **167** | | TYNIG | | | | | WGQGTQVTVSS |
| DLLBII05B08 | | | | | | | |
| **168** | | YYNIG | | | | | WGQGTQVTVSS |
| DLLBII05B09 | | | | | | | |
| **169** | | YYNIG | | | | | WGQGTQVTVSS |
| DLLBII05B11 | | LHVIG | | | | | WGQGTQVTVSS |
| **170** | | | | | | | |
| DLLBII05D11 | | YYNIG | | | | | WGQGTQVTVSS |
| **171** | | | | | | | |
| DLLBII06A02 | | KYAIG | | | | | WGQGTQVTVSS |
| **172** | | | | | | | |
| DLLBII06A05 | | YYNIG | | | | | WGQGTQVTVSS |
| **173** | | | | | | | |
| DLLBII06B11 | | | | | | | WGQGTQVTVSS |
| **174** | | | | | | | |
| DLLBII06E02 | | YYNIG | | | | | WGQGTQVTVSS |
| **175** | | | | | | | |
| DLLBII06E04 | | YYAIG | | | | | WGQGTQVTVSS |
| **176** | | | | | | | |
| DLLBII06E12 | | YHNIG | | | | | WGQGTQVTVSS |
| **177** | | | | | | | |
| DLLBII06G09 | | YYNIG | | | | | WGQGTQVTVSS |
| **178** | | | | | | | |
| DLLBII07A02 | | | | | | | WGQGTQVTVSS |
| **179** | | | | | | | |
| DLLBII07B05 | | | | | | | WGQGTQVTVSS |
| **180** | | | | | | | |
| DLLBII08A09 | | YYNIG | | | | | WGQGTQVTVSS |
| **181** | | | | | | | |
| DLLBII0 8B05 | | | | | | | WGQGTQVTVSS |
| **182** | | | | | | | |
| DLLBII08C11 | | DYAIG | | | | | WGQGTQVTVSS |
| **183** | | | | | | | |
| DLLBII08H06 | | | | | | | WGQGTQVTVSS |
| **184** | | | | | | | |
| DLLBII09C01 | | YHNIG | | | | | WGQGTQVTVSS |
| **185** | | | | | | | |
| DLLBII100G01 | | DYAIG | | | | | WGQGTQVTVSS |
| **186** | | | | | | | |
| DLLBII101B12 | | DYAIG | | | | | TGQGTQVTVSS |
| **187** | | | | | | | |
| DLLBII101E04 | | | | | | | WGQGTQVTVSS |
| **188** | | | | | | | |
| DLLBII101F01 | | | | | | | WGQGTQVTVSS |
| **189** | | | | | | | |
| DLLBII101F03 | | | | | | | WGQGTQVTVSS |
| **190** | | | | | | | |
| DLLBII101F06 | | | | | | | WGQGTQVTVSS |
| **191** | | | | | | | |
| DLLBII101F08 | | | | | | | WGQGTQVTVSS |
| **192** | | | | | | | |
| DLLBII101F10 | | VYAIG | | | | | WGQGTQVTVSS |
| **193** | | | | | | | |
| DLLBII101G02 | | | | | | | WGQGTQVTVSS |
| **194** | | | | | | | |
| DLLBII101G03 | | | | | | | WGQGTQVTVSS |
| **195** | | | | | | | |
| DLLBII101G05 | | | | | | | WGQGTQVTVSS |
| **196** | | | | | | | |
| DLLBII101G08 | | | | | | | WGQGTQVTVSS |
| **197** | | | | | | | |
| DLLBII101H02 | | | | | | | WGQGTQVTVSS |
| **198** | | | | | | | |
| DLLBII101H03 | | DYAIG | | | | | TGQGTQVTVSS |
| **199** | | | | | | | |
| DLLBII101H05 | | | | | | | RGQGTQVTVSS |
| **200** | | | | | | | |
| DLLBII101H09 | | YYTIV | | | | GPDCSSYDY | WGQGTQVTVSS |
| **201** | | | | | | | |
| DLLBII102F08 | | | | | | | WGQGTQVTVSS |
| **202** | | | | | | | |
| DLLBII102G04 | | | | | | | RGQGTQVTVSS |
| **203** | | | | | | | |
| DLLBII1 02H07 | | | | | | | RGQGTQVTVSS |
| **204** | | | | | | | |
| DLLBII102H09 | | | | | | | WGQGTQVTVSS |
| **205** | | | | | | | |
| DLLBII103A04 | | DYAIG | | | | | SGQGTQVTVSS |
| **206** | | | | | | | |
| DLLBII103B05 | | DYAIG | | | | | TGQGTQVTVSS |
| **207** | | | | | | | |
| DLLBII104A03 | | IAAMG | | | | | WGQGTQVTVSS |
| **208** | | | | | | | |
| DLLBII104A05 | | | | | | | WGQGTQVTVSS |
| **209** | | | | | | | |
| DLLBII104B02 | | | | | | | WGQGTQVTVSS |
| **210** | | | | | | | |
| DLLBII104B05 | | IDVMA | | | | | WGQGTQVTVSS |
| **211** | | | | | | | |
| DLLBII104B08 | | | | | | | WGQGTQVTVSS |
| **212** | | | | | | | |
| DLLBII104C04 | | | | | | RRNFLSNY | WGQGTQVTVSS |
| **213** | | | | | | | |
| DLLBII104C12 | | DYAIG | | | | | TGQGTQVTVSS |
| **214** | | | | | | | |
| DLLBII104G01 | | DYAIG | | | | | WGQGTQVTVSS |
| **215** | | | | | | | |
| DLLBII105G01 | | DYAIG | | | | | SGQGTQVTVSS |
| **216** | | | | | | | |
| DLLBII106A01 | | DYAIG | | | | | WGQGTQVTVSS |
| **217** | | | | | | | |
| DLLBII106F01 | | | | | | | WGQGTQVTVSS |
| **218** | | | | | | | |
| DLLBII106H01 | | | | | | | WGQGTQVTVSS |
| **219** | | | | | | | |
| DLLBII107C03 | | | | | | | WGQGTQVTVSS |
| **220** | | | | | | | |
| DLLBII112A03 | | | | | | DIY | WGRGTQVTVSS |
| **221** | | | | | | | |
| DLLBII112A04 | | GMG | | | | | WGQGTQVTVSS |
| **222** | | | | | | | |
| DLLBII112E07 | | | | | | | WGQGTQVTVSS |
| **223** | | | | | | | |
| DLLBII115A05 | | | | | | | WGQGTQVTVSS |
| **224** | | | | | | | |
| DLLBII16A03 | | YYAIG | | | | | WGQGTQVTVSS |
| **225** | | | | | | | |
| DLLBII16A07 | | DYAIG | | | | | WGQGTQVTVSS |
| **226** | | | | | | | |
| DLLBII16A09 | | YHNIG | | | | | WGQGTQVTVSS |
| **227** | | | | | | | |
| DLLBII16C11 | | YYNIG | | | | | WGQGTQVTVSS |
| **228** | | | | | | | |
| DLLBII16D11 | | YYNIG | | | | | WGQGTQVTVSS |
| **229** | | | | | | | |
| DLLBII16E02 | | DYAIG | | | | | SGQGTQVTVSS |
| **230** | | | | | | | |
| DLLBII16E08 | | YYNIG | | | | | WGQGTQVTVSS |
| **231** | | | | | | | |
| DLLBII16H02 | | YYNIG | | | | | WGQGTQVTVSS |
| **232** | | | | | | | |
| DLLBII16H09 | | | | | | | WGQGTQVTVSS |
| **233** | | | | | | | |
| DLLBII17A12 | | DYAIG | | | | | SGQGTQVTVSS |
| **234** | | | | | | | |
| DLLBII17B03 | | | | | | | WGQGTQVTVSS |
| **235** | | | | | | | |
| DLLBII1 7B09 | | DYAIG | | | | | WGQGTQVTVSS |
| **236** | | | | | | | |
| DLLBII17C01 | | DYPIG | | | | | SGQGTQVTVSS |
| **237** | | | | | | | |
| DLLBII17C08 | | | | | | | WGQGTQVTVSS |
| **238** | | | | | | | |
| DLLBII17E04 | | YYNIG | | | | | WGQGTQVTVSS |
| **239** | | | | | | | |
| DLLBII17F10 | | | | | | | WGQGTQVTVSS |
| **240** | | | | | | | |
| DLLBII17G10 | | | | | | | WGQGTQVTVSS |
| **241** | | | | | | | |
| DLLBII18D02 | | YYAIG | | | | | WGQGTQVTVSS |
| **242** | | | | | | | |
| DLLBII18F05 | | | | | | | WGQGTQVTVSS |
| **243** | | | | | | | |
| DLLBII18H08 | | YYNIG | | | | | WGQGTQVTVSS |
| **244** | | | | | | | |
| DLLBII19B09 | | YYNIG | | | | | WGQGTQVTVSS |
| **245** | | | | | | | |
| DLLBII19D04 | | | | | | | WGQGTQVTVSS |
| **246** | | | | | | | |
| DLLBII19D07 | | NYNIG | | | | | WGQGTQVTVSS |
| **247** | | | | | | | |
| DLLBII19D10 | | | | | | EMDGSRYV | EGQGTQVTVSS |
| **248** | | | | | | | |
| DLLBII19F04 | | | | | | VHPSTGFGS | WGQGTQVTVSS |
| **249** | | | | | | | |
| DLLBII19F12 | | | | | | | WGQGTQVTVSS |
| **250** | | | | | | | |
| DLLBII24C07 | | DYAIG | | | | | SGQGTQVTVSS |
| **251** | | | | | | | |
| DLLBII24D07 | | DYAIG | | | | | SGQGTQVTVSS |
| **252** | | | | | | | |
| DLLBII25G05 | | DYAIG | | | | | WGQGTQVTVSS |
| **253** | | | | | | | |
| DLLBII25H07 | | YYAIG | | | | | WGQGTQVTVSS |
| **254** | | | | | | | |
| DLLBII26C02 | | YYAIG | | | | | WGQGTQVTVSS |
| **255** | | | | | | | |
| DLLBII26H11 | | YYAIG | | | | | WGQGTQVTVSS |
| **256** | | | | | | | |
| DLLBII28B06 | | | | | | | WGQGTQVTVSS |
| **257** | | | | | | | |
| DLLBII33A06 | | NYAIG | | | | | WGQGTQVTVSS |
| **258** | | | | | | | |
| DLLBII33A10 | | | | | | | WGQGTQVTVSS |
| **259** | | | | | | | |
| DLLBII33C05 | | NYVIG | | | | | WGQGTQVTVSS |
| **260** | | | | | | | |
| DLLBII33C09 | | YYVIG | | | | | WGQGTQVTVSS |
| **261** | | | | | | | |
| DLLBII33C10 | | | | | | | WGQGTQVTVSS |
| **262** | | | | | | | |
| DLLBII33D02 | | DYAIG | | | | | WGQGTQVTVSS |
| **263** | | | | | | | |
| DLLBII33E01 | | | | | | | WGQGTQVTVSS |
| **264** | | | | | | | |
| DLLBII33E03 | | YYNIG | | | | | WGQGTQVTVSS |
| **265** | | | | | | | |
| DLLBII33F01 | | YYAIG | | | | | WGQGTQVTVSS |
| **266** | | | | | | | |
| DLLBII33F04 | | | | | | | WGQGTQVTVSS |
| **267** | | | | | | | |
| DLLBII33H04 | | YYNIG | | | | | WGQGTQVTVSS |
| **268** | | | | | | | |
| DLLBII42A08 | | | | | | | WGQGTQVTVSS |
| **269** | | | | | | | |
| DLLBII42B07 | | YYAIG | | | | | WGQGTQVTVSS |
| **270** | | | | | | | |
| DLLBII42B10 | | YYAIG | | | | | WGQGTQVTVSS |
| **271** | | | | | | | |
| DLLBII42F08 | | | | | | | WGQGTQVTVSS |
| **272** | | | | | | | |
| DLLBII42G04 | | | | | | | WGQGTQVTVSS |
| **273** | | | | | | | |
| DLLBII43A05 | | | | | | | WGQGTQVTVSS |
| **274** | | | | | | | |
| DLLBII43A10 | | YYNIG | | | | | WGQGTQVTVSS |
| **275** | | | | | | | |
| DLLBII43A12 | | | | | | | WGQGTQVTVSS |
| **276** | | | | | | | |
| DLLBII43B04 | | | | | | | WGQGTQVTVSS |
| **277** | | | | | | | |
| DLLBII43B11 | | | | | | | WGQGTQVTVSS |
| **278** | | | | | | | |
| DLLBII43C12 | | YYNIG | | | | | WGQGTQVTVSS |
| **279** | | | | | | | |
| DLLBII47A05 | | YYAIG | | | | | WGQGTQVTVSS |
| **280** | | | | | | | |
| DLLBII47D01 | | DYAIG | | | | | WGQGTQVTVSS |
| **281** | | | | | | | |
| DLLBII47E03 | | | | | | | WGQGTQVTVSS |
| **282** | | | | | | | |
| DLLBII47E12 | | | | | | | WGQGTQVTVSS |
| **283** | | | | | | | |
| DLLBII47F06 | | YYAIG | | | | | WGQGTQVTVSS |
| **284** | | | | | | | |
| DLLBII47G11 | | HYNIG | | | | | WGQGTQVTVSS |
| **285** | | | | | | | |
| DLLBII47H02 | | 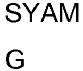 | | | | | WGQGTQVTVSS |
| **286** | | | | | | | |
| DLLBII48A01 | | YYNIG | | | | | WGQGTQVTVSS |
| **287** | | | | | | | |
| DLLBII48A08 | | | | | | | WGQGTQVTVSS |
| **288** | | | | | | | |
| DLLBII48E03 | | YYAIG | | | | | WGQGTQVTVSS |
| **289** | | | | | | | |
| DLLBII48F05 | | | | | | | WGQGTQVTVSS |
| **290** | | | | | | | |
| DLLBII49A12 | | YHNIG | | | | | WGQGTQVTVSS |
| **291** | | | | | | | |
| DLLBII49B05 | | YYAIG | | | | | WGQGTQVTVSS |
| **292** | | | | | | | |
| DLLBII49E01 | | YYNIG | | | | | WGQGTQVTVSS |
| **293** | | | | | | | |
| DLLBII49F05 | | YYNIG | | | | | WGQGTQVTVSS |
| **294** | | | | | | | |
| DLLBII49G02 | | KYSIG | | | | | WGQGTQVTVSS |
| **295** | | | | | | | |
| DLLBII49G05 | | YYAIG | | | | | WGQGTQVTVSS |
| **296** | | | | | | | |
| DLLBII49H05 | | YYNIG | | | | | WGQGTQVTVSS |
| **297** | | | | | | | |
| DLLBII55A07 | | DYAIG | | | | | WGQGTQVTVSS |
| **298** | | | | | | | |
| DLLBII55D12 | | DYAIG | | | | | TGQGTQVTVSS |
| **299** | | | | | | | |
| DLLBII56A09 | | DYAIG | | | | | TGQGTQVTVSS |
| **300** | | | | | | | |
| DLLBII56C04 | | VYAIG | | | | | WGQGTQVTVSS |
| **301** | | | | | | | |
| DLLBII56H08 | | DYAIG | | | | | TGQGTQVTVSS |
| **302** | | | | | | | |
| DLLBII58A11 | | | | | | | WGQGTQVTVSS |
| **303** | | | | | | | |
| DLLBII58B01 | | | | | | | WGQGTQVTVSS |
| **304** | | | | | | | |
| DLLBII59B01 | | | | | | | WGQGTQVTVSS |
| **305** | | | | | | | |
| DLLBII59B11 | | | | | | | WGQGTQVTVSS |
| **306** | | | | | | | |
| DLLBII61F05 | | | | | | RTSRSPRP | RGQGTQVTVSS |
| **307** | | | | | | | |
| DLLBII61F07 | | | | | | | WGQGTQVTVSS |
| **308** | | | | | | | |
| DLLBII62C11 | | | | | | GSGSWGV | HGQGTQVTVSS |
| **309** | | | | | | | |
| DLLBII78B03 | | | | | | | WGQGTQVTVSS |
| **310** | | | | | | | |
| DLLBII78B04 | | | | | | | WGQGTQVTVSS |
| **311** | | | | | | | |
| DLLBII80E08 | | | | | | | WGQGTQVTVSS |
| **312** | | | | | | | |
| DLLBII83G01 | | | | | | | WGQGTQVTVSS |
| **313** | | | | | | | |
| DLLBII83G04 | | IMG | | | | | WGQGTQVTVSS |
| **314** | | | | | | | |
| DLLBII87B06 | | DYAIG | | | | | TGQGTQVTVSS |
| **315** | | | | | | | |
| DLLBII89B04 | | DYAIG | | | | | TGQGTQVTVSS |
| **316** | | | | | | | |
| DLLBII90E10 | | DYAIG | | | | | TGQGTQVTVSS |
| **317** | | | | | | | |
| DLLBII95A01 | | | | | | | WGQGTQVTVSS |
| **318** | | | | | | | |
| DLLBII95B03 | | | | | | | WGQGTQVTVSS |
| **319** | | | | | | | |
| DLLBII95C03 | | | | | | | WGQGTQVTVSS |
| **320** | | | | | | | |
| DLLBII95D02 | | | | | | | WGQGTQVTVSS |
| **321** | | | | | | | |
| DLLBII95F02 | | | | | | | WGQGTQVTVSS |
| **322** | | | | | | | |
| DLLBII95F03 | | VYAIG | | | | | WGQGTQVTVSS |
| **323** | | | | | | | |
| DLLBII95H02 | | | | | | | WGQGTQVTVSS |
| **324** | | | | | | | |
| DLLBII96C02 | | | | | | | WGQGTQVTVSS |
| **325** | | | | | | | |
| DLLBII96C03 | | | | | | | WGQGTQVTVSS |
| **326** | | | | | | | |
| DLLBII96F02 | | | | | | | WGQGTQVTVSS |
| **327** | | | | | | | |
| DLLBII96H02 | | | | | | | WGQGTQVTVSS |
| **328** | | | | | | | |
| DLLBII97B02 | | | | | | | WGQGTQVTVSS |
| **329** | | | | | | | |
| DLLBII97D01 | | | | | | | WGQGTQVTVSS |
| **330** | | | | | | | |
| DLLBII97E01 | | | | | | | WGQGTQVTVSS |
| **331** | | | | | | | |
| DLLBII97E02 | | | | | | | WGQGTQVTVSS |
| **332** | | | | | | | |

### Example 5

### Characterization of purified anti-DII4 VHHs

Inhibitory anti-DII4 VHHs selected from the screening described in Example 4 are further purified and characterized. Selected VHHs are expressed in *E. coli* TG1 as c-myc, His6-tagged proteins. Expression is induced by addition of 1 mM IPTG and allowed to continue for 4 hours at 37°C. After spinning the cell cultures, periplasmic extracts are prepared by freeze-thawing the pellets. These extracts are used as starting material and VHHs are purified via IMAC and size exclusion chromatography (SEC) resulting in 95% purity as assessed via SDS-PAGE.

### 5.1. Evaluation of DII4 blocking VHHs in ELISA

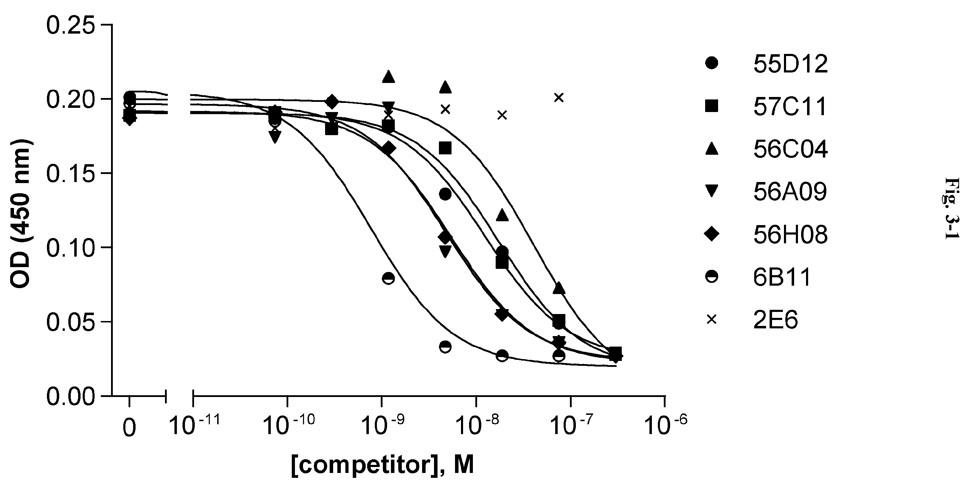

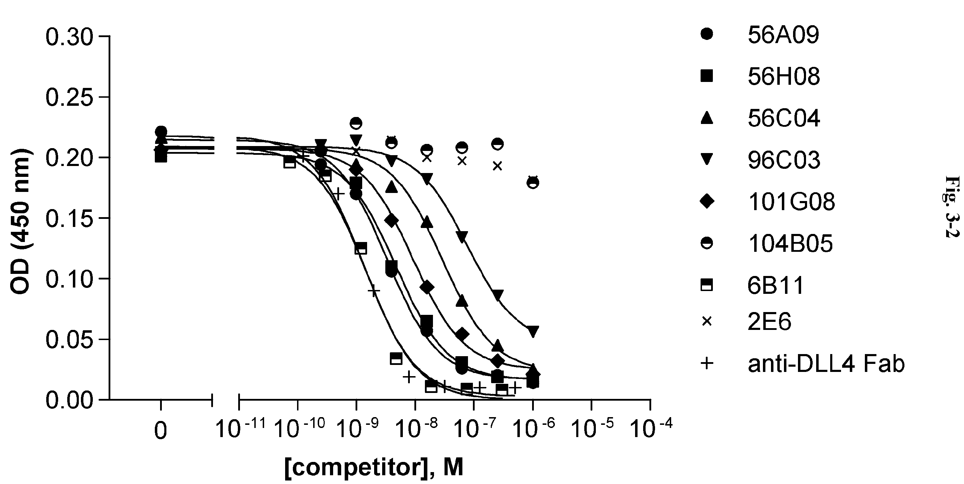

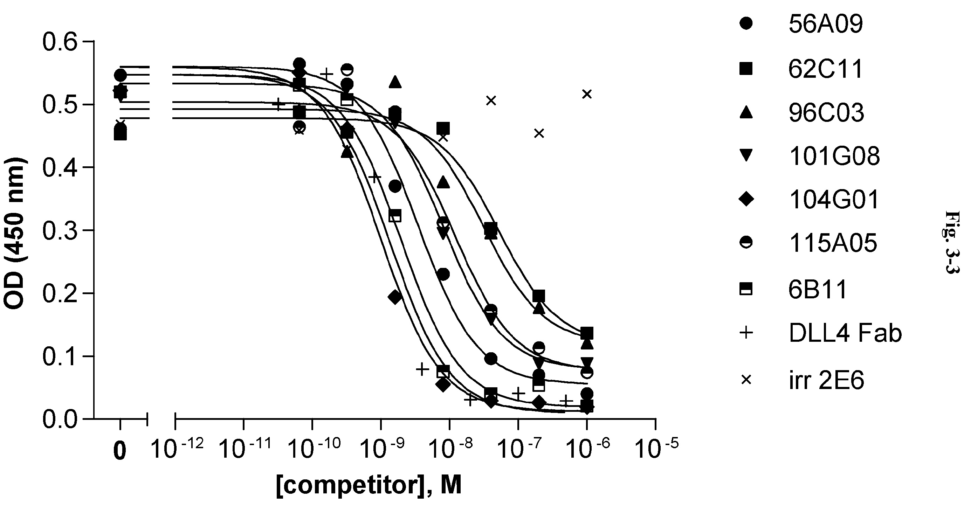

The blocking capacity of the VHHs is evaluated in a human DII4 - human Notch1/Fc blocking ELISA. In brief, 1 µg/mL of human Notch1/Fc chimera (R&D Systems, Minneapolis, MN, USA) is coated in a 96-well MaxiSorp plate (Nunc, Wiesbaden, Germany). A fixed concentration of 15 nM biotinylated human DII4 is preincubated with a dilution series of the VHH for 1 hour, after which the mixture is incubated on the coated Notch1 receptor for an additional hour. Residual binding of biotinylated human DII4 is detected using horseradish peroxidase (HRP) conjugated extravidin (Sigma, St. Louis, MO, USA) (Figure 3). Human DII4 is biotinylated as described above. The IC₅₀ values for VHHs blocking the human DII4 - human Notch1/Fc interaction are depicted in Table 6.

**Table 6: IC₅₀ (nM) values for VHHs in hDLL4/hNotch1-Fc competition ELISA**

| **VHH ID** | **IC₅₀ (nM)** |
|---|---|
| 6B11 | 1.5 |
| 55D12 | 12.3 |
| 56A09 | 4.9 |
| 56C04 | 33.9 |
| 56H08 | 6.9 |
| 57C11 | 17.3 |
| 62C11 | 72.0 |
| 96C03 | 38.4 |
| 101G08 | 9.5 |
| 104G01 | 1.1 |
| 115A05 | 9.1 |
| antiDLL4 Fab | 0.7 |

### 5.2. Evaluation of DII4 blocking VHHs in AlphaScreen

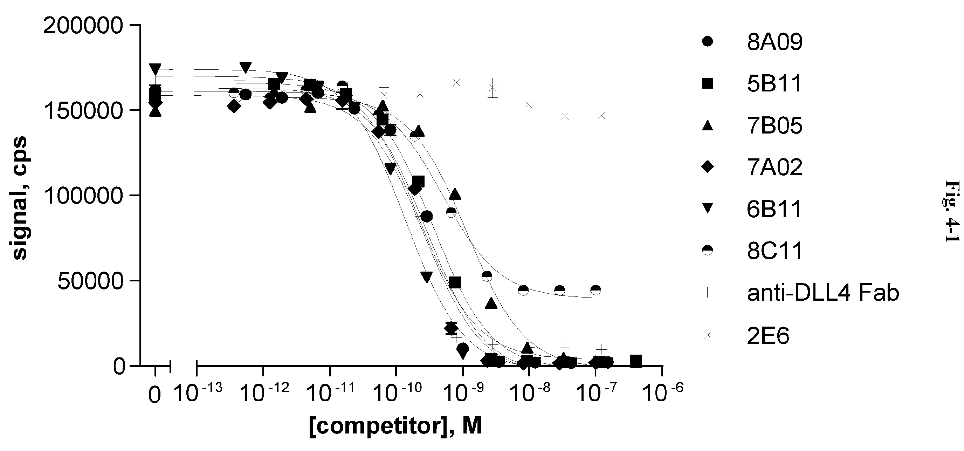

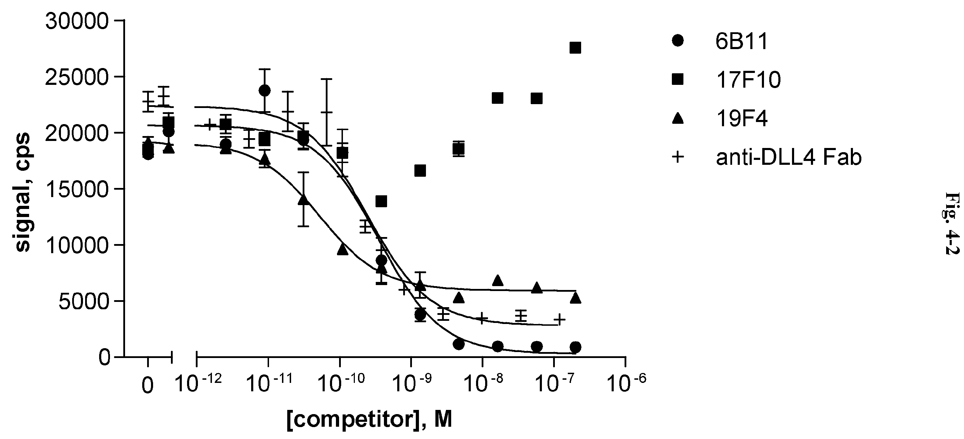

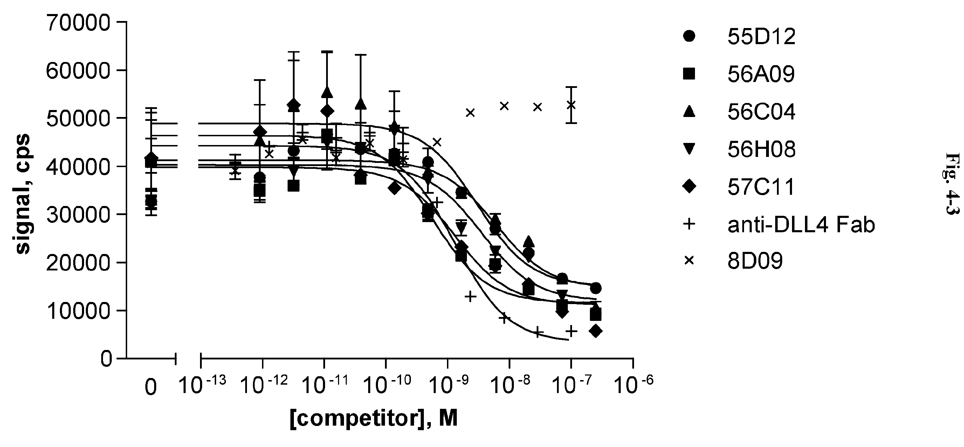

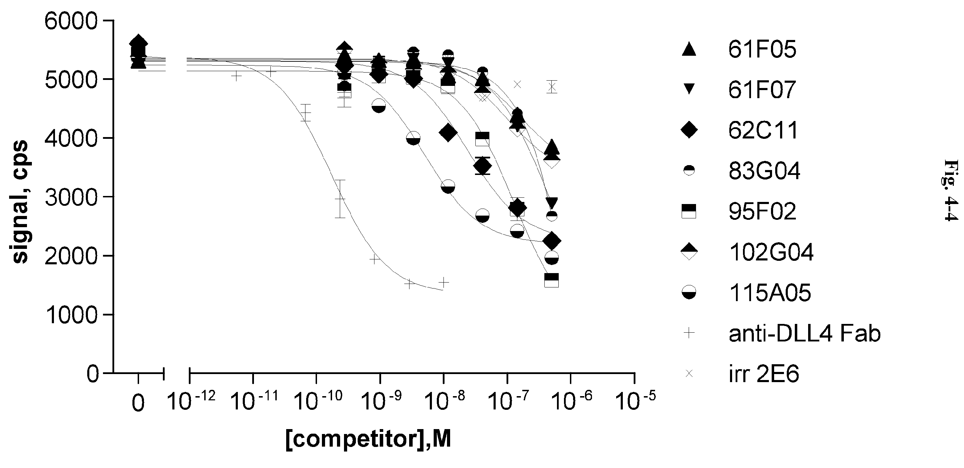

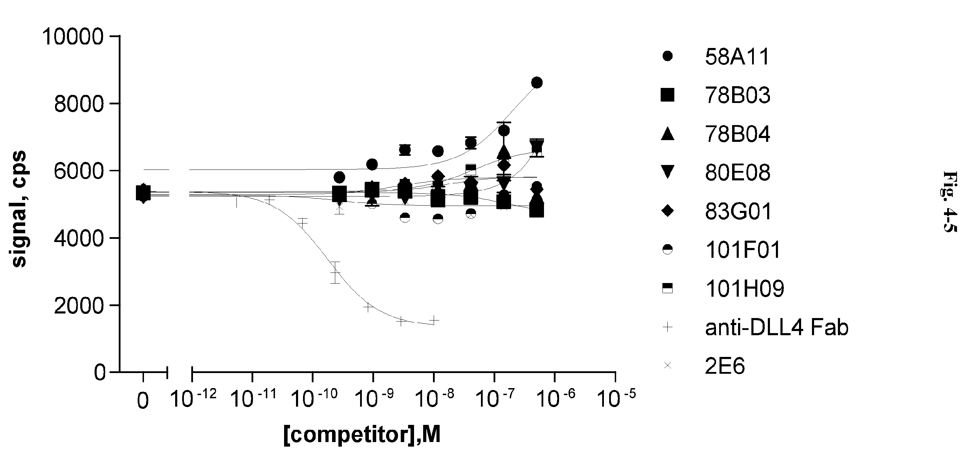

In brief, 1 nM biotinylated human DII4 is captured on streptavidin-coated donor beads (20 µg/mL), while 0.4 nM of the receptor human Notch1 (as a Fc fusion protein) is captured on anti-human Fc VHH-coated acceptor beads (20 µg/mL). Both loaded beads are incubated together with a dilution range of the competing VHH (Figure 4). The IC₅₀ values for VHHs blocking the human DII4 - human Notch1/Fc interaction are depicted in Table 7.

**Table 7: IC₅₀ (nM) values for VHHs in hDLL4/hNotch1 competition AlphaScreen**

| **VHH ID** | **IC₅₀ (nM)** |
|---|---|
| 5B11 | 0.7 |
| 6B11 | 0.3 |
| 7A02 | 0.4 |
| 7B05 | 1.1 |
| 8A09 | 0.4 |
| 8C11 | 0.7 ^{(a)} |
| 19F04 | 0.05 ^{(a)} |
| 55D12 | 2.3 |
| 56A09 | 1.2 |
| 56C04 | 5.4 |
| 56H08 | 1.6 |
| 57C11 | 2.2 |
| 62C11 | 24.1 |
| 115A05 | 5.0 |
| antiDLL4 Fab | 0.3 |

| | |
|---|---|
| ^{(a)} partial inhibitor | |

### 5.3. Inhibition by anti-DII4 VHHs of human Notch1/Fc binding to human or mouse DII4 expressed on the CHO cells

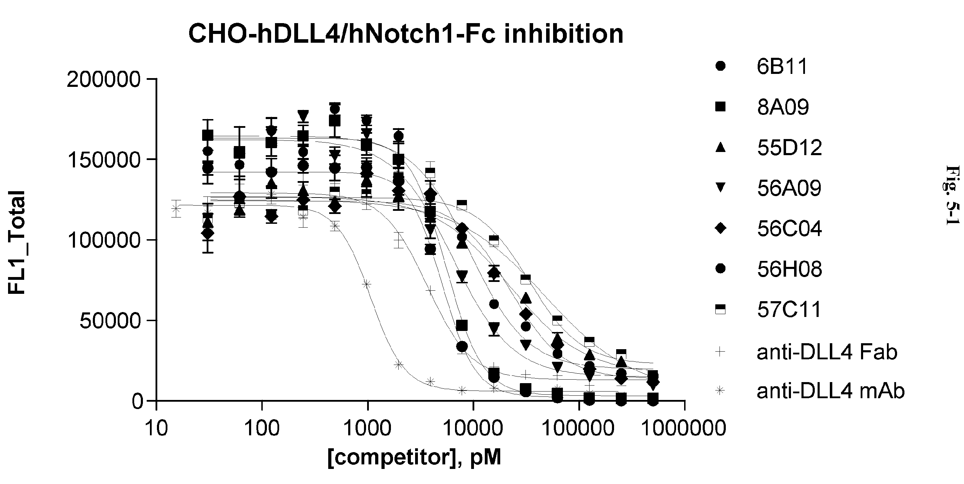

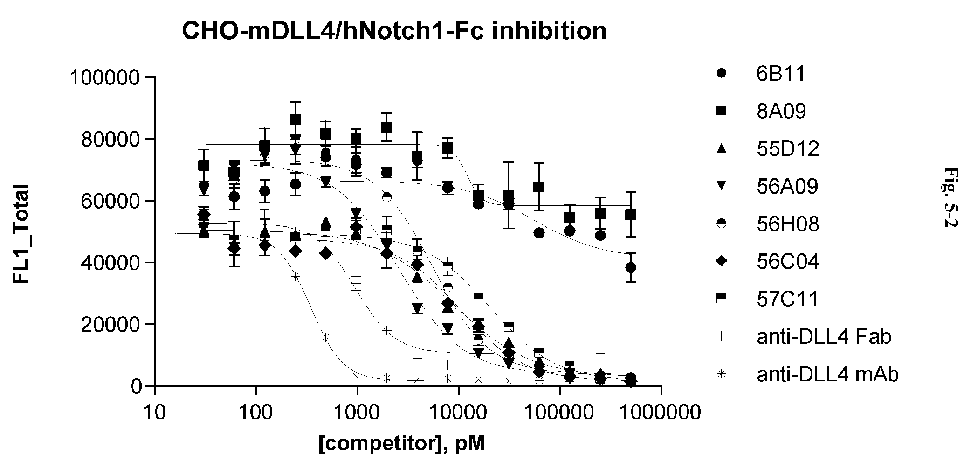

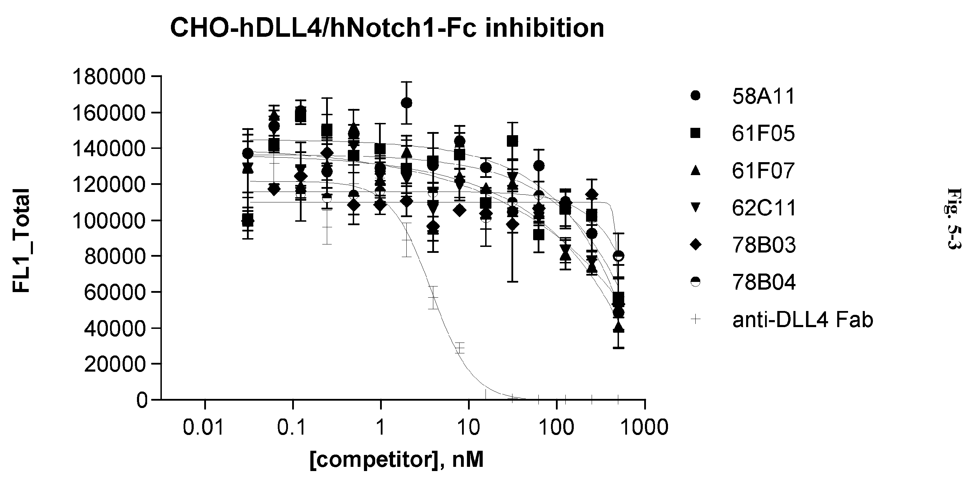

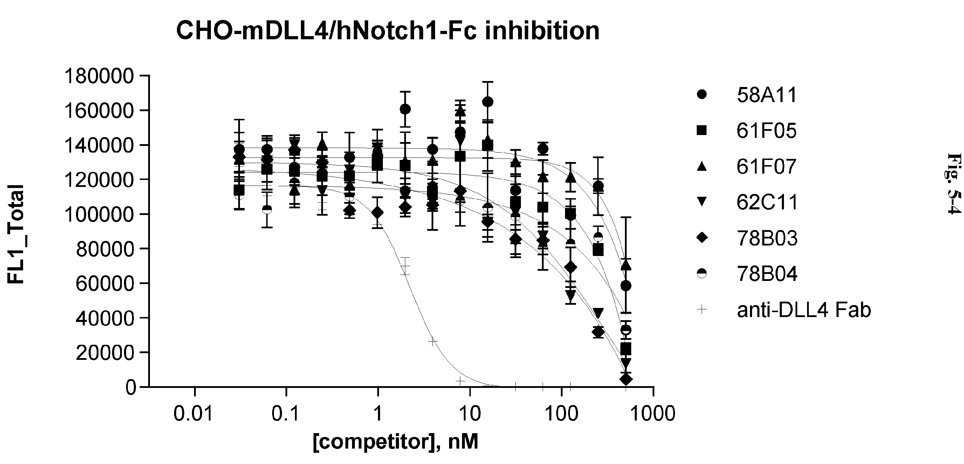

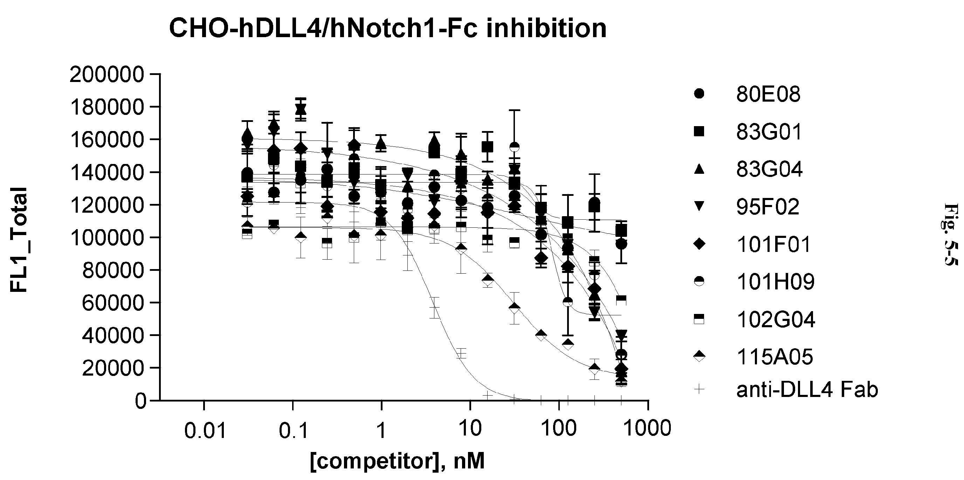

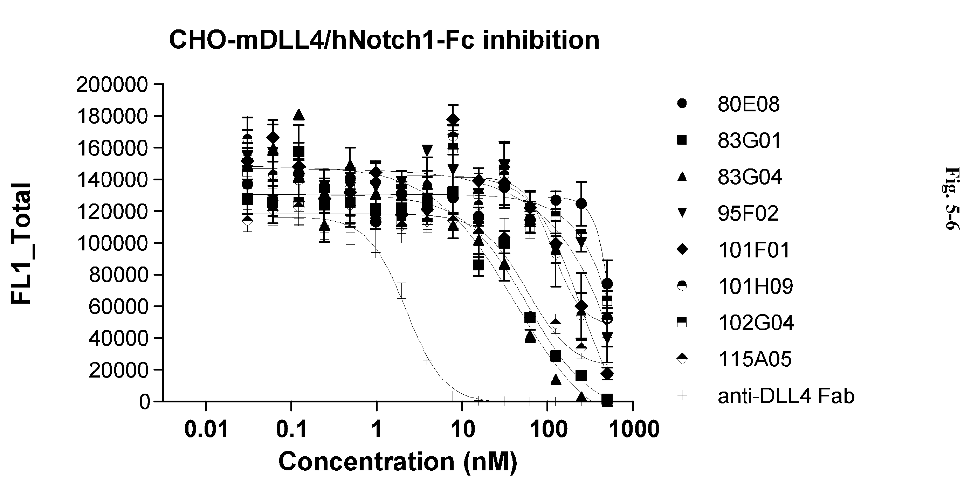

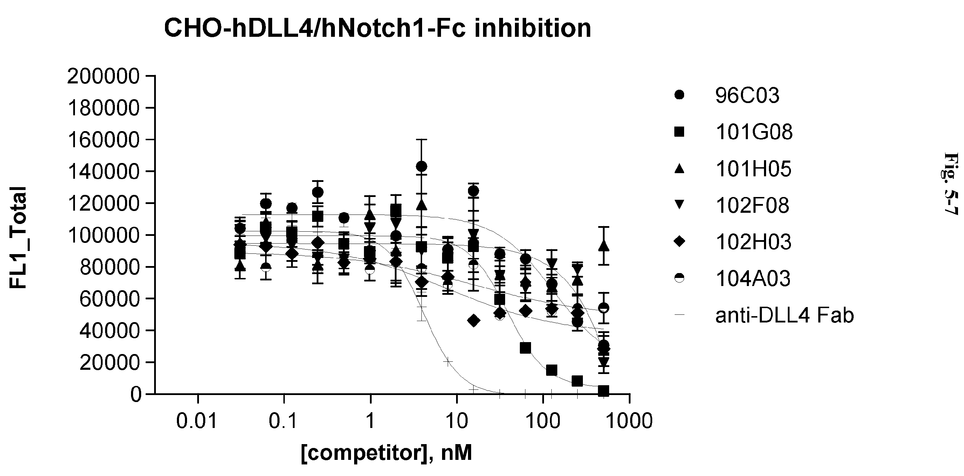

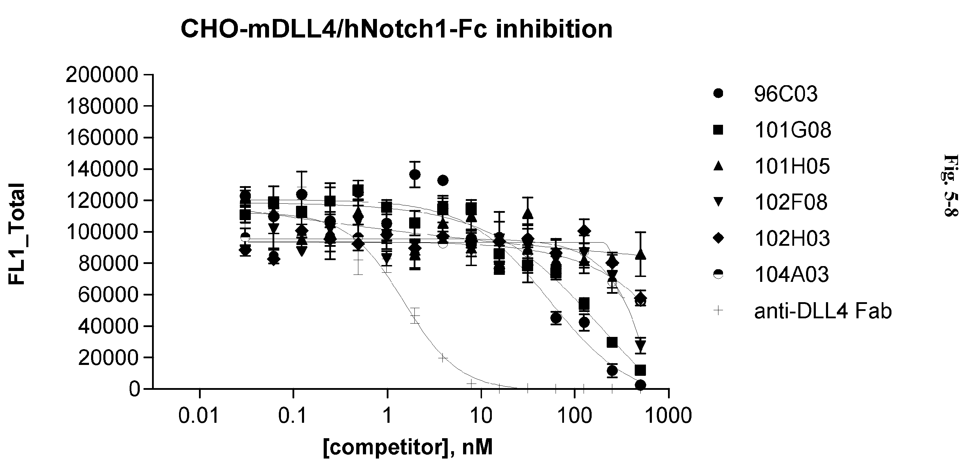

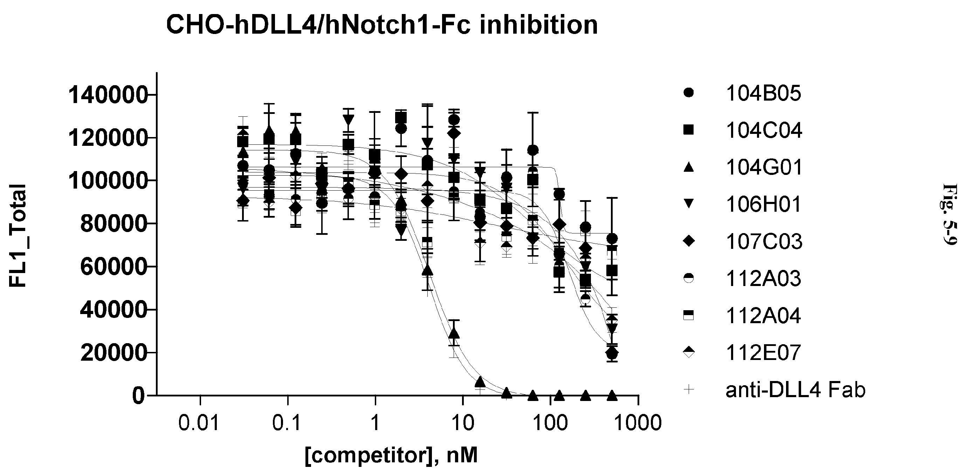

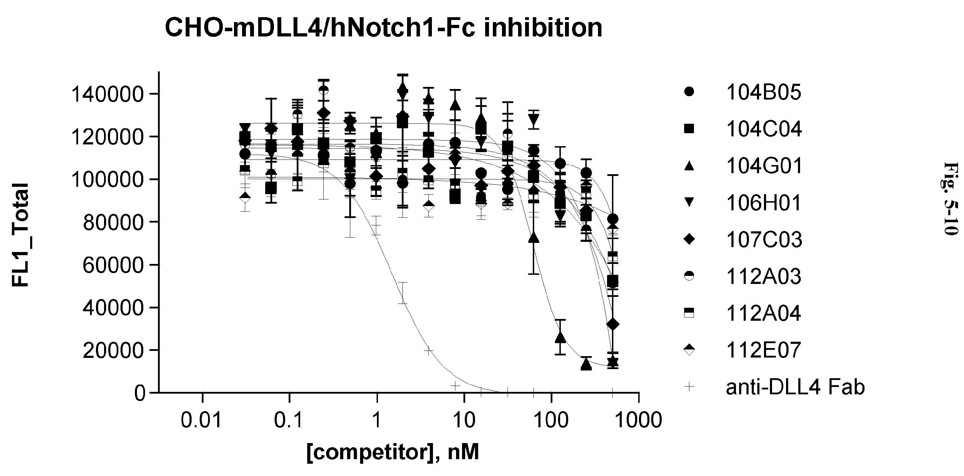

The blocking capacity of the VHHs is evaluated in a human and mouse DII4 - human Notch1/Fc competitive FMAT assay (Figure 5) as outlined in Example 4. The IC₅₀ values for VHHs blocking the interaction of human Notch1/Fc to human or mouse DII4 expressed on CHO cells are depicted in Table 8.

**Table 8: (Mean) IC₅₀ values (nM) of purified VHHs blocking the interaction of human Notch1/Fc to human or mouse DLL4 expressed on CHO cells (FMAT)**

| | **hDLL4** | **mDLL4** |
|---|---|---|
| **VHH ID** | **IC₅₀ (nM)** | **IC₅₀ (nM)** |
| 6B11 | 8.9 | - |
| 8A09 | 5.5 | - |
| 19F04 | 33.0 | - |
| 55D12 | 39.1 | 41.0 |
| 56A09 | 10.6 | 15.0 |
| 56C04 | 28.7 | 49.6 |
| 56H08 | 22.0 | 33.7 |
| 57C11 | 53.9 | 49.5 |
| 62C11 | 172.2 | 106.3 |
| 96C03 | 160.8 | 28.8 |
| 101G08 | 24.6 | 92.1 |
| 104G01 | 2.5 | - |
| 115A05 | 22.0 | 43.0 |
| antiDLL4 Fab | 5.4 | 2.3 |

### 5.4. Evaluation of DII4 blocking VHHs in reporter assay

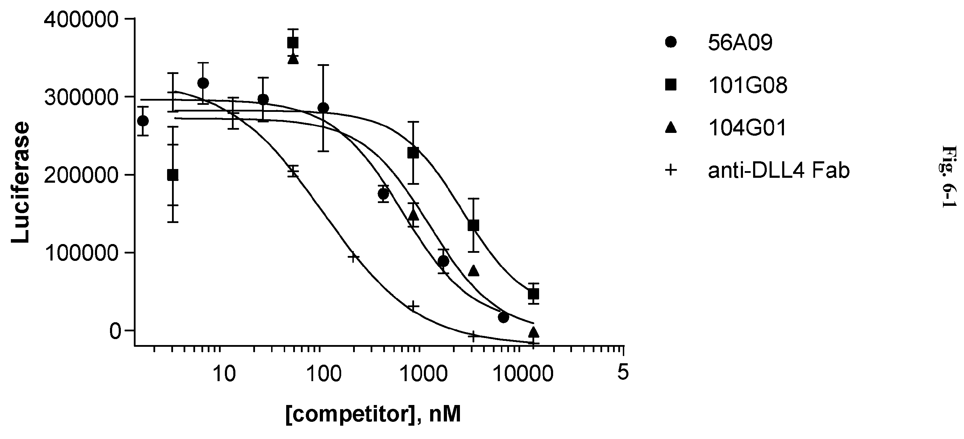

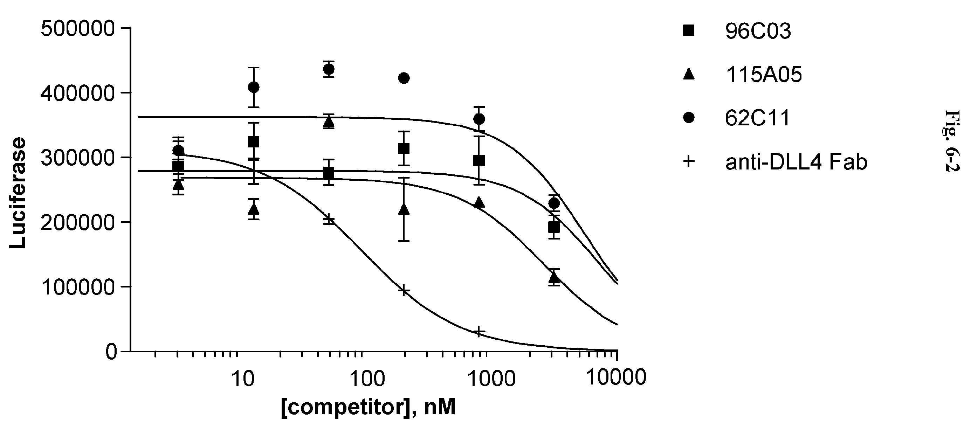

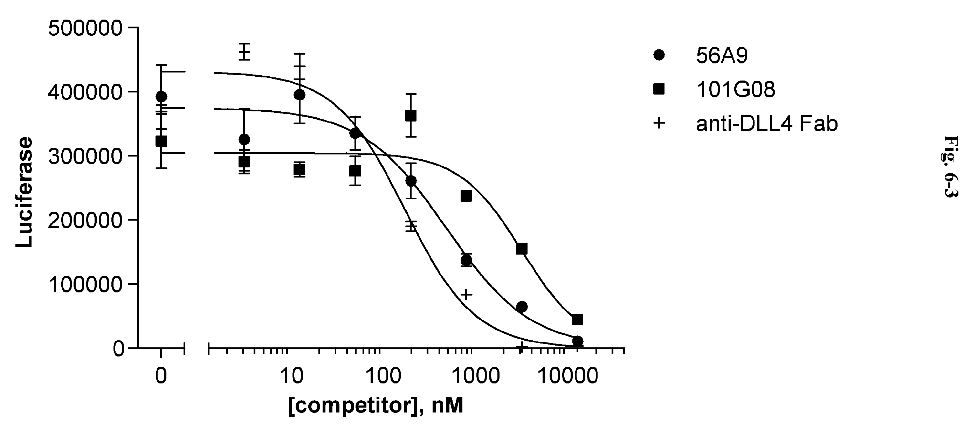

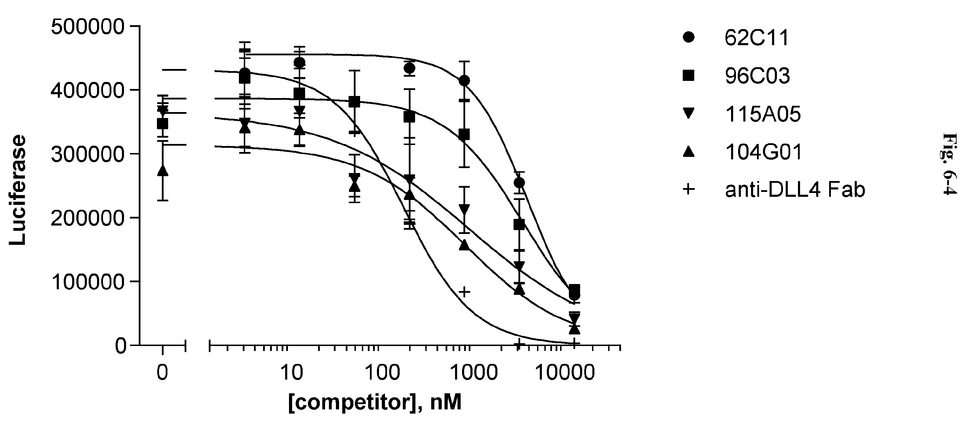

To evaluate the potency of the selected VHHs, a reporter assay is set up which is based on the γ-secretase mediated cleavage of Notch1 and release of the intracellular domain of Notch1 (NICD) upon stimulation with DII4. The Notch1-GAL4/VP16 construct is cotransfected with the pGL4.31 [Luc2P/Gal4UAS/Hygro] reporter plasmid in HEK cells resulting in a transient expression of the fusion protein. These transiently transfected cells are stimulated for 24 hours by co-culture with a HEK293-hDII4 stable cell line. Forty-eight hours post-transfection, the readout is performed. The VHHs are preincubated with the HEK293-hDII4 cells 1 hour before the start of the co-culture and are included during the co-culture (Figure 6). The IC₅₀ values of the VHHs for blocking the DII4-mediated cleavage of Notch1 and subsequent translocation of its NICD to the nucleus of the receptor cell are depicted in Table 9.

**Table 9: (Mean) IC₅₀ values (nM) of purified anti-DII4 VHHs in a DLL4/Notch1 reporter assay**

| **VHH ID** | **IC₅₀** |
|---|---|
| 56A09 | 540 |
| 62C11 | 4663 |
| 96C03 | 5156 |
| 101G08 | 2760 |
| 104G01 | 964 |
| 115A05 | 1740 |
| anti-DLL4 Fab | 133 |

### 5.5. Epitope binning

In order to determine whether VHHs can bind simultaneously to DII4 when e.g. a benchmark antibody is bound, epitope binning experiments are carried out (via Surface Plasmon Resonance (SPR) on a Biacore T100 instrument). Anti-DII4 Fab fragment is irreversibly immobilized on the reference and on the active flow cell of a CM5 sensor chip. For each sample (cycle), human DII4 is injected on the active and reference flow cell and reversibly captured by anti-DII4 Fab. Additional binding of VHHs is evaluated by injection over the immobilized surface. All VHHs and anti-DII4 Fab are injected at 100 nM with a surface contact time of 120 seconds and a flow rate of 10 uL/minute. Surface is regenerated using 10 mM glycine (pH1.5). Processed curves are evaluated with Biacore T100 Evaluation software. Table 10-A represents the sequential injection/regeneration path of analysed VHHs and controls. VHHs DLLBII56A09 (SEQ ID NO: 300), DLLBII96C03 (SEQ ID NO: 326), DLLBII101G08 (SEQ ID NO: 197) and DLLBII115A05 (SEQ ID NO: 224) are shown not to additionally bind to human DII4 captured by DII4 Fab. Injection of DII4 Fab also failed to additionally bind human DII4 indicating that all epitopes are saturated. Therefore, it can be concluded that these VHHs recognize an epitope overlapping with DII4 Fab for binding human DII4. Human-only VHHs DLLBII6B11 (SEQ ID NO: 174) and DLLBII104G01 (SEQ ID NO: 215) show additional binding on DII4 Fab captured human DII4, indicating that these VHHs that are specific for human DII4 recognize a different epitope than the human/mouse cross-reactive VHHs.

**Table 10-A: Epitope binning of anti-DLL4 VHHs - simultaneous binding with DLL4 Fab**

| **Injection step** | **Binding/** *Regeneration* | **[sample ]** | **Binding level (RU)** |
|---|---|---|---|
| 1 | hDLL4 | 100 nM | 1727 |
| 2 | DLL4 Fab | 100 nM | no binding |
| 3 | 59A9 | 100 nM | no binding |
| 4 | 6B11 | 100 nM | 405 |
| 5 | *Glycine pH1.5* | 10 mM | 90 |
| 6 | hDLL4 | 100 nM | 1349 |
| 7 | 104G1 | 100 nM | 276 |
| 8 | *Glycine pH1.5* | 10 mM | 87 |
| 9 | hDLL4 | 100 nM | 1336 |
| 10 | *Glycine pH1.5* | 10 mM | 70 |
| 11 | hDLL4 | 100 nM | 1333 |
| 12 | 96C3 | 100 nM | no binding |
| 13 | 101G8 | 100 nM | no binding |
| 14 | 115A05 | 100 nM | no binding |
| 15 | *Glycine pH1.5* | 10 mM | 70 |

### 5.6. Epitope mapping using DII4 deletion mutants

Binding of the VHHs to these DII4 mutants is assessed in Biacore. In brief, VHHs DLLBII101G08 (SEQ ID NO:197) and DLLBII115A5 (SEQ ID NO: 224) are coated on a CM4 Sensorchip and 200 nM of each deletion mutant is injected across the chip. Binding is qualitatively assessed. No binding of DLLBII56A09 (SEQ ID NO: 300), DLLBII101 G08 (SEQ ID NO: 197) and DLLBII115A05 (SEQ ID NO: 224) is observed to human and mouse DII4 mutants hDII4.1 and mDII4.8, respectively, lacking EGF-like 2 domain (Table 10-B). Indirect evidence using a hDII4/DII4 IgG competitive ELISA already pointed to this observation. In brief, 1 µg/mL of DII4 IgG is coated in a 96-well MaxiSorp plate (Nunc, Wiesbaden, Germany). A fixed concentration of 6 nM biotinylated human DII4 is preincubated with a dilution series of the VHH for 1 hour, after which the mixture is incubated on the coated IgG for an additional hour. Residual binding of biotinylated human DII4 is detected using horseradish peroxidase conjugated extravidin (Sigma, St. Louis, MO, USA) (data not shown). Human DII4 is biotinylated as described above. It is known from patent literature that the monoclonal anti-DII4 IgG (Genentech,
US 2008/0014196A1) binds to an epitope within the EGF-like 2 domain of DII4.

**Table 10-B: Epitope mapping of anti-DLL4 VHHs - binding to DLL4 deletion mutants**

| | **DLLBII56A9** | | **DLLBII101G8** | | **DLLBII115A5** | |
|---|---|---|---|---|---|---|
| **sample** | **Binding (RU)** | **kd (1/s)** | **Binding (RU)** | **kd (1/s)** | **Binding (RU)** | **kd (1/s)** |
| hDLL4 | 281 | 9.5E-04 | 373 | 2.0E-03 | 324 | 3.5E-03 |
| mDLL4 | 389 | 1.9E-03 | 502 | 6.0E-03 | 344 | 6.5E-03 |
| hDLL4.1 | no binding | | no binding | | no binding | |
| hDLL4.3 | 125 | 7.4E-04 | 198 | 4.65E-03 | 137 | 3.5E-03 |
| hDLL4.5 | 143 | 1.2E-03 | 266 | 2.19E-03 | 162 | 4.2E-03 |
| hDLL4.6 | 136 | 1.1E-03 | 229 | 2.20E-03 | 152 | 4.1E-03 |
| mDLL4.8 | no binding | | no binding | | no binding | |
| mDLL4.10 | 141 | 1.1E-03 | 189 | 5.14E-03 | 121 | 3.8E-03 |
| mDLL4.11 | 132 | 1.6E-03 | 210 | 6.16E-03 | 121 | 6.6E-03 |
| mDLL4.12 | 161 | 1.3E-03 | 244 | 4.52E-03 | 152 | 3.1E-03 |

### 5.7. Determining the affinity of the hDII4 - VHH interaction

Kinetic analysis to determine the affinity of the DII4 - VHH interaction is performed by Surface Plasmon Resonance (SPR) on a Biacore T100 instrument. Recombinant human DII4 is immobilized onto a CM5 chip via amine coupling using EDC and NHS) or biotinylated human DII4 is captured on a SA chip (streptavidin surface). Purified VHHs or Fab fragment are injected for 2 minutes at different concentrations (between 10 and 300 nM) and allowed to dissociate for 20 min at a flow rate of 45 µl/min. Between sample injections, the surfaces are regenerated with 10 mM glycine pH1.5 and 100 mM HCl. HBS-N (Hepes buffer pH7.4) is used as running buffer. If possible, data are evaluated by fitting a 1:1 interaction model (Langmuir binding) onto the binding curves. The affinity constant K_{D} is calculated from resulting association and dissociation rate constants (kₐ) and (k_{d}). The affinities of the anti-DII4 VHHs are depicted in Table 11.

**Table 11: Affinity K_{D} (nM) of purified VHHs for recombinant human DLL4**

| | **rhDLL4** | | |
|---|---|---|---|
| **VHH ID** | **kₐ (M⁻¹.s⁻¹)** | **k_{d} (s⁻¹)** | **K_{D} (nM)** |
| 56A09 | 1.7E+05 | 9.3E-04 | 5.6 |
| 56C04 | 1.1E+05 | 4.9E-03 | 45 |
| 56H08 | 1.2E+05 | 1.1E-03 | 9.4 |
| 62C11 | 1.2E+06 | 1.3E-01 | 120 |
| 96C03 | 1.6E+05 | 4.8E-02 | 310 |
| 101G08 | 4.3E+04 | 2.2E-03 | 52 |
| 104G01 ^{(a)} | 1.2E+05 - 1.5E+05 | 3E-03 - 6E-04 | 4-24 |
| 115A05 | 1.5E+05 | 3.9E-03 | 25 |
| antiDLL4 Fab | 2.3E+05 | 3.4E-04 | 1.5 |

| | | | |
|---|---|---|---|
| ^{(a)} heterogeneous binding curve resulting in no 1:1 fit | | | |

### 5.8. Binding to orthologues (mDII4, cDII4) and family members (hJagged-1,hDLL1)

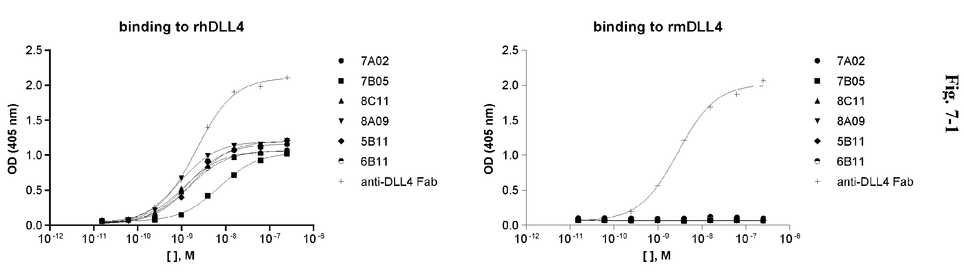

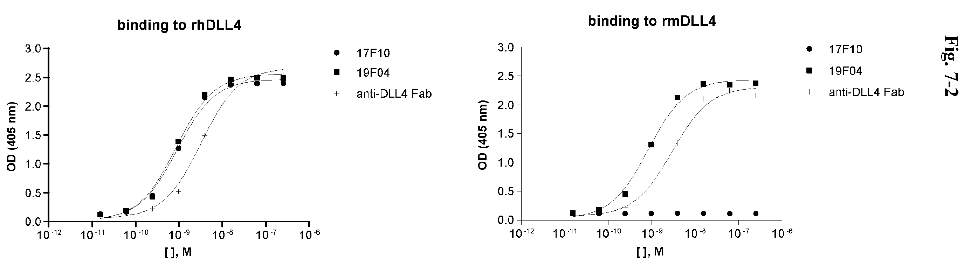

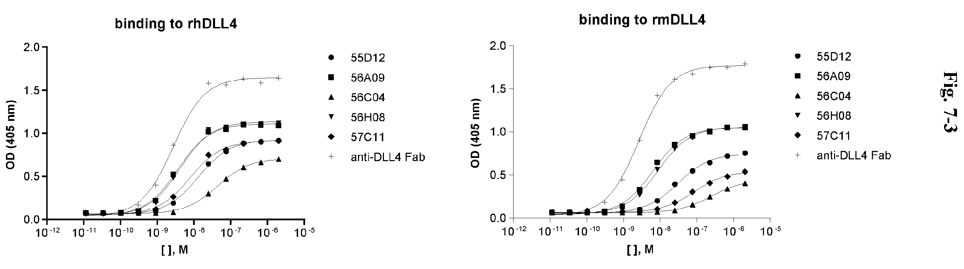

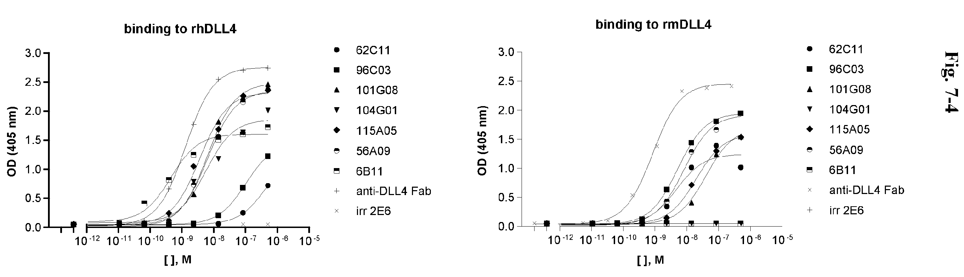

In order to determine cross-reactivity to mouse DII4 a binding ELISA is performed. In brief, recombinant mouse DII4 (R&D Systems, Minneapolis, MS, USA) is coated overnight at 4°C at 1 µg/mL in a 96-well MaxiSorp plate (Nunc, Wiesbaden, Germany). Wells are blocked with a casein solution (1% in PBS). VHHs are applied as dilution series and binding is detected using a mouse anti-myc (Roche) and an anti-mouse-AP conjugate (Sigma, St Louis, MO, USA) (Figure 7). As reference, binding to human DII4 is measured. EC₅₀ values are summarized in Table 12.

**Table 12: EC₅₀ (nM) values for VHHs in a recombinant human DLL4 and mouse DLL4 binding ELISA**

| | **rhDLL4** | **rmDLL4** |
|---|---|---|
| **VHH ID** | **EC₅₀ (nM)** | **EC₅₀ (nM)** |
| 5B11 | 1.8 | - |
| 6B11 | 1.4 | - |
| 7A02 | 1.4 | - |
| 7B05 | 7.2 | - |
| 8A09 | 0.9 | - |
| 8C11 | 1.1 | - |
| 17F10 | 0.9 | - |
| 19F04 | 0.9 | 0.8 |
| 55D12 | 13.1 | 30.0 |
| 56A09 | 3.6 | 6.3 |
| 56C04 | 44.3 | 244.0 |
| 56H08 | 4.1 | 8.7 |
| 57C11 | 7.9 | 83.4 |
| 62C11 | 137.0 | 13.1 |
| 96C03 | 86.5 | 8.7 |
| 101G08 | 8.9 | 53.9 |
| 104G01 | 8.4 | - |
| 115A05 | 5.0 | 33.4 |
| antiDLL4 Fab | 3.0 | 3.0 |

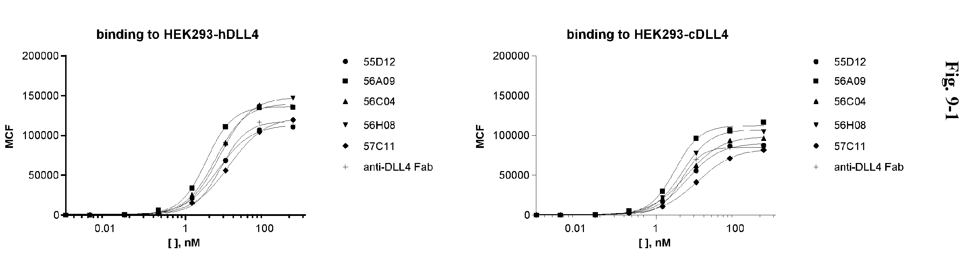

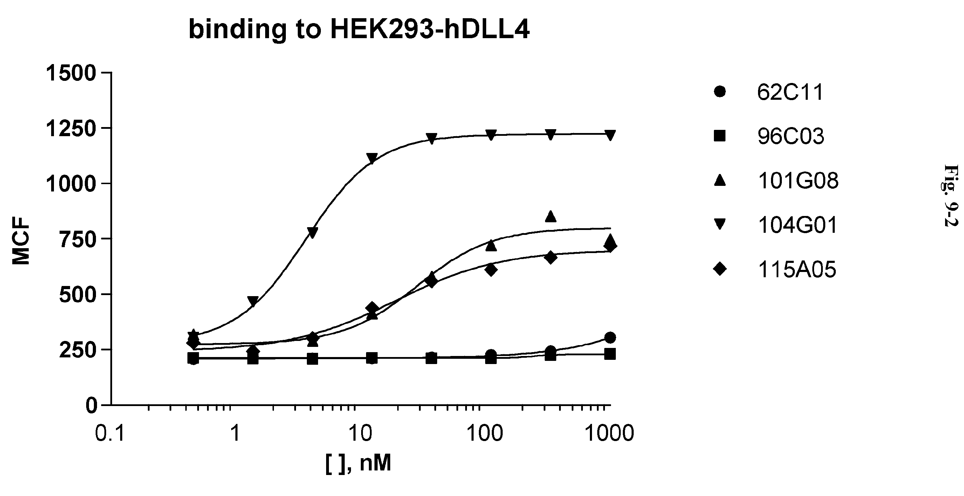

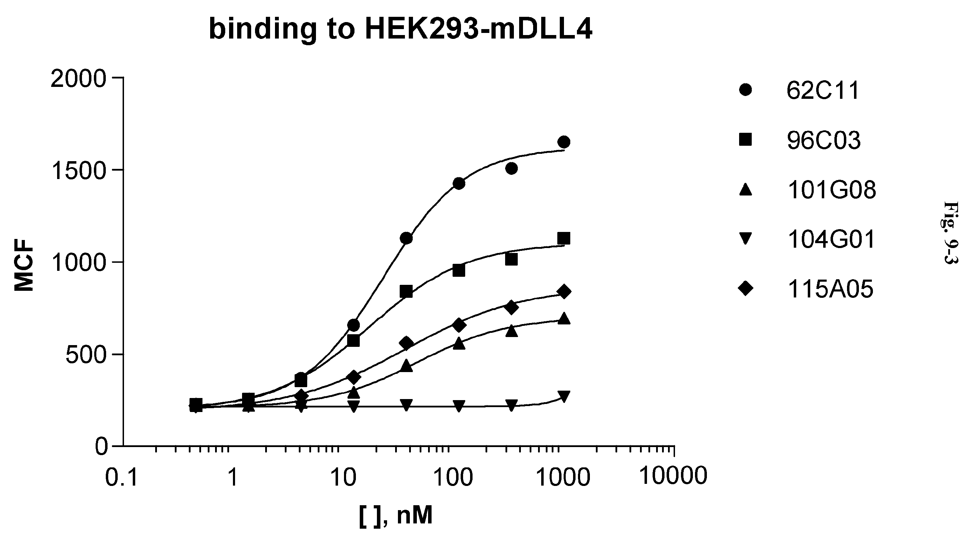

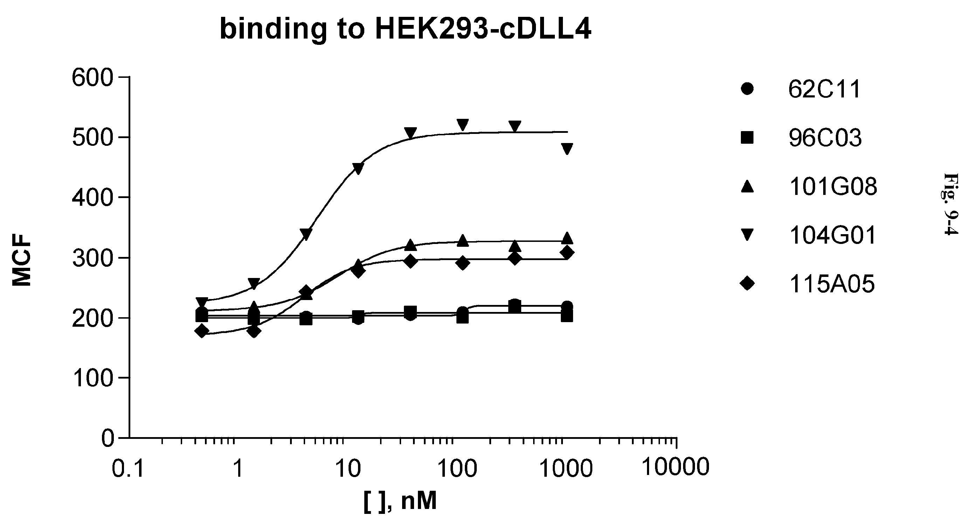

In order to determine the cynomologus cross-reactivity of the VHHs, a FACS binding experiment is performed. Cynomolgus DII4 expressing HEK293 cells (transient or stable transfection) are used for a titration binding experiment of the VHHs. After a 30 minutes incubation on ice, all samples are washed and detection is performed by applying anti-c-myc∼Alexa647 (Santa Cruz Biotechnology, Santa Cruz, CA, USA). Human and mouse DII4 overexpressing HEK293 cells are taken as reference. The mean MCF value is determined on the FACS Array and used for calculation of the EC₅₀ value (see Figure 9).

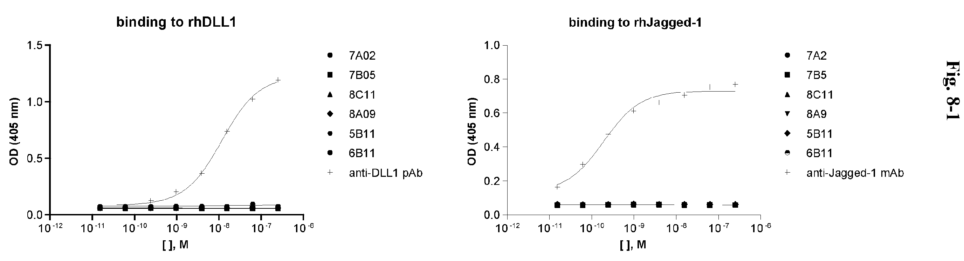

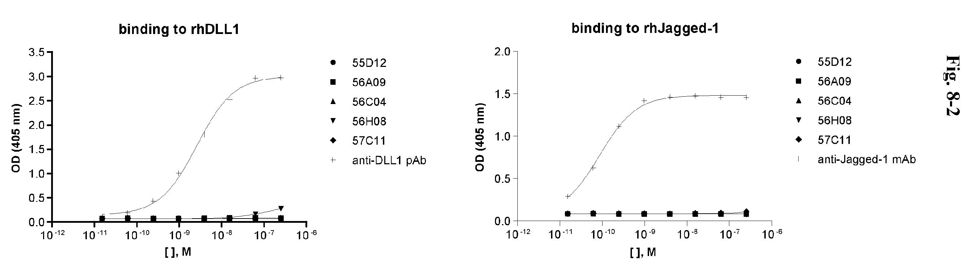

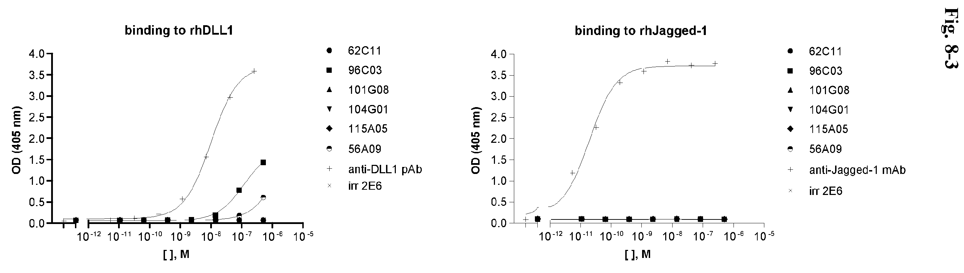

Absence of binding to homologous ligands human DLL1 and human Jagged-1 is assessed via solid phase binding assay (ELISA). In brief, human DLL1 (Alexis, San Diego, CA, USA) and human Jagged-1 (Alexis, San Diego, CA, USA) are coated overnight at 4°C at 1 µg/mL in a 96-well MaxiSorp plate (Nunc, Wiesbaden, Germany). Wells are blocked with a casein solution (1% in PBS). VHHs are applied as dilution series and binding is detected using a mouse anti-myc (Roche) and an anti-mouse-AP conjugate (Sigma, St. Louis, MO, USA). All anti-DII4 VHHs are considered as being non-cross reactive to these homologous ligands (Figure 8).

### 5.9. Evaluation of VHHs in blocking DII4- mediated HUVEC proliferation

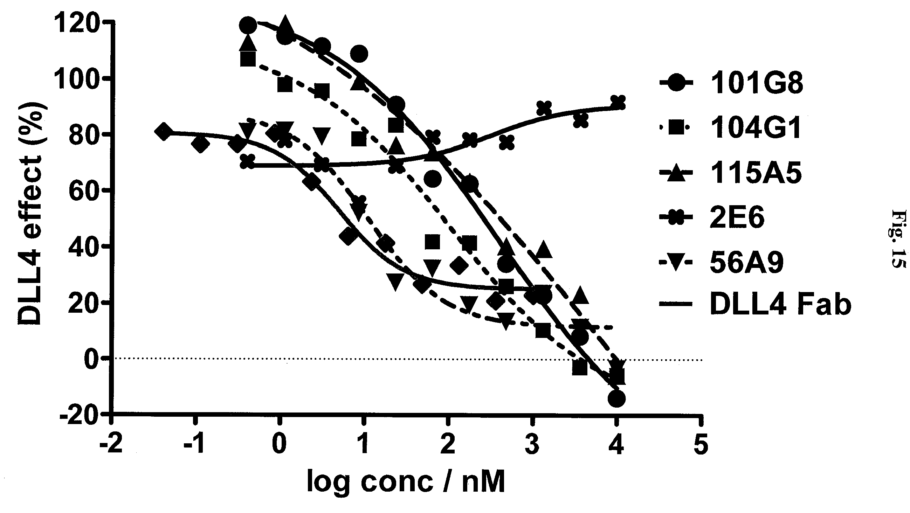

The potency of the selected VHHs is evaluated in a proliferation assay, as described by Ridgway et al., Nature. 2006 Dec 21;444(7122):1083-7), in modified form. In brief, 96-well tissue culture plates are coated with purified DII4-His (RnD Systems; C-terminal His-tagged human DII4, amino acid 27-524, 0.75ml/well, 10 ng/ml) in coating buffer (PBS, 0.1 % BSA). Wells are washed in PBS before 4000 HUVE cells/well are seeded in quadruplicate. Cell proliferation is measured by [³H]-Thymidine incorporation on day 4. The results, shown in Figure 15, demonstrate that the DLL4 VHHs DLLBII101 G08, DLLBII104G01, DLLBII115A05, DLLBII56A09 and the DLL4 Fab inhibit the DLL4-dependent effect on HUVEC proliferation in a dose-dependent manner, the IC₅₀ values are summarized in Table 13. The tested VHHs achieve a complete inhibition of the DLL4-dependent effect at 10µM.

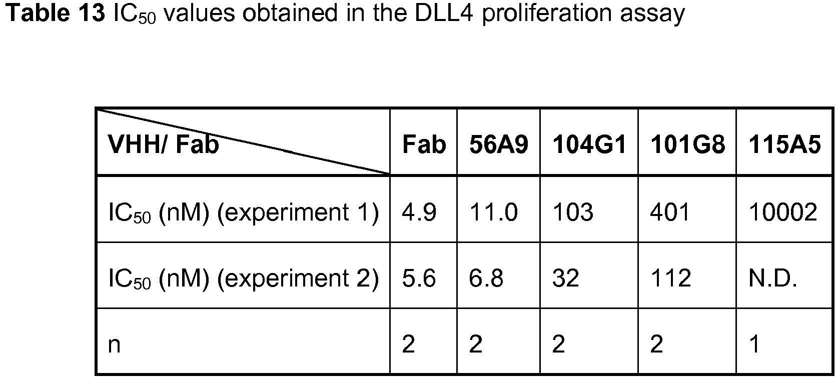

### Example 6

### Affinity maturation of selected anti-DII4 VHHs

VHHs DLLBII101G08 and DLLBII115A05 are subjected to two cycles of affinity maturation.

In a first cycle, amino acid substitutions are introduced randomly in both framework (FW) and complementary determining regions (CDR) using the error-prone PCR method. Mutagenesis is performed in a two-round PCR-based approach (Genemorph II Random Mutagenesis kit obtained from Stratagene, La Jolla, CA, USA) using 1 ng of the DLLBII101G08 or DLLBII115A05 cDNA template, followed by a second error-prone PCR using 0.1 ng of product of round 1. After a polish step, PCR products are inserted via unique restriction sites into a vector designed to facilitate phage display of the VHH library. Consecutive rounds of in-solution selections are performed using decreasing concentrations of biotinylated recombinant human DLL4 (biot-rhDLL4) and trypsin elutions. Affinity-driven selections in a third round using cold rhDLL4 (at least 100x excess over biot-rhDLL4) are also performed. No selections on murine DLL4 are included as (conservation of) cross-reactivity is assessed at the screening level. Individual mutants are produced as recombinant protein using an expression vector derived from pUC119, which contains the LacZ promoter, a resistance gene for ampicillin, a multiple cloning site and an ompA leader sequence (pAX50). *E. coli* TG1 cells are transformed with the expression vector library and plated on agar plates (LB + Amp + 2% glucose). Single colonies are picked from the agar plates and grown in 1 mL 96-deep-well plates. VHH expression is induced by adding IPTG (1 mM). Periplasmic extracts (in a volume of ∼ 80 uL) are prepared according to standard methods and screened for binding to recombinant human and mouse DII4 in a ProteOn (BioRad, Hercules, CA, USA) off-rate assay. In brief, a GLC ProteOn Sensor chip is coated with recombinant human DII4 on the "ligand channels" L2 and L4 (with L1/L3 as reference channel), while "ligand channels" L3 and L6 is coated with mouse DII4. Periplasmic extract of affinity matured clones is diluted 1/10 and injected across the "analyte channels" A1-A6. An average off-rate is calculated of the wild type clones present in the plate and served as a reference to calculate off-rate improvements.

In a second cycle, a combinatorial library is created by simultaneously randomising the susceptible positions identified in cycle one. For this, the full length DLLBII101G8 or DLLBII115A05 cDNA is synthesized by overlap PCR using oligonucleotides degenerated (NNS) at the randomisation positions and a rescue PCR is performed. A list of the primers used for generating the combinatorial library can be found in Table 14 and SEQ ID NOs: 427 to 457. The randomised VHH genes are inserted into a phage display vector (pAX50) using specific restriction sites as described above (Example 2). Preparation of periplasmic extracts of individual VHH clones is performed as described before.

**Table 14: Oligonucleotides affinity maturation libraries**

| **101G08 combinatorial library oligonucleotides** | **115A5 combinatorial library oligonucleotides** |
|---|---|
| >101G08CL_fwd1-bis | >115A05CL_fwd_1 |
| | |
| >101G08CL_fwd_2 | >115A5CL_rev1-bis |
| | |
| | >115A05CL_fwd_2 |
| >101G08CL_fwd_3 | |
| | >115A05CL1_rev_2 |
| >101G08CL_fwd_4 | |
| | >115A05CL_fwd_3 |
| >101G08CL_fwd_5 | |
| | >115A05CL_rev_3 |
| >101G08CL_fwd_6-4 | |
| | >115A05CL_fwd_4 |
| >101G08CL_fwd_6-3 | |
| | >115A05CL_rev_4 |
| >101G08CL_fwd_6-2 | |
| | >115A05CL_fwd_5 |
| >101G08CL_fwd_6-1 | |
| | >115A05CL_rev_5 |
| >101G08CL_rev_2-2 | |
| | |
| | >115A05CL_fwd_6-1 |
| >101G08CL_rev_2-1 | |
| | |
| | >115A05CL_fwd_6-2 |
| >101G08CL_rev_3 | |
| | |
| | >115A05CL_rev_6 |
| >101G08CL_rev_4 | |
| | |
| | >115A05CL2_rev_2-2 |
| >101G08CL_rev_5 | |
| | |
| | >115A05CL2_rev_2-1 |
| >101G08CL_rev_6 | |
| | |
| >101G08CL_rev1-bis | |
| | |

Screening for binding to recombinant human DII4 in a ProteOn off-rate assay identifies clones with up to 38-fold (DLLBII101G08) and 11-fold (DLLBII115A05) improved off-rates (Table 15).

**Table 15: Off-rate screening of DLLBII101G08 and DLLBII115A05 affinity-matured clones.**

| | hDLL4 k_{d} (s⁻¹) | fold | mDLL4 k_{d} (s⁻¹) | fold |
|---|---|---|---|---|
| *DLLBII101G08* | *2.2E-03* | *1* | *6.7E-03* | *1* |
| DLLBII129D08 | 5.9E-05 | 38 | 1.9E-04 | 35 |
| DLLBII129H04 | 6.8E-05 | 33 | 2.5E-04 | 27 |
| DLLBII129G10 | 7.3E-05 | 31 | 2.6E-04 | 26 |
| DLLBII129H07 | 7.4E-05 | 30 | 2.5E-04 | 27 |
| DLLBII129B02 | 7.6E-05 | 30 | 2.6E-04 | 26 |
| DLLBII129E11 | 8.0E-05 | 28 | 2.5E-04 | 26 |
| DLLBII130F06 | 6.5E-05 | 27 | 2.6E-04 | 19 |
| DLLBII130B03 | 6.7E-05 | 27 | 2.4E-04 | 20 |
| DLLBII129D01 | 8.5E-05 | 26 | 2.6E-04 | 26 |
| DLLBII130D06 | 6.9E-05 | 26 | 3.1E-04 | 16 |
| DLLBII129G09 | 8.8E-05 | 26 | 3.4E-04 | 20 |
| DLLBII129B05 | 9.3E-05 | 24 | 3.4E-04 | 20 |
| DLLBII130E03 | 7.5E-05 | 24 | 2.7E-04 | 18 |
| DLLBII129H05 | 9.4E-05 | 24 | 3.5E-04 | 19 |
| DLLBII130A05 | 7.5E-05 | 24 | 3.0E-04 | 17 |
| DLLBII130B02 | 7.8E-05 | 23 | 2.9E-04 | 17 |
| DLLBII129H02 | 9.9E-05 | 23 | 3.4E-04 | 19 |
| DLLBII130B04 | 8.3E-05 | 22 | 2.9E-04 | 17 |
| DLLBII129E07 | 1.1E-04 | 21 | 2.8E-04 | 24 |
| DLLBII129E03 | 1.1E-04 | 20 | 3.6E-04 | 18 |
| DLLBII129A03 | 1.2E-04 | 19 | 3.8E-04 | 18 |

The best top DLLBII101G08 variants and DLLBII115A05 variants are cloned into expression vector pAX100 in frame with a C-terminal c-myc tag and a (His)6 tag. Off-rates on recombinant mouse DII4 are also improved. VHHs are produced in *E. coli* as His6-tagged proteins and purified by IMAC and SEC. Sequences are represented in Tables 16-A (LLBII101G08) and 16-B (DLLBII115A05), respectively.

**Table 16-A Framework and CDR region sequences of DLLBII101G08 variants**

| VHH ID | | | | | | | |
|---|---|---|---|---|---|---|---|
| **SEQ ID NO** | **FR1** | **CDR1** | **FR2** | **CDR2** | **FR3** | **CDR3** | **FR4** |
| DLLBII12 9A03 | | | | | | | |
| **354** | | | | | | | |
| DLLBII12 9B02 | | | | | | | |
| **355** | | | | | | | |
| DLLBII12 9B05 | | | | | | | |
| **356** | | | | | | | |
| DLLBII12 9D01 | | | | | | | |
| **357** | | | | | | | |
| DLLBII12 9D08 | | | | | | | |
| **358** | | | | | | | |
| DLLBII12 9E03 | | | | | | | |
| **359** | | | | | | | |
| DLLBII12 9E07 | | | | | | | |
| **360** | | | | | | | |
| DLLBII12 9E11 | | | | | | | |
| **361** | | | | | | | |
| DLLBII12 9G09 | | | | | | | |
| **362** | | | | | | | |
| DLLBII12 9G10 | | | | | | | |
| **363** | | | | | | | |
| DLLBII12 9H02 | | | | | | | |
| **364** | | | | | | | |
| DLLBII12 9H04 | | | | | | | |
| **365** | | | | | | | |
| DLLBII12 9H05 | | | | | | | |
| **366** | | | | | | | |
| DLLBII12 9H07 | | | | | | | |
| **367** | | | | | | | |
| DLLBII13 0A05 | | | | | | | |
| **368** | | | | | | | |
| DLLBII13 0B02 | | | | | | | |
| **369** | | | | | | | |
| DLLBII13 0B03 | | | | | | | |
| **370** | | | | | | | |
| DLLBII13 0B04 | | | | | | | |
| **371** | | | | | | | |
| DLLBII13 0D06 | | | | | | | |
| **372** | | | | | | | |
| DLLBII13 0E03 | | | | | | | |
| **373** | | | | | | | |
| DLLBII13 0F06 | | | | | | | |
| **374** | | | | | | | |

**Table 16-B Framework and CDR region sequences of DLLBII115A05 variants**

| VHH ID | **FR1** | **CDR1** | **FR2** | **CDR2** | **FR3** | **CDR3** | **FR4** |
|---|---|---|---|---|---|---|---|
| **SEQ ID NO** | | | | | | | |
| DLLBII1 33A05 | | | | | | | |
| **396** | | | | | | | |
| DLLBII1 33A09 | | | | | | | |
| **397** | | | | | | | |
| DLLBII1 33A12 | | | | | | | |
| **398** | | | | | | | |
| DLLBII1 33D06 | | | | | | | |
| **399** | | | | | | | |
| DLLBII1 33F01 | | | | | | | |
| **400** | | | | | | | |
| DLLBII1 33F06 | | | | | | | |
| **401** | | | | | | | |
| DLLBII1 33G05 | | | | | | | |
| **402** | | | | | | | |
| DLLBII1 33H03 | | | | | | | |
| **403** | | | | | | | |
| DLLBII1 34B11 | | | | | | | |
| **404** | | | | | | | |
| DLLBII1 34D10 | | | | | | | |
| **405** | | | | | | | |
| DLLBII1 35H04 | | | | | | | |
| **406** | | | | | | | |
| DLLBII1 36C07 | | | | | | | |
| **407** | | | | | | | |
| DLLBII1 36D01 | | | | | | | |
| **408** | | | | | | | |
| DLLBII1 36H03 | | | | | | | |
| **409** | | | | | | | |
| DLLBII1 37A04 | | | | | | | |
| **410** | | | | | | | |
| DLLBII1 37A06 | | | | | | | |
| **411** | | | | | | | |
| DLLBII1 37B06 | | | | | | | |
| **412** | | | | | | | |
| DLLBII1 37C04 | | | | | | | |
| **413** | | | | | | | |
| DLLBII1 37F04 | | | | | | | |
| **414** | | | | | | | |
| DLLBII1 38F12 | | | | | | | |
| **415** | | | | | | | |
| DLLBI 15 | | | | | | | |
| **416** | | | | | | | |

### Example 7

### Characterization of affinity matured purified anti-DII4 VHHs

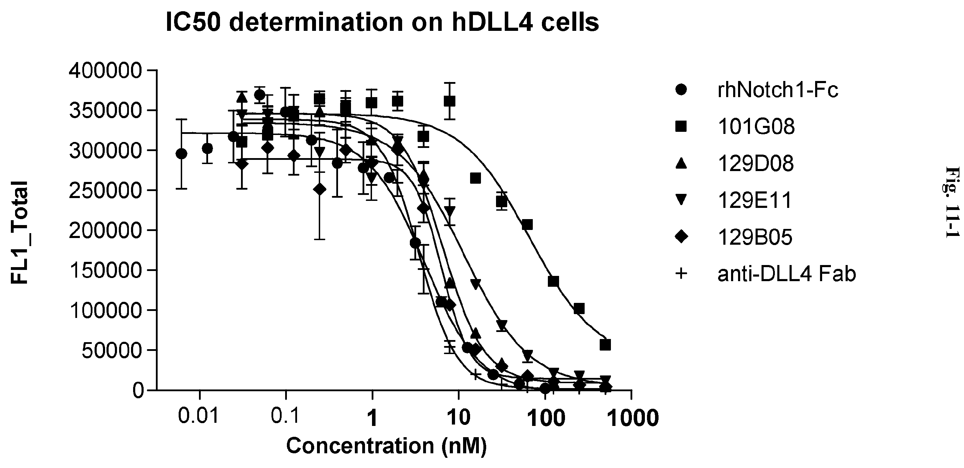

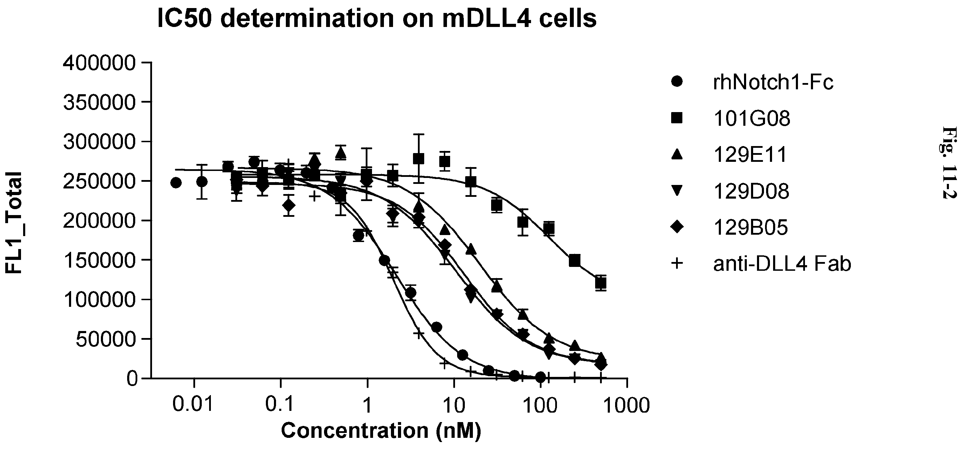

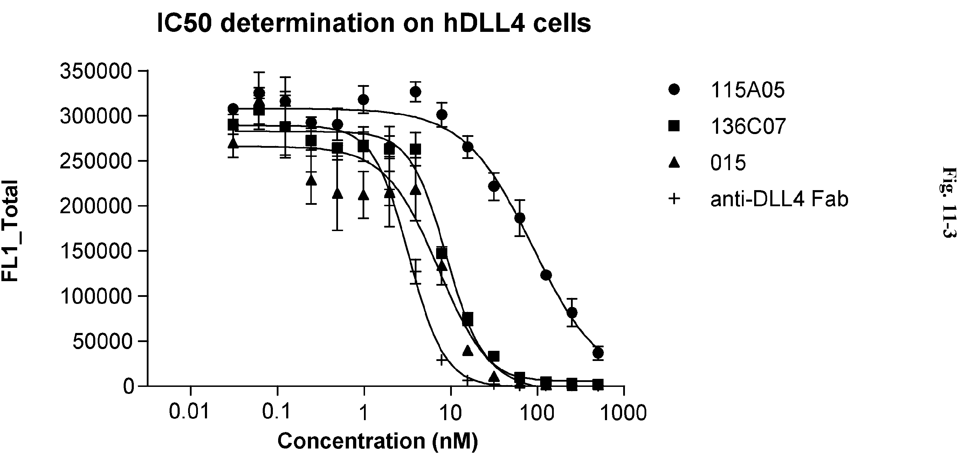

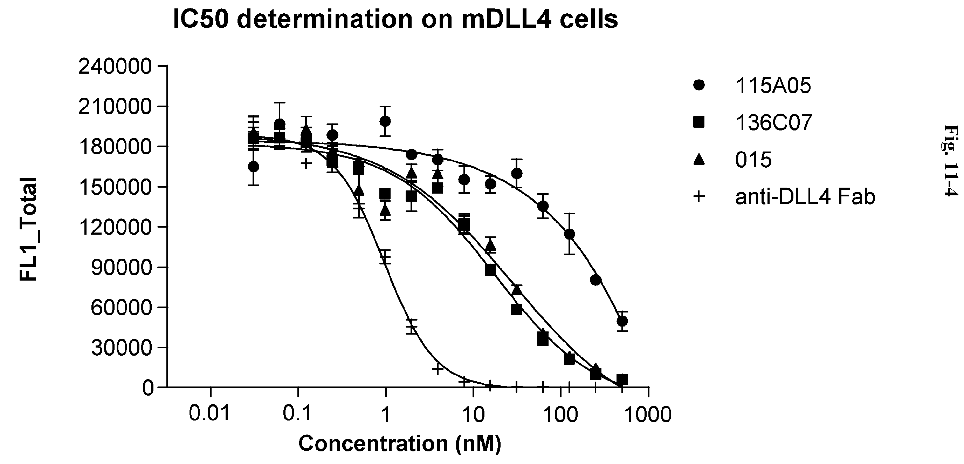

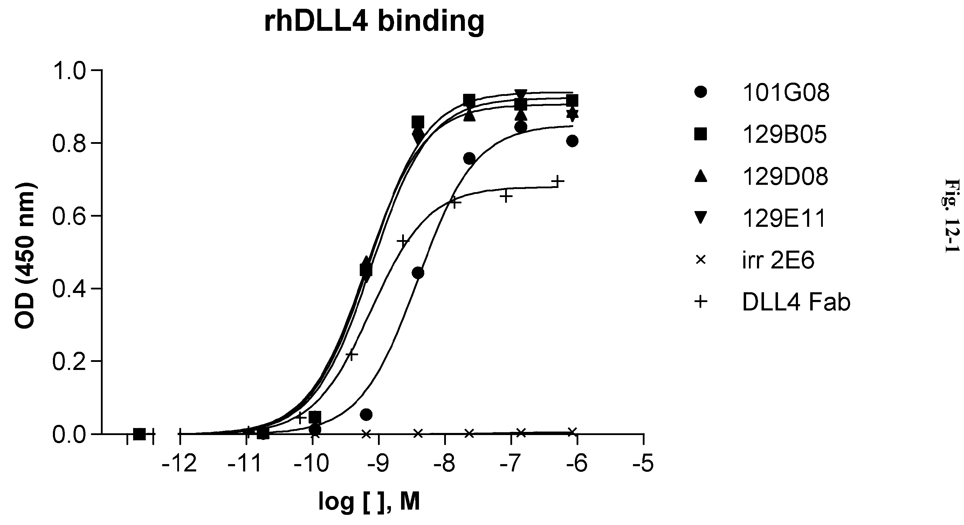

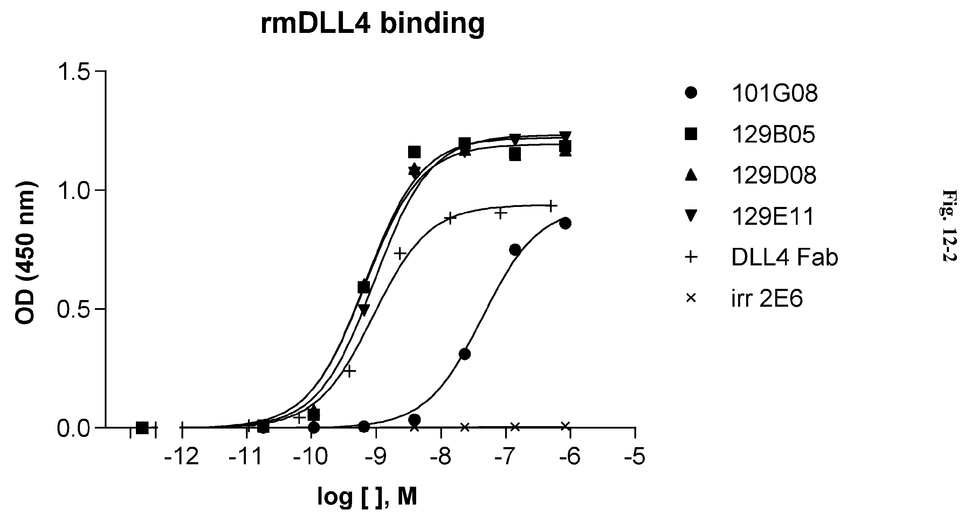

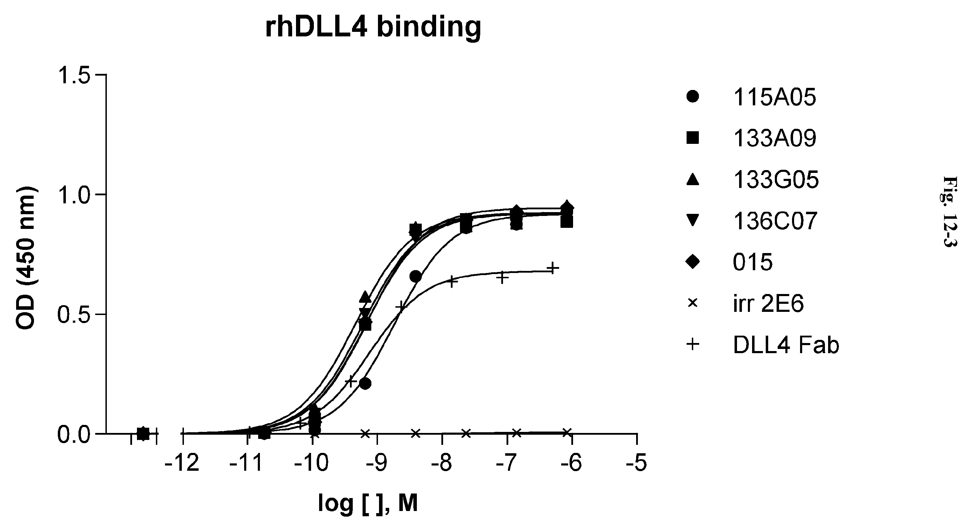

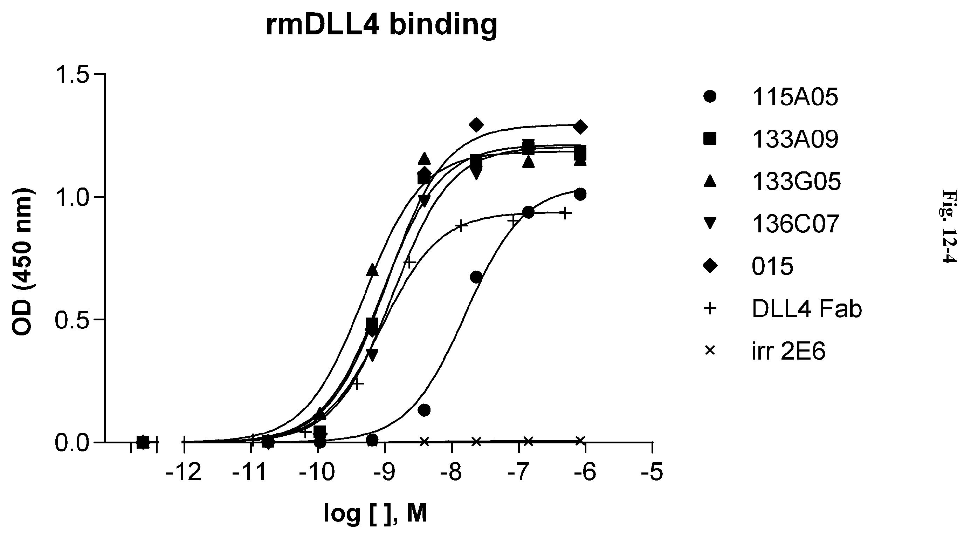

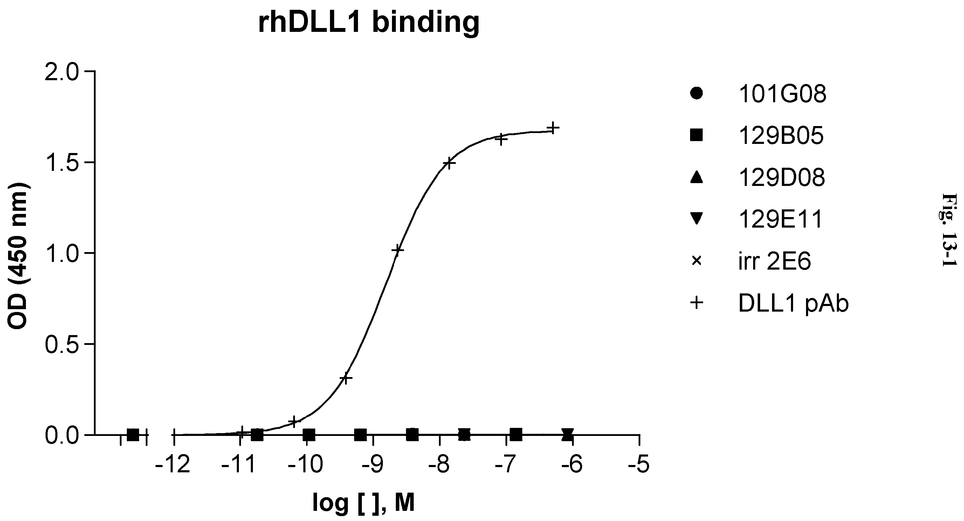

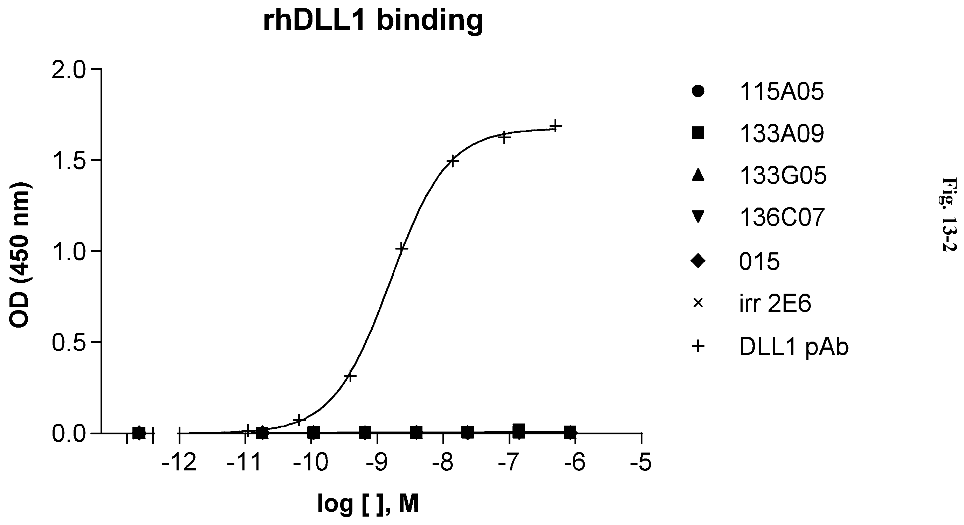

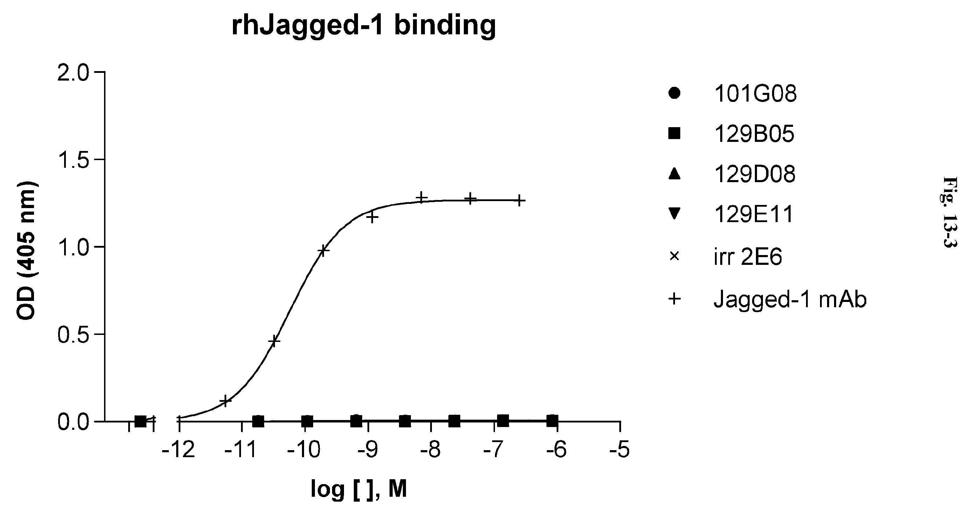

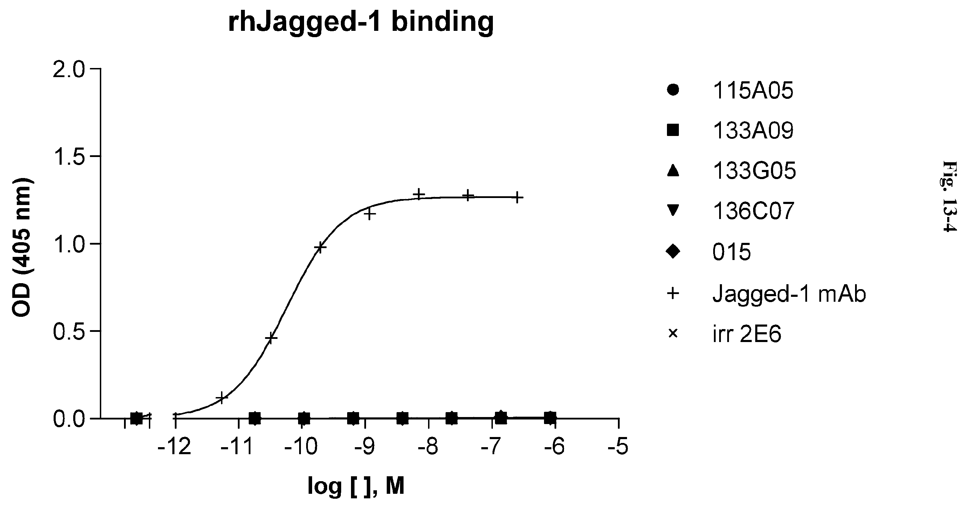

Affinity-matured variants of VHHs DLLBII101G08 and DLLBII115A05 are expressed and purified as described above (Example 6). VHHs are characterized in the rhDLL1/ rhJAG1 binding ELISA and hDII4/ mDII4/ cynoDII4 FACS (Example 5.8; Table 20; Figure 12 and 13), the rhDII4 - rhNotch1 competition ELISA (Example 5.1; Table 17; Figure 10), the competition rhNotch1 - CHO-hDII4 FMAT (Example 5.3; Table 18; Figure 11).

Characterization data are summarized in Table 21. Overall, the affinity matured VHHs show clear improvements in affinity and potency, while their binding to mDII4 and cyno DII4 is maintained and no binding to hDLL1 or hJAG1 is observed

**Table 17: IC₅₀ (nM) values for affinity matured VHHs in hDLL4/hNotch1-Fc competition ELISA**

| **VHH ID** | **IC₅₀ (nM)** |
|---|---|
| *101G08* | *10.0* |
| 129A03 | 1.8 |
| 129B05 | 0.9 |
| 129D08 | 1.2 |
| 129E11 | 1.3 |
| 129H07 | 1.0 |
| 130B03 | 1.5 |
| 130F06 | 1.3 |
| anti-DLL4 Fab | 1.5 |
| *115A05* | *7.5* |
| 133A05 | 2.1 |
| 133A09 | 1.5 |
| 133G05 | 2.0 |
| 134D10 | 1.3 |
| 136C07 | 1.4 |
| 015 | 0.9 |
| anti-DLL4 Fab | 1.2 |

**Table 18: IC₅₀ values (nM) of purified affinity matured VHHs blocking the interaction of human Notch1/Fc to human or mouse DLL4 expressed on CHO cells (FMAT)**

| | **hDLL4** | **mDLL4** |
|---|---|---|
| **VHH ID** | **IC₅₀ (nM)** | **IC₅₀ (nM)** |
| *101G08* | *69.3* | *140.5* |
| 129B05 | 7.4 | 14.4 |
| 129D08 | 7.8 | 11.0 |
| 129E11 | 8.1 | 12.3 |
| anti-DLL4 Fab | 5.5 | 3.0 |
| *115A05* | *106.7* | *348.9* |
| 133A09 | 6.6 | 18.6 |
| 133G05 | 5.9 | 12.0 |
| 136C07 | 8.0 | 31.2 |
| 015 | 5.7 | 21.2 |
| anti-DLL4 Fab | 3.4 | 1.6 |

**Table 19: Affinity K_{D} (nM) of purified affinity matured VHHs on recombinant human DLL4 and mouse DLL4**

| | **rhDLL4** | | | **rmDLL4** | | |
|---|---|---|---|---|---|---|
| **VHH ID** | **kₐ (M⁻¹s⁻¹)** | **k_{d} (s⁻¹)** | **K_{D} (nM)** | **kₐ (M⁻¹s⁻¹)** | **k_{d} (s⁻¹)** | **K_{D} (nM)** |
| *101G08 (wt)* | *4.8E*+*04* | *2.3E-03* | *48.0* | *9.4E*+*04* | *5.6E-03* | *60.0* |
| 129A03 | 2.1E+05 | 1.2E-04 | 0.5 | | | |
| 129B05 | 2.3E+05 | 7.9E-05 | 0.3 | 2.7E+05 | 3.1 E-04 | 1.1 |
| 129D08 | 1.8E+05 | 6.4E-05 | 0.4 | 2.7E+05 | 2.0E-04 | 0.8 |
| 129E11 | 1.9E+05 | 9.0E-05 | 0.5 | 2.5E+05 | 2.9E-04 | 1.2 |
| 129H07 | 1.6E+05 | 7.3E-05 | 0.5 | | | |
| 130B03 | 2.2E+05 | 6.8E-05 | 0.3 | | | |
| 130F06 | 2.0E+05 | 8.0E-05 | 0.4 | | | |
| anti-DLL4 Fab | 2.3E+05 | 3.4E-04 | 1.5 | | | |
| | | | | | | |

| **VHH ID** | **kₐ (M⁻¹s⁻ 1)** | **k_{d} (s⁻¹)** | **K_{D} (nM)** | **kₐ (M⁻¹s⁻¹)** | **k_{d} (s⁻¹)** | **K_{D} (nM)** |
|---|---|---|---|---|---|---|
| *115A05 (wt)* | *2.5E*+*05* | *4.0E-03* | *16.0* | *1.7E*+*05* | *9.1E-03* | *53. 0* |
| 133A09 | 4.4E+05 | 9.0E-04 | 2.1 | 3.5E+05 | 2.7E-03 | 7.8 |
| 133G05 | 5.9E+05 | 4.7E-04 | 0.8 | 4.7E+05 | 1.6E-03 | 3.4 |
| 136C07 | 6.2E+05 | 3.9E-04 | 0.6 | 5.0E+05 | 1.3E-03 | 2.6 |
| 015 | 4.5E+05 | 4.7E-04 | 1.0 | 3.5E+05 | 1.5E-03 | 4.3 |
| anti-DLL4 Fab | 2.3E+05 | 3.4E-04 | 1.5 | | | |

**Table 20: EC₅₀ (nM) values of affinity matured VHHs for binding on CHO-hDLL4, CHO-mDLL4 and CHO-cDLL4 (FACS)**

| | **hDLL4** | **mDLL4** | **cDLL4** |
|---|---|---|---|
| **VHH ID** | **EC₅₀ (nM)** | **EC₅₀ (nM)** | **EC₅₀ (nM)** |
| *101G08(wt)* | *17.5* | | *11.2* |
| 129B05 | 9.7 | 3.9 | 3.9 |
| 129D08 | 9.6 | 3.7 | 3.8 |
| 129E11 | 1.4 | 4.1 | 4.2 |
| anti-DLL4 Fab | 5.6 | 2.1 | 2.5 |
| *115A05(wt)* | *11.3* | | 13.8 |
| 133A09 | 7.2 | 1.7 | 2.3 |
| 133G05 | 8.5 | 2.8 | 2.7 |
| 136C07 | 10.9 | 8.3 | 3.5 |
| 015 | 14.8 | 7.0 | 5.1 |
| anti-DLL4 Fab | 5.6 | 2.1 | 2.5 |

**Table 21: Characteristics of affinity-matured VHHs derived from DLLBII101G08 and DLLBII115A05**

| | | | ELISA | FMAThDLL4 | FMATmDLL4 | FACS | FACS | FACS | ELISA | ELISA |
|---|---|---|---|---|---|---|---|---|---|---|
| | K_{D} (nM) hDL L4 | K_{D} (nM ) mD LL4 | IC₅₀ (nM ) | IC₅₀ (nM ) | IC₅₀ (nM) | EC₅₀ (nM ) | EC₅₀ (nM ) | EC₅₀ (nM ) | hDLL1 | hJag-1 |
| *101G08* | *48.0* | *60.0* | *10.0* | *69.3* | *140. 5* | *17.5* | *NF* | *11. 2* | *nb* | *nb* |
| 129A03 | 0.5 | | 1.8 | | | | | | | |
| 129B05 | 0.3 | 1.1 | 0.9 | 7.4 | 14.4 | 9.7 | 3.9 | 3.9 | nb | nb |
| 129D08 | 0.4 | 0.8 | 1.2 | 7.8 | 11.0 | 9.6 | 3.7 | 3.8 | nb | nb |
| 129E11 | 0.5 | 1.2 | 1.3 | 8.1 | 12.3 | 10.4 | 4.1 | 4.2 | nb | nb |
| 129H07 | 0.5 | | 1.0 | | | | | | | |
| 130B03 | 0.3 | | 1.5 | | | | | | | |
| 130F06 | 0.4 | | 1.3 | | | | | | | |
| DLL4Fab | 1.5 | | 1.5 | 5.5 | 3.0 | 5.6 | 2.1 | 2.5 | | |
| *115A05* | *16.0* | *53. 0* | *7.5* | *106.7* | *348.9* | *11.3* | *NF* | *13.8* | *nb* | *nb* |
| 133A05 | | | 2.1 | | | | | | | |
| 133A09 | 2.1 | 7.8 | 1.5 | 6.6 | 18.6 | 7.2 | 1.7 | 2.3 | nb | nb |
| 133G05 | 0.8 | 3.4 | 2.0 | 5.9 | 12.0 | 8.5 | 2.8 | 2.7 | nb | nb |
| 134D10 | | | 1.3 | | | | | | | |
| 136C07 | 0.6 | 2.6 | 1.4 | 8.0 | 31.2 | 10.9 | 8.3 | 3.5 | nb | nb |
| 015 | 1.0 | 4.3 | 0.9 | 5.7 | 21.2 | 14.8 | 7.0 | 5.1 | nb | nb |
| DLL4Fab | 1.5 | | 1.2 | 3.4 | 1.6 | 5.6 | 2.1 | 2.5 | | |

| | | | | | | | | | | |
|---|---|---|---|---|---|---|---|---|---|---|
| nb: no binding | | | | | | | | | | |

### Example 8

### Construction, production and characterization of bispecific VHHs targeting DLL4 and Ang2 using anti-serum albumin binding as half-life extension

In a first cycle, the anti-DLL4 VHH DLLBII00018 (US 2011/0172398 A1), the sequence of which is indicated in US2011/0172398 as SEQ ID NO:35: and in Table 23 as follows:

| Sequence IDs and AA sequences of monovalent sequence-optimized anti-DLL4 VHHs of parental DLLBII129B05 (FR, framework; CDR, complementary determining region) | | | | | | | |
|---|---|---|---|---|---|---|---|
| VHH ID SEQ ID NO | FR1 | CDR1 | FR2 | CDR2 | FR3 | CDR3 | FR4 |
| DLLBII018 35 | | SYAMA | | | | | , |

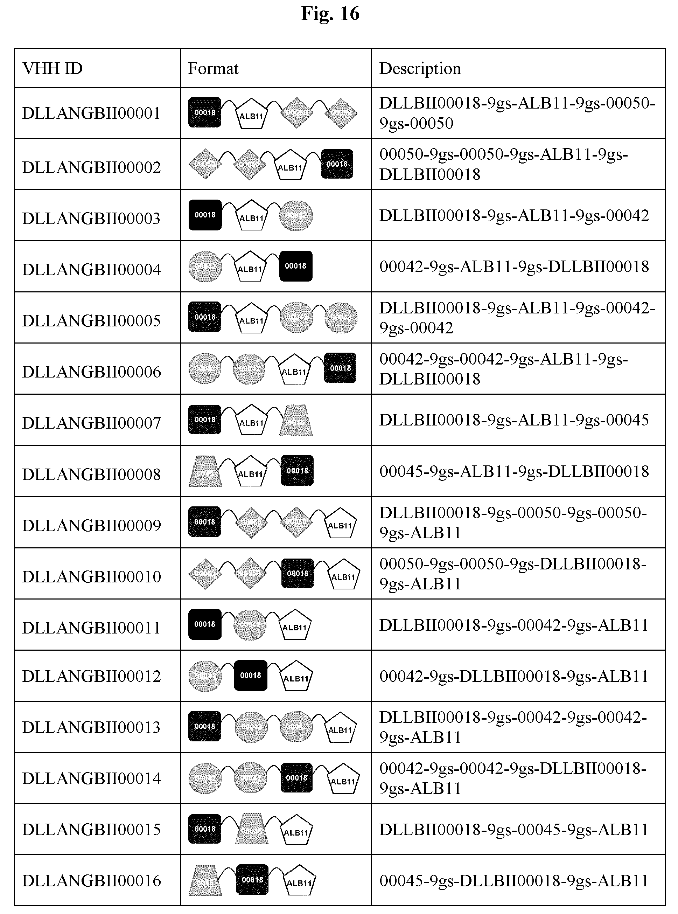

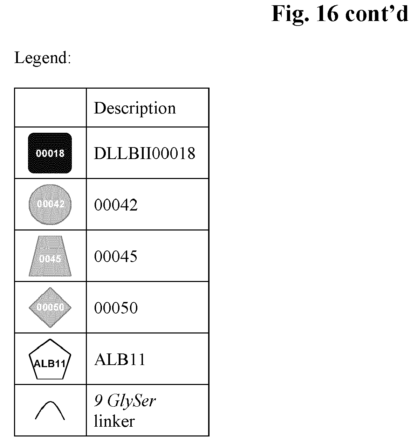

and the cycle 1 sequence optimized anti-Ang2 VHHs 00042 (SEQ ID NO: 482), 00045 (SEQ ID NO: 484) and 00050 (SEQ ID NO:483) are used as building blocks to generate bispecific VHHs DLLANGBII00001-00016. A genetic fusion to a serum albumin binding VHH is used as half-life extension methodology. Building blocks are linked via a 9 Gly-Ser flexible linker. VHHs are produced and purified as described in Example 5. An overview of the format and sequence of all bispecific VHHs is depicted in Figure 16 and Table 22-A (linker sequences underlined), SEQ ID Nos 460-475. Expression levels are indicated in Table 22-B.

**Table 22-A**

| Sequences of bispecific VHH targeting DLL4 and Ang2 | |
|---|---|
| VHH ID | AA sequence |
| DLLANGBII00001 | |
| DLLANGBII00002 | |
| DLLANGBII00003 | |
| DLLANGBII00004 | |
| DLLANGBII00005 | |
| DLLANGBII00006 | |
| DLLANGBII00007 | |
| DLLANGBII00008 | |
| DLLANGBII00009 | |
| DLLANGBII00010 | |
| DLLANGBII00011 | |
| DLLANGBII00012 | |
| DLLANGBII00013 | |
| DLLANGBII00014 | |
| DLLANGBII00015 | |
| DLLANGBII00016 | |

**Table 22-B**

| VHH ID | Format | Expression (mg/L) |
|---|---|---|
| DLLANGBII00001 | 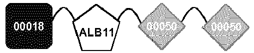 | 46 |
| DLLANGBII00002 | 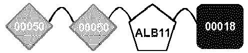 | -n.e. |
| DLLANGBII00003 | 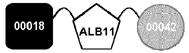 | 133 |
| DLLANGBII00004 | 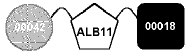 | 57 |
| DLLANGBII00005 | 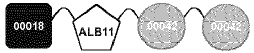 | 83 |
| DLLANGBII00006 | 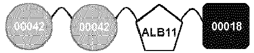 | 24 |
| DLLANGBII00007 | 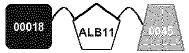 | 46 |
| DLLANGBII00008 | 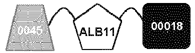 | 18 |
| DLLANGBII00009 | 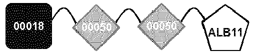 | 30 |
| DLLANGBII00010 | 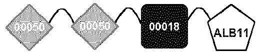 | 13 |
| DLLANGBII00011 | 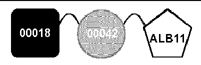 | 125 |
| DLLANGBII00012 | 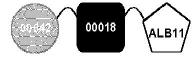 | 75 |
| DLLANGBII00013 | 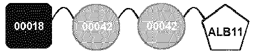 | 26 |
| DLLANGBII00014 | 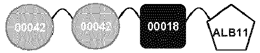 | 57 |
| DLLANGBII00015 | 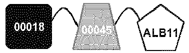 | 61 |
| DLLANGBII00016 | 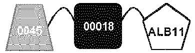 | 3 |

| | | |
|---|---|---|
| n.e.: no expression performed | | |

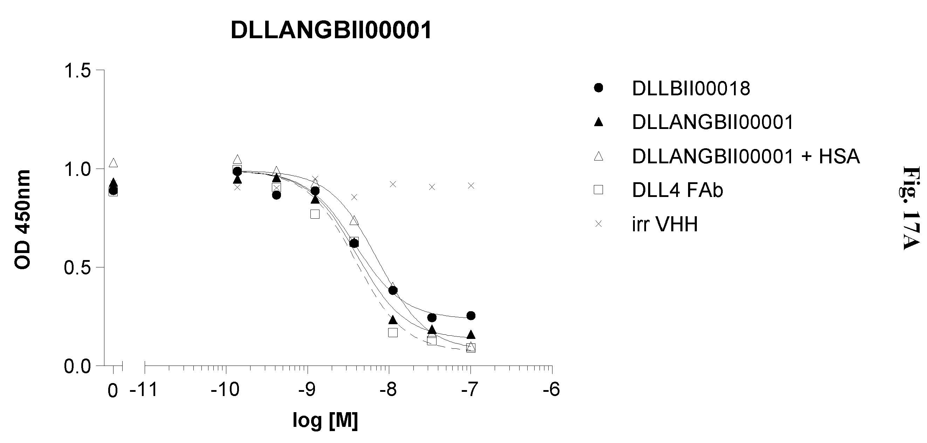

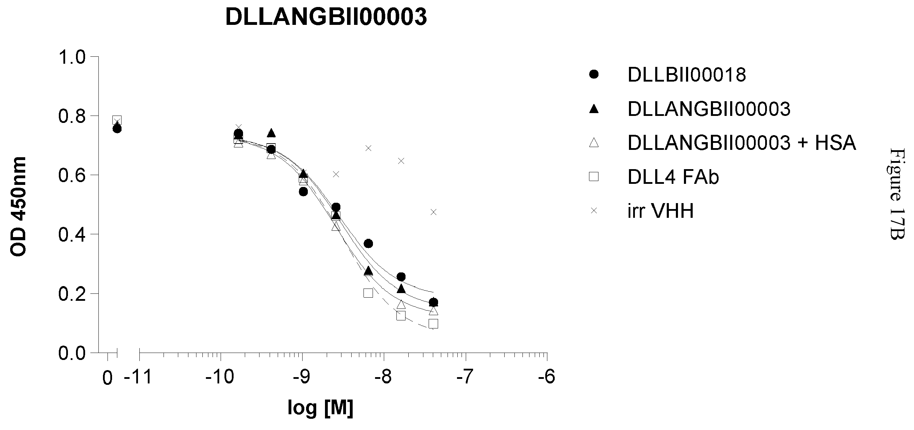

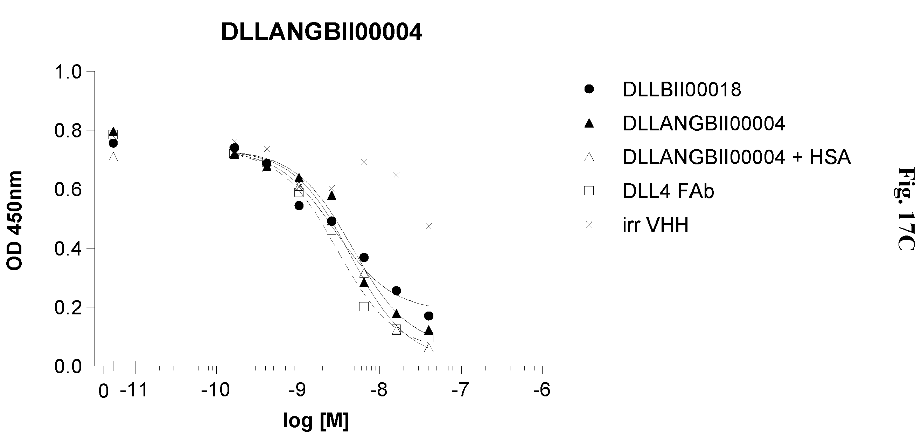

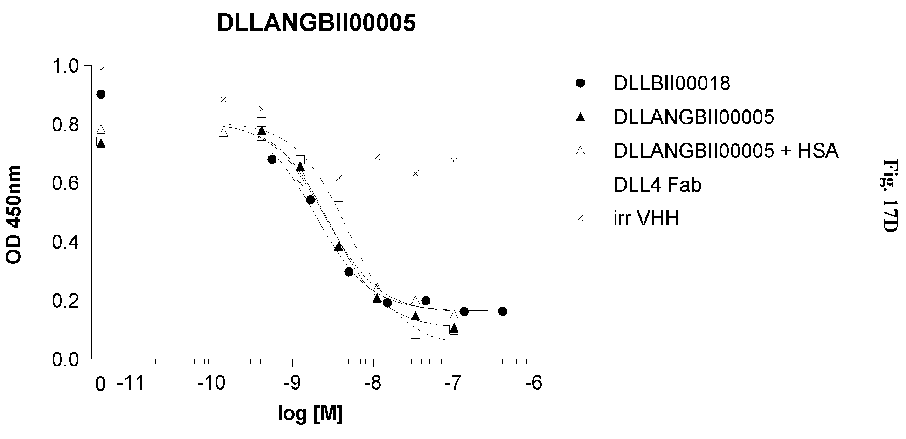

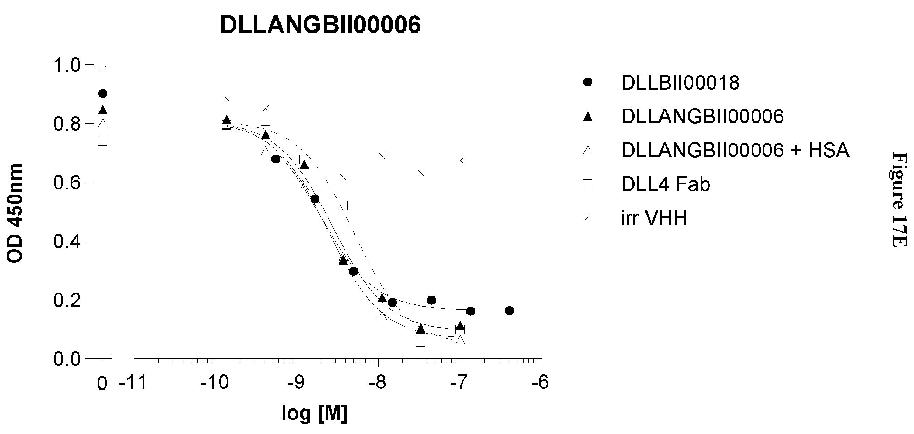

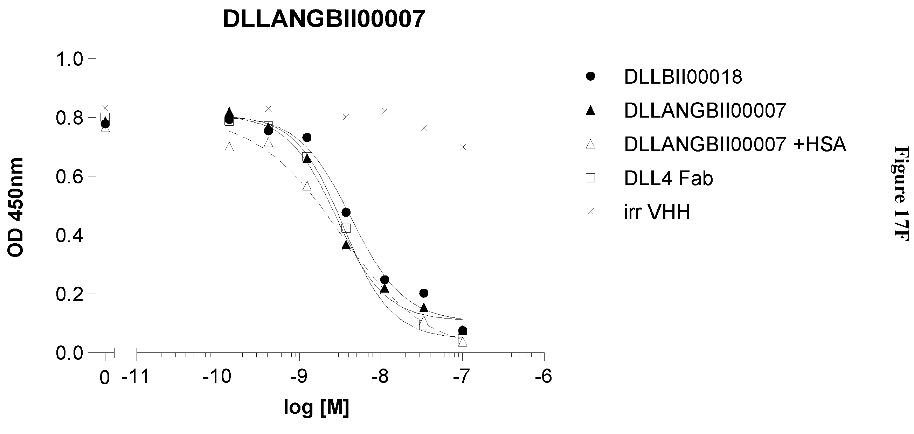

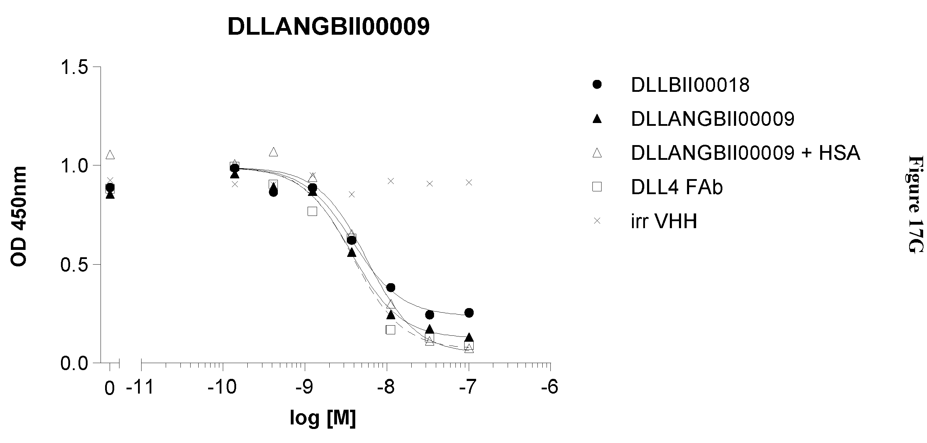

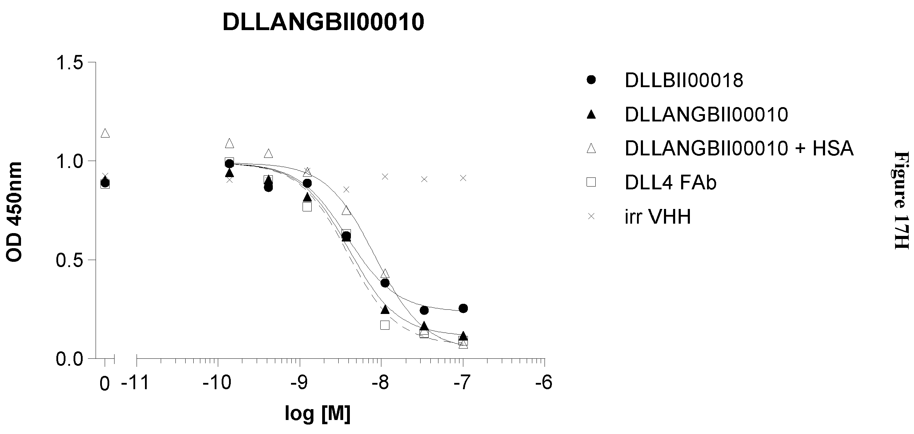

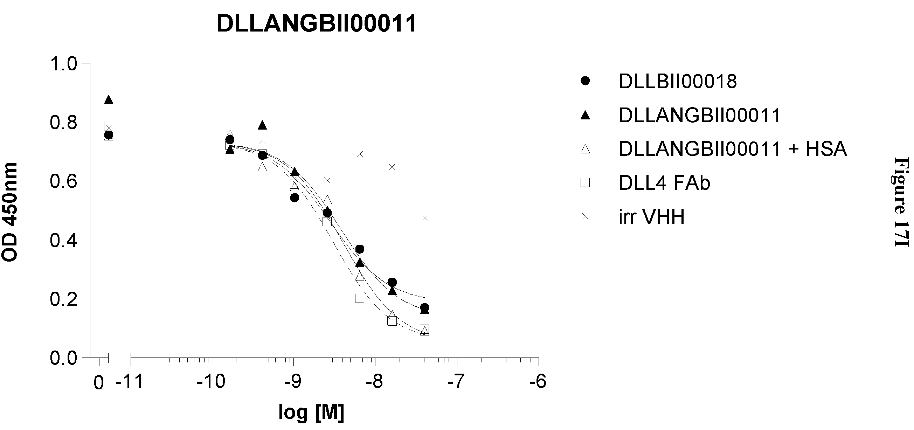

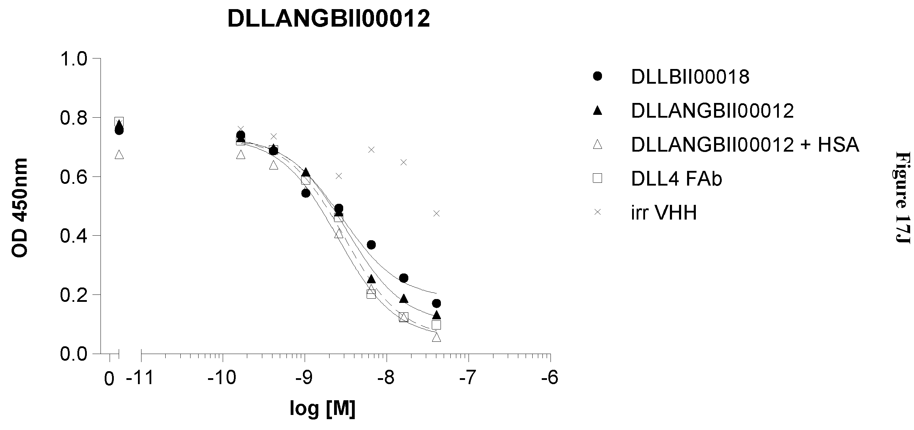

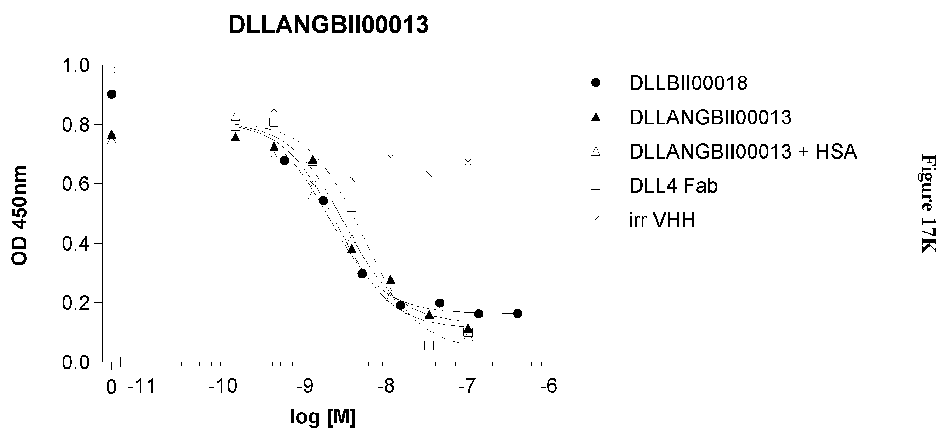

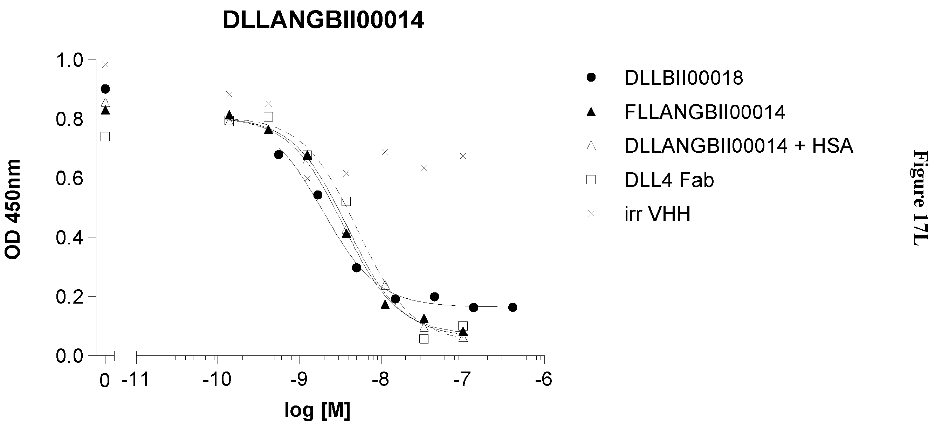

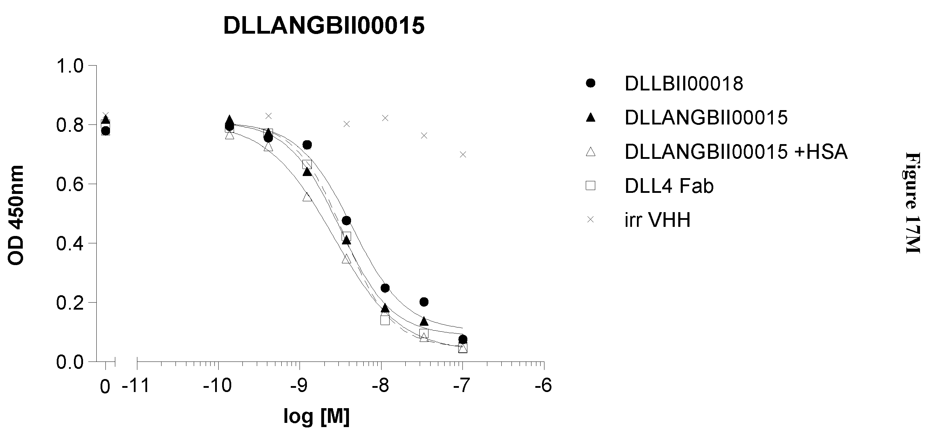

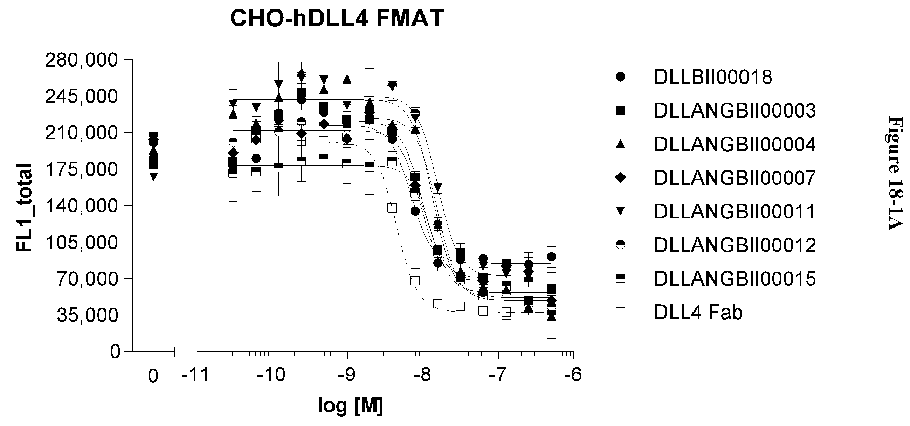

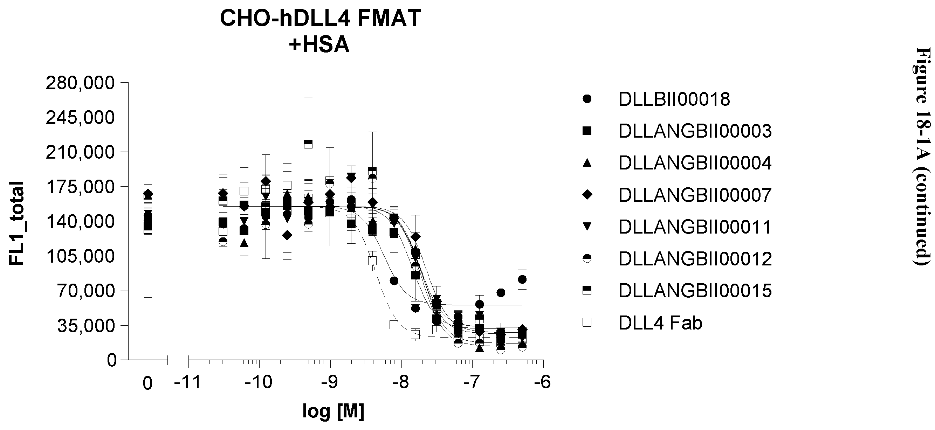

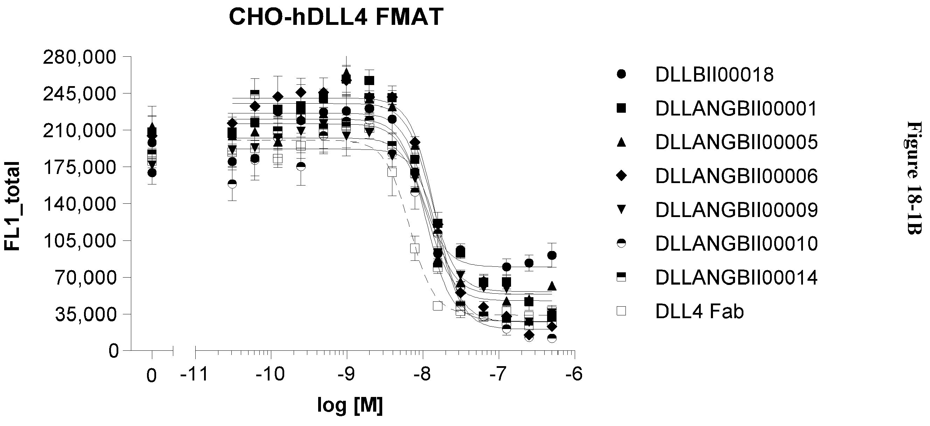

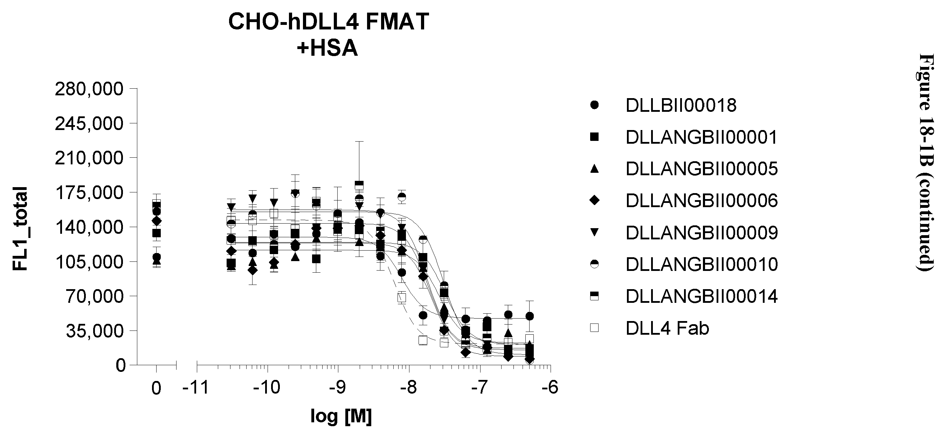

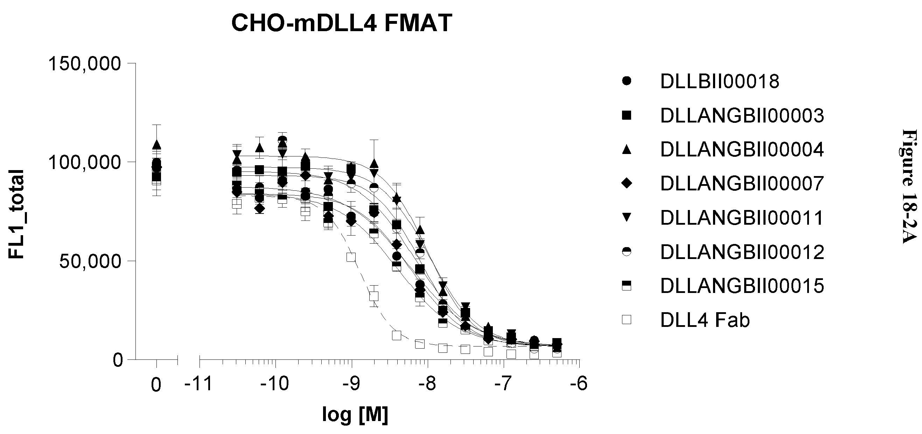

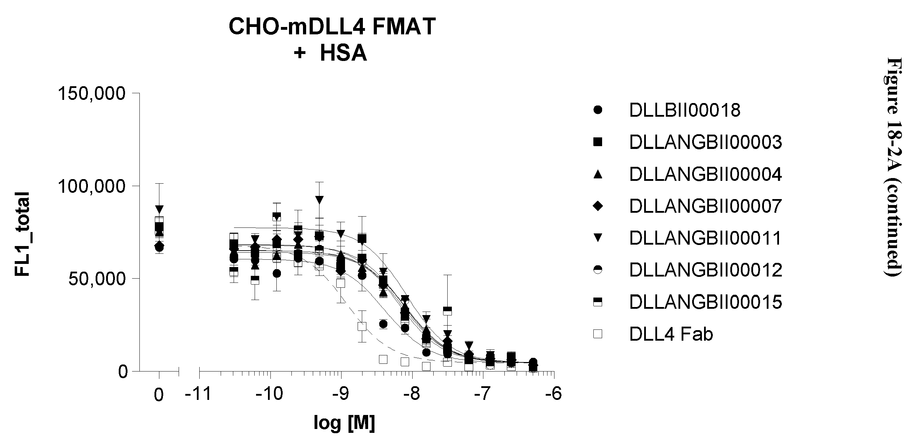

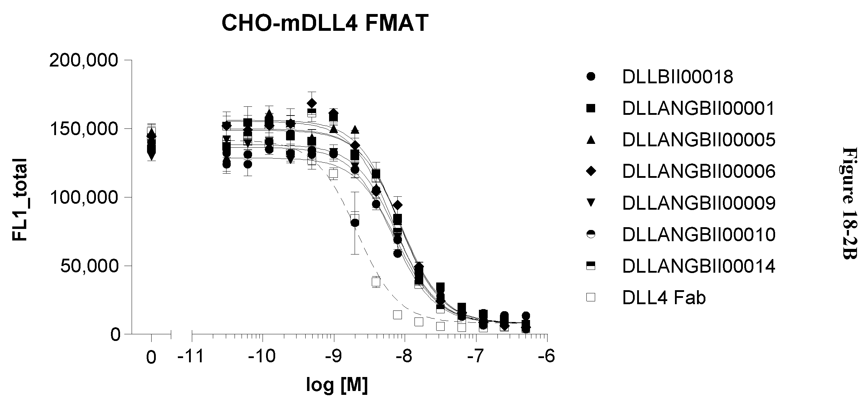

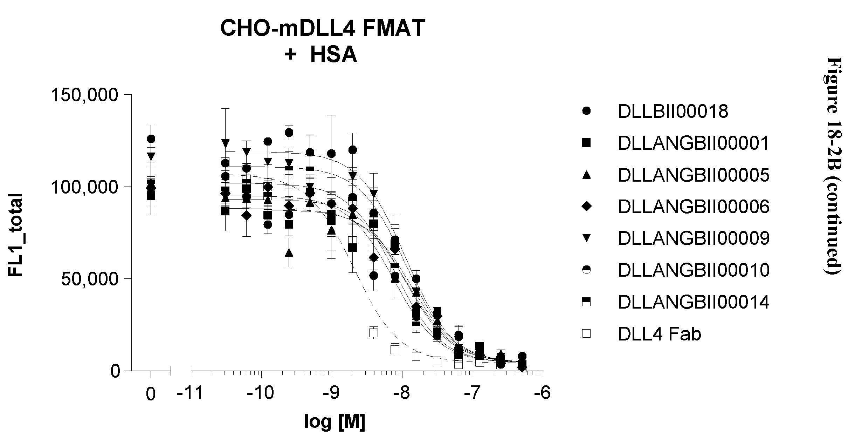

To explore the anti-DLL4 blocking properties in comparison with the monovalent building block DLLBII00018, all purified bispecific VHHs are analyzed in the hDLL4/hNotch1 competition ELISA (see Example 5.1 as described in patent US 2011/0172398 A1) (Figure 17) and the CHO-hDLL4 / CHO-mDLL4 competition FMAT (see Example 5.3 as described in patent US 2011/0172398 A1) (Figure 18). Here, the ELISA competition assay is performed with a fixed concentration of 8 nM biotinylated hDLL4. Both ELISA and the FMAT competition assay are also performed after preincubation of the VHH with 12.5 µM and 25 µM human serum albumin, respectively. A summary of IC₅₀ values and % inhibition is shown in Table 23.

**Table 23: IC₅₀ values (nM) and % inhibition in hDLL4/hNotch1 competition ELISA and CHO-hDLL4 and CHO-mDLL4 competition FMAT.**

| | | | hDLL4 ELISA | | CHO-hDLL4 FMAT | | CHO-mDLL4 FMAT | |
|---|---|---|---|---|---|---|---|---|
| VHH ID | Format | HSA | IC₅₀ (nM) | % inh | IC₅₀ (nM) | % inh | IC₅₀ (nM) | % inh |
| DLLBII00018 | 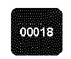 | - | 4.4 | 85 | 6.3 | 66 | 4.5 | 93 |
| | | + | n.d. | n.d. | 4.2 | 67 | 4.3 | 95 |
| DLLANGBII00001 | 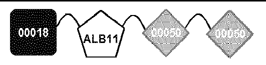 | - | 4.8 | 91 | 8.1 | 76 | 6.5 | 95 |
| | | + | 5.4 | 94 | 17.7 | 87 | 10.0 | 97 |
| DLLANGBII00002 | 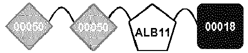 | - | n.d. | n.d. | n.d. | n.d. | n.d. | n.d. |
| | | + | n.d. | n.d. | n.d. | n.d. | n.d. | n.d. |
| DLLANGBII00009 | 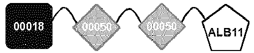 | - | 4.9 | 92 | 10.5 | 80 | 7. | 96 |
| | | + | 5.5 | 99 | 14.0 | 91 | 12.1 | 97 |
| DLLANGBII00010 | 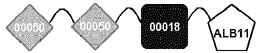 | - | 5.2 | 95 | 14.4 | 96 | 7.4 | 98 |
| | | + | 10.0 | 100 | 26.2 | 99 | 11.3 | 98 |
| DLL4 Fab | | - | 3.9 | 97 | 3.9 | 85 | 1.8 | 99 |
| | | + | n.d. | n.d. | 3.2 | 85 | 1.5 | 97 |
| DLLBII00018 | 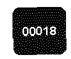 | - | 2.6 | 84 | 5.2 | 65 | 3.8 | 96 |
| | | + | n.d. | n.d. | 3.5 | 70 | 3.7 | 98 |
| DLLANGBII00003 | 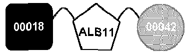 | - | 2.1 | 87 | 6.2 | 70 | 5.3 | 99 |
| | | + | 2.6 | 92 | 11.4 | 90 | 6.7 | 101 |
| DLLANGBII00004 | 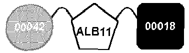 | - | 3.1 | 92 | 8.1 | 81 | 5.1 | 100 |
| | | + | 3.2 | 97 | 12.1 | 98 | 6.0 | 101 |
| DLLANGBII00005 | 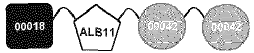 | - | 2.4 | 87 | 7.3 | 74 | 6.4 | 94 |
| | | + | 3.0 | 83 | 13.1 | 87 | 8.9 | 99 |
| DLLANGBII00006 | 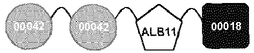 | - | 2.7 | 93 | 8.5 | 88 | 6.8 | 97 |
| | | + | 3.1 | 94 | 15.4 | 99 | 8.8 | 100 |
| DLLANGBII00011 | 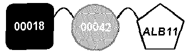 | - | 2.5 | 87 | 9.0 | 71 | 6.0 | 100 |
| | | + | 3.2 | 93 | 9.1 | 87 | 6.3 | 101 |
| DLLANGBII00012 | 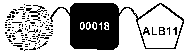 | - | 2.3 | 89 | 8.3 | 82 | 4.1 | 99 |
| | | + | 2.5 | 95 | 10.4 | 99 | 5.8 | 101 |
| DLLANGBII00013 | 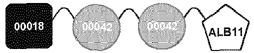 | - | 2.2 | 88 | n.d. | n.d. | n.d. | n.d. |
| | | + | 2.3 | 89 | n.d. | n.d. | n.d. | n.d. |
| DLLANGBII00014 | 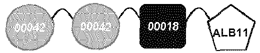 | - | 2.6 | 92 | 6.4 | 86 | 5.8 | 98 |
| | | + | 4.4 | 93 | 12.7 | 93 | 7.6 | 100 |
| DLL4 Fab | | - | 3.4 | 93 | 3.6 | 85 | 1.2 | 99 |
| | | + | n.d. | n.d. | 3.1 | 88 | 1.2 | 99 |
| DLLBII00018 | 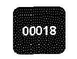 | - | 3.5 | 82 | 4.2 | 64 | 3.3 | 99 |
| | | + | n.d. | n.d. | 3.0 | 73 | 3.1 | 101 |
| DLLANGBII00007 | 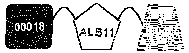 | - | 2.4 | 86 | 7.1 | 73 | 4.9 | 99 |
| | | + | 5.2 | 97 | 12.6 | 90 | 5.9 | 101 |
| DLLANGBII00008 | 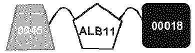 | | n.d. | n.d. | n.d. | n.d. | n.d. | n.d. |
| | | + | n.d. | n.d. | n.d. | n.d. | n.d. | n.d. |
| DLLANGBII00015 | 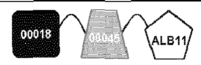 | - | 2.8 | 88 | 6.7 | 73 | 3.0 | 99 |
| | | + | 3.2 | 95 | 9.4 | 91 | 5.1 | 101 |
| DLLANGBII00016 | 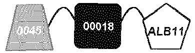 | - | n.d. | n.d. | n.d. | n.d. | n.d. | n.d. |
| | | + | n.d. | n.d | n.d. | n.d | n.d. | n.d. |
| DLL4 Fab | | - | 3.1 | 95 | 3.3 | 84 | 0.8 | 99 |
| | | + | n.d. | n.d. | 3.0 | 91 | 1.0 | 101 |

| | | | | | | | | |
|---|---|---|---|---|---|---|---|---|
| n.d., not determined | | | | | | | | |

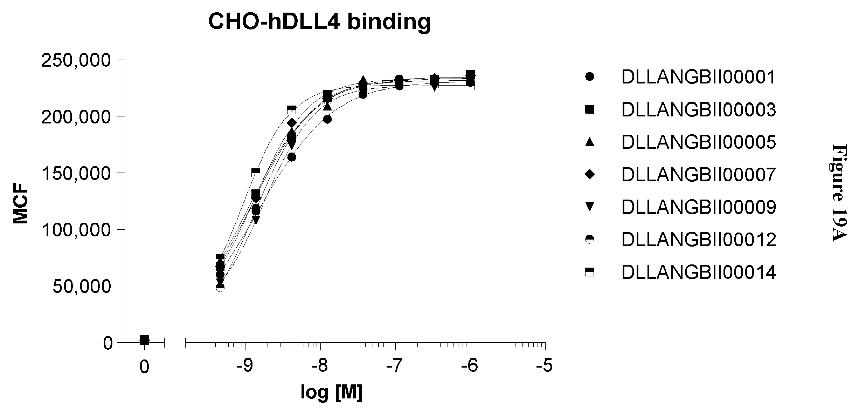

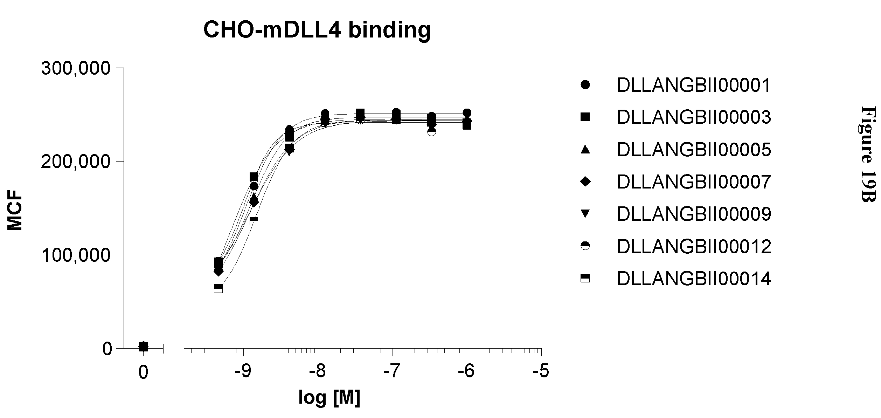

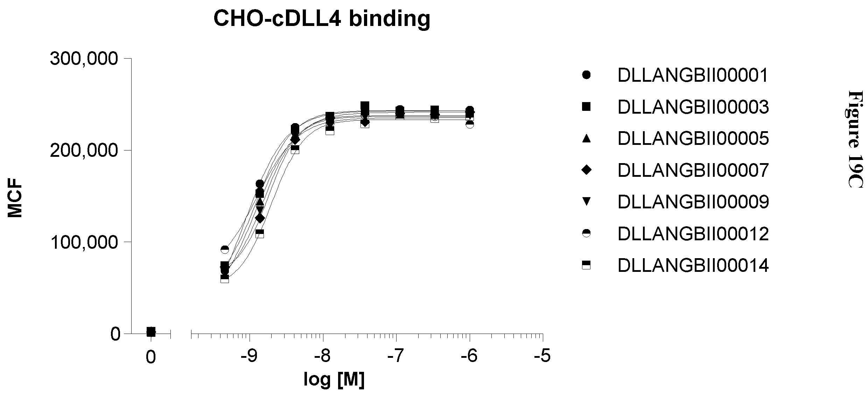

Additionally, in order to determine cross-reactivity of the bispecific VHHs to murine and cynomolgus DLL4, a FACS binding experiment is performed. Briefly, CHO cells overexpressing mouse and cynomolgus DLL4 are used for a titration binding experiment of the VHHs. After a 30 min incubation on ice, all samples are washed and a 2-step detection using anti-c-myc followed by goat-anti mouse IgG-PE labeled is performed. CHO cells overexpressing human DLL4 are taken as reference. The mean MCF value is determined using a FACS Array and used for calculation of the EC₅₀ value (Table 24; Figure 19).

**Table 24: EC₅₀ values of bispecific VHHs binding to human, mouse and cyno DLL4 overexpressed on CHO cells (FACS)**

| | CHO-hDLL4 EC₅₀ (nM) | CHO-mDLL4 EC₅₀ (nM) | CHO-cDLL4 EC₅₀ (nM) |
|---|---|---|---|
| DLLBII00018 | 1.5 | 1.2 | 0.8 |
| DLLANGBII00001 | 1.2 | 0.9 | 0.8 |
| DLLANGBII00003 | 1.1 | 0.9 | 0.7 |
| DLLANGBII00005 | 1.1 | 1.1 | 0.8 |
| DLLANGBII00007 | 1.5 | 1.2 | 0.9 |
| DLLANGBII00009 | 1.4 | 1.1 | 0.8 |
| DLLANGBII00012 | 0.8 | 0.8 | 0.7 |
| DLLANGBII00014 | 1.8 | 1.4 | 1.2 |
| DLL4Fab | 7.5 | 2.3 | 1.1 |

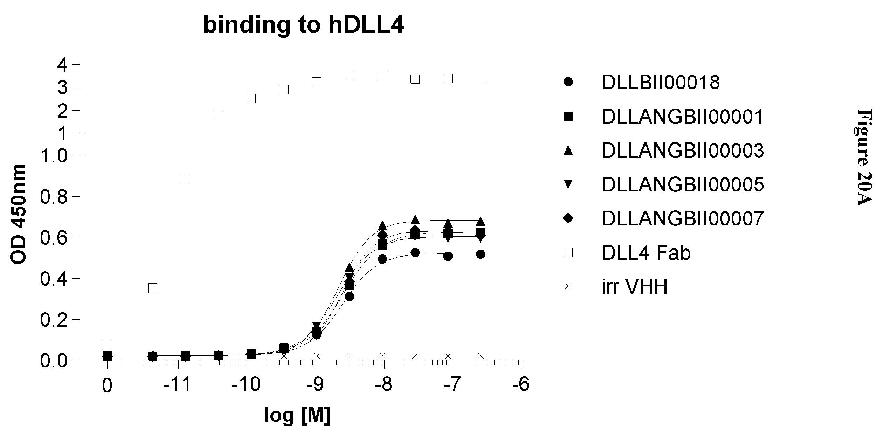

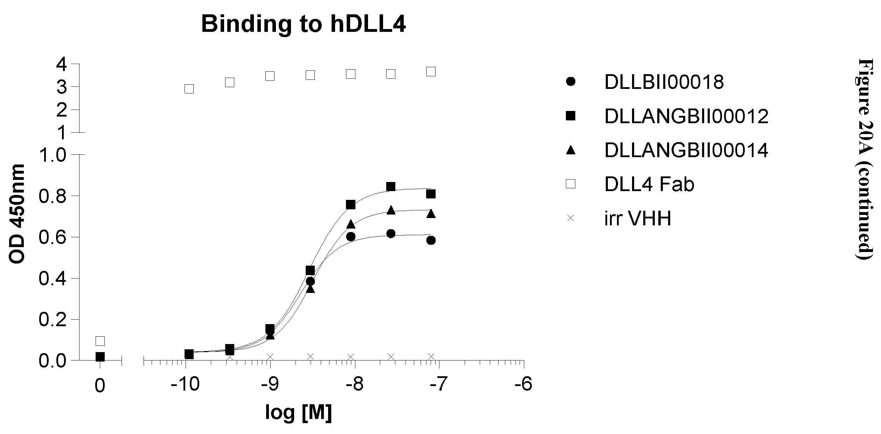

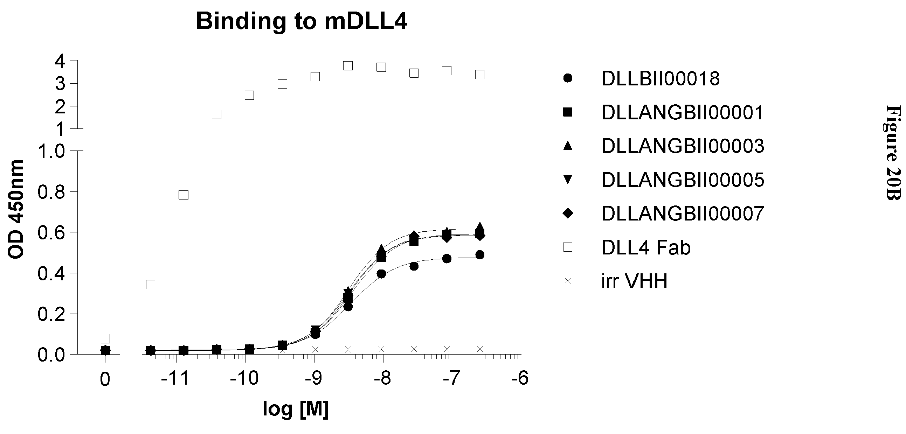

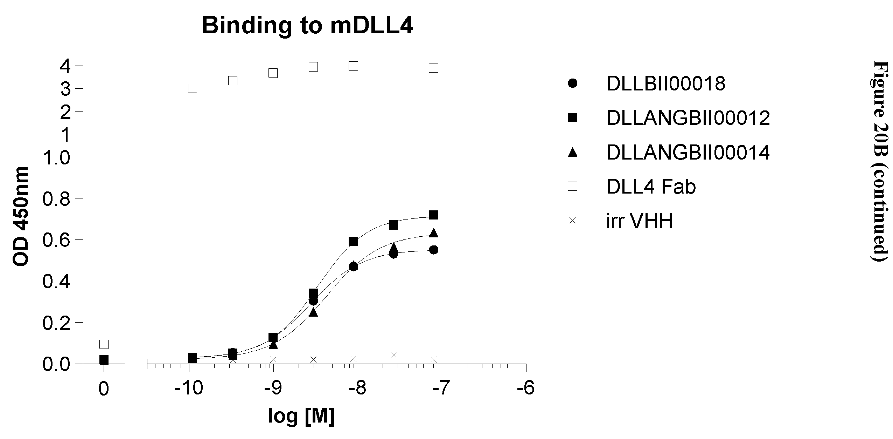

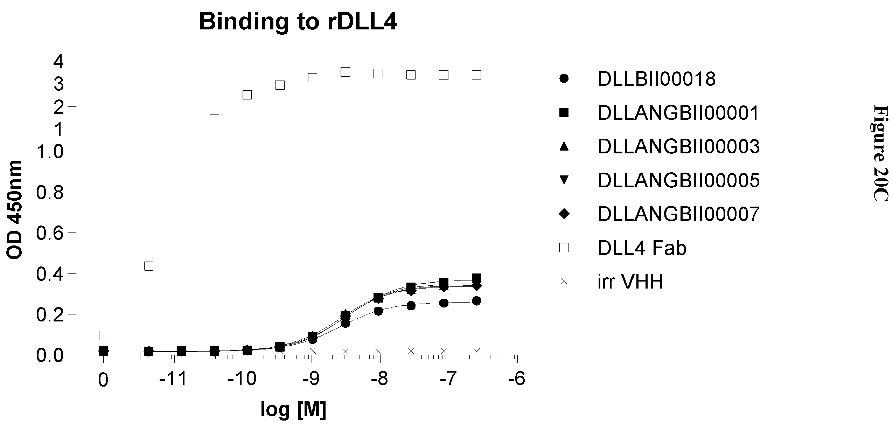

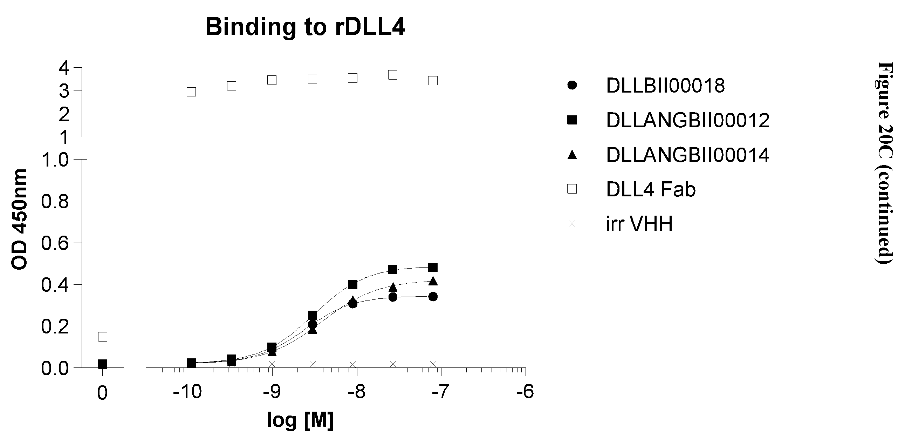

In order to determine cross-reactivity to mouse DLL4 and rat DLL4, a binding ELISA is performed. In brief, recombinant mouse DLL4 (R&D Systems, Minneapolis, MI, USA) and rat DLL4 is coated overnight at 4°C in a 96-well MaxiSorp plate (Nunc, Wiesbaden, Germany). Wells are blocked with a 1% casein solution. VHHs are applied as dilution series and binding is detected biotinylated anti-VHH 1A4 followed by extravidin-HRP. 1A4 is an anti-VHH VHH (generetad in-house by Ablynx NV). As reference binding to human DLL4 is measured. EC₅₀ values are summarized in Table 25 and Figure 20.

**Table 25: EC₅₀ values of bispecific VHHs binding to human, mouse and rat DLL4 (ELISA)**

| | hDLL4 EC₅₀ (nM) | mDLL4 EC₅₀ (nM) | rDLL4 EC₅₀ (nM) |
|---|---|---|---|
| DLLBII00018 | 2.5 | 3.3 | 2.6 |
| DLLANGBII00001 | 2.5 | 3.6 | 3.4 |
| DLLANGBII00003 | 2.1 | 3.2 | 2.7 |
| DLLANGBII00005 | 2.0 | 3.1 | 2.9 |
| DLLANGBII00007 | 2.4 | 3.3 | 2.8 |
| DLLANGBII00012 | 2.9 | 3.3 | 3.1 |
| DLLANGBII00014 | 3.2 | 4.2 | 3.8 |

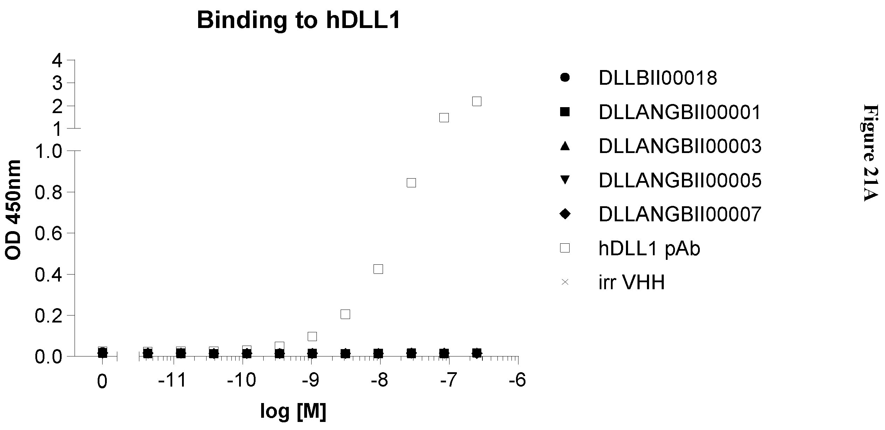

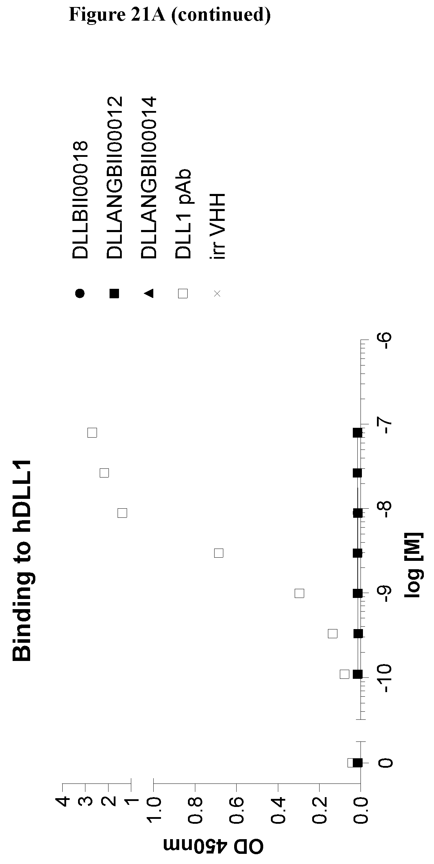

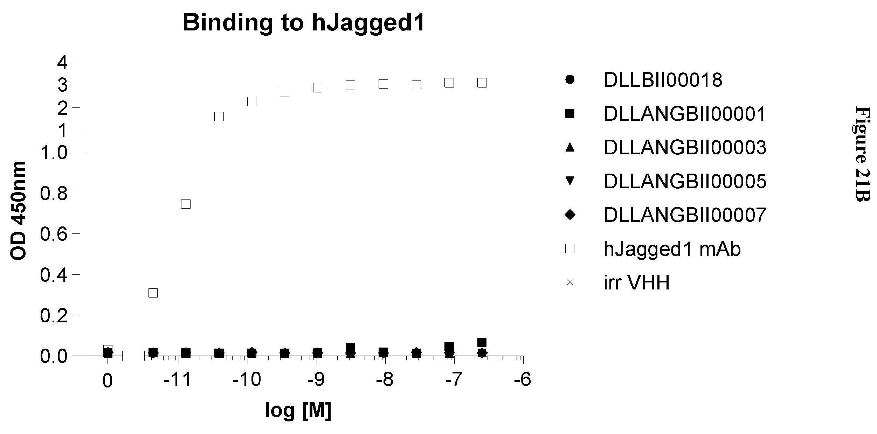

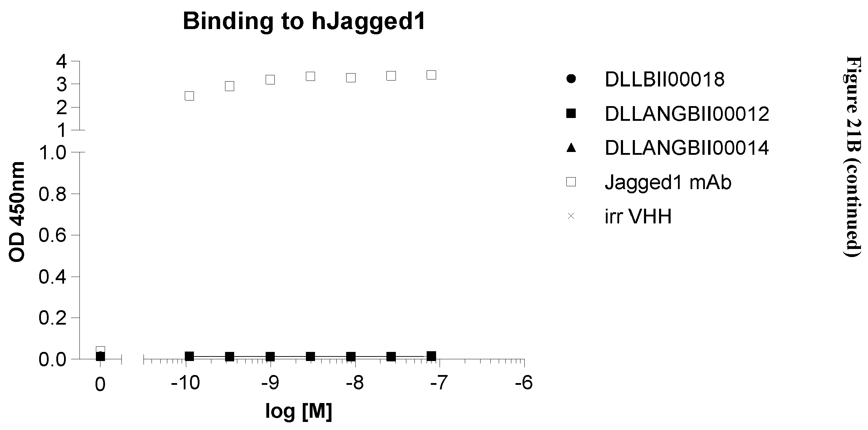

Absence of binding to the homologous human ligands DLL1 and Jagged-1 is assessed via a solid phase binding assay (ELISA). In brief, 1 µg/mL of recombinant human DLL1 (Alexis, San Diego, CA, USA) or recombinant human Jagged-1 (Alexis, San Diego, CA, USA) is coated overnight at 4°C in a 96-well MaxiSorp plate (Nunc, Wiesbaden, Germany). Wells are blocked with a 1% casein solution. VHHs are applied as dilution series and binding is detected biotinylated anti-VHH 1A4 followed by extravidin-HRP. All bispecific VHH are considered as being non-cross reactive to these homologous ligands. Results are shown in Figure 21.

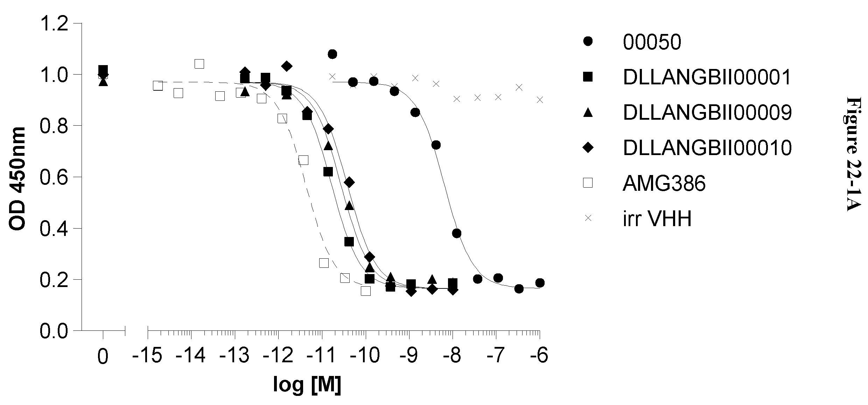

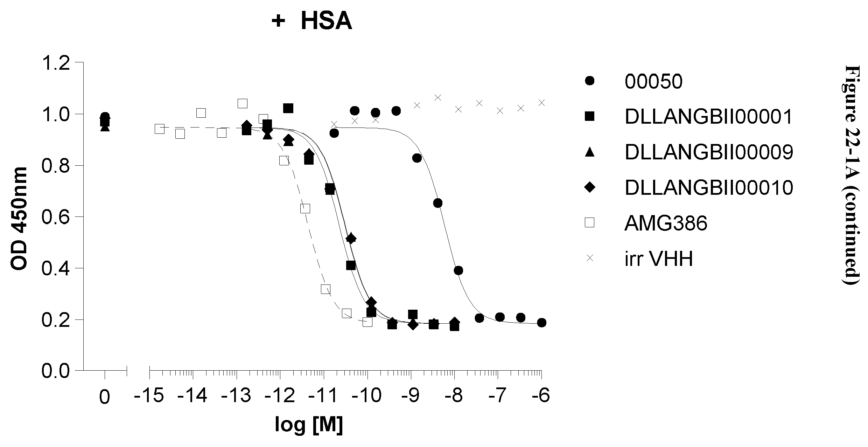

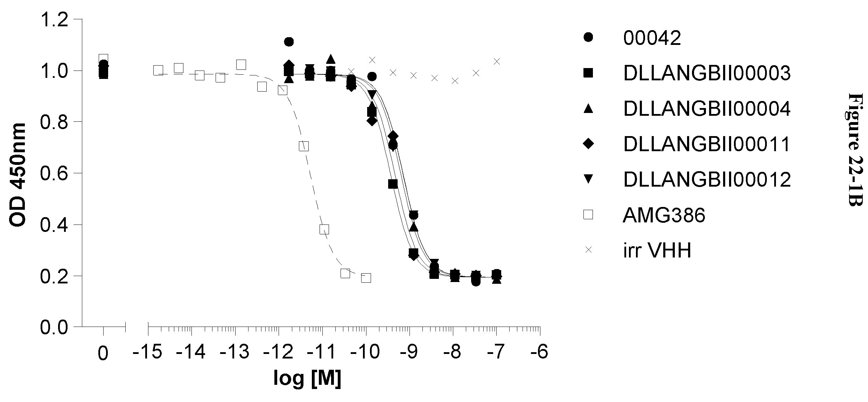

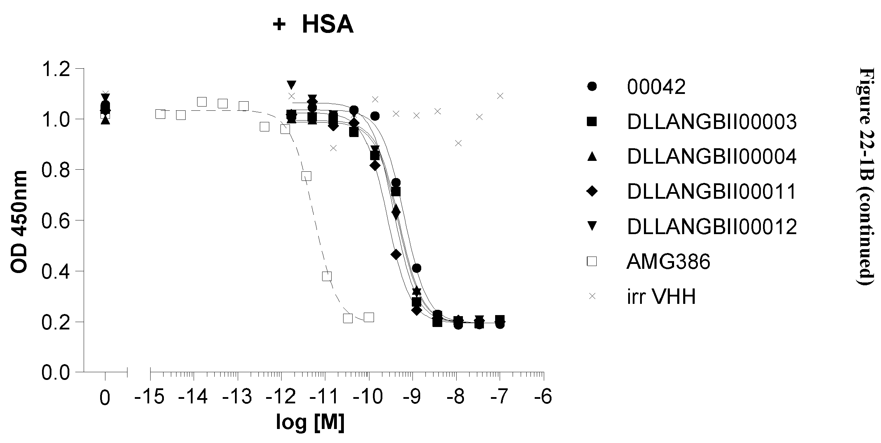

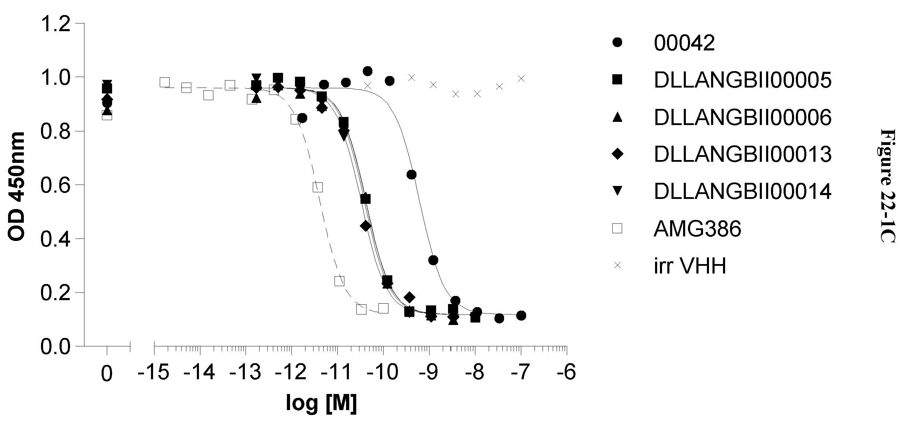

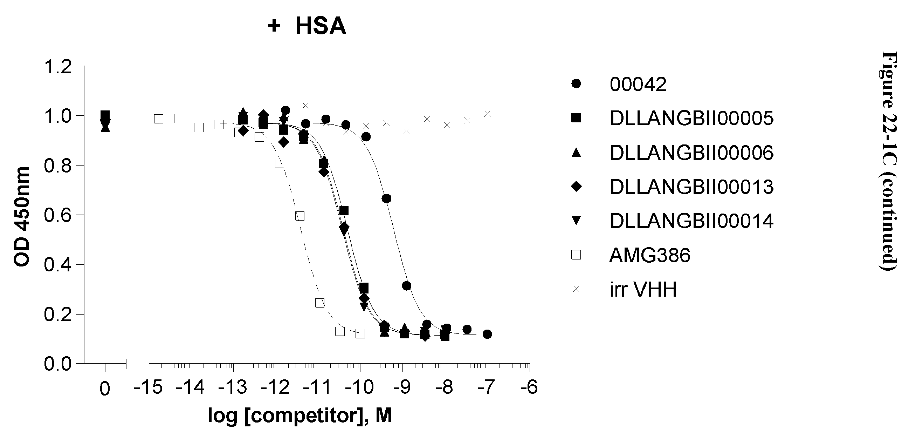

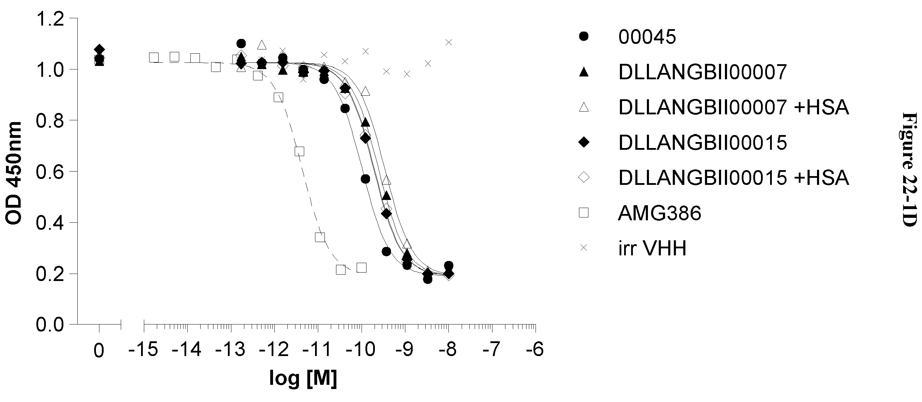

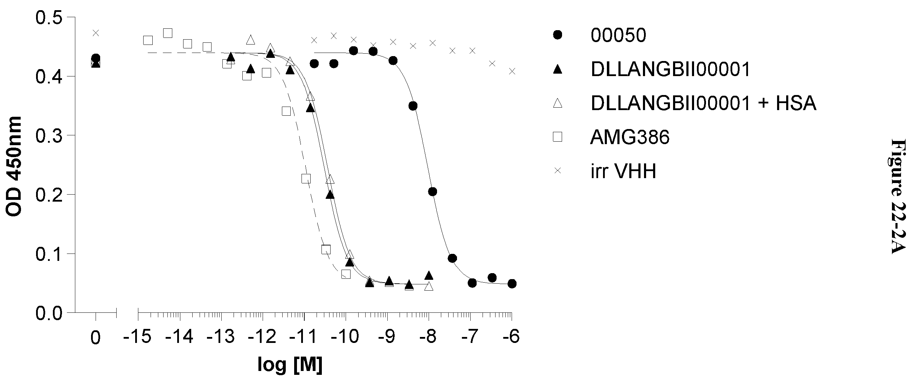

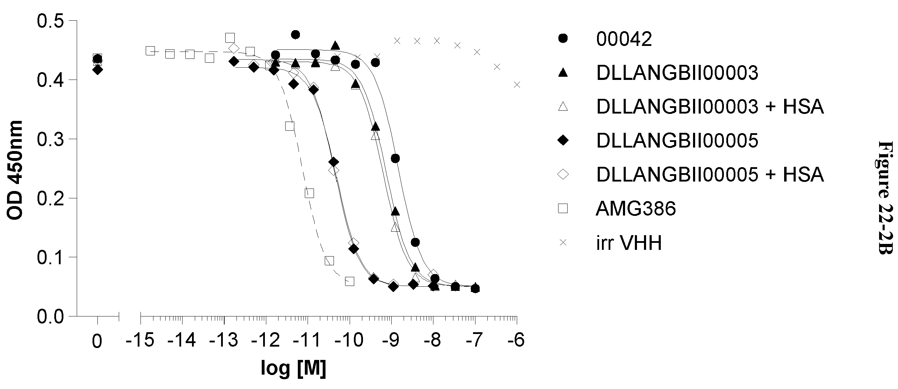

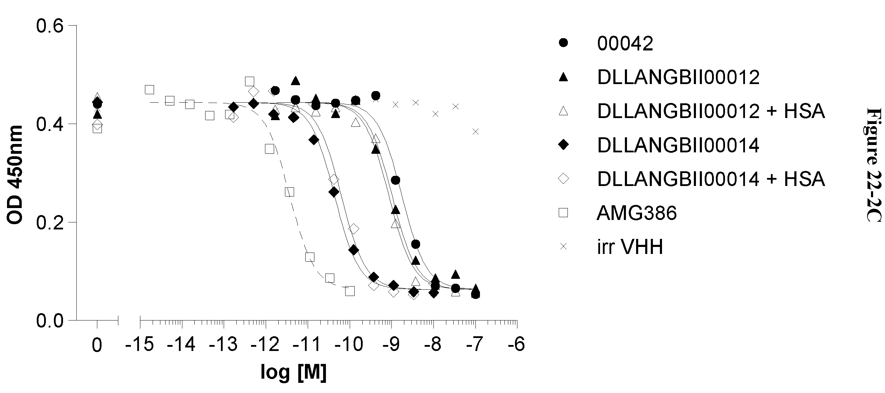

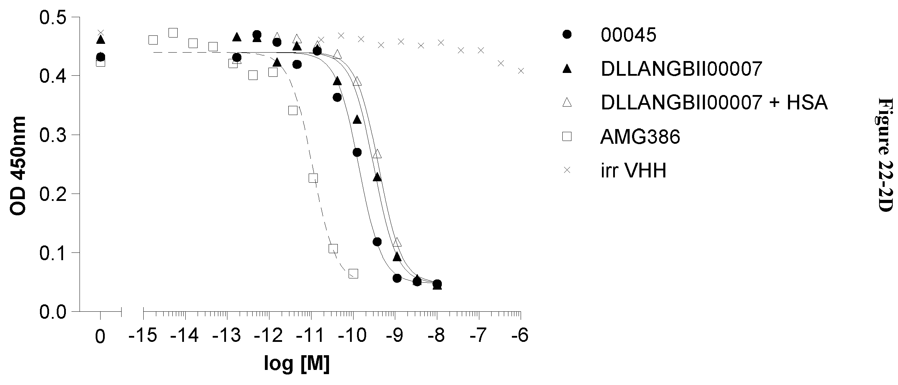

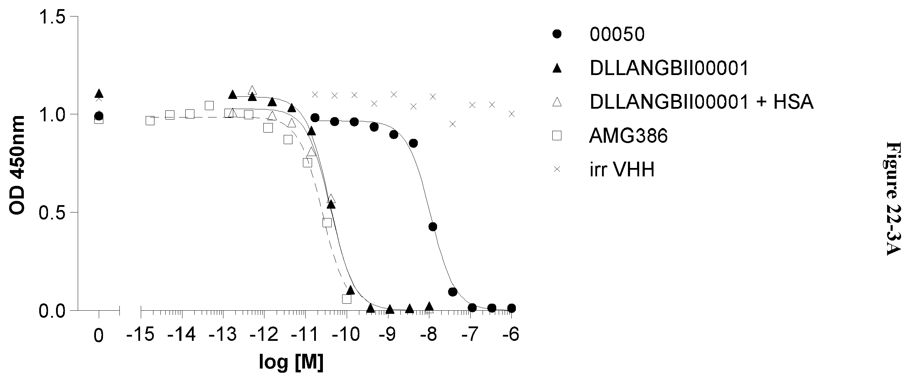

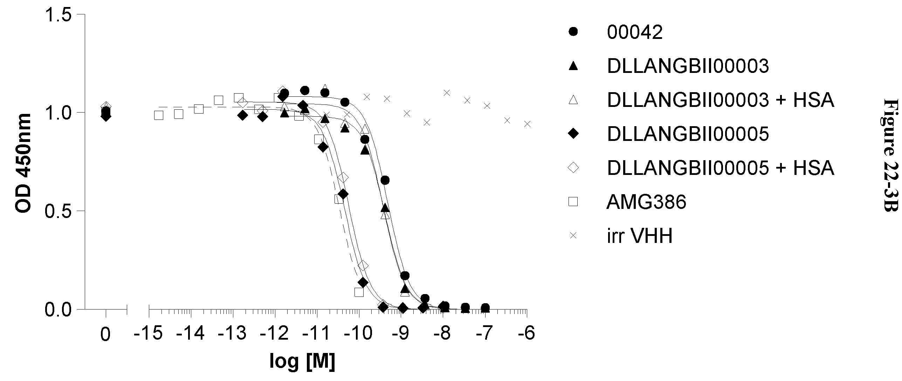

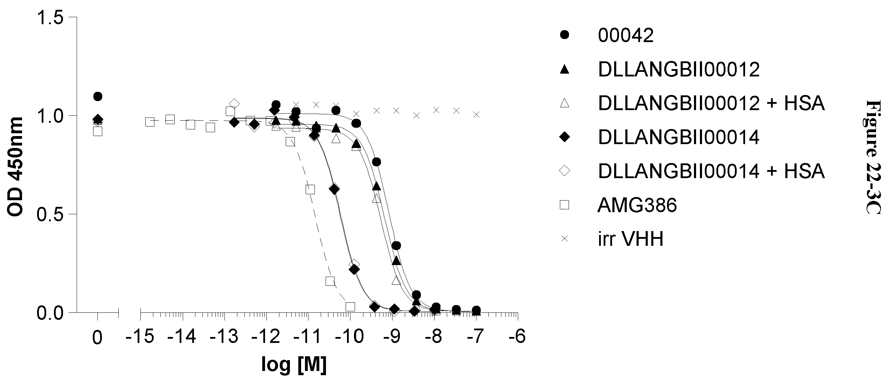

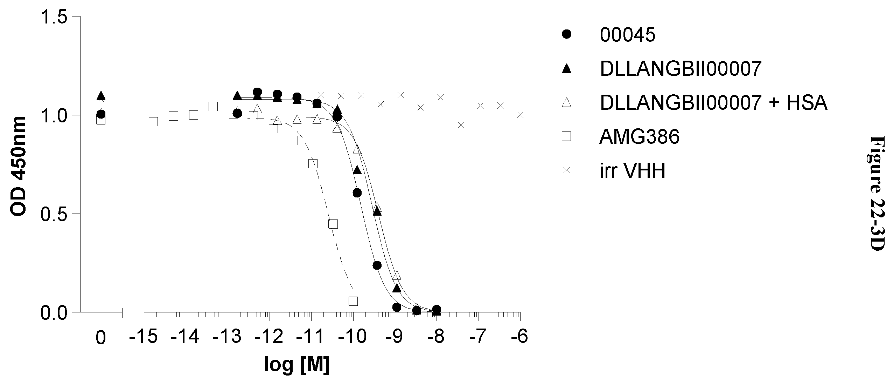

To explore the anti-Ang2 blocking properties in comparison with the monovalent anti-Ang2 building blocks 00042, 00045 and 00050, all purified bispecific VHHs are analyzed in a human Ang2/hTie2-Fc (Figure 22-1), mouse Ang2/mTie2 (Figure 22-2) and cyno Ang2/cTie2 (Figure 22-3) competition ELISA. This assay is also performed after incubation of the VHH with 0.5 µM human serum albumin. A summary of IC₅₀ and % inhibition values is shown in Table 26.

**Table 26: IC₅₀ values (pM) and % inhibition in human, mouse and cyno Ang2/Tie2 competition ELISA**

| | | | hAng2 | | mAng2 | | cAng2 | |
|---|---|---|---|---|---|---|---|---|
| VHH ID | Format | HSA | IC₅₀ (pM) | % inh | IC50 (pM) | % inh | IC₅₀ (pM) | % inh |
| 00050 | 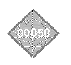 | - | 7,063 | 100 | 10,556 | 100 | 11,363 | 100 |
| | | + | 6,569 | 100 | n.d. | n.d. | n.d. | n.d. |
| DLLANGBII00001 | 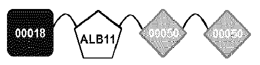 | - | 18 | 100 | 34 | 100 | 40 | 100 |
| | | + | 21 | 100 | 38 | 100 | 41 | 100 |
| DLLANGBII00002 | 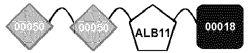 | - | n.d. | n.d. | n.d. | n.d. | n.d. | n.d. |
| | | + | n.d. | n.d. | n.d. | n.d. | n.d. | n.d. |
| DLLANGBII00009 | 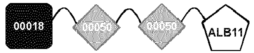 | - | 24 | 100 | n.d. | n.d. | n.d. | n.d. |
| | | + | 29 | 100 | n.d. | n.d. | n.d. | n.d. |
| DLLANGBII00010 | 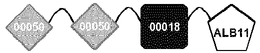 | - | 38 | 100 | n.d. | n.d. | n.d. | n.d. |
| | | + | 33 | 100 | n.d. | n.d. | n.d. | n.d. |
| AMG386 | | - | 4 | 100 | 8 | 100 | 21 | 100 |
| | | + | 4 | 100 | n.d. | n.d. | n.d. | n.d. |
| 00042 | 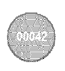 | - | 684 | 100 | 1619 | 100 | 733 | 100 |
| | | + | 589 | 100 | n.d. | n.d. | n.d. | n.d. |
| DLLANGBII00003 | 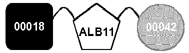 | - | 349 | 100 | 894 | 100 | 472 | 100 |
| | | + | 406 | 100 | 668 | 100 | 407 | 100 |
| DLLANGBII00004 | 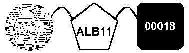 | - | 590 | 100 | n.d. | n.d. | n.d. | n.d. |
| | | + | 501 | 100 | n.d. | n.d. | n.d. | n.d. |
| DLLANGBII00005 | 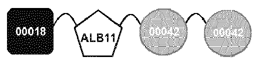 | - | 36 | 100 | 47 | 100 | 46 | 100 |
| | | + | 50 | 100 | 54 | 100 | 58 | 100 |
| DLLANGBII00006 | 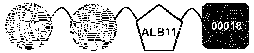 | - | 34 | 100 | n.d. | n.d. | n.d. | n.d. |
| | | + | 42 | 100 | n.d. | n.d. | n.d. | n.d. |
| DLLANGBII00011 | 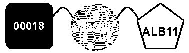 | - | 342 | 100 | n.d. | n.d. | n.d. | n.d. |
| | | + | 227 | 100 | n.d. | n.d. | n.d. | n.d. |
| DLLANGBII00012 | 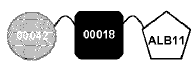 | - | 609 | 100 | 955 | 100 | 596 | 100 |
| | | + | 364 | 100 | 702 | 100 | 430 | 100 |
| DLLANGBII00013 | 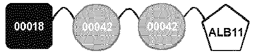 | - | 30 | 100 | n.d. | n.d. | n.d. | n.d. |
| | | + | 37 | 100 | n.d. | n.d. | n.d. | n.d. |
| DLLANGBII00014 | 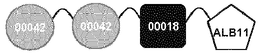 | - | 33 | 100 | 37 | 100 | 42 | 100 |
| | | + | 33 | 100 | 50 | 100 | 46 | 100 |
| AMG386 | | - | 4 | 100 | 5 | 100 | 17 | 100 |
| | | + | 4 | 100 | n.d. | n.d. | n.d. | n.d. |
| 00045 | 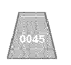 | - | 106 | 100 | 146 | 100 | 164 | 100 |
| | | + | n.d. | n.d. | n.d. | n.d. | n.d. | n.d. |
| DLLANGBII00007 | 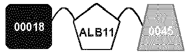 | - | 242 | 100 | 364 | 100 | 312 | 100 |
| | | + | 337 | 100 | 428 | 100 | 452 | 100 |
| DLLANGBII00008 | 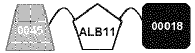 | - | n.d. | n.d. | n.d. | n.d. | n.d. | n.d. |
| | | + | n.d. | n.d. | n.d. | n.d. | n.d. | n.d. |
| DLLANGBII00015 | 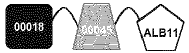 | - | 201 | 100 | n.d. | n.d. | n.d. | n.d. |
| | | + | 207 | 100 | n.d. | n.d. | n.d. | n.d. |
| DLLANGBII00016 | 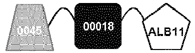 | - | n.d. | n.d. | n.d. | n.d. | n.d. | n.d. |
| | | + | n.d. | n.d. | n.d. | n.d. | n.d. | n.d. |
| AMG386 | | - | 4 | 100 | 8 | 100 | 21 | 100 |
| | | + | n.d. | n.d. | n.d. | n.d. | n.d. | n.d. |

| | | | | | | | | |
|---|---|---|---|---|---|---|---|---|
| n.d., not determined | | | | | | | | |

Affinities of certain DLL4-Ang2 bispecific VHHs for human serum albumin have been determined (see Example 5) and are shown in Table 27. The affinity constant K_{D} is calculated from resulting association and dissociation rate constants kₐ and k_{d}.

**Table 27: Affinity K_{D} of purified VHHs for human serum albumin (HSA)**

| | | kₐ (1/Ms) | k_{d} (1/s) | K_{D} (nM) |
|---|---|---|---|---|
| ALB11 | 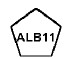 | 5.2E+05 | 1.8E-03 | 4 |
| DLLANGBII00001 | 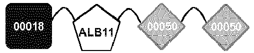 | 7.8E+04 | 4.9E-03 | 63 |
| DLLANGBII00003 | 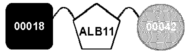 | 1.2E+05 | 4.7E-03 | 39 |
| DLLANGBII00005 | 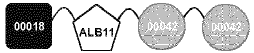 | 7.5E+04 | 4.6E-03 | 61 |
| DLLANGBII00007 | 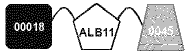 | 1.1E+05 | 4.6E-03 | 42 |
| DLLANGBII00012 | 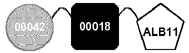 | 8.4E+04 | 5.5E-03 | 66 |
| DLLANGBII00014 | 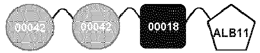 | 5.7E+04 | 5.8E-03 | 102 |

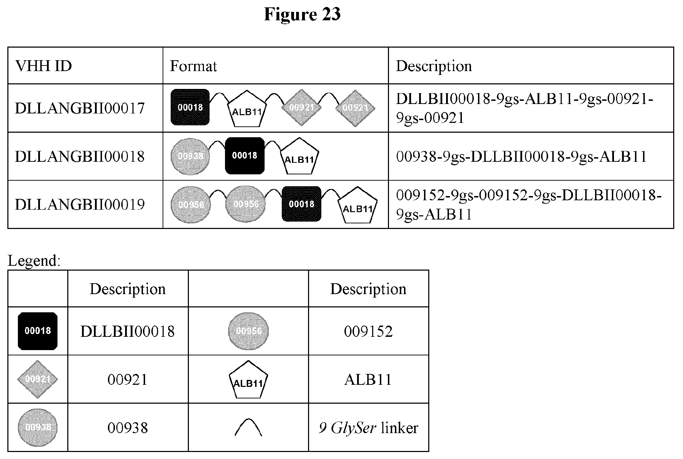

In a second cycle, the anti-DLL4 VHH DLLBII00018 (US 2011/0172398 A1), having the sequence as shown at the beginning of Example 8, and the final sequence optimized anti-Ang2 VHHs 00921 (SEQ ID NO: 485), 00938 (SEQ ID NO:486) and 00956 (SEQ ID NO:488) are used as building blocks to generate bispecific VHHs DLLANGBII00017-00019. A genetic fusion to a serum albumin binding VHH is used as half-life extension methodology. Building blocks are linked via a 9 Gly-Ser flexible linker. An overview of the format and sequence of all bispecific VHHs is depicted in Figure 23 and Table 28 (linker sequences underlined), SEQ ID Nos 476-478.
DLLANGBII00018 (SEQ ID NO: 477) is an embodiment of the invention.

**Table 28**

| Sequences of bispecific VHH targeting DLL4 and Ang2 | |
|---|---|
| VHH ID | AA sequence |
| DLLANGBII00017 | |
| DLLANGBII00018 | |
| DLLANGBII00019 | |

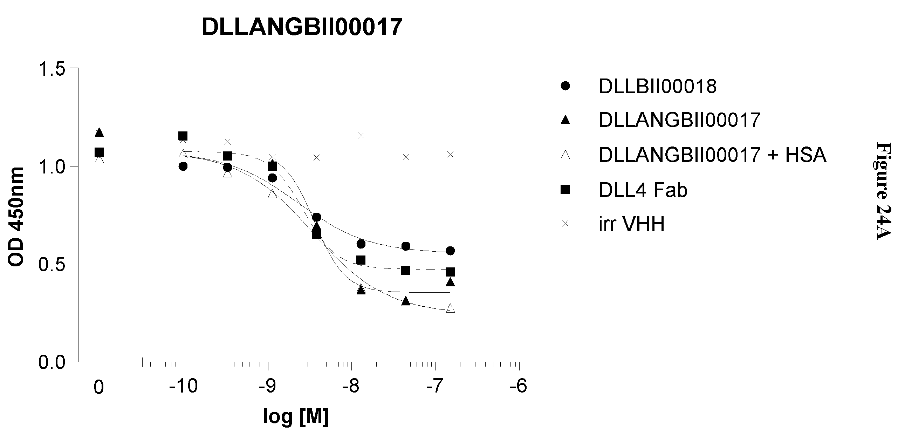

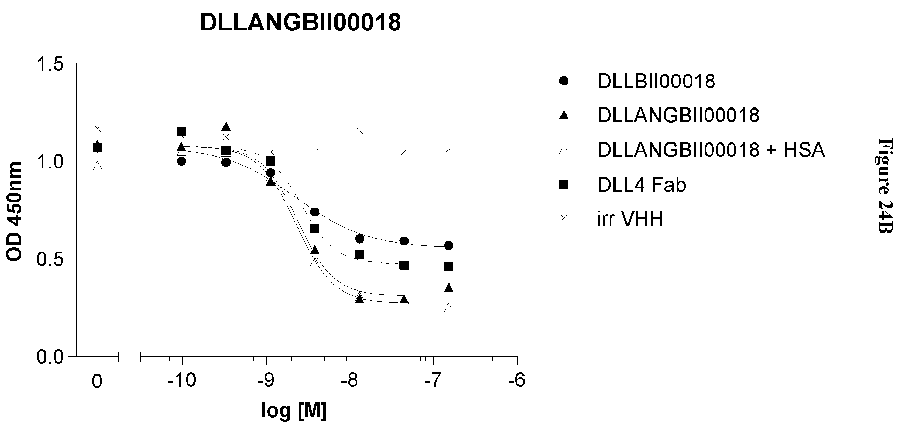

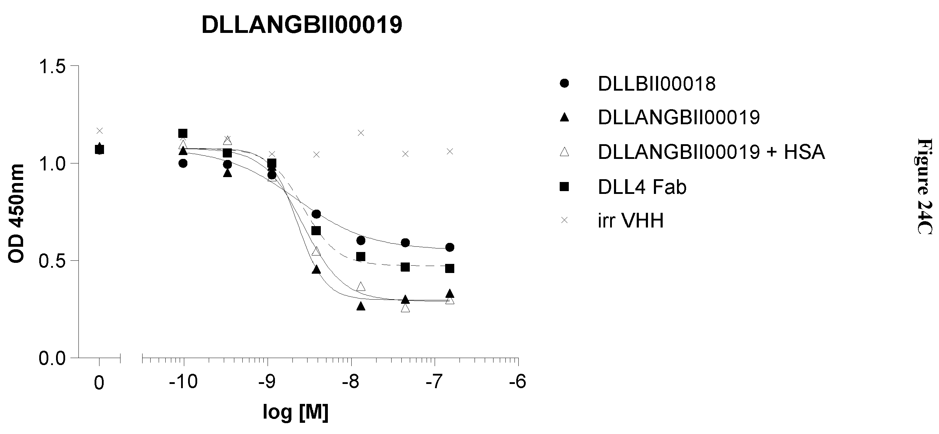

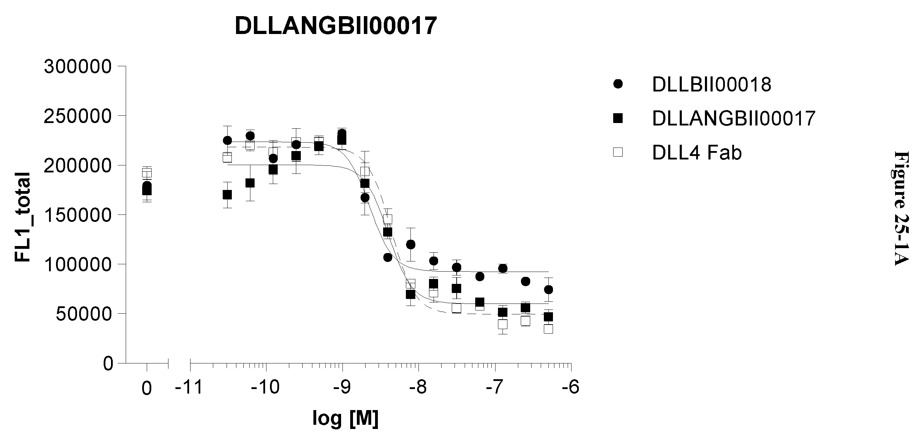

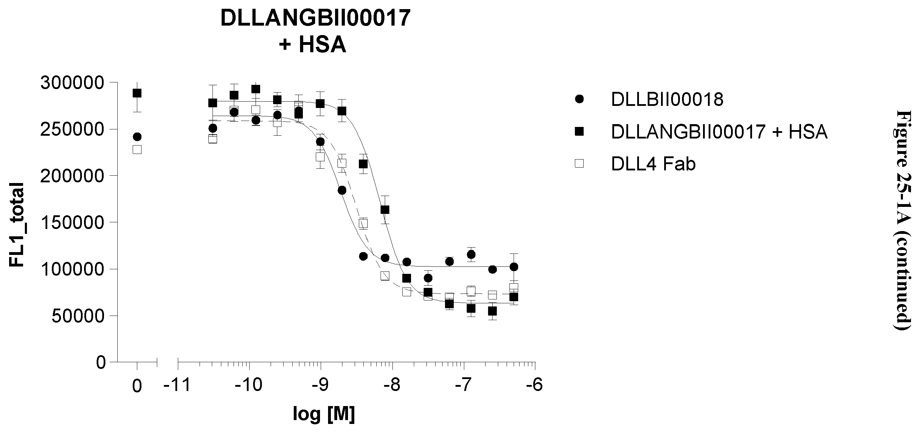

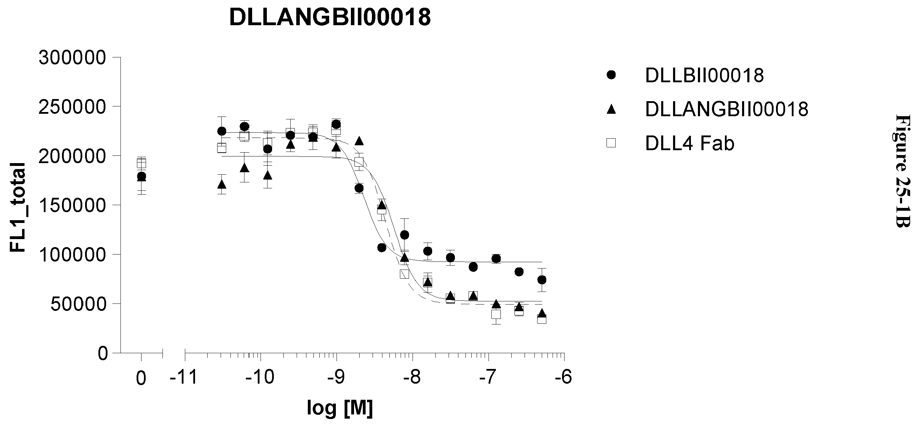

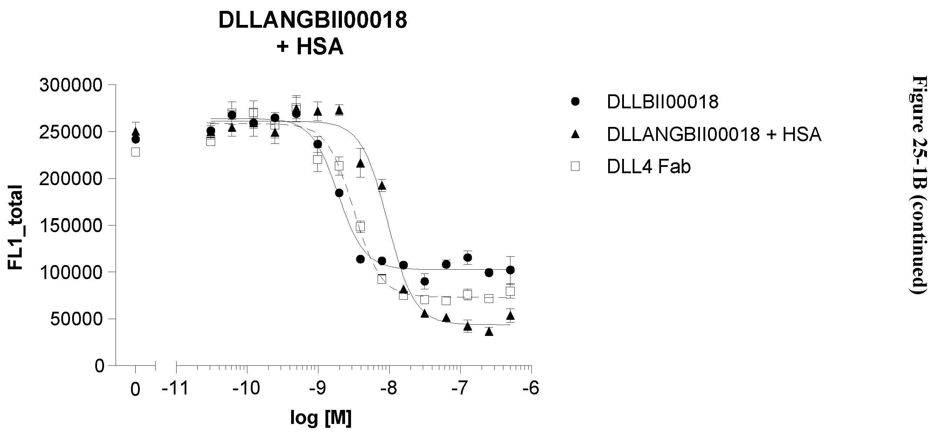

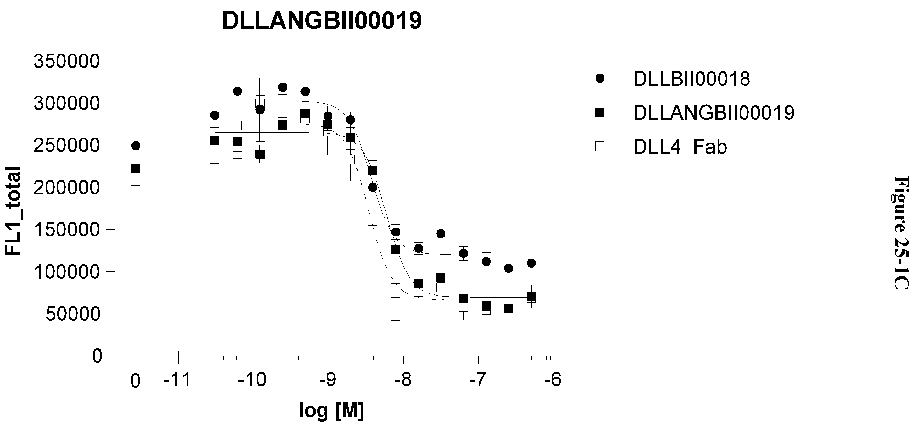

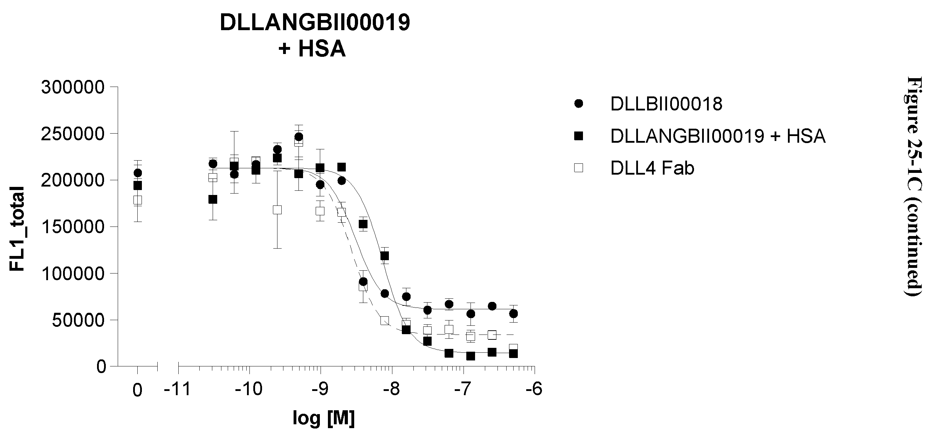

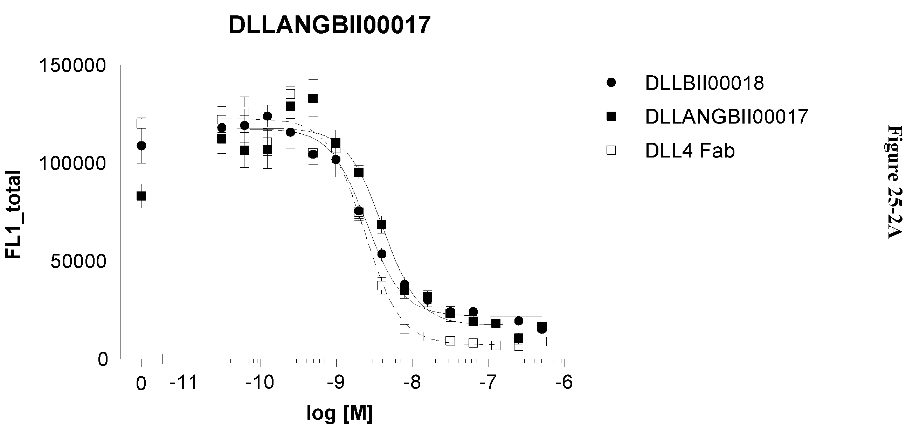

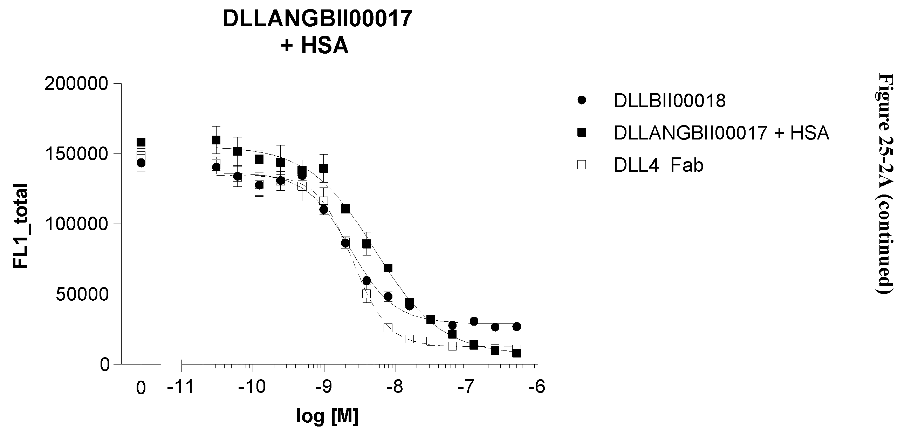

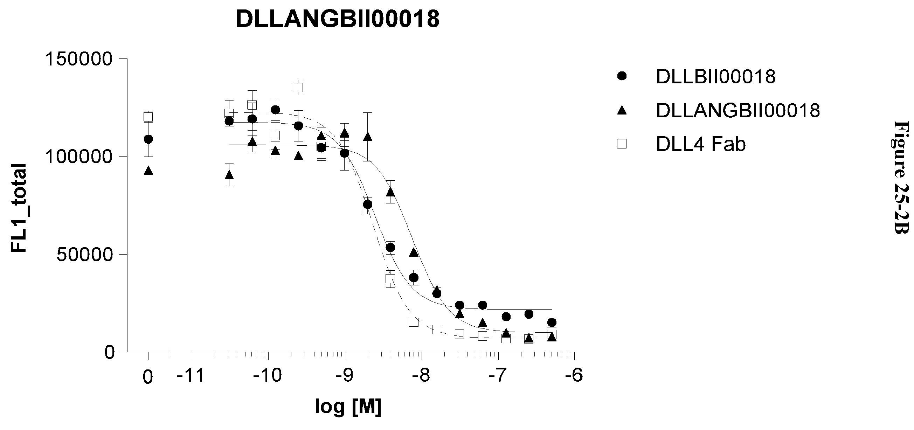

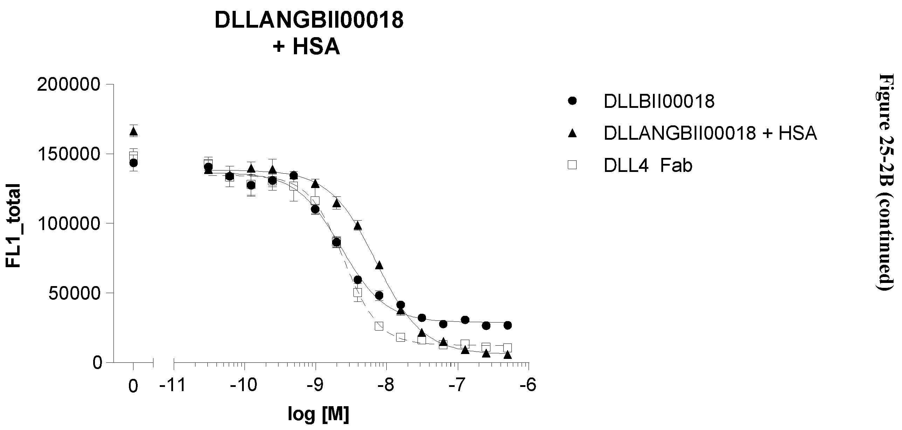

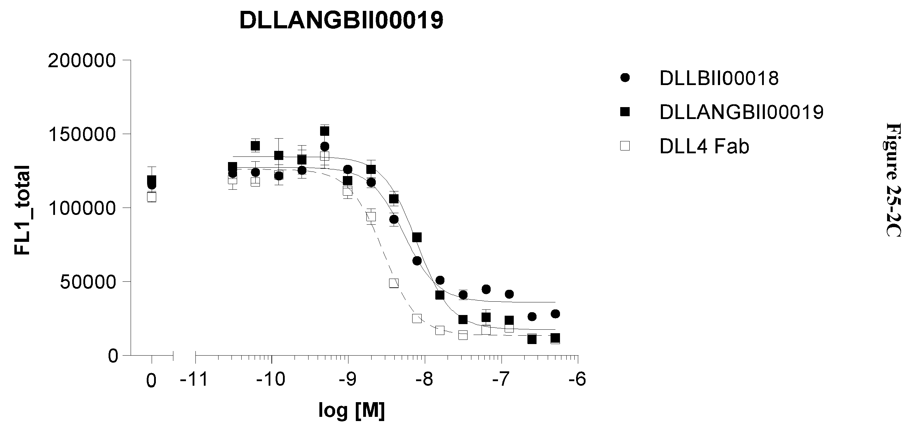

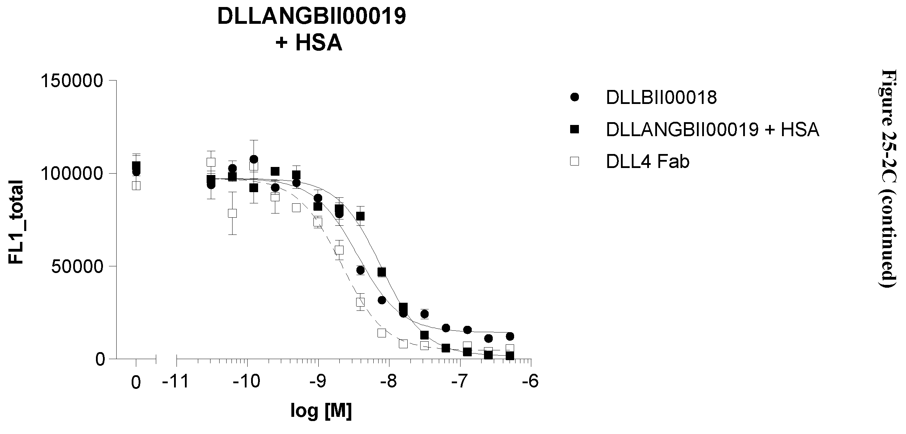

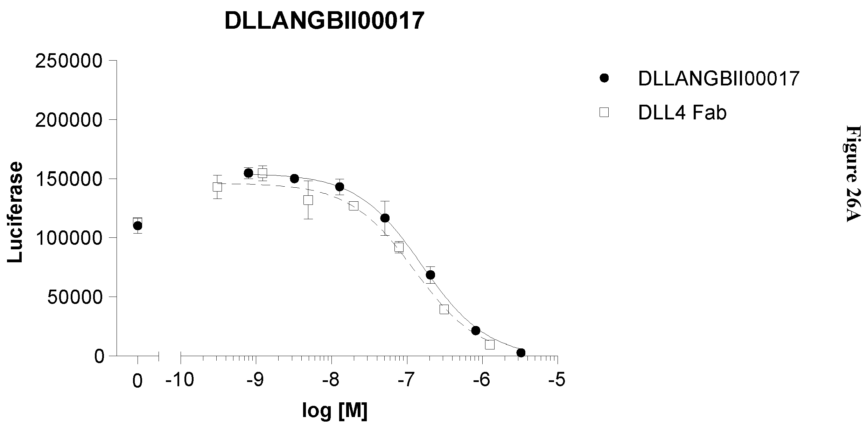

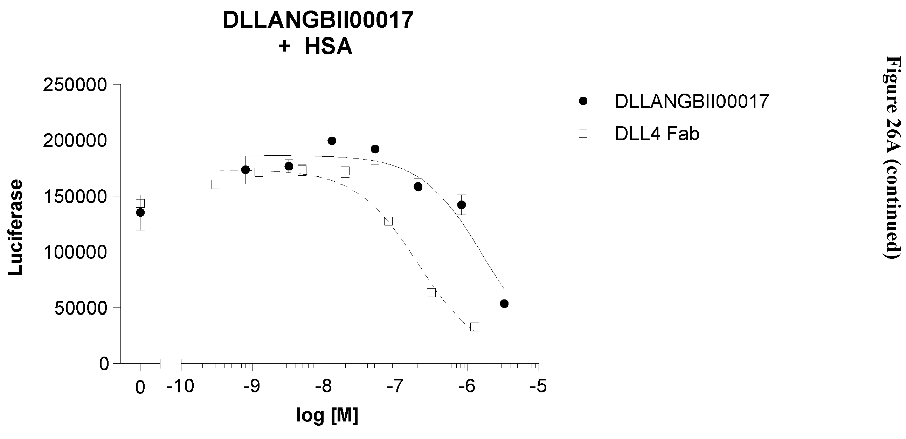

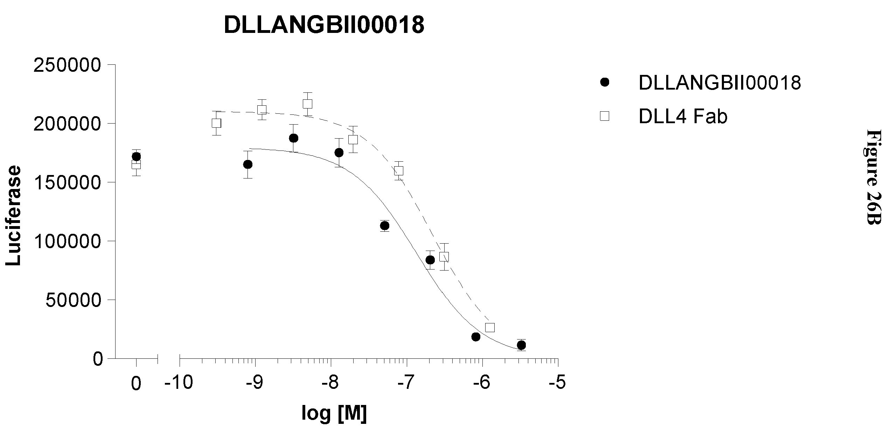

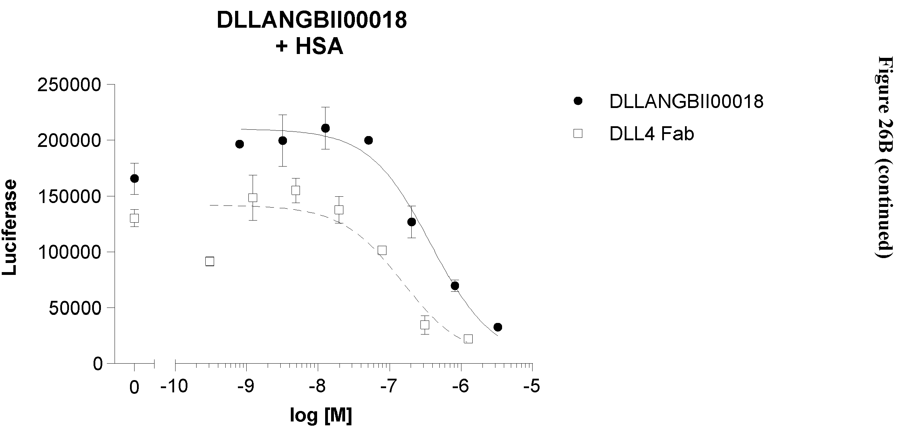

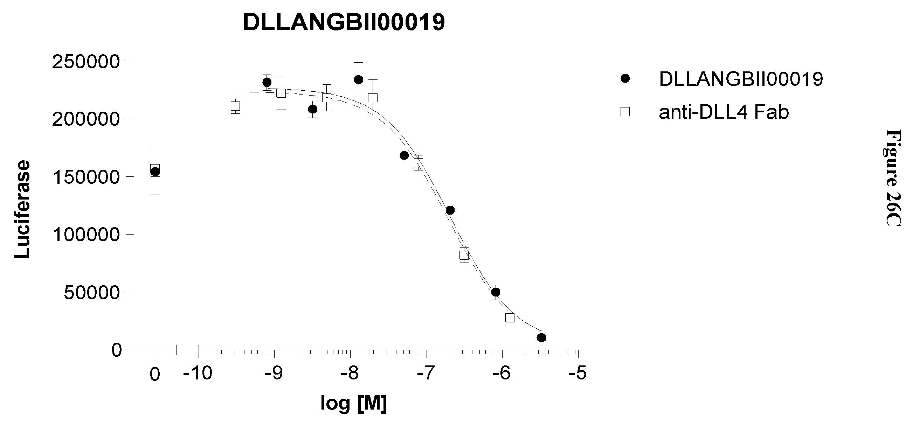

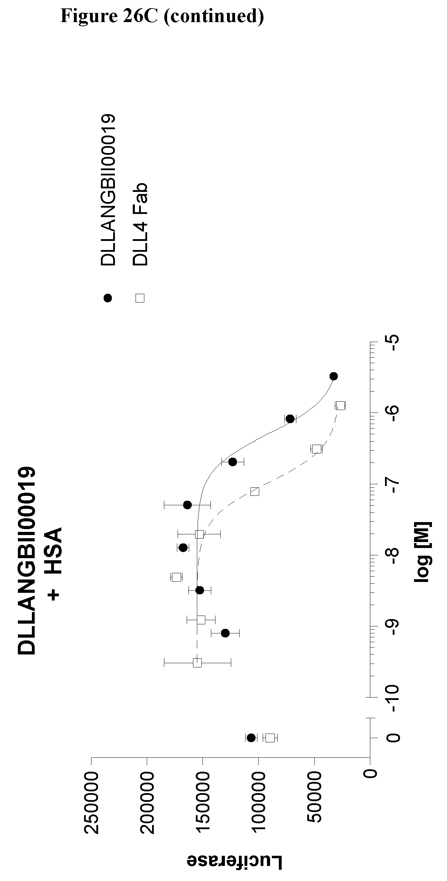

To explore the anti-DLL4 blocking properties in comparison with the monovalent building block DLLBII00018, all purified bispecific VHHs are analyzed in the hDLL4/hNotch1 competition ELISA (see Example 5.1 as described in patent US 2011/0172398 A1) (Figure 24), the CHO-hDLL4 / CHO-mDLL4 competition FMAT (see Example 5.3 as described in patent US 2011/0172398 A1) (Figure 25) and the hDLL4 mediated Notch1 activation (reporter gene) assay (see Example 5.4 as described in patent US 2011/0172398 A1) (Figure 26). Here, the ELISA competition assay is performed with a fixed concentration of 8 nM biotinylated hDLL4. The ELISA competition assay, the FMAT competition assays and the reporter gene assay are also performed after preincubation of the VHH with 12.5 µM, 25 µM and 162 µM human serum albumin, respectively. A summary of IC₅₀ values and % inhibition is shown in Table 29.

**Table 29: IC₅₀ values (nM) and % inhibition in hDLL4/hNotch1 competition ELISA, CHO-hDLL4 and CHO-mDLL4 competition FMAT and hDLL4 mediated Notch1 activation (reporter gene) assay**

| | | | hDLL4 ELISA | | CHO-hDLL4 FMAT^{(a)} | | CHO-mDLL4 FMAT ^{(a)} | | hDLL4 reporter | |
|---|---|---|---|---|---|---|---|---|---|---|
| VHH ID | Format | HSA | IC₅₀ (nM) | % inh | IC₅₀ (nM) | % inh | IC₅₀ (nM) | % inh | IC₅₀ (nM) | % inh |
| DLLBII00018 | 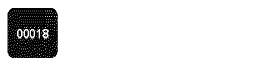 | - | 1.8 | 64 | 4.2 | 73 | 4.5 | 88 | 162 | 100 |
| | | + | 1.8 | 75 | 3.6 | 75 | 3.6 | 89 | 144 | 100 |
| DLLANGBII00017 | 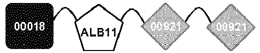 | - | 1.5 | 81 | 5.8 | 82 | 6.2 | 93 | 197 | 100 |
| | | + | 2.4 | 94 | 9.5 | 91 | 6.1 | 100 | 1,708 | ^{(b)} |
| DLLANGBII00018 | 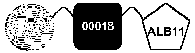 | - | 1.5 | 75 | 6.4 | 85 | 6.2 | 97 | 140 | 100 |
| | | + | 3.1 | 91 | 10.2 | 97 | 6.0 | 101 | 514 | 100 |
| DLLANGBII00019 | 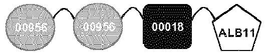 | - | 1.5 | 76 | 6.2 | 92 | 6.0 | 97 | 214 | 100 |
| | | + | 2.8 | 89 | 9.5 | 110 | 7.1 | 106 | 642 | 100 |
| DLL4 Fab | | - | 2.7 | 73 | 5.6 | 90 | 3.2 | 99 | 139 | 100 |
| | | + | 2.7 | 86 | 4.7 | 90 | 2.9 | 102 | 156 | 100 |

| | | | | | | | | | | |
|---|---|---|---|---|---|---|---|---|---|---|
| (a) tagged versions of VHH were used as these had a higher purity which avoided assay interference at higher VHH concentration. (b) maximum inhibition not reached at highest VHH concentration | | | | | | | | | | |

Binding to human DLL4, mouse DLL4 and rat DLL4 is assessed in Biacore. Briefly, kinetic analysis of the bispecific VHHs is performed by SPR on a Biacore T100 instrument. Recombinant human DLL4 (R&D Systems, Minneapolis, MI, USA) and mouse DLL4 (R&D Systems, Minneapolis, MI, USA) are immobilized on a CM5 chip via amine coupling. VHHs are injected over these surfaces at different concentrations between 2.5 and 1,800 nM. Samples are injected for 2 min and allowed to dissociate for 20 min at a flow rate of 45 µl/min. Between sample injections, the surfaces were regenerated with a 100s pulse of 10mM glycine pH 1.5. Association/dissociation data are evaluated by fitting a 1:1 interaction model (Langmuir binding). The affinity constant K_{D} is calculated from resulting association and dissociation rate constants kₐ and k_{d} (Table 30).

**Table 30: Binding kinetcs of bispecific VHHs for binding to human and mouse DLL4 (Biacore)**

| | hDLL4 | | | mDLL4 | | |
|---|---|---|---|---|---|---|
| | kₐ (1/Ms) | kₐ (1/s) | K_{D} (nM) | kₐ (1/Ms) | kₐ (1/s) | K_{D} (nM) |
| DLLANGBII00017 | 1.6E+05 | 9.5E-05 | 0.6 | 9.1E+05 | 2.6E-04 | 2.4 |
| DLLANGBII00018 | 2.0E+05 | 9.3E-05 | 0.5 | 1.3E+05 | 2.9E-04 | 1.9 |
| DLLANGBII00019 | 1.1E+05 | 7.8E-05 | 0.7 | 1.5E+05 | 2.8E-04 | 3.0 |

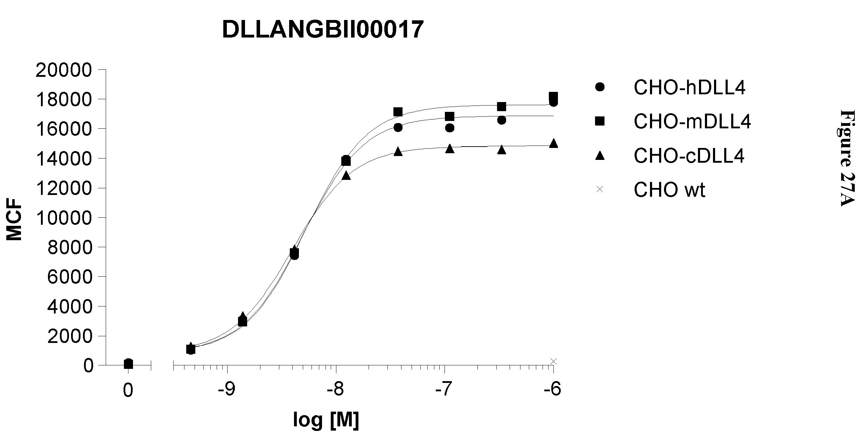

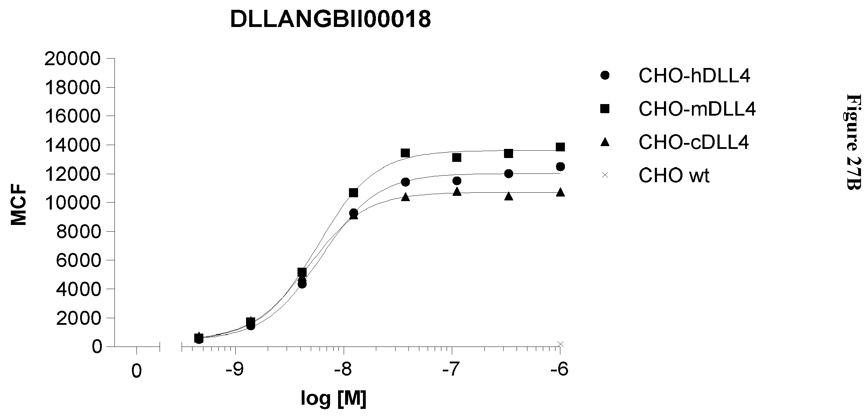

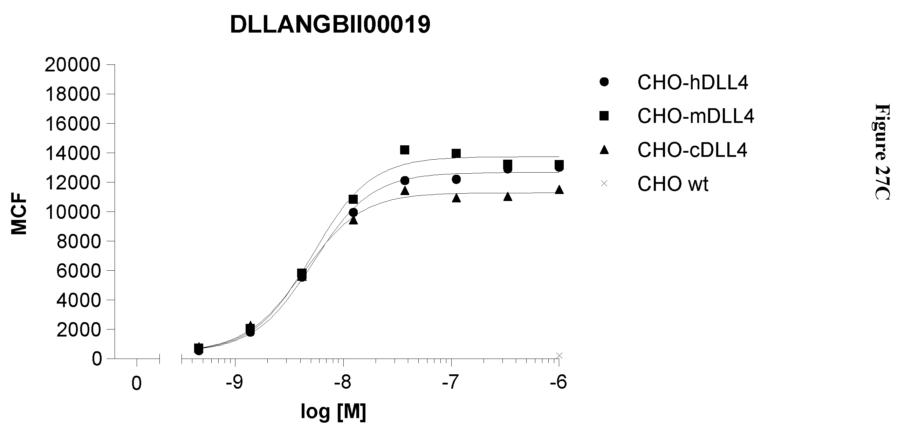

Additionally, in order to determine cross-reactivity of the bispecific VHHs to murine and cynomolgus DLL4, a FACS binding experiment is performed. Briefly, CHO cells overexpressing mouse and cynomolgus DLL4 are used for a titration binding experiment of the VHHs. After a 30 min incubation on ice, all samples are washed and a 2-step detection using biotinylated anti-VHH 1A4 followed by PE labeled streptavidin is performed. CHO cells overexpressing human DLL4 are taken as reference. The mean MCF value is determined using a FACS Array and used for calculation of the EC₅₀ value (Table 31; Figure 27).

**Table 31: EC₅₀ values of bispecific VHHs binding to human, mouse and cyno DLL4 overexpressed on CHO cells (FACS)**

| | CHO-hDLL4 EC₅₀ (nM) | CHO-mDLL4 EC₅₀ (nM) | CHO-cDLL4 EC₅₀ (nM) |
|---|---|---|---|
| DLLANGBII00017 | 6.0 | 6.2 | 4.7 |
| DLLANGBII00018 | 7.9 | 6.7 | 5.6 |
| DLLANGBII00019 | 6.7 | 6.3 | 5.3 |
| DLL4Fab | 7.0 | 6.0 | 5.3 |

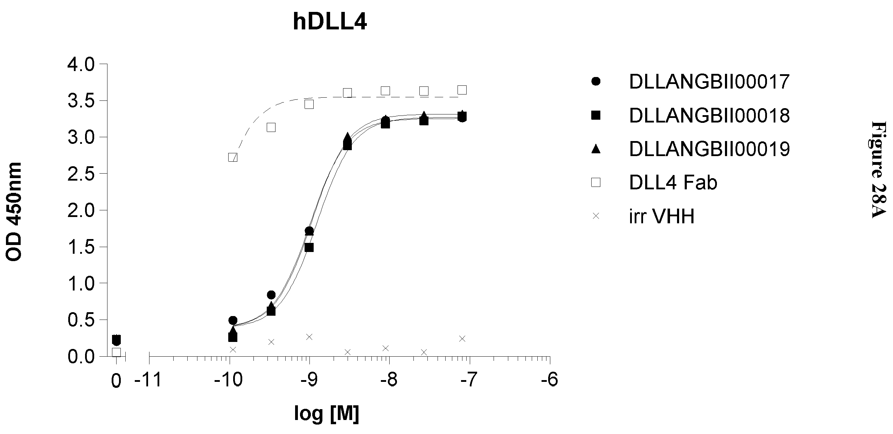

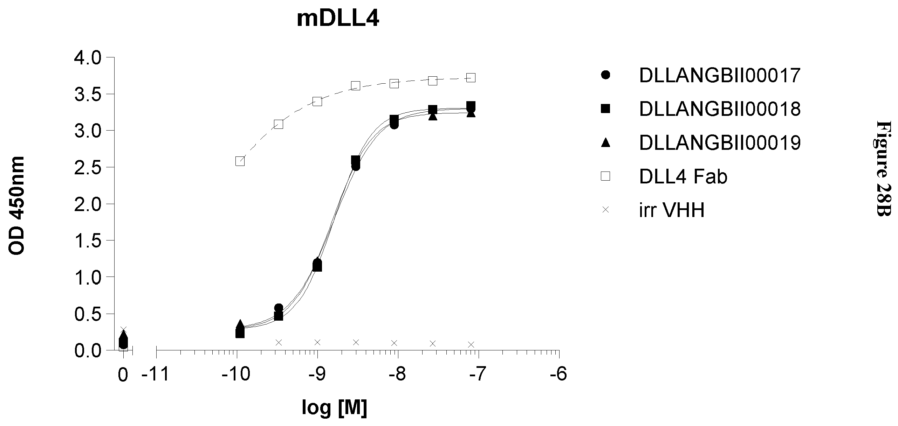

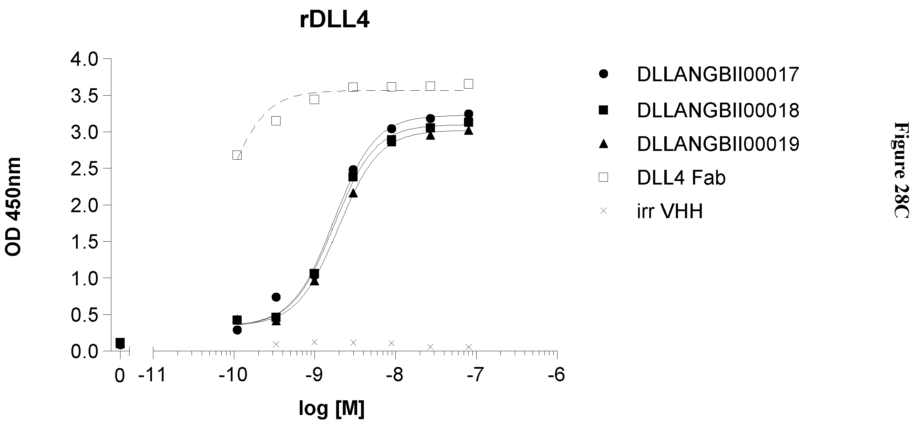

In order to determine cross-reactivity to mouse DLL4 and rat DLL4, a binding ELISA is performed. In brief, recombinant mouse DLL4 (R&D Systems, Minneapolis, MI, USA) and rat DLL4 is coated overnight at 4°C in a 96-well MaxiSorp plate (Nunc, Wiesbaden, Germany). Wells are blocked with a 1% casein solution. VHHs are applied as dilution series and binding is detected biotinylated anti-VHH 1A4 followed by extravidin-HRP. As reference binding to human DLL4 is measured. EC₅₀ values are summarized in Table 32 and Figure 28.

**Table 32: EC₅₀ values of bispecific VHHs binding to human, mouse and rat DLL4 (ELISA)**

| | hDLL4 EC₅₀ (nM) | mDLL4 EC₅₀ (nM) | cDLL4 EC₅₀ (nM) |
|---|---|---|---|
| DLLANGBII00017 | 1.0 | 1.6 | 1.7 |
| DLLANGBII00018 | 1.2 | 1.6 | 1.7 |
| DLLANGBII00019 | 1.1 | 1.5 | 1.9 |

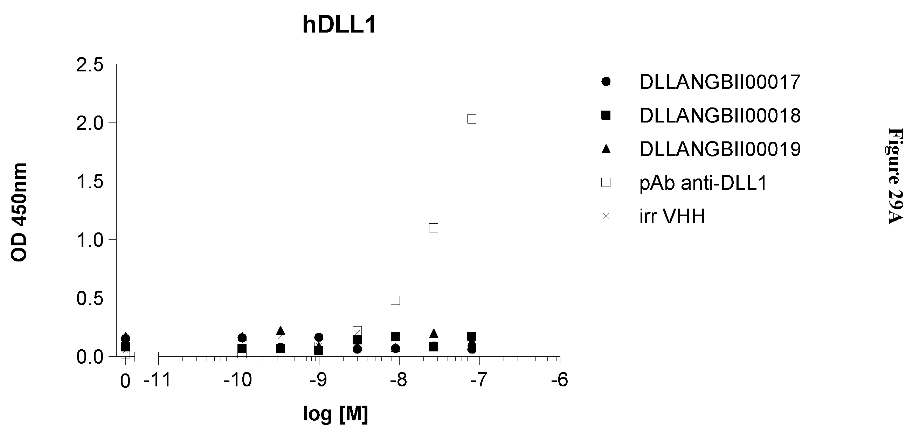

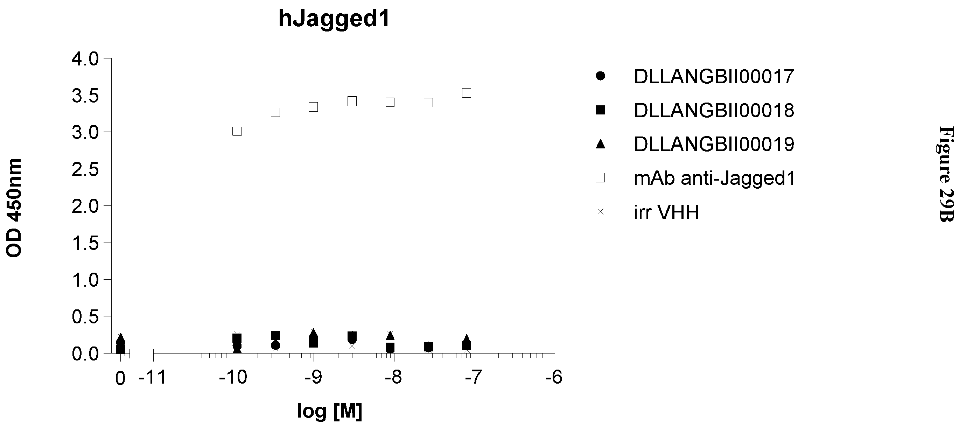

Absence of binding to the homologous human ligands DLL1 and Jagged-1 is assessed via a solid phase binding assay (ELISA). In brief, 1 µg/mL of recombinant human DLL1 (Alexis, San Diego, CA, USA) or recombinant human Jagged-1 (Alexis, San Diego, CA, USA) is coated overnight at 4°C in a 96-well MaxiSorp plate (Nunc, Wiesbaden, Germany). Wells are blocked with a 1% casein solution. VHHs are applied as dilution series and binding is detected biotinylated anti-VHH 1A4 followed by extravidin-HRP. All bispecific VHH are considered as being non-cross reactive to these homologous ligands. Results are shown in Figure 29.

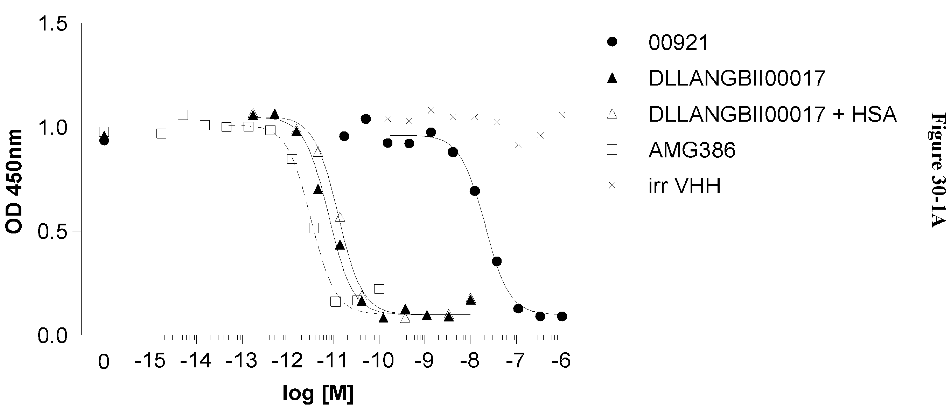

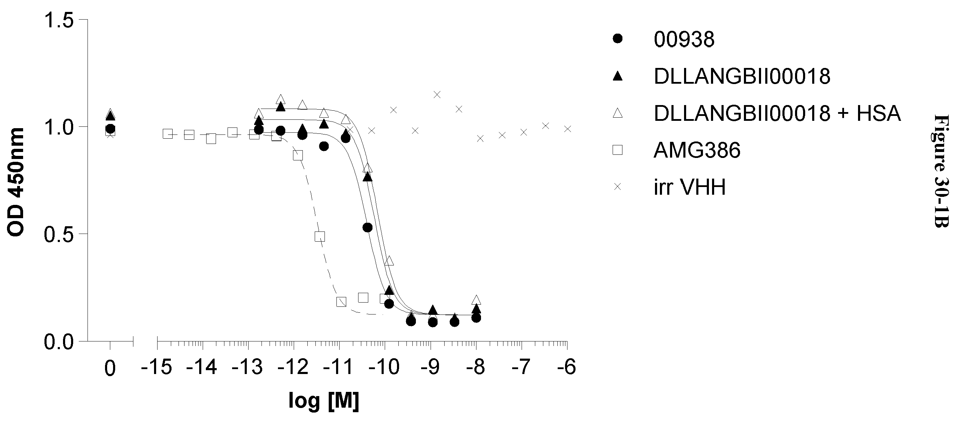

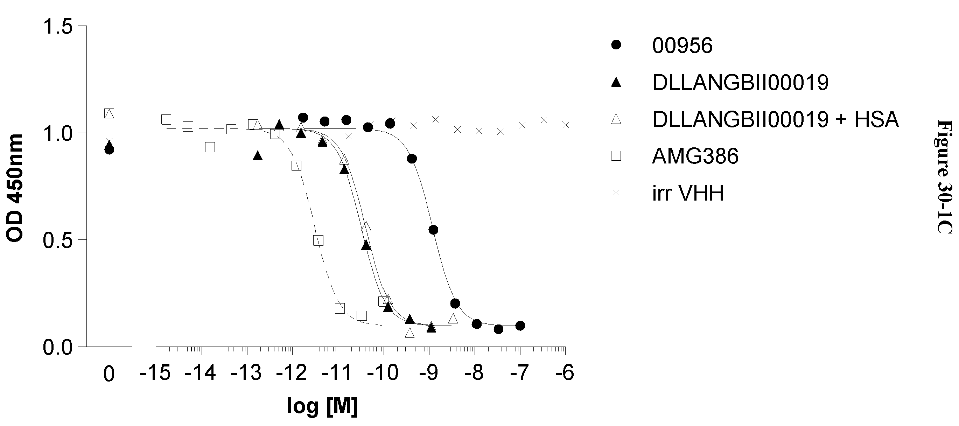

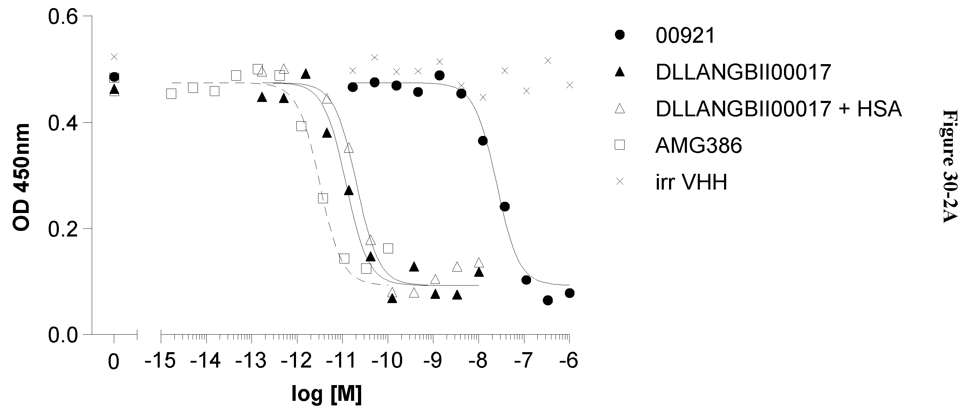

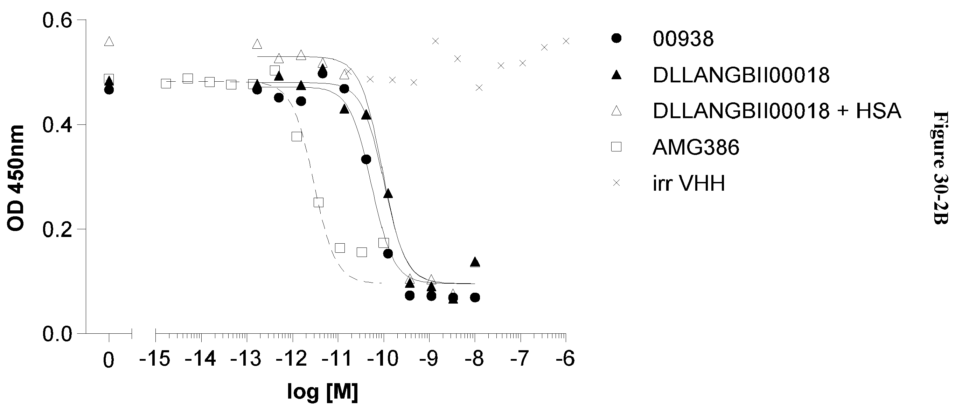

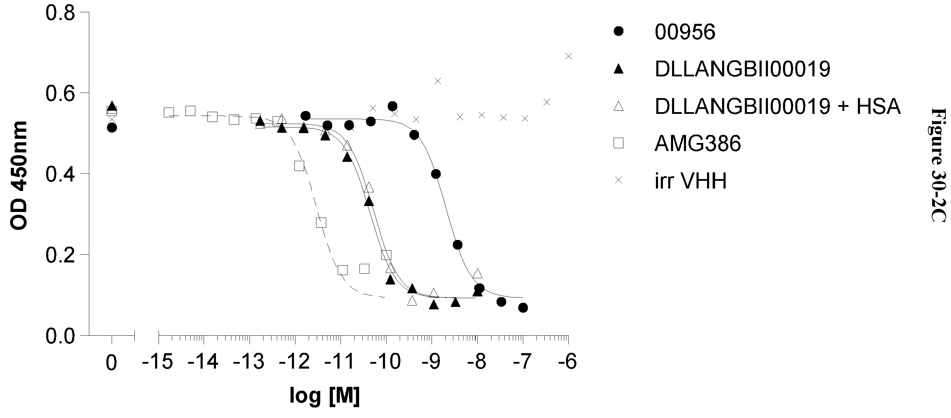

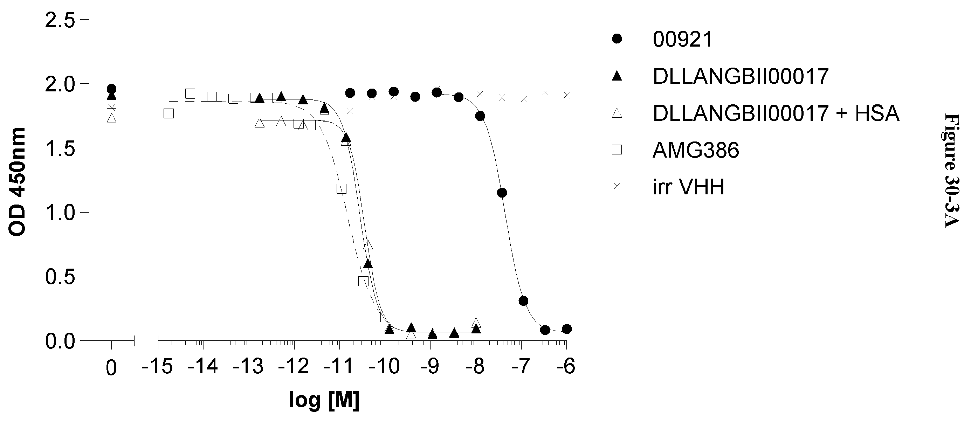

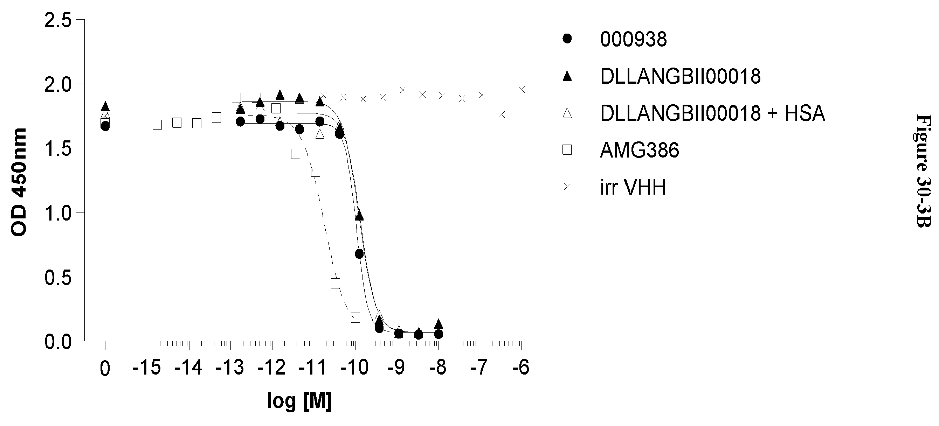

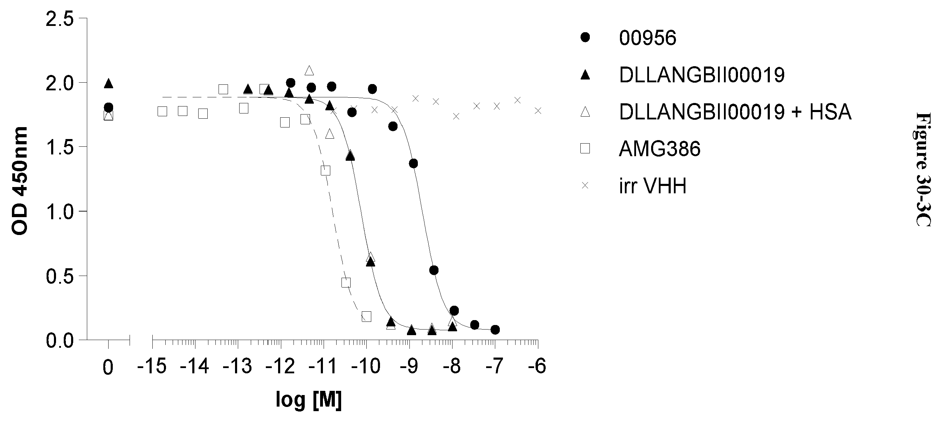

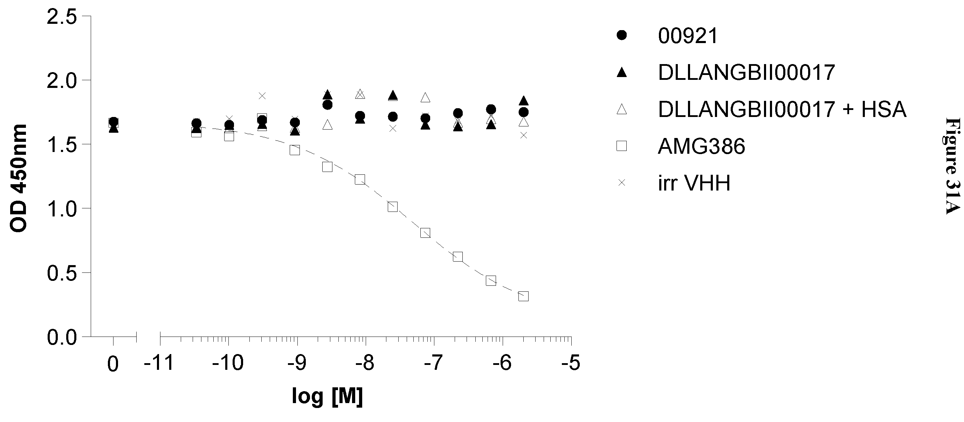

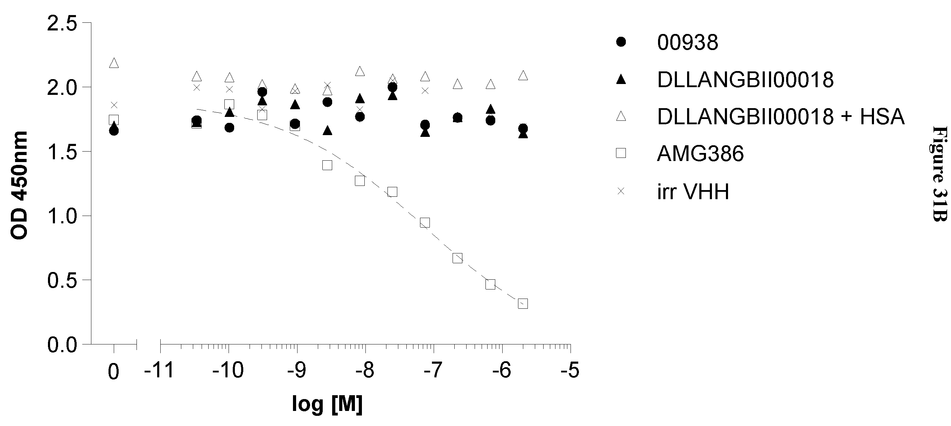

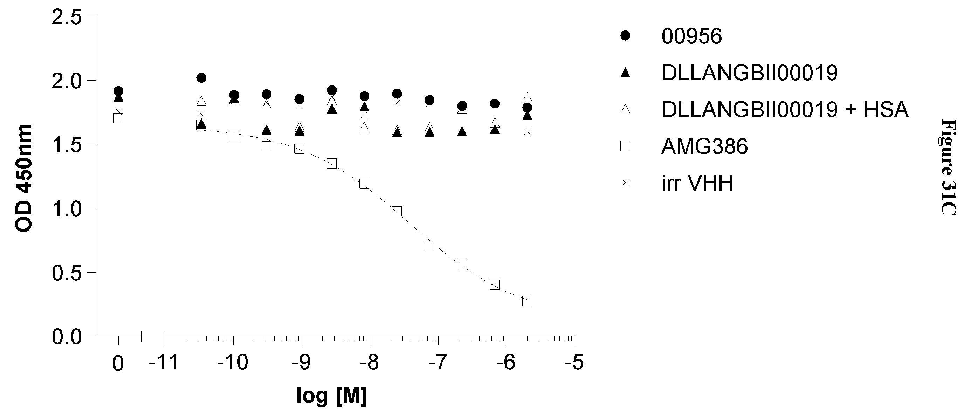

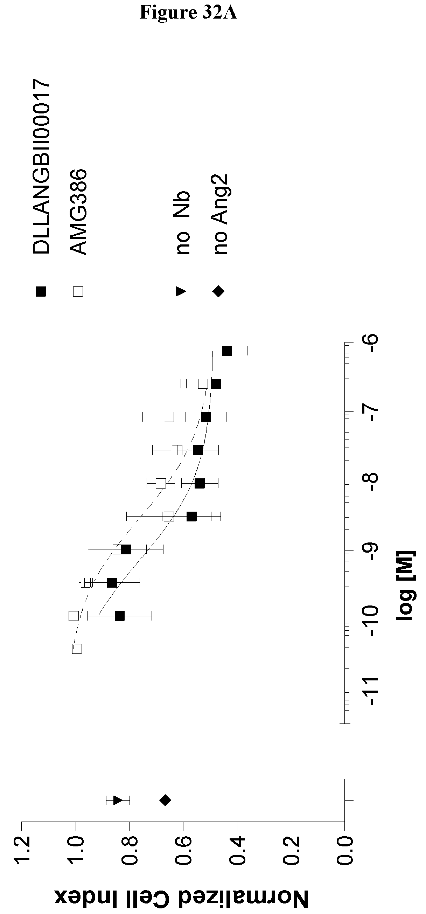

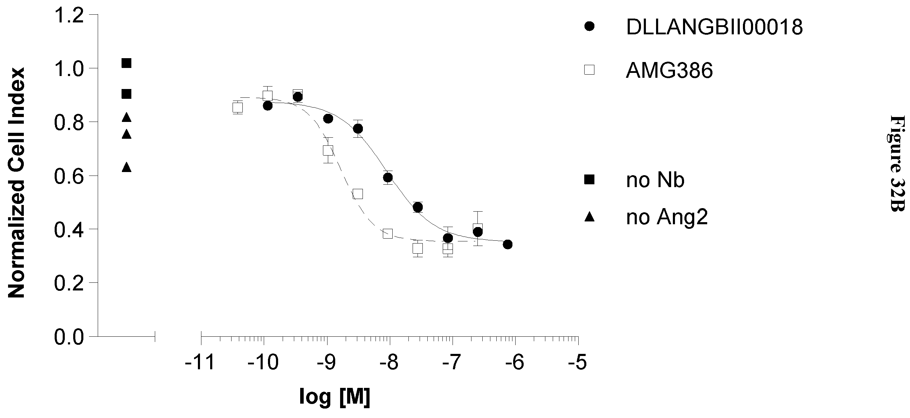

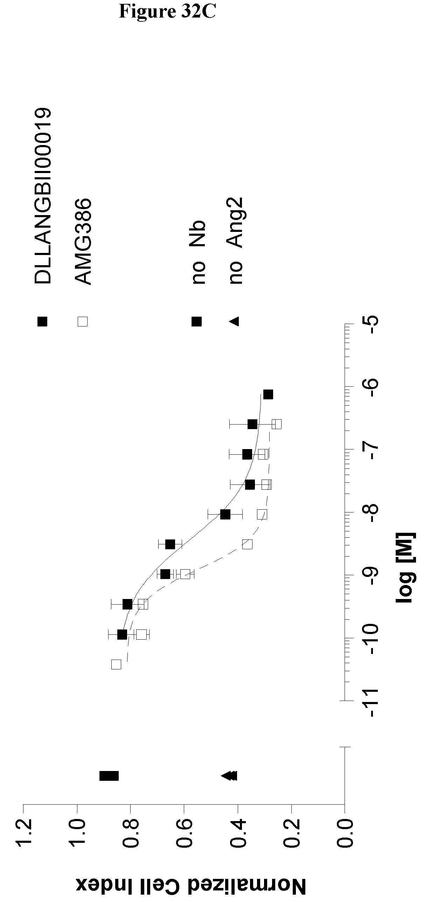

To explore the anti-Ang2 blocking properties in comparison with the final sequence optimized monovalent anti-Ang2 building blocks 00921, 00938 and 00956, all purified bispecific VHHs are analyzed in a human Ang2/hTie2 (Figure 30-1), mouse Ang2/mTie2 (Figure 30-2), cyno Ang2/cTie2 (Figure 30-3), a hAng1/hTie2 (Figure 31) competition ELISA and the hAng2 mediated HUVEC survival assay (Figure 32). A summary of IC₅₀ and % inhibition values is shown in Table 33.

**Table 33: IC₅₀ values (pM) and % inhibition in human, mouse and cyno Ang2/Tie2 competition ELISA, hAng1 competition ELISA and hAng2 mediated HUVEC survival assay**

| | | | hAng2 | | mAng2 | | cAng2 | | hAng1/hAng2 IC₅₀ ratio | HUVEC survival | |
|---|---|---|---|---|---|---|---|---|---|---|---|
| VHH ID | Format | HSA | IC₅₀ (pM) | % inh | IC50 (pM) | % inh | IC₅₀ (pM) | % inh | | IC₅₀ (nM) | % inh |
| 00921 | 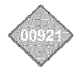 | - | 20,400 | 100 | 27,200 | 100 | 43,400 | 100 | >98 | 18.8 | 100 |
| | | + | n.d. | n.d. | n.d. | n.d. | n.d. | n.d. | n.d. | n.d. | n.d. |
| DLLANGBII000017 | 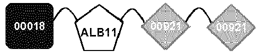 | - | 8 | 100 | 12 | 100 | 28 | 100 | > 257,632 | tbd | tbd |
| | | + | 13 | 100 | 20 | 100 | 35 | 100 | > 158,855 | n.d. | n.d. |
| AMG386 | | - | 3 | 100 | 3 | 100 | 15 | 100 | 14,421 | tbd | tbd |
| | | + | n.d. | n.d. | n.d. | n.d. | n.d. | n.d. | n.d. | n.d. | n.d. |
| 00938 | 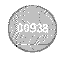 | - | 40 | 100 | 62 | 100 | 105 | 100 | > 50,234 | 4.3 | 100 |
| | | + | n.d. | n.d. | n.d. | n.d. | n.d. | n.d. | n.d. | n.d. | n.d. |
| DLLANGBII00018 | 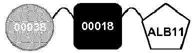 | - | 55 | 100 | 85 | 100 | 130 | 100 | > 36,392 | 4.0 | 100 |
| | | + | 61 | 100 | 91 | 100 | 131 | 100 | > 32,684 | n.d. | n.d. |
| AMG386 | | - | 3 | 100 | 3 | 100 | 19 | 100 | 17,452 | 1.7 | 100 |
| | | + | n.d. | n.d. | n.d. | n.d. | n.d. | n.d. | n.d. | n.d. | n.d. |
| 00956 | 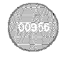 | - | 1,180 | 100 | 2,230 | 100 | 2,030 | 100 | > 1,698 | 6.8 | 100 |
| | | + | n.d. | n.d. | n.d. | n.d. | n.d. | n.d. | n.d. | n.d. | n.d. |
| DLLANGBII00019 | 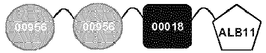 | - | 32 | 100 | 45 | 100 | 76 | 100 | > 61,802 | 3.6 | 100 |
| | | + | 40 | 100 | 51 | 100 | 76 | 100 | > 50,234 | n.d. | n.d. |
| AMG386 | | - | 3 | 100 | 2 | 100 | 16 | 100 | 11,482 | 1.2 | 100 |
| | | + | n.d. | n.d. | n.d. | n.d. | n.d. | n.d. | n.d. | n.d. | n.d. |

| | | | | | | | | | | | |
|---|---|---|---|---|---|---|---|---|---|---|---|
| n.d., not determined; tbd, to be determined | | | | | | | | | | | |

Affinities of DLLANGBII00017-18-19 for human, mouse, cyno and rat Ang2 (see Example 5) have been determined and are shown in Table 34.

**Table 34: Binding kinetics of purified VHHs for recombinant human, cyno, mouse and rat Ang2**

| | human Ang2-FLD | | | cyno Ang2-FLD | | |
|---|---|---|---|---|---|---|
| | kₐ (1/Ms) | k_{d} (1/s) | K_{D} (M) | kₐ (1/Ms) | k_{d} (1/s) | K_{D} (M) |
| DLLANGBII00017 | 1.90E+06 | 1.30E-02 | 6.60E-09 | 2.50E+06 | 1.20E-02 | 4.70E-09 |
| DLLANGBII00018 | 8.80E+05 | 3.30E-05 | 3.80E-11 | 1.30E+06 | 3.20E-05 | 2.40E-11 |
| DLLANGBII00019 | 5.10E+05 | 1.60E-03 | 3.10E-09 | 6.30E+05 | 1.30E-03 | 2.10E-09 |

| | mouse Ang2-FLD | | | rat Ang2-FLD | | |
|---|---|---|---|---|---|---|
| | kₐ (1/Ms) | k_{d} (1/s) | K_{D} (M) | kₐ (1/Ms) | k_{d} (1/s) | K_{D} (M) |
| DLLANGBII00017 | 9.10E+05 | 1.50E-02 | 1.70E-08 | 6.70E+05 | 3.30E-02 | 4.90E-08 |
| DLLANGBII00018 | 4.40E+05 | 6.90E-05 | 1.60E-10 | 3.30E+05 | 9.00E-05 | 2.70E-10 |
| DLLANGBII00019 | 3.80E+05 | 3.80E-03 | 1.00E-08 | 2.80E+05 | 6.00E-03 | 2.10E-08 |

Affinities of DLLANGBII00017-18-19 for human, mouse and cyno serum albumin have been determined (Example 5) and are shown in Table 35. The affinity constant K_{D} is calculated from resulting association and dissociation rate constants kₐ and k_{d}.

**Table 35: Binding kinetics of purified VHHs for recombinant human, mouse and cyno serum albumin**

| | HSA | | | CSA | | |
|---|---|---|---|---|---|---|
| | kₐ (1/Ms) | k_{d} (1/s) | K_{D} (nM) | kₐ (1/Ms) | k_{d} (1/s) | K_{D} (nM) |
| ALB11 | 4.5E+05 | 1.7E-03 | 3.8E-09 | 4.2E+05 | 1.7E-03 | 3.9E-09 |
| DLLANGBII00017 | 1.4E+05 | 4.4E-03 | 3.1E-08 | 1.4E+05 | 4.2E-03 | 2.9E-08 |
| DLLANGBII00018 | 1.6E+05 | 4.7E-03 | 2.9E-08 | 1.6E+05 | 4.6E-03 | 2.9E-08 |
| DLLANGBII00019 | 8.1E+04 | 5.6E-03 | 6.9E-08 | 8.1E+04 | 5.5E-03 | 6.8E-08 |

| | MSA | | | | | |
|---|---|---|---|---|---|---|
| | kₐ (1/Ms) | k_{d} (1/s) | K_{D} (nM) | | | |
| ALB11 | 5.5E+05 | 3.0E-02 | 5.5E-08 | | | |
| DLLANGBII00017 | 1.4E+05 | 1.1E-01 | 7.7E-07 | | | |
| DLLANGBII00018 | * | * | * | | | |
| DLLANGBII00019 | * | * | * | | | |

| | | | | | | |
|---|---|---|---|---|---|---|
| * could not be properly fitted | | | | | | |

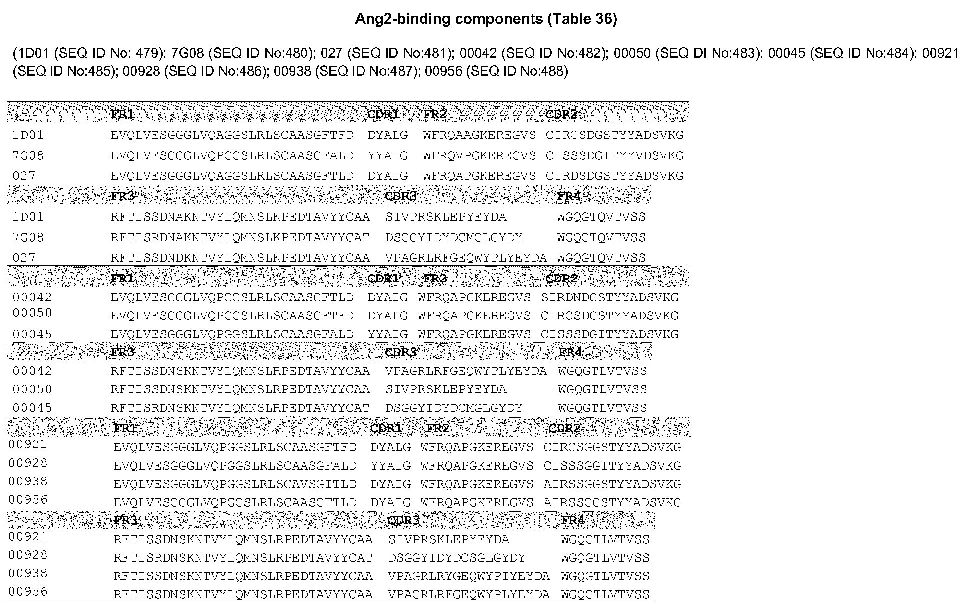

## Claims

1. A binding molecule comprising
- one Ang2-binding component,
- one DII4-binding component, and
- one serum albumin binding component,
wherein said Ang2-, DII4- and serum albumin binding components are immunoglobulin single variable domains, each immunoglobulin single variable domain consisting of four framework regions and three complementarity determining regions (CDRs), and
wherein said immunoglobulin single variable domains are VHHs, and
wherein said binding molecule comprises
- one Ang2-binding VHH, having
a CDR1 having the sequence DYAIG, as shown in SEQ ID NO: 513,
a CDR2 having the sequence AIRSSGGSTYYADSVKG, as shown in SEQ ID NO: 514, and
a CDR3 having the sequence VPAGRLRYGEQWYPIYEYDA, as shown in SEQ ID
NO: 515,
- the DLLII00018 domain as the DII4-binding component, which domain has the sequence: and
- the AlbII domain, as shown in SEQ ID NO: 519, as the serum albumin binding component,
in this order.

2. The binding molecule of claim 1, being DLLANGBII00018, as shown in SEQ ID NO: 477.

3. A nucleic acid molecule encoding a binding molecule of claim 1 or claim 2.

4. A vector containing a nucleic acid molecule of claim 3.

5. A host cell containing a nucleic acid molecule of claim 3 or a vector of claim 4.

6. A pharmaceutical composition containing at least one binding molecule of claim 1 or claim 2 as the active ingredient.

7. The pharmaceutical composition of claim 6 for use in the treatment of cancer and cancerous diseases.

8. The pharmaceutical composition of claim 6 for use in the treatment of eye diseases.

## Patentansprüche

1. Bindungsmolekül, umfassend
- eine Ang2-bindende Komponente,
- eine DII4-bindende Komponente und
- eine Serumalbumin-bindende Komponente,
wobei die Ang2-, DII4- und Serumalbumin-bindenden Komponenten variable Immunglobulin-Einzeldomänen sind, wobei jede variable Immunglobulin-Einzeldomäne aus vier Gerüstregionen und drei komplementaritätsbestimmenden Regionen (CDRs) besteht, und
wobei die variablen Immunglobulin-Einzeldomänen VHHs sind, und
wobei das Bindungsmolekül umfasst
- eine Ang2-bindende VHH mit
einer CDR1 mit der Sequenz DYAIG, wie in SEQ ID NO: 513 gezeigt,
einer CDR2 mit der Sequenz AIRSSGGSTYYADSVKG, wie in SEQ ID NO: 514 gezeigt, und
einer CDR3 mit der Sequenz VPAGRLRYGEQWYPIYEYDA, wie in SEQ ID NO: 515 gezeigt,
- die DLLBII00018-Domäne als die DII4-bindende Komponente, wobei die Domäne die Sequenz: und
- die Alb11-Domäne, wie in SEQ ID NO: 519 gezeigt, als die Serumalbumin-bindende Komponente,
in dieser Reihenfolge.

2. Bindungsmolekül von Anspruch 1, welches DLLANGBII00018, wie in SEQ ID NO: 477 gezeigt, ist.

3. Nucleinsäuremolekül, kodierend ein Bindungsmolekül von Anspruch 1 oder Anspruch 2.

4. Vektor, enthaltend ein Nucleinsäuremolekül von Anspruch 3.

5. Wirtszelle, enthaltend ein Nucleinsäuremolekül von Anspruch 3 oder einen Vektor von Anspruch 4.

6. Pharmazeutische Zusammensetzung, enthaltend mindestens ein Bindungsmolekül von Anspruch 1 oder Anspruch 2 als den Wirkstoff.

7. Pharmazeutische Zusammensetzung von Anspruch 6 zur Verwendung bei der Behandlung von Krebs und Krebserkrankungen.

8. Pharmazeutische Zusammensetzung von Anspruch 6 zur Verwendung bei der Behandlung von Augenerkrankungen.

## Revendications

1. Molécule de liaison comprenant
- un composant de liaison à Ang2
- un composant de liaison à DII4, et
- un composant de liaison à la sérumalbumine,
dans laquelle lesdits composants de liaison à Ang2, à DII4 et à la sérumalbumine sont des domaines variables uniques d'immunoglobuline, chaque domaine variable unique d'immunoglobuline consistant en quatre régions de structure et trois régions de détermination de la complémentarité (CDR), et
dans laquelle lesdits domaines variables uniques d'immunoglobuline sont des VHH, et
dans laquelle ladite molécule de liaison comprend
- une VHH de liaison à Ang2, comportant une CDR1 ayant la séquence DYAIG, telle que représentée par SEQ ID NO : 513,
une CDR2 ayant la séquence AIRSSGGSTYYADSVKG , telle que représentée par SEQ ID NO : 514, et
une CDR3 ayant la séquence VPAGRLRYGEQWYPIYEYDA, telle que représentée par SEQ ID NO : 515,
- le domaine DLLBII00018 en tant que composant de liaison à DII4, ledit domaine ayant la séquence : et
- le domaine Alb11, tel que représenté par SEQ ID NO : 519, en tant que composant de liaison à la sérumalbumine,
dans cet ordre.

2. Molécule de liaison selon la revendication 1, qui est DLLANGBII00018, telle que représentée par SEQ ID NO : 477.

3. Molécule d'acide nucléique codant une molécule de liaison selon la revendication 1 ou la revendication 2.

4. Vecteur contenant une molécule d'acide nucléique selon la revendication 3.

5. Cellule hôte contenant une molécule d'acide nucléique selon la revendication 3 ou un vecteur selon la revendication 4.

6. Composition pharmaceutique contenant au moins une molécule de liaison selon la revendication 1 ou la revendication 2 en tant que principe actif.

7. Composition pharmaceutique selon la revendication 6, pour son utilisation dans le traitement d'un cancer et des maladies cancéreuses.

8. Composition pharmaceutique selon la revendication 6, pour son utilisation dans le traitement des maladies oculaires.


### SEQUENCE LISTING

<110> Boehringer Ingelheim International GmbH
<120> Bispecific binding molecules binding to D114 and Ang2
<130> 12-0331-pct
<160> 540
<170> PatentIn version 3.3
<210> 1
   <211> 16
   <212> PRT
   <213> Homo sapiens
<400> 1
   Pro Phe Ala Tyr Tyr Ser Asp Leu Cys Gly Val Asn Gly Val Asp Tyr
   1               5                   10                  15
<210> 2
   <211> 16
   <212> PRT
   <213> Lama Glama
<400> 2
   Pro Phe Ser Tyr Tyr Ser His Leu Cys Gly Val Asn Gly Tyr Asp Tyr
   1               5                   10                  15
<210> 3
   <211> 16
   <212> PRT
   <213> Lama Glama
<400> 3
   Pro Phe Ser Tyr Tyr Ser Ser Leu Cys Gly Val Asn Glu Tyr Asp Tyr
   1               5                   10                  15
<210> 4
   <211> 15
   <212> PRT
   <213> Lama Glama
<400> 4
   Pro Trp Asp Ser Trp Tyr Cys Gly Ile Gly Asn Asp Tyr Asp Tyr
   1               5                   10                  15
<210> 5
   <211> 16
   <212> PRT
   <213> Lama Glama
<400> 5
   Pro Phe Ile His Tyr Ser Asp Leu Cys Gly Val Asn Gly Tyr Asp Tyr
   1               5                   10                  15
<210> 6
   <211> 22
   <212> PRT
   <213> Lama Glama
<400> 6
   Asp Pro Ile His Asn Cys Tyr Ser Gly Ser Ser Tyr Tyr Tyr Ser Pro
   1               5                   10                  15
   Glu Ala Val Tyr Asp Tyr
               20
<210> 7
   <211> 16
   <212> PRT
   <213> Lama Glama
<400> 7
   Pro Phe Ala His Tyr Ser Asp Leu Cys Gly Val Asn Gly Tyr Asp Tyr
   1               5                   10                  15
<210> 8
   <211> 15
   <212> PRT
   <213> Lama Glama
<400> 8
   Ser Gly Ser Tyr Tyr Tyr Pro Thr Asp Val His Glu Tyr Asp Tyr
   1               5                   10                  15
<210> 9
   <211> 16
   <212> PRT
   <213> Lama Glama
<400> 9
   Pro Phe Glu Tyr Tyr Ser Asp Leu Cys Gly Val Asn Gly Tyr Asp Tyr
   1               5                   10                  15
<210> 10
   <211> 21
   <212> PRT
   <213> Lama Glama
<400> 10
   Asp Pro Ile His Asn Cys Tyr Ser Gly Arg Tyr Tyr Tyr Ser Pro Glu
   1               5                   10                  15
   Ala Val Tyr Glu Tyr
               20
<210> 11
   <211> 16
   <212> PRT
   <213> Lama Glama
<400> 11
   Pro Phe Ser His Tyr Ser Asp Leu Cys Gly Val Asn Ala Ile Asp Tyr
   1               5                   10                  15
<210> 12
   <211> 16
   <212> PRT
   <213> Lama Glama
<400> 12
   Pro Phe Glu Tyr Tyr Ser Asp Leu Cys Gly Val Asn Gly Tyr Asp Tyr
   1               5                   10                  15
<210> 13
   <211> 15
   <212> PRT
   <213> Lama Glama
<400> 13
   Ser Gly Ser Tyr Tyr Tyr Pro Thr Asp Val Phe Glu Tyr Asp Tyr
   1 5 10 15
<210> 14
   <211> 21
   <212> PRT
   <213> Lama Glama
<400> 14
   His Pro Leu Gln Asn Cys Cys Gly Gly Ser Ala Tyr Ala Ser Pro Glu
   1 5 10 15
   Ala Val Tyr Glu Tyr
   20
<210> 15
   <211> 16
   <212> PRT
   <213> Lama Glama
<400> 15
   Pro Phe Ala Tyr Tyr Ser Asn Leu Cys Gly Val Asn Gly Tyr Asp Tyr
   1 5 10 15
<210> 16
   <211> 21
   <212> PRT
   <213> Lama Glama
<400> 16
   Asp Pro Ile His Asn Cys Tyr Ser Gly Asn Tyr Tyr Ala Ser Pro Glu
   1 5 10 15
   Ala Val Tyr Asp Tyr
   20
<210> 17
   <211> 15
   <212> PRT
   <213> Lama Glama
<400> 17
   Asp Pro Leu Val Cys Gly Tyr Asn Asp Pro Arg Leu Ala Asp Tyr
   1 5 10 15
<210> 18
   <211> 16
   <212> PRT
   <213> Lama Glama
<400> 18
   Pro Phe Ala His Tyr Ser Asp Leu Cys Gly Val Asn Gly Tyr Asp Tyr
   1 5 10 15
<210> 19
   <211> 16
   <212> PRT
   <213> Lama Glama
<400> 19
   Pro Phe Thr His Tyr Ser Asp Leu Cys Gly Val Asn Glu Tyr Asp Tyr
   1 5 10 15
<210> 20
   <211> 11
   <212> PRT
   <213> Lama Glama
<400> 20
   Pro Gly Ile Ala Ala Cys Arg Gly Ile His Tyr
   1 5 10
<210> 21
   <211> 11
   <212> PRT
   <213> Lama Glama
<210> 22
   <211> 14
   <212> PRT
   <213> Lama Glama
<400> 22
   Pro Arg Gly Trp Gly Pro Thr Gly Pro His Glu Tyr Gly Tyr
   1 5 10
<210> 23
   <211> 15
   <212> PRT
   <213> Lama Glama
<400> 23
   Ser Phe Gln Ser Gly Ala Ala Pro Gly Ala Asn Phe Tyr Asp Tyr
   1 5 10 15
<210> 24
   <211> 12
   <212> PRT
   <213> Lama Glama
<400> 24
   Pro Ala Pro Gly Ser Ser Gly Tyr Glu Tyr Asp Tyr
   1 5 10
<210> 25
   <211> 12
   <212> PRT
   <213> Lama Glama
<400> 25
   Pro Ser Pro Gly Ser Ser Gly Tyr Glu Tyr Asp Tyr
   1 5 10
<210> 26
   <211> 14
   <212> PRT
   <213> Lama Glama
<400> 26
   Ser Leu Arg Gly Trp Asp Thr Thr Arg Ile Asp Tyr Glu Tyr
   1 5 10
<210> 27
   <211> 11
   <212> PRT
   <213> Lama Glama
<400> 27
   Pro Gly Ile Ala Ala Cys Arg Gly Ile His Tyr
   1 5 10
<210> 28
   <211> 14
   <212> PRT
   <213> Lama Glama
<400> 28
   Pro Arg Gly Trp Gly Pro Thr Gly Pro His Glu Tyr Gly Tyr
   1 5 10
<210> 29
   <211> 12
   <212> PRT
   <213> Lama Glama
<400> 29
   Pro Ala Pro Gly Ser Ser Gly Tyr Glu Tyr Asp Tyr
   1 5 10
<210> 30
   <211> 12
   <212> PRT
   <213> Lama Glama
<400> 30
   Pro Ser Pro Gly Ser Ser Gly Tyr Glu Tyr Asp Tyr
   1 5 10
<210> 31
   <211> 14
   <212> PRT
   <213> Lama Glama
<400> 31
   Arg Ala Ala Asp Thr Arg Leu Gly Pro Tyr Glu Tyr Asp Tyr
   1 5 10
<210> 32
   <211> 14
   <212> PRT
   <213> Lama Glama
<400> 32
   Pro Arg Gly Trp Gly Pro Thr Gly Pro His Glu Tyr Gly Tyr
   1 5 10
<210> 33
   <211> 11
   <212> PRT
   <213> Lama Glama
<400> 33
   Pro Gly Ile Ala Ala Cys Arg Gly Ile His Tyr
   1 5 10
<210> 34
   <211> 13
   <212> PRT
   <213> Lama Glama
<400> 34
   Gly Arg Gly Phe Tyr His Asp Tyr Ser Ser Tyr Glu Tyr
   1 5 10
<210> 35
   <211> 9
   <212> PRT
   <213> Lama Glama
<400> 35
   Gly Pro Asp Cys Ser Ser Tyr Asp Tyr
   1               5
<210> 36
   <211> 14
   <212> PRT
   <213> Lama Glama
<400> 36
   Ser Leu Arg Gly Trp Asp Thr Thr Arg Ile Asp Tyr Glu Tyr
   1               5                   10
<210> 37
   <211> 13
   <212> PRT
   <213> Lama Glama
<400> 37
   Ala Arg Gly Asp Ile Asp Val Tyr Thr Leu Ser Asp Ser
   1               5                   10
<210> 38
   <211> 13
   <212> PRT
   <213> Lama Glama
<400> 38
   Ala Arg Gly Asp Ile Asp Val Tyr Thr Leu Ser Asp Ser
   1               5                   10
<210> 39
   <211> 14
   <212> PRT
   <213> Lama Glama
<400> 39
   Pro Arg Gly Trp Gly Pro Thr Gly Pro His Glu Tyr Gly Tyr
   1               5                   10
<210> 40
   <211> 20
   <212> PRT
   <213> Lama Glama
<400> 40
   Arg Leu Phe Ser Gly Gly Cys Ala Val Val Ala Gly Thr Ser Trp Ala
   1               5                   10                  15
   Asp Phe Gly Ser
               20
<210> 41
   <211> 20
   <212> PRT
   <213> Lama Glama
<400> 41
   Arg Leu Phe Lys Gly Gly Cys Ala Val Val Ala Gly Thr Ser Trp Ala
   1               5                   10                  15
   Asp Phe Gly Ser
               20
<210> 42
   <211> 12
   <212> PRT
   <213> Lama Glama
<400> 42
   Leu Ala Thr Thr Val Thr Pro Ser Trp Val Asn Tyr
   1               5                   10
<210> 43
   <211> 14
   <212> PRT
   <213> Lama Glama
<400> 43
   Pro Arg Gly Trp Gly Pro Thr Gly Pro His Glu Tyr Asp Tyr
   1               5                   10
<210> 44
   <211> 14
   <212> PRT
   <213> Lama Glama
<400> 44
   Ser Leu Arg Gly Trp Asp Thr Thr Trp Ile Asp Tyr Glu Tyr
   1               5                   10
<210> 45
   <211> 13
   <212> PRT
   <213> Lama Glama
<400> 45
   Asp Ser Arg Arg Gly Gly Val Gly Asn Phe Phe Arg Ser
   1 5 10
<210> 46
   <211> 14
   <212> PRT
   <213> Lama Glama
<400> 46
   Pro Arg Gly Trp Gly Pro Thr Gly Pro Ile Glu Tyr Ala Tyr
   1 5 10
<210> 47
   <211> 8
   <212> PRT
   <213> Lama Glama
<400> 47
   Arg Arg Asn Phe Leu Ser Asn Tyr
   1 5
<210> 48
   <211> 11
   <212> PRT
   <213> Lama Glama
<400> 48
   Pro Gly Ile Ala Ala Cys Arg Gly Ile His Tyr
   1 5 10
<210> 49
   <211> 14
   <212> PRT
<210> 50
   <211> 20
   <212> PRT
   <213> Lama Glama
<400> 50
   Arg Leu Phe Ser Gly Gly Cys Ala Val Val Ala Arg Thr Ser Trp Ala
   1 5 10 15
   Asp Phe Gly Ser
   20
<210> 51
   <211> 11
   <212> PRT
   <213> Lama Glama
<400> 51
   Pro Gly Ile Ala Ala Cys Arg Gly Ile His Tyr
   1 5 10
<210> 52
   <211> 14
   <212> PRT
   <213> Lama Glama
<400> 52
   Pro Arg Gly Trp Gly Pro Thr Gly Pro His Glu Tyr Gly Tyr
   1 5 10
<210> 53
   <211> 11
   <212> PRT
   <213> Lama Glama
<400> 53
   Pro Val Lys Val Ala Gly Leu Glu Tyr Ala Tyr
   1 5 10
<210> 54
   <211> 13
   <212> PRT
   <213> Lama Glama
<400> 54
   Asp Val Gln His Ser Ala Trp Leu Lys Pro Leu Thr Tyr
   1 5 10
<210> 55
   <211> 3
   <212> PRT
   <213> Lama Glama
<400> 55
   Asp Ile Tyr
   1
<210> 56
   <211> 15
   <212> PRT
   <213> Lama Glama
<400> 56
   Pro Tyr Tyr Ser Asp Phe Glu Gly Thr Thr Thr Glu Tyr Asp Tyr
   1 5 10 15
<210> 57
   <211> 16
   <212> PRT
   <213> Lama Glama
<400> 57
   Thr Thr Arg Gly Arg Tyr Ser Ala Leu Ser Ala Ser Ala Tyr Asp Tyr
   1 5 10 15
<210> 58
   <211> 19
   <212> PRT
   <213> Lama Glama
<400> 58
   Asp Arg Tyr Ile Arg Ala Arg Gln Gly Asp Tyr Trp Gly Ala Tyr Glu
   1 5 10 15
   Tyr Asp Tyr
<210> 59
   <211> 21
   <212> PRT
   <213> Lama Glama
<400> 59
   Asp Pro Ile His Asn Cys Tyr Ser Gly Arg Tyr Tyr Ala Ser Pro Asp
   1 5 10 15
   Ala Val Tyr Asp Tyr
   20
<210> 60
   <211> 15
   <212> PRT
   <213> Lama Glama
<400> 60
   Asp Pro Leu Val Cys Gly Tyr Asn Asp Pro Arg Leu Ala Asp Tyr
   1 5 10 15
<210> 61
   <211> 16
   <212> PRT
   <213> Lama Glama
<400> 61
   Pro Phe Asn His Tyr Ser Asp Leu Cys Gly Val Asn Ala Ile Asp Tyr
   1 5 10 15
<210> 62
   <211> 16
   <212> PRT
   <213> Lama Glama
<400> 62
   Pro Phe Ser Tyr Tyr Ser Ser Leu Cys Gly Val Asn Glu Tyr Asp Tyr
   1 5 10 15
<210> 63
   <211> 16
   <212> PRT
   <213> Lama Glama
<400> 63
   Pro Phe Ser Tyr Tyr Asn Asn Leu Cys Gly Val Asn Gly Val Asp Tyr
   1 5 10 15
<210> 64
   <211> 20
   <212> PRT
   <213> Lama Glama
<400> 64
   Arg Leu Phe Ser Gly Gly Cys Ala Val Val Ala Gly Thr Ser Trp Ala
   1 5 10 15
   Asp Phe Gly Ser
   20
<210> 65
   <211> 16
   <212> PRT
   <213> Lama Glama
<400> 65
   Pro Phe Ala His Tyr Ser Asp Leu Cys Gly Val Asn Gly Tyr Asp Tyr
   1 5 10 15
<210> 66
   <211> 16
   <212> PRT
   <213> Lama Glama
<400> 66
   Pro Phe Ala Tyr Tyr Ser Asp Leu Cys Gly Val Asn Glu Tyr Asp Tyr
   1 5 10 15
<210> 67
   <211> 15
   <212> PRT
   <213> Lama Glama
<400> 67
   Pro His Ser Asp Tyr Asp Glu Glu Ala Pro Ser Asp Phe Gly Ser
   1 5 10 15
<210> 68
   <211> 20
   <212> PRT
   <213> Lama Glama
<400> 68
   Arg Leu Phe Ser Gly Gly Cys Ala Val Val Val Gly Thr Ser Trp Ala
   1 5 10 15
   Asp Phe Gly Ser
   20
<210> 69
   <211> 11
   <212> PRT
   <213> Lama Glama
<400> 69
   Ser Leu Gly Ser Ser Trp Cys Ala Tyr Asp Tyr
   1 5 10
<210> 70
   <211> 15
   <212> PRT
   <213> Lama Glama
<400> 70
   Asp Pro Leu Val Cys Gly Tyr Asn Asp Pro Arg Leu Ala Asp Tyr
   1 5 10 15
<210> 71
   <211> 20
   <212> PRT
   <213> Lama Glama
<400> 71
   Arg Leu Phe Ser Gly Gly Cys Ala Val Val Ala Gly Thr Ser Trp Ala
   1 5 10 15
   Asp Phe Gly Ser
   20
<210> 72
   <211> 15
   <212> PRT
   <213> Lama Glama
<400> 72
   Ser Gly Ser Tyr Tyr Tyr Pro Thr Asp Val His Glu Tyr Ala Tyr
   1 5 10 15
<210> 73
   <211> 16
   <212> PRT
   <213> Lama Glama
<400> 73
   Pro Phe Glu Tyr Tyr Ser Ala Tyr Cys Gly Val Asn Arg Tyr Asp Tyr
   1 5 10 15
<210> 74
   <211> 18
   <212> PRT
   <213> Lama Glama
<400> 74
   Ala His Asp Asn Tyr Trp Phe Thr Asp Asp Ser Leu Gly Arg Gly Leu
   1 5 10 15
   Lys Tyr
<210> 75
   <211> 11
   <212> PRT
   <213> Lama Glama
<400> 75
   Lys Thr Phe Gly Ser Asn Trp Tyr Asp Asp Tyr
   1 5 10
<210> 76
   <211> 21
   <212> PRT
   <213> Lama Glama
<400> 76
   Asp Pro Ile His Asn Cys Tyr Ser Gly Arg Tyr Tyr Ala Ser Pro Glu
   1 5 10 15
   Ala Val Tyr Asp Tyr
   20
<210> 77
   <211> 21
   <212> PRT
   <213> Lama Glama
<400> 77
   Asp Pro Phe His Asn Cys Tyr Ser Gly Ser His Tyr Ser Ser Pro Glu
   1 5 10 15
   Ala Val Tyr Glu Tyr
   20
<210> 78
   <211> 16
   <212> PRT
   <213> Lama Glama
<400> 78
   Pro Phe Glu Tyr Tyr Ser Asp Leu Cys Gly Val Asn Gly Tyr Asp Tyr
   1 5 10 15
<210> 79
   <211> 16
   <212> PRT
   <213> Lama Glama
<400> 79
   Pro Phe Asn Tyr Tyr Ser Asp Leu Cys Gly Val Asn Gly Val Asp Tyr
   1 5 10 15
<210> 80
   <211> 15
   <212> PRT
   <213> Lama Glama
<400> 80
   Ser Gly Ser Tyr Tyr Tyr Pro Thr Asp Val His Glu Tyr Ala Tyr
   1 5 10 15
<210> 81
   <211> 16
   <212> PRT
   <213> Lama Glama
<400> 81
   Pro Phe Ala His Tyr Ser Asp Leu Cys Gly Val Asn Gly Tyr Asp Tyr
   1 5 10 15
<210> 82
   <211> 8
   <212> PRT
   <213> Lama Glama
<400> 82
   Glu Met Asp Gly Ser Arg Tyr Val
   1 5
<210> 83
   <211> 9
   <212> PRT
   <213> Lama Glama
<400> 83
   Val His Pro Ser Thr Gly Phe Gly Ser
   1 5
<210> 84
   <211> 15
   <212> PRT
   <213> Lama Glama
<400> 84
   Pro His Ser Asp Tyr Asp Glu Glu Ala Pro Ser Asp Phe Gly Ser
   1 5 10 15
<210> 85
   <211> 20
   <212> PRT
   <213> Lama Glama
<400> 85
   Arg Leu Phe Ser Gly Gly Cys Ala Val Val Ala Ser Thr Ser Trp Ala
   1 5 10 15
   Asp Phe Gly Ser
   20
<210> 86
   <211> 20
   <212> PRT
   <213> Lama Glama
<400> 86
   Arg Leu Phe Arg Gly Gly Cys Ala Val Val Ala Gly Thr Ser Trp Ala
   1 5 10 15
   Asp Phe Gly Ser
   20
<210> 87
   <211> 15
   <212> PRT
   <213> Lama Glama
<400> 87
   Asp Pro Leu Val Cys Gly Tyr Asn Asp Pro Arg Leu Ala Asp Tyr
   1 5 10 15
<210> 88
   <211> 21
   <212> PRT
   <213> Lama Glama
<400> 88
   Asp Pro Ile His Asn Cys Tyr Ser Gly Arg Tyr Tyr Ala Ser Pro Asp
   1 5 10 15
   Ala Val Tyr Glu Tyr
   20
<210> 89
   <211> 21
   <212> PRT
   <213> Lama Glama
<400> 89
   Asp Pro Ile His Asn Cys Tyr Ser Gly Asn Gly Tyr Asp Ser Pro Glu
   1 5 10 15
   Ala Val Tyr Asp Tyr
   20
<210> 90
   <211> 21
   <212> PRT
   <213> Lama Glama
<400> 90
   Asp Pro Phe His Asn Cys Tyr Ser Gly Ser Ala Tyr Ser Ser Pro Glu
   1 5 10 15
   Ala Val Tyr Glu Tyr
   20
<210> 91
   <211> 11
   <212> PRT
   <213> Lama Glama
<400> 91
   Pro Val Lys Val Ala Gly Leu Glu Tyr Asp Tyr
   1 5 10
<210> 92
   <211> 11
   <212> PRT
   <213> Lama Glama
<400> 92
   Ser Val Gly Ser Ser Trp Cys Ala Tyr Asp Tyr
   1 5 10
<210> 93
   <211> 11
   <212> PRT
   <213> Lama Glama
<400> 93
   Ser Leu Gly Ser Ser Trp Cys Ala Tyr Asp Tyr
   1 5 10
<210> 94
   <211> 14
   <212> PRT
   <213> Lama Glama
<400> 94
   Asp Ser Leu Pro Cys Tyr Tyr Asp Lys Met Val Tyr Asp Tyr
   1 5 10
<210> 95
   <211> 14
   <212> PRT
   <213> Lama Glama
<400> 95
   Asp Ser Phe Ala Cys Asp Tyr Gly Lys Met Ile Tyr Asp Tyr
   1 5 10
<210> 96
   <211> 11
   <212> PRT
   <213> Lama Glama
<400> 96
   Ser Leu Gly Ser Ser Trp Cys Ala Tyr Asp Tyr
   1 5 10
<210> 97
   <211> 15
   <212> PRT
   <213> Lama Glama
<400> 97
   Asp Pro Leu Val Cys Gly Tyr Asn Asp Pro Arg Leu Ala Asp Tyr
   1 5 10 15
<210> 98
   <211> 15
   <212> PRT
   <213> Lama Glama
<400> 98
   Ser Gly Ser Tyr Tyr Tyr Pro Thr Asp Val His Glu Tyr Asp Tyr
   1 5 10 15
<210> 99
   <211> 16
   <212> PRT
   <213> Lama Glama
<400> 99
   Pro Phe Asn Tyr Tyr Ser Asn Leu Cys Gly Val Asn Gly Val Asp Tyr
   1 5 10 15
<210> 100
   <211> 21
   <212> PRT
   <213> Lama Glama
<400> 100
   Asp Pro Ile His Asn Cys Tyr Ser Gly Ser His Tyr Tyr Ser Pro Glu
   1 5 10 15
   Ala Val Tyr Glu Tyr
   20
<210> 101
   <211> 21
   <212> PRT
   <213> Lama Glama
<400> 101
   Asp Pro Ile His Asn Cys Tyr Ser Gly Ser Asp Tyr Ala Ser Pro Glu
   1               5                   10                  15
   Ala Val Tyr Glu Tyr
               20
<210> 102
   <211> 16
   <212> PRT
   <213> Lama Glama
<400> 102
   Pro Phe Ile His Tyr Ser Asp Leu Cys Gly Val Asn Gly Asn Asp Tyr
   1               5                   10                  15
<210> 103
   <211> 15
   <212> PRT
   <213> Lama Glama
<400> 103
   Ser Gly Ser Tyr Tyr Tyr Pro Thr Asp Val His Glu Tyr Asp Tyr
   1               5                   10                  15
<210> 104
   <211> 14
   <212> PRT
   <213> Lama Glama
<400> 104
   Ala Pro Ile Phe Glu Cys Pro Ser Gly Glu Ile Tyr Asp Tyr
   1               5                   10
<210> 105
   <211> 21
   <212> PRT
   <213> Lama Glama
<400> 105
   Asp Pro Ile His Asn Cys Tyr Ser Gly Thr Tyr Tyr Ala Ser Pro Glu
   1               5                   10                  15
   Ala Val Tyr Glu Tyr
               20
<210> 106
   <211> 21
   <212> PRT
   <213> Lama Glama
<400> 106
   Asp Pro Ile His Asn Cys Tyr Ser Gly Ser Tyr Tyr Ala Ser Pro Glu
   1 5 10 15
   Ala Val Tyr Asp Tyr
   20
<210> 107
   <211> 15
   <212> PRT
   <213> Lama Glama
<400> 107
   Ser Gly Ser Tyr Tyr Tyr Pro Thr Asp Val His Glu Tyr Ala Tyr
   1 5 10 15
<210> 108
   <211> 15
   <212> PRT
   <213> Lama Glama
<400> 108
   Ser Gly Ser Tyr Tyr Tyr Pro Thr Asp Val His Glu Tyr Ala Tyr
   1 5 10 15
<210> 109
   <211> 16
   <212> PRT
   <213> Lama Glama
<400> 109
   Pro Phe Ala Tyr Tyr Ser Asp Leu Cys Gly Val Asn Glu Tyr Asp Tyr
   1 5 10 15
<210> 110
   <211> 16
   <212> PRT
   <213> Lama Glama
<400> 110
   Pro Phe Asn His Tyr Ser Phe Leu Cys Gly Val Asn Glu Tyr Asp Tyr
   1 5 10 15
<210> 111
   <211> 15
   <212> PRT
   <213> Lama Glama
<400> 111
   Ser Gly Ser Tyr Tyr Tyr Pro Thr Glu Val Tyr Glu Tyr Asp Tyr
   1 5 10 15
<210> 112
   <211> 15
   <212> PRT
   <213> Lama Glama
<400> 112
   Ser Gly Ser Tyr Tyr Tyr Pro Thr Asp Val His Glu Tyr Asp Tyr
   1 5 10 15
<210> 113
   <211> 16
   <212> PRT
   <213> Lama Glama
<400> 113
   Pro Phe Glu Tyr Tyr Ser Asp Leu Cys Gly Val Asn Gly Tyr Asp Tyr
   1 5 10 15
<210> 114
   <211> 21
   <212> PRT
   <213> Lama Glama
<400> 114
   Asp Pro Ile His Asn Cys Tyr Gly Gly Ser Tyr Tyr Ala Ser Pro Glu
   1 5 10 15
   Ala Val Tyr Glu Tyr
   20
<210> 115
   <211> 12
   <212> PRT
   <213> Lama Glama
<400> 115
   Ala Gly Ala Ser Ser Trp Cys Phe Pro Pro Gly Tyr
   1 5 10
<210> 116
   <211> 21
   <212> PRT
   <213> Lama Glama
<400> 116
   Asp Pro Ile His Asn Cys Tyr Ser Gly Ile Tyr Tyr Ala Ser Pro Glu
   1 5 10 15
   Ala Val Tyr Asp Tyr
   20
<210> 117
   <211> 15
   <212> PRT
   <213> Lama Glama
<400> 117
   Ser Gly Ser Tyr Tyr Tyr Pro Thr Asp Val His Glu Tyr Asp Tyr
   1 5 10 15
<210> 118
   <211> 21
   <212> PRT
   <213> Lama Glama
<400> 118
   Asp Pro Ile His Asn Cys Tyr Ser Gly Arg Tyr Tyr Ala Ser Pro Asp
   1 5 10 15
   Ala Val Tyr Asp Tyr
   20
<210> 119
   <211> 16
   <212> PRT
   <213> Lama Glama
<400> 119
   Pro Phe Ser Tyr Tyr Ser Asp Leu Cys Gly Val Asn Gly Tyr Asp Tyr
   1 5 10 15
<210> 120
   <211> 15
   <212> PRT
   <213> Lama Glama
<400> 120
   Ser Gly Ser Tyr Tyr Tyr Pro Ser Asp Val His Glu Tyr Asp Tyr
   1 5 10 15
<210> 121
   <211> 16
   <212> PRT
   <213> Lama Glama
<400> 121
   Pro Phe Ser Tyr Tyr Ser Gly Leu Cys Gly Val Asn Gly Val Asp Tyr
   1 5 10 15
<210> 122
   <211> 11
   <212> PRT
   <213> Lama Glama
<400> 122
   Ser Leu Gly Ser Ser Trp Cys Ala Tyr Asp Tyr
   1 5 10
<210> 123
   <211> 21
   <212> PRT
   <213> Lama Glama
<400> 123
   Asp Pro Val His Asn Cys Tyr Ser Gly Arg Tyr Tyr Ala Ser Pro Asp
   1 5 10 15
   Ala Val Tyr Glu Tyr
   20
<210> 124
   <211> 15
   <212> PRT
   <213> Lama Glama
<400> 124
   Ser Gly Ser Tyr Tyr Tyr Pro Thr Asp Val His Glu Tyr Ala Tyr
   1 5 10 15
<210> 125
   <211> 16
   <212> PRT
   <213> Lama Glama
<400> 125
   Pro Phe Ser His Tyr Asn Asp Leu Cys Gly Val Asn Ala Ile Asp Tyr
   1 5 10 15
<210> 126
   <211> 21
   <212> PRT
   <213> Lama Glama
<400> 126
   Asp Pro Ile His Asn Cys Tyr Ser Gly Asn Gly Tyr Asp Ser Pro Glu
   1 5 10 15
   Ala Val Tyr Asp Tyr
   20
<210> 127
   <211> 16
   <212> PRT
   <213> Lama Glama
<400> 127
   Pro Phe Glu Tyr Tyr Ser Asp Leu Cys Gly Val Asn Gly Tyr Asp Tyr
   1 5 10 15
<210> 128
   <211> 16
   <212> PRT
   <213> Lama Glama
<400> 128
   Pro Phe Glu Tyr Tyr Ser Asn Leu Cys Gly Val Asn Gly Tyr Asp Tyr
   1 5 10 15
<210> 129
   <211> 21
   <212> PRT
   <213> Lama Glama
<400> 129
   Asp Pro Leu His Asn Cys Tyr Ser Gly Arg Gly Tyr Tyr Ser Pro Glu
   1 5 10 15
   Ala Val Tyr Glu Tyr
   20
<210> 130
   <211> 21
   <212> PRT
   <213> Lama Glama
<400> 130
   Asp Pro Ile His Asn Cys Tyr Ser Gly Ser Tyr Tyr Ala Ser Pro Glu
   1 5 10 15
   Ala Val Tyr Glu Tyr
   20
<210> 131
   <211> 16
   <212> PRT
   <213> Lama Glama
<400> 131
   Pro Phe Glu Tyr Tyr Ser Asn Leu Cys Gly Val Asn Gly Tyr Asp Tyr
   1 5 10 15
<210> 132
   <211> 11
   <212> PRT
   <213> Lama Glama
<400> 132
   Pro Gly Ile Ala Ala Cys Arg Gly Ile His Tyr
   1 5 10
<210> 133
   <211> 11
   <212> PRT
   <213> Lama Glama
<400> 133
   Pro Gly Ile Ala Ala Cys Arg Gly Ile His Tyr
   1 5 10
<210> 134
   <211> 11
   <212> PRT
   <213> Lama Glama
<400> 134
   Pro Gly Ile Ala Ala Cys Arg Gly Ile His Tyr
   1 5 10
<210> 135
   <211> 11
   <212> PRT
   <213> Lama Glama
<400> 135
   Pro Gly Ile Ala Ala Cys Arg Gly Ile His Tyr
   1 5 10
<210> 136
   <211> 11
   <212> PRT
   <213> Lama Glama
<400> 136
   Pro Gly Ile Ala Ala Cys Arg Gly Ile His Tyr
   1 5 10
<210> 137
   <211> 17
   <212> PRT
   <213> Lama Glama
<400> 137
   Lys Pro Asn Leu Lys Tyr Gly Ser Tyr Trp Pro Pro Arg Gly Tyr Asp
   1 5 10 15
   Tyr
<210> 138
   <211> 17
   <212> PRT
   <213> Lama Glama
<400> 138
   Lys Pro Asn Leu Lys Tyr Gly Ser Thr Trp Pro Pro Arg Gly Tyr Asp
   1               5                   10                  15
   Tyr
<210> 139
   <211> 17
   <212> PRT
   <213> Lama Glama
<400> 139
   Lys Pro Asn Leu Lys Tyr Gly Ser Tyr Trp Pro Pro Arg Gly Tyr Asp
   1               5                   10                  15
   Tyr
<210> 140
   <211> 17
   <212> PRT
   <213> Lama Glama
<400> 140
   Lys Pro Asn Leu Lys Tyr Gly Ser Asp Trp Pro Pro Arg Gly Tyr Asp
   1               5                   10                  15
   Tyr
<210> 141
   <211> 8
   <212> PRT
   <213> Lama Glama
<400> 141
   Arg Thr Ser Arg Ser Pro Arg Pro
   1               5
<210> 142
   <211> 15
   <212> PRT
   <213> Lama Glama
<400> 142
   Ser Asn Tyr Tyr Ser Val Tyr Asp Asp Arg Pro Val Met Asp Tyr
   1               5                   10                  15
<210> 143
   <211> 7
   <212> PRT
   <213> Lama Glama
<400> 143
   Gly Ser Gly Ser Trp Gly Val
   1 5
<210> 144
   <211> 19
   <212> PRT
   <213> Lama Glama
<400> 144
   Asn Glu Gly Tyr Cys Ser Gly Tyr Gly Cys Tyr Glu Asp Ser Gly Gln
   1 5 10 15
   Tyr Asp Tyr
<210> 145
   <211> 19
   <212> PRT
   <213> Lama Glama
<400> 145
   Asn Glu Gly Tyr Cys Ser Gly Tyr Gly Cys Tyr Glu Asp Ser Gly Gln
   1 5 10 15
   Tyr Asp Tyr
<210> 146
   <211> 12
   <212> PRT
   <213> Lama Glama
<400> 146
   Gly Gly Arg Ser Phe Leu Pro Phe Val Pro Ala Tyr
   1 5 10
<210> 147
   <211> 19
   <212> PRT
   <213> Lama Glama
<400> 147
   Asn Glu Gly Tyr Cys Ser Gly Tyr Gly Cys Tyr Glu Asp Ser Gly Gln
   1 5 10 15
   Tyr Asp Tyr
<210> 148
   <211> 19
   <212> PRT
   <213> Lama Glama
<400> 148
   Asn Gly Gly Tyr Cys Ser Gly Tyr Gly Cys Tyr Glu Asp Ser Gly Gln
   1 5 10 15
   Tyr Asp Tyr
<210> 149
   <211> 11
   <212> PRT
   <213> Lama Glama
<400> 149
   Pro Gly Ile Ala Ala Cys Arg Gly Ile His Tyr
   1 5 10
<210> 150
   <211> 11
   <212> PRT
   <213> Lama Glama
<400> 150
   Pro Gly Ile Ala Ala Cys Arg Gly Ile His Tyr
   1 5 10
<210> 151
   <211> 11
   <212> PRT
   <213> Lama Glama
<400> 151
   Pro Gly Ile Ala Ala Cys Arg Gly Ile His Tyr
   1 5 10
<210> 152
   <211> 14
   <212> PRT
   <213> Lama Glama
<400> 152
   Pro Arg Gly Trp Gly Pro Thr Gly Pro Ile Glu Tyr Ala Tyr
   1 5 10
<210> 153
   <211> 14
   <212> PRT
   <213> Lama Glama
<400> 153
   Pro Arg Gly Trp Gly Pro Thr Gly Pro Ile Glu Tyr Gly Tyr
   1 5 10
<210> 154
   <211> 14
   <212> PRT
   <213> Lama Glama
<400> 154
   Pro Arg Gly Trp Gly Pro Thr Gly Pro His Glu Tyr Gly Tyr
   1 5 10
<210> 155
   <211> 14
   <212> PRT
   <213> Lama Glama
<400> 155
   Pro Arg Gly Trp Gly Pro Thr Gly Pro His Glu Tyr Ala Tyr
   1 5 10
<210> 156
   <211> 12
   <212> PRT
   <213> Lama Glama
<400> 156
   Pro Ala Pro Gly Ser Ser Gly Tyr Glu Tyr Asp Tyr
   1 5 10
<210> 157
   <211> 11
   <212> PRT
   <213> Lama Glama
<400> 157
   Pro Gly Ile Ala Ala Cys Arg Gly Ile His Tyr
   1 5 10
<210> 158
   <211> 14
   <212> PRT
   <213> Lama Glama
<400> 158
   Pro Arg Gly Trp Gly Pro Thr Gly Pro His Glu Tyr Ala Tyr
   1 5 10
<210> 159
   <211> 14
   <212> PRT
   <213> Lama Glama
<400> 159
   Pro Arg Gly Trp Gly Pro Thr Gly Pro Leu Glu Tyr Gly Tyr
   1 5 10
<210> 160
   <211> 14
   <212> PRT
   <213> Lama Glama
<400> 160
   Pro Arg Gly Trp Gly Pro Thr Gly Pro His Glu Tyr Gly Tyr
   1 5 10
<210> 161
   <211> 14
   <212> PRT
   <213> Lama Glama
<400> 161
   Pro Arg Gly Trp Gly Pro Thr Gly Pro His Glu Tyr Gly Tyr
   1 5 10
<210> 162
   <211> 14
   <212> PRT
   <213> Lama Glama
<400> 162
   Pro Arg Gly Trp Gly Pro Thr Gly Pro His Glu Tyr Gly Tyr
   1 5 10
<210> 163
   <211> 14
   <212> PRT
   <213> Lama Glama
<400> 163
   Pro Arg Gly Trp Gly Pro Thr Gly Pro His Glu Tyr Gly Tyr
   1 5 10
<210> 164
   <211> 14
   <212> PRT
   <213> Lama Glama
<400> 164
   Pro Arg Gly Trp Gly Pro Thr Gly Pro His Glu Tyr Ala Tyr
   1 5 10
<210> 165
   <211> 14
   <212> PRT
   <213> Lama Glama
<400> 165
   Pro Arg Gly Trp Gly Pro Thr Gly Pro His Glu Tyr Gly Tyr
   1 5 10
<210> 166
   <211> 14
   <212> PRT
   <213> Lama Glama
<400> 166
   Pro Arg Gly Trp Gly Pro Thr Gly Pro His Glu Tyr Ala Tyr
   1 5 10
<210> 167
   <211> 125
   <212> PRT
   <213> Lama Glama
<400> 167
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Thr Tyr
   20 25 30
   Asn Ile Gly Trp Phe Arg Gln Ala Pro Gly Lys Glu Arg Glu Trp Val
   35 40 45
   Ser Cys Ile Ser Ser Ser Asp Gly Ser Thr Asn Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Asn Leu Lys Pro Glu Asp Thr Ala Val Tyr Tyr Cys
   85 90 95
   Ala Ala Pro Phe Ala Tyr Tyr Ser Asp Leu Cys Gly Val Asn Gly Val
   100 105 110
   Asp Tyr Trp Gly Gln Gly Thr Gln Val Thr Val Ser Ser
   115 120 125
<210> 168
   <211> 125
   <212> PRT
   <213> Lama Glama
<400> 168
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Asn Ile Gly Trp Phe Arg Gln Ala Pro Gly Lys Glu Arg Glu Trp Val
           35                  40                  45
   Ser Cys Ile Asn Ser Ser Asp Gly Ser Thr Tyr Tyr Thr Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Ile Tyr Tyr Cys
                   85                  90                  95
   Ala Ala Pro Phe Ser Tyr Tyr Ser His Leu Cys Gly Val Asn Gly Tyr
               100                 105                 110
   Asp Tyr Trp Gly Gln Gly Thr Gln Val Thr Val Ser Ser
           115                 120                 125
<210> 169
   <211> 125
   <212> PRT
   <213> Lama Glama
<400> 169
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Asn Ile Gly Trp Phe Arg Gln Ala Pro Gly Lys Glu Arg Glu Trp Val
           35                  40                  45
   Ser Cys Ile Ser Ser Ser Asp Gly Ser Thr Ala Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Ser Tyr Tyr Ser Ser Leu Cys Gly Val Asn Glu Tyr
               100                 105                 110
   Asp Tyr Trp Gly Gln Gly Thr Gln Val Thr Val Ser Ser
           115                 120                 125
<210> 170
   <211> 124
   <212> PRT
   <213> Lama Glama
<400> 170
   Glu Val Gln Leu Val Glu Ser Gly Gly Gly Leu Val Gln Pro Gly Gly
   1               5                   10                  15
   Ser Leu Arg Leu Ser Cys Ala Ile Ser Gly Phe Thr Leu Asp Leu His
               20                  25                  30
   Val Ile Gly Trp Leu Arg Gln Ala Pro Gly Lys Glu Arg Glu Trp Val
           35                  40                  45
   Ser Cys Ile Ser Ser Ser Asp Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Trp Asp Ser Trp Tyr Cys Gly Ile Gly Asn Asp Tyr Asp
   100 105 110
   Tyr Trp Gly Gln Gly Thr Gln Val Thr Val Ser Ser
   115 120
<210> 171
   <211> 125
   <212> PRT
   <213> Lama Glama
<400> 171
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Tyr Tyr
   20 25 30
   Asn Ile Gly Trp Phe Arg Gln Ala Pro Gly Lys Glu Arg Glu Trp Val
   35 40 45
   Ser Cys Ile Arg Gly Ser Asn Gly Ser Thr Gly Tyr Thr Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Ile His Tyr Ser Asp Leu Cys Gly Val Asn Gly Tyr
   100 105 110
   Asp Tyr Trp Gly Gln Gly Thr Gln Val Thr Val Ser Ser
   115 120 125
<210> 172
   <211> 131
   <212> PRT
   <213> Lama Glama
<400> 172
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Lys Tyr
               20                  25                  30
   Ala Ile Gly Trp Phe Arg Gln Ala Pro Gly Lys Glu Arg Glu Gly Val
           35                  40                  45
   Ser Cys Ile Ser Ser Arg Gly Gly Ser Thr Tyr Tyr Val Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Ile His Asn Cys Tyr Ser Gly Ser Ser Tyr Tyr Tyr
               100                 105                 110
   Ser Pro Glu Ala Val Tyr Asp Tyr Trp Gly Gln Gly Thr Gln Val Thr
           115                 120                 125
   Val Ser Ser
       130
<210> 173
   <211> 125
   <212> PRT
   <213> Lama Glama
<400> 173
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Asn Ile Gly Trp Phe Arg Gln Ala Pro Gly Lys Glu Arg Glu Trp Val
           35                  40                  45
   Ser Cys Ile Thr Ser Ser Asn Gly Ser Thr Tyr Tyr Thr Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Ala His Tyr Ser Asp Leu Cys Gly Val Asn Gly Tyr
               100                 105                 110
   Asp Tyr Trp Gly Gln Gly Thr Gln Val Thr Val Ser Ser
   115                 120                 125
<210> 174
   <211> 123
   <212> PRT
   <213> Lama Glama
<400> 174
   Glu Val Gln Leu Val Glu Ser Glu Gly Gly Leu Val Gln Ala Gly Gly
   1               5                   10                  15
   Ser Leu Arg Leu Ser Cys Ala Ala Ser Gly Ser Thr Phe Ser Ser Tyr
               20                  25                  30
   Ala Met Gly Trp Tyr Arg Gln Ala Pro Gly Lys Gln Arg Glu Leu Val
           35                  40                  45
   Ala Val Ile Ser Asn Gly Gly Ile Thr Asn Tyr Pro Asn Ser Val Lys
       50                  55                  60
   Gly Arg Phe Thr Ile Ser Arg Asp Asn Ala Lys Asn Thr Val Tyr Leu
   65                  70                  75                  80
   Gln Met Asn Ser Leu Lys Pro Glu Asp Thr Ala Val Tyr Tyr Cys Phe
                   85                  90                  95
   Tyr Ser Gly Ser Tyr Tyr Tyr Pro Thr Asp Val His Glu Tyr Asp Tyr
               100                 105                 110
   Trp Gly Gln Gly Thr Gln Val Thr Val Ser Ser
           115                 120
<210> 175
   <211> 125
   <212> PRT
   <213> Lama Glama
<400> 175
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Asn Ile Gly Trp Phe Arg Gln Ala Pro Gly Lys Glu Arg Glu Trp Val
           35                  40                  45
   Ser Cys Ile Asn Ser Ser Asp Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Glu Tyr Tyr Ser Asp Leu Cys Gly Val Asn Gly Tyr
               100                 105                 110
   Asp Tyr Trp Gly Gln Gly Thr Gln Val Thr Val Ser Ser
           115                 120                 125
<210> 176
   <211> 130
   <212> PRT
   <213> Lama Glama
<400> 176
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Tyr Tyr
               20                  25                  30
   Ala Ile Gly Trp Phe Arg Gln Ala Pro Gly Lys Glu Arg Glu Gly Ile
           35                  40                  45
   Ser Cys Ile Ser Ser Arg Gly Gly Ser Thr Phe Tyr Val Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Ile His Asn Cys Tyr Ser Gly Arg Tyr Tyr Tyr Ser
               100                 105                 110
   Pro Glu Ala Val Tyr Glu Tyr Trp Gly Gln Gly Thr Gln Val Thr Val
           115                 120                 125
   Ser Ser
       130
<210> 177
   <211> 125
   <212> PRT
   <213> Lama Glama
<400> 177
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Tyr His
   20 25 30
   Asn Ile Gly Trp Phe Arg Gln Ala Pro Gly Lys Glu Arg Glu Trp Val
   35 40 45
   Ser Cys Ile Ser Ser Ser Gly Gly Ser Thr Ala Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Ser His Tyr Ser Asp Leu Cys Gly Val Asn Ala Ile
   100 105 110
   Asp Tyr Trp Gly Gln Gly Thr Gln Val Thr Val Ser Ser
   115 120 125
<210> 178
   <211> 125
   <212> PRT
   <213> Lama Glama
<400> 178
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Tyr Tyr
   20 25 30
   Asn Ile Gly Trp Phe Arg Gln Ala Pro Gly Lys Glu Arg Glu Trp Val
   35 40 45
   Ser Cys Ile Asn Ser Ser Asp Gly Ser Thr Tyr Tyr Ala Asp Ser Val
   50 55 60
   Lys Gly Arg Phe Thr Val Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Glu Tyr Tyr Ser Asp Leu Cys Gly Val Asn Gly Tyr
   100 105 110
   Asp Tyr Trp Gly Gln Gly Thr Gln Val Thr Val Ser Ser
   115 120 125
<210> 179
   <211> 123
   <212> PRT
   <213> Lama Glama
<400> 179
   Glu Val Gln Leu Val Glu Ser Gly Gly Gly Leu Val Gln Ala Gly Gly
   1 5 10 15
   Ser Leu Arg Leu Ser Cys Ala Ala Ser Gly Ser Thr Phe Asn Ser Tyr
   20 25 30
   Ala Met Gly Trp Tyr Arg Gln Ala Pro Gly Lys Gln Arg Glu Trp Val
   35 40 45
   Ala Ala Phe Ser Thr Gly Gly Ser Thr Asn Tyr Ala Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr Tyr Cys Phe
   85 90 95
   Tyr Ser Gly Ser Tyr Tyr Tyr Pro Thr Asp Val Phe Glu Tyr Asp Tyr
   100 105 110
   Trp Gly Gln Gly Thr Gln Val Thr Val Ser Ser
   115 120
<210> 180
   <211> 130
   <212> PRT
   <213> Lama Glama
<400> 180
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Ala Leu Asp Tyr Tyr
               20                  25                  30
   Ala Val Gly Trp Phe Arg Gln Ala Pro Gly Lys Glu Arg Glu Gly Val
           35                  40                  45
   Ser Cys Ile Ser Ser Arg Gly Gly Ser Thr Phe Tyr Ala Asp Ser Val
       50                  55                  60
   Lys Gly Arg Phe Thr Thr Ser Arg Asn Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala His Pro Leu Gln Asn Cys Cys Gly Gly Ser Ala Tyr Ala Ser
               100                 105                 110
   Pro Glu Ala Val Tyr Glu Tyr Trp Gly Gln Gly Thr Gln Val Thr Val
           115                 120                 125
   Ser Ser
       130
<210> 181
   <211> 125
   <212> PRT
   <213> Lama Glama
<400> 181
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Asn Ile Gly Trp Phe Arg Gln Ala Pro Gly Lys Glu Arg Glu Trp Val
           35                  40                  45
   Ser Cys Ile Asn Ser Ser Asp Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Ala Tyr Tyr Ser Asn Leu Cys Gly Val Asn Gly Tyr
   100 105 110
   Asp Tyr Trp Gly Gln Gly Thr Gln Val Thr Val Ser Ser
   115 120 125
<210> 182
   <211> 130
   <212> PRT
   <213> Lama Glama
<400> 182
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Ala Leu Asp Tyr Tyr
   20 25 30
   Ala Val Gly Trp Phe Arg Gln Ala Pro Gly Lys Glu Arg Glu Gly Val
   35 40 45
   Ser Cys Ile Ser Ser Arg Gly Gly Ser Thr Tyr Tyr Val Asp Ser Val
   50 55 60
   Lys Gly Arg Phe Thr Thr Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Asp Pro Ile His Asn Cys Tyr Ser Gly Asn Tyr Tyr Ala Ser
   100 105 110
   Pro Glu Ala Val Tyr Asp Tyr Trp Gly Gln Gly Thr Gln Val Thr Val
   115 120 125
   Ser Ser
   130
<210> 183
   <211> 124
   <212> PRT
   <213> Lama Glama
<400> 183
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser His Asp Arg Thr Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Leu Val Cys Gly Tyr Asn Asp Pro Arg Leu Ala Asp
               100                 105                 110
   Tyr Trp Gly Gln Gly Thr Gln Val Thr Val Ser Ser
           115                 120
<210> 184
   <211> 125
   <212> PRT
   <213> Lama Glama
<400> 184
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Asn Ile Gly Trp Phe Arg Gln Ala Pro Gly Lys Glu Arg Glu Trp Val
           35                  40                  45
   Ser Cys Ile Thr Ser Ser Tyr Gly Ser Thr Tyr Tyr Thr Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Ala His Tyr Ser Asp Leu Cys Gly Val Asn Gly Tyr
               100                 105                 110
   Asp Tyr Trp Gly Gln Gly Thr Gln Val Thr Val Ser Ser
           115                 120                 125
<210> 185
   <211> 125
   <212> PRT
   <213> Lama Glama
<400> 185
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr His
               20                  25                  30
   Asn Ile Gly Trp Phe Arg Gln Ala Pro Gly Lys Glu Arg Glu Trp Val
           35                  40                  45
   Ser Cys Ile Ser Ser Ser Asp Gly Arg Thr Ala Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Thr His Tyr Ser Asp Leu Cys Gly Val Asn Glu Tyr
               100                 105                 110
   Asp Tyr Trp Gly Gln Gly Thr Gln Val Thr Val Ser Ser
           115                 120                 125
<210> 186
   <211> 120
   <212> PRT
   <213> Lama Glama
<400> 186
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Pro Glu Gly Ile
           35                  40                  45
   Gly Cys Ile Ser Ser Ser Gly Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Thr Pro Gly Ile Ala Ala Cys Arg Gly Ile His Tyr Trp Gly Gln
   100 105 110
   Gly Thr Gln Val Thr Val Ser Ser
   115 120
<210> 187
   <211> 120
   <212> PRT
   <213> Lama Glama
<400> 187
   Lys Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Pro Glu Gly Ile
   35 40 45
   Ser Cys Ile Ser Ser Ser Gly Gly Ile Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Thr Pro Gly Ile Ala Ala Cys Arg Gly Ile His Tyr Thr Gly Gln
   100 105 110
   Gly Thr Gln Val Thr Val Ser Ser
   115 120
<210> 188
   <211> 123
   <212> PRT
   <213> Lama Glama
<400> 188
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Asn Tyr
               20                  25                  30
   Asp Met Ser Trp Val Arg Gln Ala Pro Gly Lys Gly Pro Glu Trp Val
           35                  40                  45
   Ser Ala Ile Asn Ser Gly Gly Gly Thr Thr Tyr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Thr Pro Arg Gly Trp Gly Pro Thr Gly Pro His Glu Tyr Gly Tyr
               100                 105                 110
   Trp Gly Gln Gly Thr Gln Val Thr Val Ser Ser
           115                 120
<210> 189
   <211> 124
   <212> PRT
   <213> Lama Glama
<400> 189
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Asn Tyr
               20                  25                  30
   Ala Met Gly Trp Phe Arg Gln Ala Pro Gly Lys Glu Arg Glu Phe Val
           35                  40                  45
   Ala Ala Ile Ser Trp Ser Gly Gly Asp Thr Tyr Tyr Ala Asp Ser Val
       50                  55                  60
   Lys Gly Arg Phe Thr Ile Ser Arg Asp Asn Ala Lys Asn Thr Val Cys
   65                  70                  75                  80
   Leu Gln Met Asn Ser Leu Lys Pro Glu Asp Thr Ala Val Tyr Tyr Cys
                   85                  90                  95
   Ala Ala Ser Phe Gln Ser Gly Ala Ala Pro Gly Ala Asn Phe Tyr Asp
   100 105 110
   Tyr Trp Gly Gln Gly Thr Gln Val Thr Val Ser Ser
   115 120
<210> 190
   <211> 121
   <212> PRT
   <213> Lama Glama
<400> 190
   Glu Val Gln Leu Val Glu Ser Glu Gly Gly Ser Val Gln Ala Gly Gly
   1 5 10 15
   Ser Leu Arg Leu Ser Cys Ala Ala Ser Gly Arg Thr Phe Ser Ser Tyr
   20 25 30
   Ala Met Gly Trp Phe Arg Gln Ala Pro Gly Lys Glu Arg Glu Phe Val
   35 40 45
   Ala Ala Ile Asn Trp Ser Gly Gly Tyr Thr Tyr Tyr Ala Asp Ser Val
   50 55 60
   Arg Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Ala Pro Gly Ser Ser Gly Tyr Glu Tyr Asp Tyr Trp Gly
   100 105 110
   Gln Gly Thr Gln Val Thr Val Ser Ser
   115 120
<210> 191
   <211> 121
   <212> PRT
   <213> Lama Glama
<400> 191
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Phe Ser Ser Tyr
   20 25 30
   Ala Met Gly Trp Phe Arg Gln Ala Pro Gly Lys Glu Arg Glu Phe Val
   35 40 45
   Ala Ala Ile Phe Trp Ser Gly Gly Ser Thr Tyr Tyr Ala Asp Ser Val
   50 55 60
   Arg Gly Arg Phe Thr Ile Ser Arg Asp Ile Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Ser Pro Gly Ser Ser Gly Tyr Glu Tyr Asp Tyr Trp Gly
   100 105 110
   Gln Gly Thr Gln Val Thr Val Ser Ser
   115 120
<210> 192
   <211> 123
   <212> PRT
   <213> Lama Glama
<400> 192
   Glu Val Gln Leu Val Glu Ser Gly Gly Gly Leu Val Gln Thr Gly Asp
   1 5 10 15
   Ser Leu Arg Leu Ser Cys Ala Ala Ser Gly Ser Thr Phe Ser Asn Tyr
   20 25 30
   Arg Met Gly Trp Phe Arg Gln Gly Pro Gly Lys Glu Arg Glu Phe Val
   35 40 45
   Ala Ala Ile Gly Arg Asn Gly Gln Asn Thr Tyr Tyr Thr Asp Ser Val
   50 55 60
   Lys Gly Arg Phe Thr Val Thr Arg Asp Asn Ala Lys Asn Met Met Tyr
   65 70 75 80
   Leu Gln Met Asn Ser Leu Lys Pro Glu Asp Ser Ala Val Tyr Thr Cys
   85 90 95
   Ala Ala Ser Leu Arg Gly Trp Asp Thr Thr Arg Ile Asp Tyr Glu Tyr
   100 105 110
   Trp Gly Gln Gly Thr Gln Val Thr Val Ser Ser
   115 120
<210> 193
   <211> 120
   <212> PRT
   <213> Lama Glama
<400> 193
   Glu Val Gln Leu Val Glu Ser Gly Gly Gly Leu Val Gln Pro Gly Gly
   1               5                   10                  15
   Ser Leu Arg Leu Ser Cys Thr Ala Ser Gly Phe Thr Phe Asp Val Tyr
               20                  25                  30
   Ala Ile Gly Trp Phe Arg Gln Ala Pro Gly Lys Glu Pro Glu Gly Ile
           35                  40                  45
   Ser Cys Ile Ser Ser Ser Gly Ser Ile Thr Tyr Tyr Ala Asp Ser Val
       50                  55                  60
   Lys Gly Arg Phe Thr Thr Ser Arg Asp Ser Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Pro Gly Ile Ala Ala Cys Arg Gly Ile His Tyr Trp Gly Gln
               100                 105                 110
   Gly Thr Gln Val Thr Val Ser Ser
           115                 120
<210> 194
   <211> 123
   <212> PRT
   <213> Lama Glama
<400> 194
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Asn Tyr
               20                  25                  30
   Asp Met Ser Trp Val Arg Gln Ala Pro Gly Lys Gly Pro Glu Trp Val
           35                  40                  45
   Ser Ala Ile Asn Ser Gly Gly Asp Thr Thr Tyr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Thr Pro Arg Gly Trp Gly Pro Thr Gly Pro His Glu Tyr Gly Tyr
               100                 105                 110
   Trp Gly Gln Gly Thr Gln Val Thr Val Ser Ser
           115                 120
<210> 195
   <211> 121
   <212> PRT
   <213> Lama Glama
<400> 195
   Glu Val Gln Leu Val Glu Ser Arg Gly Gly Leu Val Gln Ala Gly Gly
   1               5                   10                  15
   Ser Leu Arg Leu Ser Cys Ala Ala Ser Gly Arg Thr Phe Asn Ser Tyr
               20                  25                  30
   Ala Met Gly Trp Phe Arg Gln Ala Pro Gly Lys Glu Arg Glu Phe Val
           35                  40                  45
   Ala Thr Ile Asn Trp Ser Gly Gly Ser Thr Tyr Tyr Ala Asp Ser Val
       50                  55                  60
   Lys Gly Arg Phe Thr Ile Ser Arg Asp Asn Ala Lys Asn Thr Ala Tyr
   65                  70                  75                  80
   Leu Gln Met Asn Ser Leu Lys Pro Glu Asp Thr Ala Val Tyr Tyr Cys
                   85                  90                  95
   Ala Ala Pro Ala Pro Gly Ser Ser Gly Tyr Glu Tyr Asp Tyr Trp Gly
               100                 105                 110
   Gln Gly Thr Gln Val Thr Val Ser Ser
           115                 120
<210> 196
   <211> 121
   <212> PRT
   <213> Lama Glama
<400> 196
   Glu Val Gln Leu Val Glu Ser Gly Gly Gly Ser Val Gln Ala Gly Gly
   1               5                   10                  15
   Ser Leu Arg Leu Ser Cys Ala Ala Ser Gly Arg Thr Phe Ser Ser Tyr
               20                  25                  30
   Ala Met Gly Trp Phe Arg Gln Ala Pro Gly Lys Glu Arg Glu Phe Val
           35                  40                  45
   Ala Ala Val Tyr Trp Ser Gly Gly Ser Thr Tyr Tyr Ala Asp Ser Val
       50                  55                  60
   Arg Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Ser Pro Gly Ser Ser Gly Tyr Glu Tyr Asp Tyr Trp Gly
               100                 105                 110
   Gln Gly Thr Gln Val Thr Val Ser Ser
           115                 120
<210> 197
   <211> 123
   <212> PRT
   <213> Lama Glama
<400> 197
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Phe Arg Gln Ala Pro Gly Lys Glu Arg Glu Phe Val
           35                  40                  45
   Ala Ala Ile Arg Trp Ser Gly Gly Thr Ala Tyr Tyr Ala Asp Ser Val
       50                  55                  60
   Gln Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Asn Arg Ala Ala Asp Thr Arg Leu Gly Pro Tyr Glu Tyr Asp Tyr
               100                 105                 110
   Trp Gly Gln Gly Thr Gln Val Thr Val Ser Ser
   115 120
<210> 198
   <211> 123
   <212> PRT
   <213> Lama Glama
<400> 198
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Gly Asn Tyr
   20 25 30
   Asp Met Ser Trp Val Arg Gln Ala Pro Gly Lys Gly Pro Glu Trp Val
   35 40 45
   Ser Ala Ile Asn Ser Gly Gly Gly Ile Thr Tyr Tyr Ala Asp Phe Val
   50 55 60
   Lys Gly Arg Phe Thr Ile Ser Arg Asp Asn Ala Lys Asn Thr Leu Tyr
   65 70 75 80
   Leu Gln Met Ser Ser Leu Lys Pro Glu Asp Thr Ala Val Tyr Tyr Cys
   85 90 95
   Ala Thr Pro Arg Gly Trp Gly Pro Thr Gly Pro His Glu Tyr Gly Tyr
   100 105 110
   Trp Gly Gln Gly Thr Gln Val Thr Val Ser Ser
   115 120
<210> 199
   <211> 120
   <212> PRT
   <213> Lama Glama
<400> 199
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Pro Glu Gly Ile
   35 40 45
   Ser Cys Ile Ser Ser Ser Gly Gly Ile Thr Tyr Tyr Thr Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Thr Pro Gly Ile Ala Ala Cys Arg Gly Ile His Tyr Thr Gly Gln
   100 105 110
   Gly Thr Gln Val Thr Val Ser Ser
   115 120
<210> 200
   <211> 122
   <212> PRT
   <213> Lama Glama
<400> 200
   Glu Val Gln Leu Val Glu Ser Gly Gly Gly Ser Ala Gln Ala Gly Gly
   1 5 10 15
   Ser Leu Arg Leu Ser Cys Ala Ala Ser Gly Arg Thr Ser Ser Thr Tyr
   20 25 30
   Ala Met Gly Trp Phe Arg Gln Ala Pro Gly Lys Glu His Glu Phe Val
   35 40 45
   Ser Ala Ile Gly Arg Gly Thr Gly Ala Thr Ser Tyr Gly Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Gln Leu Glu Asp Thr Gly Asp Tyr Tyr Cys
   85 90 95
   Val Ala Gly Arg Gly Phe Tyr His Asp Tyr Ser Ser Tyr Glu Tyr Arg
   100 105 110
   Gly Gln Gly Thr Gln Val Thr Val Ser Ser
   115 120
<210> 201
   <211> 118
   <212> PRT
   <213> Lama Glama
<400> 201
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Gly Tyr Tyr
               20                  25                  30
   Thr Ile Val Trp Phe Arg Gln Ala Pro Gly Lys Glu Arg Lys Gly Val
           35                  40                  45
   Ser Cys Ile Ser Ser Arg Asp Gly Ser Arg Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Arg Met Asn Ser Leu Lys Pro Glu Asp Thr Ala Val Tyr Tyr Cys
                   85                  90                  95
   Ala Ala Gly Pro Asp Cys Ser Ser Tyr Asp Tyr Trp Gly Gln Gly Thr
               100                 105                 110
   Gln Val Thr Val Ser Ser
           115
<210> 202
   <211> 123
   <212> PRT
   <213> Lama Glama
<400> 202
   Glu Val Gln Leu Val Glu Ser Gly Gly Gly Leu Val Gln Thr Gly Asp
   1               5                   10                  15
   Ser Leu Arg Leu Ser Cys Ala Ala Ser Gly Ser Thr Phe Ser Asn Tyr
               20                  25                  30
   Arg Met Gly Trp Phe Arg Gln Gly Pro Gly Lys Glu Arg Glu Phe Val
           35                  40                  45
   Ala Ala Ile Gly Arg Asn Gly Gln Asn Thr Tyr Tyr Thr Asp Ser Val
       50                  55                  60
   Lys Gly Arg Phe Thr Val Thr Arg Asp Asn Ala Lys Asn Met Val Tyr
   65                  70                  75                  80
   Leu Gln Met Asn Ser Leu Lys Pro Glu Asp Ser Ala Val Tyr Thr Cys
   85 90 95
   Ala Ala Ser Leu Arg Gly Trp Asp Thr Thr Arg Ile Asp Tyr Glu Tyr
   100 105 110
   Trp Gly Gln Gly Thr Gln Val Thr Val Ser Ser
   115 120
<210> 203
   <211> 121
   <212> PRT
   <213> Lama Glama
<400> 203
   Glu Val Gln Leu Met Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Arg Ile Thr Ser Gly Gly Arg Thr Thr Tyr Arg Asp Ser Val Lys
   50 55 60
   Gly Arg Phe Thr Ile Ser Arg Asp Asn Ser Lys Asn Thr Leu Tyr Leu
   65 70 75 80
   Gln Met Asn Ser Leu Lys Pro Glu Asp Thr Ala Leu Tyr Tyr Cys Ala
   85 90 95
   Lys Ala Arg Gly Asp Ile Asp Val Tyr Thr Leu Ser Asp Ser Arg Gly
   100 105 110
   Gln Gly Thr Gln Val Thr Val Ser Ser
   115 120
<210> 204
   <211> 121
   <212> PRT
   <213> Lama Glama
<400> 204
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Arg Ile Thr Ser Gly Gly Arg Ala Thr Tyr Arg Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Lys Pro Glu Asp Thr Ala Leu Tyr Tyr Cys Ala
                   85                  90                  95
   Lys Ala Arg Gly Asp Ile Asp Val Tyr Thr Leu Ser Asp Ser Arg Gly
               100                 105                 110
   Gln Gly Thr Gln Val Thr Val Ser Ser
           115                 120
<210> 205
   <211> 123
   <212> PRT
   <213> Lama Glama
<400> 205
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Asn Tyr
               20                  25                  30
   Asp Met Ser Trp Val Arg Arg Pro Pro Gly Lys Gly Pro Glu Trp Val
           35                  40                  45
   Ser Ala Ile Asn Ser Gly Gly Gly Ser Thr Tyr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Thr Pro Arg Gly Trp Gly Pro Thr Gly Pro His Glu Tyr Gly Tyr
               100                 105                 110
   Trp Gly Gln Gly Thr Gln Val Thr Val Ser Ser
                       115                 120
<210> 206
   <211> 129
   <212> PRT
   <213> Lama Glama
<400> 206
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr Tyr Tyr Ala Asp Ser Val
       50                  55                  60
   Lys Gly Arg Phe Thr Val Ser Ser Asn Asn Ala Asp Asp Thr Val Tyr
   65                  70                  75                  80
   Leu Gln Met Asn Ser Leu Lys Pro Glu Asp Thr Ala Val Tyr Tyr Cys
                   85                  90                  95
   Ala Val Arg Leu Phe Ser Gly Gly Cys Ala Val Val Ala Gly Thr Ser
               100                 105                 110
   Trp Ala Asp Phe Gly Ser Ser Gly Gln Gly Thr Gln Val Thr Val Ser
           115                 120                 125
   Ser
<210> 207
   <211> 129
   <212> PRT
   <213> Lama Glama
<400> 207
   Ala Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr His Tyr Ala Asp Ser Val
       50                  55                  60
   Lys Gly Arg Phe Thr Ile Ser Ser Asp Lys Val Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Val Arg Leu Phe Lys Gly Gly Cys Ala Val Val Ala Gly Thr Ser
               100                 105                 110
   Trp Ala Asp Phe Gly Ser Thr Gly Gln Gly Thr Gln Val Thr Val Ser
           115                 120                 125
   Ser
<210> 208
   <211> 119
   <212> PRT
   <213> Lama Glama
<400> 208
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Asp Ile Pro Arg Ile Ala
               20                  25                  30
   Ala Met Gly Trp Tyr Arg Gln Ala Pro Gly Lys Gln Arg Glu Leu Val
           35                  40                  45
   Ala Thr Val Ser Asn Ala Ala Thr Thr Arg Tyr Ala Asp Ser Ala Lys
       50                  55                  60
   Gly Arg Phe Thr Ile Ser Arg Asp Asn Ala Lys Thr Val Ser Leu Gln
   65                  70                  75                  80
   Met Asp Asn Leu Lys Pro Glu Asp Thr Gly Val Tyr Tyr Cys Tyr Ser
                   85                  90                  95
   Leu Ala Thr Thr Val Thr Pro Ser Trp Val Asn Tyr Trp Gly Gln Gly
               100                 105                 110
   Thr Gln Val Thr Val Ser Ser
           115
<210> 209
   <211> 123
   <212> PRT
   <213> Lama Glama
<400> 209
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ala Phe Gly Tyr Tyr
               20                  25                  30
   Asp Met Ser Trp Val Arg Gln Ala Pro Gly Lys Gly Pro Glu Trp Val
           35                  40                  45
   Ser Ala Ile Asn Ser Gly Gly Gly Ser Thr Tyr Tyr Ala Asp Ser Val
       50                  55                  60
   Lys Gly Arg Phe Thr Ile Ser Arg Asp Asn Ala Lys Asn Met Leu Tyr
   65                  70                  75                  80
   Leu Gln Met Asn Ser Leu Lys Pro Glu Asp Thr Ala Val Tyr Tyr Cys
                   85                  90                  95
   Ala Thr Pro Arg Gly Trp Gly Pro Thr Gly Pro His Glu Tyr Asp Tyr
               100                 105                 110
   Trp Gly Gln Gly Thr Gln Val Thr Val Ser Ser
           115                 120
<210> 210
   <211> 123
   <212> PRT
   <213> Lama Glama
<400> 210
   Glu Val Gln Leu Val Glu Ser Gly Gly Gly Leu Val Gln Ala Gly Gly
   1               5                   10                  15
   Ser Leu Arg Leu Ser Cys Asp Ala Ser Gly Arg Gly Phe Ser Tyr Tyr
               20                  25                  30
   Arg Met Gly Trp Phe Arg Gln Ala Pro Gly Lys Glu Arg Glu Phe Val
           35                  40                  45
   Ala Ala Ile Gly Lys Ser Gly Arg Asn Thr Tyr Tyr Gly Asp Tyr Val
       50                  55                  60
   Lys Gly Arg Phe Thr Val Ser Arg Asp Asn Ala Lys Asn Thr Val Tyr
   65 70 75 80
   Leu Gln Met Thr Ser Leu Lys Pro Glu Asp Thr Ala Val Tyr Thr Cys
   85 90 95
   Ala Ala Ser Leu Arg Gly Trp Asp Thr Thr Trp Ile Asp Tyr Glu Tyr
   100 105 110
   Trp Gly Gln Gly Thr Gln Val Thr Val Ser Ser
   115 120
<210> 211
   <211> 121
   <212> PRT
   <213> Lama Glama
<400> 211
   Glu Val Gln Leu Val Glu Ser Gly Gly Gly Ser Val Gln Ala Gly Gly
   1 5 10 15
   Ser Leu Arg Leu Ser Cys Ala Ala Ser Gly Ser Ile Ser Arg Ile Asp
   20 25 30
   Val Met Ala Trp Tyr Arg Gln Ala Pro Gly Lys Glu Arg Glu Leu Val
   35 40 45
   Ala Ser Ile Ser Ser Gly Gly Ser Thr Asn Tyr Ala Asp Ser Val Lys
   50 55 60
   Gly Arg Phe Thr Ile Ser Arg Glu Tyr Phe Lys Asn Met Met Tyr Leu
   65 70 75 80
   Gln Met Asn Ser Leu Lys Phe Glu Asp Thr Ala Val Tyr Tyr Cys Asn
   85 90 95
   Ala Asp Ser Arg Arg Gly Gly Val Gly Asn Phe Phe Arg Ser Trp Gly
   100 105 110
   Gln Gly Thr Gln Val Thr Val Ser Ser
   115 120
<210> 212
   <211> 123
   <212> PRT
   <213> Lama Glama
<400> 212
   Glu Val Gln Leu Val Glu Ser Arg Gly Gly Leu Val Gln Pro Gly Gly
   1               5                   10                  15
   Ser Leu Arg Leu Ser Cys Ala Ala Ser Gly Phe Thr Phe Gly Ser Tyr
               20                  25                  30
   Asp Met Ser Trp Val Arg Gln Ala Pro Gly Lys Gly Pro Glu Trp Val
           35                  40                  45
   Ser Ala Ile Asn Ser Gly Gly Gly Ser Thr Tyr Tyr Ala Asp Ser Val
       50                  55                  60
   Lys Gly Arg Phe Thr Ile Ser Arg Asp Asn Ala Lys Ser Thr Leu Tyr
   65                  70                  75                  80
   Leu Gln Met Asn Ser Leu Lys Pro Glu Asp Thr Ala Val Tyr Tyr Cys
                   85                  90                  95
   Ala Ile Pro Arg Gly Trp Gly Pro Thr Gly Pro Ile Glu Tyr Ala Tyr
               100                 105                 110
   Trp Gly Gln Gly Thr Gln Val Thr Val Ser Ser
           115                 120
<210> 213
   <211> 116
   <212> PRT
   <213> Lama Glama
<400> 213
   Glu Val Gln Leu Val Glu Ser Gly Gly Gly Leu Val Gln Ala Gly Gly
   1               5                   10                  15
   Ser Leu Arg Leu Ser Cys Ala Ala Ala Gly Ser Thr Phe Ser Ser Tyr
               20                  25                  30
   Val Met Gly Trp Tyr Arg Gln Ala Pro Gly Lys Gln Arg Glu Leu Val
           35                  40                  45
   Ala His Ile Ser Thr Arg Gly Ile Thr Tyr Tyr Ala Asp Ser Val Lys
       50                  55                  60
   Gly Arg Phe Thr Ile Ser Arg Asp Asn Ala Lys Asn Thr Met Tyr Leu
   65                  70                  75                  80
   Gln Met Asn Ser Leu Lys Pro Glu Asp Thr Ala Val Tyr Tyr Cys Asn
                   85                  90                  95
   Thr Arg Arg Asn Phe Leu Ser Asn Tyr Trp Gly Gln Gly Thr Gln Val
   100 105 110
   Thr Val Ser Ser
   115
<210> 214
   <211> 120
   <212> PRT
   <213> Lama Glama
<400> 214
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Pro Glu Gly Ile
   35 40 45
   Ser Cys Ile Ser Ser Ser Gly Gly Ile Thr Tyr Tyr Ala Asp Ser Val
   50 55 60
   Lys Gly Arg Phe Thr Ile Ser Arg Asp Asn Ala Lys Ser Thr Val Tyr
   65 70 75 80
   Leu Gln Met Asn Ser Leu Lys Pro Glu Asp Thr Ala Val Tyr Tyr Cys
   85 90 95
   Ala Thr Pro Gly Ile Ala Ala Cys Arg Gly Ile His Tyr Thr Gly Gln
   100 105 110
   Gly Thr Gln Val Thr Val Ser Ser
   115 120
<210> 215
   <211> 123
   <212> PRT
   <213> Lama Glama
<400> 215
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Ala Trp Cys Asp Ser Ser Trp Tyr Arg Ser Phe Val Gly Tyr
               100                 105                 110
   Trp Gly Gln Gly Thr Gln Val Thr Val Ser Ser
           115                 120
<210> 216
   <211> 129
   <212> PRT
   <213> Lama Glama
<400> 216
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Asp Ser Thr Tyr Tyr Ala Asp Ser Val
   50 55 60
   Lys Gly Arg Phe Thr Ile Ser Ser Asn Asn Ala Lys Asn Thr Ala Tyr
   65 70 75 80
   Leu Gln Met Asn Ser Leu Lys Pro Glu Asp Thr Ala Val Tyr Tyr Cys
   85 90 95
   Ala Val Arg Leu Phe Ser Gly Gly Cys Ala Val Val Ala Arg Thr Ser
   100 105 110
   Trp Ala Asp Phe Gly Ser Ser Gly Gln Gly Thr Gln Val Thr Val Ser
   115 120 125
   Ser
<210> 217
   <211> 120
   <212> PRT
   <213> Lama Glama
<400> 217
   Glu Val Gln Leu Val Glu Ser Gly Gly Gly Phe Val Gln Pro Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Pro Glu Glu Ile
           35                  40                  45
   Ser Cys Ile Ser Ser Ser Gly Gly Ile Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Pro Gly Ile Ala Ala Cys Arg Gly Ile His Tyr Trp Gly Gln
               100                 105                 110
   Gly Thr Gln Val Thr Val Ser Ser
           115                 120
<210> 218
   <211> 123
   <212> PRT
   <213> Lama Glama
<400> 218
   Glu Val Gln Leu Val Glu Ser Gly Gly Gly Leu Val Gln Ser Gly Gly
   1               5                   10                  15
   Ser Leu Arg Leu Ser Cys Ala Ala Ser Gly Phe Thr Phe Gly Ser Tyr
               20                  25                  30
   Asp Met Ser Trp Val Arg Gln Ala Pro Gly Lys Gly Pro Glu Trp Val
           35                  40                  45
   Ser Ala Ile Asn Ser Gly Gly Gly Ile Thr Tyr Tyr Ala Asp Ser Val
       50                  55                  60
   Asn Gly Arg Phe Thr Ile Ser Arg Asp Asn Thr Lys Asn Thr Leu Tyr
   65 70 75 80
   Leu Gln Met Asn Ser Leu Lys Pro Glu Asp Thr Ala Val Tyr Tyr Cys
   85 90 95
   Ala Thr Pro Arg Gly Trp Gly Pro Thr Gly Pro His Glu Tyr Gly Tyr
   100 105 110
   Trp Gly Gln Gly Thr Gln Val Thr Val Ser Ser
   115 120
<210> 219
   <211> 119
   <212> PRT
   <213> Lama Glama
<400> 219
   Glu Val Gln Leu Val Glu Ser Gly Gly Gly Leu Val Gln Ala Gly Gly
   1 5 10 15
   Ser Leu Arg Leu Ser Cys Ala Ala Ser Gly Ser Thr Phe Ser Asn Tyr
   20 25 30
   Ala Met Gly Trp Tyr Arg Gln Ala Pro Gly Lys Gln Arg Glu Leu Val
   35 40 45
   Val Gly Ile Ser Ser Asp Gly Ser Thr His Tyr Ala Asp Ser Ala Lys
   50 55 60
   Gly Arg Phe Thr Ile Ser Arg Asp Asp Ala Lys Asn Thr Val Tyr Leu
   65 70 75 80
   Gln Met Asn Ser Leu Lys Thr Glu Asp Thr Ala Val Tyr Tyr Cys Tyr
   85 90 95
   Val Pro Val Lys Val Ala Gly Leu Glu Tyr Ala Tyr Trp Gly Gln Gly
   100 105 110
   Thr Gln Val Thr Val Ser Ser
   115
<210> 220
   <211> 121
   <212> PRT
   <213> Lama Glama
<400> 220
   Glu Val Gln Leu Val Glu Ser Gly Gly Gly Leu Val Gln Pro Gly Gly
   1 5 10 15
   Ser Leu Arg Leu Ser Cys Glu Val Ser Gly Ser Ile Gly Ser Val Ser
   20 25 30
   Asp Met Arg Trp Tyr Arg Gln Ala Pro Gly Leu Gln Tyr Glu Leu Val
   35 40 45
   Ala Arg Ile Thr Ser Gly Ser Ile Thr Asp Tyr Ser Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr Tyr Cys Asn
   85 90 95
   Ala Asp Val Gln His Ser Ala Trp Leu Lys Pro Leu Thr Tyr Trp Gly
   100 105 110
   Gln Gly Thr Gln Val Thr Val Ser Ser
   115 120
<210> 221
   <211> 111
   <212> PRT
   <213> Lama Glama
<400> 221
   Glu Val Gln Leu Val Glu Ser Gly Gly Gly Leu Val Gln Pro Gly Gly
   1 5 10 15
   Ser Leu Arg Leu Ser Cys Ala Ala Ser Gly Ile Arg Phe Ser Ile Asn
   20 25 30
   Gly Met Gly Trp Tyr Arg Gln Ala Pro Gly Lys Gln Arg Glu Ala Val
   35 40 45
   Ala Thr Ile Thr Arg Gly Gly Ile Arg Asp Tyr Thr Asp Ser Val Lys
   50 55 60
   Gly Arg Phe Thr Ile Ser Arg Asp Ile Ala Arg Asn Thr Val Tyr Leu
   65 70 75 80
   Gln Met Asn Asn Leu Lys Pro Glu Asp Ser Ala Val Tyr Tyr Cys Asn
   85 90 95
   Ile Asp Ile Tyr Trp Gly Arg Gly Thr Gln Val Thr Val Ser Ser
   100 105 110
<210> 222
   <211> 121
   <212> PRT
   <213> Lama Glama
<400> 222
   Glu Val Gln Leu Val Glu Ser Gly Gly Gly Leu Val Gln Ala Gly Gly
   1 5 10 15
   Ser Leu Arg Leu Ser Cys Ala Ala Phe Gly Arg Thr Pro Tyr Gly Met
   20 25 30
   Gly Trp Phe Arg Gln Ala Pro Gly Lys Glu Arg Glu Phe Val Ala Ala
   35 40 45
   Ile Thr Ser Asp Gly Ser Thr Asn Tyr Ala Asp Ser Val Lys Gly Arg
   50 55 60
   Phe Thr Ile Ser Arg Asp Asn Ala Lys Asn Ala Val Ser Leu Gln Met
   65 70 75 80
   Asn Ser Leu Lys Pro Glu Asp Thr Ala Val Tyr Tyr Cys Thr Ala Pro
   85 90 95
   Tyr Tyr Ser Asp Phe Glu Gly Thr Thr Thr Glu Tyr Asp Tyr Trp Gly
   100 105 110
   Gln Gly Thr Gln Val Thr Val Ser Ser
   115 120
<210> 223
   <211> 128
   <212> PRT
   <213> Lama Glama
<400> 223
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Val Arg Ser Tyr
   20 25 30
   Ala Thr Gly Trp Phe Arg Gln Ala Pro Gly Lys Glu Arg Glu Phe Val
   35 40 45
   Ala Ala Leu Arg Trp Ser Ile Gly Ser Ile Ala Ser Val Tyr Tyr Asp
   50 55 60
   Asp Ser Val Lys Gly Arg Phe Thr Ile Ser Gly Asp Asn Ala Glu Asn
   65 70 75 80
   Thr Val Tyr Leu Gln Met Asn Ala Leu Lys Pro Glu Asp Thr Ala Ile
   85 90 95
   Tyr Tyr Cys Ala Ser Thr Thr Arg Gly Arg Tyr Ser Ala Leu Ser Ala
   100 105 110
   Ser Ala Tyr Asp Tyr Trp Gly Gln Gly Thr Gln Val Thr Val Ser Ser
   115 120 125
<210> 224
   <211> 128
   <212> PRT
   <213> Lama Glama
<400> 224
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Gly Ser Tyr
   20 25 30
   Asp Met Ser Trp Val Arg Arg Ser Pro Gly Lys Gly Pro Glu Trp Val
   35 40 45
   Ser Ser Ile Asn Ser Gly Gly Gly Ser Thr Tyr Tyr Ala Asp Phe Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Ala Asp Arg Tyr Ile Arg Ala Arg Gln Gly Asp Tyr Trp Gly Ala
   100 105 110
   Tyr Glu Tyr Asp Tyr Trp Gly Gln Gly Thr Gln Val Thr Val Ser Ser
   115 120 125
<210> 225
   <211> 130
   <212> PRT
   <213> Lama Glama
<400> 225
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Arg Gly Gly Ser Thr Tyr Tyr Glu Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Ile His Asn Cys Tyr Ser Gly Arg Tyr Tyr Ala Ser
               100                 105                 110
   Pro Asp Ala Val Tyr Asp Tyr Trp Gly Gln Gly Thr Gln Val Thr Val
           115                 120                 125
   Ser Ser
       130
<210> 226
   <211> 124
   <212> PRT
   <213> Lama Glama
<400> 226
   Lys Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser His Asp Gly Thr Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Leu Val Cys Gly Tyr Asn Asp Pro Arg Leu Ala Asp
               100                 105                 110
   Tyr Trp Gly Gln Gly Thr Gln Val Thr Val Ser Ser
           115                 120
<210> 227
   <211> 125
   <212> PRT
   <213> Lama Glama
<400> 227
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Tyr His
   20 25 30
   Asn Ile Gly Trp Phe Arg Gln Ala Pro Gly Lys Glu Arg Glu Trp Val
   35 40 45
   Ser Cys Ile Ser Ser Ser Gly Gly Ser Thr Ala Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Asn His Tyr Ser Asp Leu Cys Gly Val Asn Ala Ile
   100 105 110
   Asp Tyr Trp Gly Gln Gly Thr Gln Val Thr Val Ser Ser
   115 120 125
<210> 228
   <211> 125
   <212> PRT
   <213> Lama Glama
<400> 228
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Tyr Tyr
   20 25 30
   Asn Ile Gly Trp Phe Arg Gln Ala Pro Gly Lys Glu Arg Glu Trp Val
   35 40 45
   Ser Cys Ile Ser Ser Ser Asp Gly Ser Thr Ala Tyr Ala Asp Ser Val
   50 55 60
   Lys Gly Arg Phe Thr Ile Ser Arg Asp Asn Gly Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Ser Tyr Tyr Ser Ser Leu Cys Gly Val Asn Glu Tyr
   100 105 110
   Asp Tyr Trp Gly Gln Gly Thr Gln Val Thr Val Ser Ser
   115 120 125
<210> 229
   <211> 125
   <212> PRT
   <213> Lama Glama
<400> 229
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Tyr Tyr
   20 25 30
   Asn Ile Gly Trp Phe Arg Gln Ala Pro Gly Lys Glu Arg Glu Trp Val
   35 40 45
   Ser Cys Ile Ser Ser Thr Asn Gly Asn Thr Tyr Tyr Ala Asp Ser Val
   50 55 60
   Lys Gly Arg Phe Ser Ile Ser Arg Asp Asn Ala Arg Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Ser Tyr Tyr Asn Asn Leu Cys Gly Val Asn Gly Val
   100 105 110
   Asp Tyr Trp Gly Gln Gly Thr Gln Val Thr Val Ser Ser
   115 120 125
<210> 230
   <211> 129
   <212> PRT
   <213> Lama Glama
<400> 230
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Asp Ser Thr Tyr Tyr Ala Asp Ser Val
   50 55 60
   Lys Gly Arg Phe Thr Ile Ser Ser Asn Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Val Arg Leu Phe Ser Gly Gly Cys Ala Val Val Ala Gly Thr Ser
   100 105 110
   Trp Ala Asp Phe Gly Ser Ser Gly Gln Gly Thr Gln Val Thr Val Ser
   115 120 125
   Ser
<210> 231
   <211> 125
   <212> PRT
   <213> Lama Glama
<400> 231
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Tyr Tyr
   20 25 30
   Asn Ile Gly Trp Phe Arg Gln Ala Pro Gly Lys Glu Arg Glu Gly Val
   35 40 45
   Ser Cys Ile Thr Ser Ser Asn Gly Ser Thr Tyr Tyr Thr Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Ala His Tyr Ser Asp Leu Cys Gly Val Asn Gly Tyr
   100 105 110
   Asp Tyr Trp Gly Gln Gly Thr Gln Val Thr Val Ser Ser
   115 120 125
<210> 232
   <211> 125
   <212> PRT
   <213> Lama Glama
<400> 232
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
   20 25 30
   Asn Ile Gly Trp Phe Arg Gln Ala Pro Gly Lys Glu Arg Glu Trp Val
   35 40 45
   Ser Cys Ile Ser Ser Ser Asp Gly Ser Thr Gly Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Ala Tyr Tyr Ser Asp Leu Cys Gly Val Asn Glu Tyr
   100 105 110
   Asp Tyr Trp Gly Gln Gly Thr Gln Val Thr Val Ser Ser
   115 120 125
<210> 233
   <211> 123
   <212> PRT
   <213> Lama Glama
<400> 233
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Ser Thr Phe Thr Ser Tyr
               20                  25                  30
   Ala Met Gly Trp Tyr Arg Gln Ala Pro Gly Lys Gln Arg Glu Leu Val
           35                  40                  45
   Ala Ala Ile Ser Ser Asp Asp Ser Thr Tyr Tyr Ala Asp Cys Val Lys
       50                  55                  60
   Gly Arg Phe Thr Ile Ser Arg Asp Tyr Ala Lys Asn Thr Val Tyr Leu
   65                  70                  75                  80
   Gln Met Asn Ser Leu Lys Pro Glu Asp Thr Ala Val Tyr Tyr Cys Asn
                   85                  90                  95
   Ala Pro His Ser Asp Tyr Asp Glu Glu Ala Pro Ser Asp Phe Gly Ser
               100                 105                 110
   Trp Gly Gln Gly Thr Gln Val Thr Val Ser Ser
           115                 120
<210> 234
   <211> 129
   <212> PRT
   <213> Lama Glama
<400> 234
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Asp Ser Thr Tyr Tyr Ala Asp Ser Val
       50                  55                  60
   Lys Gly Arg Phe Thr Ile Ser Arg Asn Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Val Arg Leu Phe Ser Gly Gly Cys Ala Val Val Val Gly Thr Ser
               100                 105                 110
   Trp Ala Asp Phe Gly Ser Ser Gly Gln Gly Thr Gln Val Thr Val Ser
           115                 120                 125
   Ser
<210> 235
   <211> 120
   <212> PRT
   <213> Lama Glama
<400> 235
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Glu Asn Tyr
               20                  25                  30
   Ala Leu Gly Trp Phe Arg Gln Ala Pro Gly Lys Glu Arg Glu Trp Val
           35                  40                  45
   Ser Cys Ile Ser Ser Ser Asp Gly Thr Thr Tyr Tyr Ala Asp Ser Val
       50                  55                  60
   Lys Gly Arg Phe Thr Ile Ser Arg Asp Asn Val Lys Asn Thr Val Tyr
   65                  70                  75                  80
   Leu Gln Met Asn Arg Leu Lys Pro Glu Asp Thr Ala Ile Tyr Tyr Cys
                   85                  90                  95
   Ala Leu Ser Leu Gly Ser Ser Trp Cys Ala Tyr Asp Tyr Trp Gly Gln
               100                 105                 110
   Gly Thr Gln Val Thr Val Ser Ser
           115                 120
<210> 236
   <211> 124
   <212> PRT
   <213> Lama Glama
<400> 236
   Glu Val Gln Leu Met Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Tyr Asp Gly Thr Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Leu Val Cys Gly Tyr Asn Asp Pro Arg Leu Ala Asp
               100                 105                 110
   Tyr Trp Gly Gln Gly Thr Gln Val Thr Val Ser Ser
           115                 120
<210> 237
   <211> 129
   <212> PRT
   <213> Lama Glama
<400> 237
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Pro Ile Gly Trp Leu Arg Gln Ala Pro Gly Lys Glu Arg Glu Gly Val
           35                  40                  45
   Ser Cys Ile Ser Ser Ser Asp Asp Ser Thr Tyr Tyr Ala Asp Ser Val
       50                  55                  60
   Lys Gly Arg Phe Thr Ile Ser Ser Asn Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Val Arg Leu Phe Ser Gly Gly Cys Ala Val Val Ala Gly Thr Ser
               100                 105                 110
   Trp Ala Asp Phe Gly Ser Ser Gly Gln Gly Thr Gln Val Thr Val Ser
           115                 120                 125
   Ser
<210> 238
   <211> 123
   <212> PRT
   <213> Lama Glama
<400> 238
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Ser Thr Phe Ser Ser Tyr
               20                  25                  30
   Ala Met Gly Trp Tyr Arg Gln Ala Pro Gly Lys Gln Arg Glu Leu Val
           35                  40                  45
   Ala Val Ile Ser Ser Gly Asp Arg Thr Asn Tyr Leu Asp Ser Val Lys
       50                  55                  60
   Gly Arg Phe Thr Ile Ser Arg Asp Asn Ala Lys Asn Thr Val Tyr Leu
   65                  70                  75                  80
   Gln Met Asn Ser Leu Lys Pro Glu Asp Thr Ala Val Tyr Tyr Cys Phe
                   85                  90                  95
   Tyr Ser Gly Ser Tyr Tyr Tyr Pro Thr Asp Val His Glu Tyr Ala Tyr
               100                 105                 110
   Trp Gly Gln Gly Thr Gln Val Thr Val Ser Ser
           115                 120
<210> 239
   <211> 125
   <212> PRT
   <213> Lama Glama
<400> 239
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
   20 25 30
   Asn Ile Gly Trp Phe Arg Gln Ala Pro Gly Lys Glu Arg Glu Trp Val
   35 40 45
   Ser Cys Ile Ser Ser Gly Asp Gly Ser Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Glu Tyr Tyr Ser Ala Tyr Cys Gly Val Asn Arg Tyr
   100 105 110
   Asp Tyr Trp Gly Gln Gly Thr Gln Val Thr Val Ser Ser
   115 120 125
<210> 240
   <211> 127
   <212> PRT
   <213> Lama Glama
<400> 240
   Glu Val Gln Leu Val Glu Ser Gly Gly Gly Leu Val Gln Ala Gly Gly
   1 5 10 15
   Ser Leu Arg Leu Ser Cys Ala Ser Ser Gly Arg Thr Leu Leu Asn Tyr
   20 25 30
   Ala Met Gly Trp Phe Arg Gln Ala Pro Gly Lys Glu Arg Glu Phe Val
   35 40 45
   Ser Gly Ile Asn Trp Ser Gly Gly Ser Thr Tyr Tyr Ala Asp Ser Val
   50 55 60
   Lys Gly Arg Phe Thr Ile Ser Arg Asp Asn Ala Glu Asn Thr Val Tyr
   65 70 75 80
   Leu His Met Asn Ser Leu Lys Pro Glu Asp Thr Ala Val Tyr Tyr Cys
   85 90 95
   Ala Ala Ala His Asp Asn Tyr Trp Phe Thr Asp Asp Ser Leu Gly Arg
   100 105 110
   Gly Leu Lys Tyr Trp Gly Gln Gly Thr Gln Val Thr Val Ser Ser
   115 120 125
<210> 241
   <211> 120
   <212> PRT
   <213> Lama Glama
<400> 241
   Glu Val Gln Leu Val Glu Ser Gly Gly Gly Leu Val Gln Ala Gly Gly
   1 5 10 15
   Ser Leu Arg Leu Ser Cys Ala Ala Ser Gly Ser Thr Phe Ser Ser Tyr
   20 25 30
   Ala Met Gly Trp Tyr Arg His Gln Ala Pro Gly Lys Gln Arg Glu Leu
   35 40 45
   Val Ala Ala Ile Ser Ser Asp Gly Ser Thr His Tyr Ala Asp Ser Val
   50 55 60
   Lys Gly Arg Phe Thr Ile Ser Arg Asp Asn Ala Lys Asn Thr Met Tyr
   65 70 75 80
   Leu Gln Met Asn Ser Leu Lys Pro Glu Asp Thr Ala Val Tyr Tyr Cys
   85 90 95
   Asn Thr Lys Thr Phe Gly Ser Asn Trp Tyr Asp Asp Tyr Trp Gly Gln
   100 105 110
   Gly Thr Gln Val Thr Val Ser Ser
   115 120
<210> 242
   <211> 130
   <212> PRT
   <213> Lama Glama
<400> 242
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Leu Asp Tyr Tyr
   20 25 30
   Ala Ile Gly Trp Phe Arg Gln Ala Pro Gly Lys Glu Arg Glu Gly Val
   35 40 45
   Ser Cys Ile Ser Ser Arg Gly Gly Ser Thr Tyr Tyr Thr Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Gly Val Tyr Tyr Cys
   85 90 95
   Ala Ala Asp Pro Ile His Asn Cys Tyr Ser Gly Arg Tyr Tyr Ala Ser
   100 105 110
   Pro Glu Ala Val Tyr Asp Tyr Trp Gly Gln Gly Thr Gln Val Thr Val
   115 120 125
   Ser Ser
   130
<210> 243
   <211> 130
   <212> PRT
   <213> Lama Glama
<400> 243
   Glu Val Gln Leu Val Glu Ser Gly Gly Gly Leu Val Gln Ala Gly Gly
   1 5 10 15
   Ser Leu Arg Leu Ser Cys Ala Ala Ser Ala Phe Thr Leu Asp Tyr Tyr
   20 25 30
   Ala Val Gly Trp Phe Arg Gln Ala Pro Gly Lys Glu Arg Glu Gly Val
   35 40 45
   Ser Cys Ile Ser Ser Ser Gly Gly Ser Thr Tyr Tyr Glu Asp Ser Val
   50 55 60
   Lys Gly Arg Phe Thr Ile Ser Arg Asp Asn Ala Lys Asn Thr Val Tyr
   65 70 75 80
   Leu Gln Met Asn Asn Leu Lys Pro Glu Asp Thr Ala Val Tyr Tyr Cys
   85 90 95
   Ala Ala Asp Pro Phe His Asn Cys Tyr Ser Gly Ser His Tyr Ser Ser
   100 105 110
   Pro Glu Ala Val Tyr Glu Tyr Trp Gly Gln Gly Thr Gln Val Thr Val
   115 120 125
   Ser Ser
   130
<210> 244
   <211> 125
   <212> PRT
   <213> Lama Glama
<400> 244
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Asn Ile Gly Trp Phe Arg Gln Ala Pro Gly Lys Gly Arg Glu Trp Val
           35                  40                  45
   Ser Cys Ile Asn Ser Ser Asp Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Glu Tyr Tyr Ser Asp Leu Cys Gly Val Asn Gly Tyr
               100                 105                 110
   Asp Tyr Trp Gly Gln Gly Thr Gln Val Thr Val Ser Ser
           115                 120                 125
<210> 245
   <211> 125
   <212> PRT
   <213> Lama Glama
<400> 245
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Asn Ile Gly Trp Phe Arg Gln Ala Pro Gly Lys Glu Arg Glu Trp Val
           35                  40                  45
   Ser Cys Ile Ser Ser Ser Asp Gly Arg Thr Asn Tyr Val Asp Ser Val
   50 55 60
   Lys Gly Arg Phe Thr Met Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Asn Tyr Tyr Ser Asp Leu Cys Gly Val Asn Gly Val
   100 105 110
   Asp Tyr Trp Gly Gln Gly Thr Gln Val Thr Val Ser Ser
   115 120 125
<210> 246
   <211> 123
   <212> PRT
   <213> Lama Glama
<400> 246
   Glu Val Gln Leu Val Glu Ser Gly Gly Gly Leu Val Gln Ala Gly Gly
   1 5 10 15
   Ser Leu Arg Leu Ser Cys Ala Ala Ser Gly Ser Thr Phe Ser Ser Tyr
   20 25 30
   Ala Met Gly Trp Tyr Arg Gln Ala Pro Gly Asn Gln Arg Glu Leu Val
   35 40 45
   Ala Val Ile Ser Ser Gly Gly Ser Thr Asn Tyr Ala Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr Cys Cys Phe
   85 90 95
   Tyr Ser Gly Ser Tyr Tyr Tyr Pro Thr Asp Val His Glu Tyr Ala Tyr
   100 105 110
   Trp Gly Gln Gly Thr Gln Val Thr Val Ser Ser
   115 120
<210> 247
   <211> 125
   <212> PRT
   <213> Lama Glama
<400> 247
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Asn Tyr
               20                  25                  30
   Asn Ile Gly Trp Phe Arg Gln Ala Pro Gly Lys Glu Arg Glu Trp Val
           35                  40                  45
   Ser Cys Ile Thr Ser Ser Asn Gly Ser Thr Tyr Tyr Thr Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Ala His Tyr Ser Asp Leu Cys Gly Val Asn Gly Tyr
               100                 105                 110
   Asp Tyr Trp Gly Gln Gly Thr Gln Val Thr Val Ser Ser
           115                 120                 125
<210> 248
   <211> 117
   <212> PRT
   <213> Lama Glama
<400> 248
   Glu Val Gln Leu Val Glu Ser Gly Gly Gly Leu Val Gln Ala Gly Asp
   1 5 10 15
   Ser Leu Arg Leu Ser Cys Ala Ala Ser Gly Arg Thr Val Gly Ser Tyr
   20 25 30
   Asp Met Ser Trp Val Arg Gln Gly Pro Gly Lys Glu Arg Glu Trp Val
   35 40 45
   Ser Ser Ile Asn Ser Gly Val Gly Lys Thr Tyr Tyr Ala Asp Ser Val
   50 55 60
   Lys Gly Arg Phe Thr Ile Phe Arg Asp Asn Ala Lys Asn Met Val Tyr
   65 70 75 80
   Leu Gln Met Asn Asn Leu Lys Pro Glu Asp Thr Ala Val Tyr Tyr Cys
   85 90 95
   Ala Thr Glu Met Asp Gly Ser Arg Tyr Val Glu Gly Gln Gly Thr Gln
   100 105 110
   Val Thr Val Ser Ser
   115
<210> 249
   <211> 117
   <212> PRT
   <213> Lama Glama
<400> 249
   Glu Val Gln Leu Val Glu Ser Gly Gly Gly Leu Val Gln Ala Glu Gly
   1 5 10 15
   Ser Leu Arg Leu Ser Cys Ala Ala Ser Gly Ser Thr Phe Ser Thr Tyr
   20 25 30
   Ala Met Ala Trp Tyr Arg Gln Ala Pro Gly Lys Gln Arg Glu Leu Val
   35 40 45
   Ala Gly Ile Ser Phe Asp Gly Ser Thr His Tyr Ala Glu Ser Val Lys
   50 55 60
   Gly Arg Phe Thr Ile Ser Arg Asp Asp Ala Lys Asn Thr Val Ser Leu
   65 70 75 80
   Gln Met Asn Ser Leu Lys Pro Glu Asp Ala Ala Val Tyr Tyr Cys Tyr
   85 90 95
   Ser Val His Pro Ser Thr Gly Phe Gly Ser Trp Gly Gln Gly Thr Gln
   100 105 110
   Val Thr Val Ser Ser
   115
<210> 250
   <211> 123
   <212> PRT
   <213> Lama Glama
<400> 250
   Glu Val Gln Leu Val Glu Ser Gly Gly Gly Leu Val Gln Ala Gly Gly
   1 5 10 15
   Ser Leu Arg Leu Ser Cys Thr Ala Ser Gly Ser Thr Phe Thr Ser Tyr
   20 25 30
   Ala Met Gly Trp Tyr Arg Gln Ala Pro Gly Lys Gln Arg Glu Leu Val
   35 40 45
   Ala Ala Ile Ser Ser Asp Asp Ser Thr Tyr Tyr Ala Asp Cys Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr Tyr Cys Asn
   85 90 95
   Ala Pro His Ser Asp Tyr Asp Glu Glu Ala Pro Ser Asp Phe Gly Ser
   100 105 110
   Trp Gly Gln Gly Thr Gln Val Thr Val Ser Ser
   115 120
<210> 251
   <211> 129
   <212> PRT
   <213> Lama Glama
<400> 251
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Asp Ser Thr Tyr Tyr Ala Asp Ser Val
   50 55 60
   Lys Gly Arg Phe Thr Ile Ser Ser Asn Asn Ala Lys Asn Thr Val Tyr
   65 70 75 80
   Leu Thr Met Asn Ser Leu Lys Pro Glu Asp Thr Ala Val Tyr Tyr Cys
   85 90 95
   Ala Val Arg Leu Phe Ser Gly Gly Cys Ala Val Val Ala Ser Thr Ser
   100 105 110
   Trp Ala Asp Phe Gly Ser Ser Gly Gln Gly Thr Gln Val Thr Val Ser
   115 120 125
   Ser
<210> 252
   <211> 129
   <212> PRT
   <213> Lama Glama
<400> 252
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr Tyr Tyr Ala Asp Ser Val
       50                  55                  60
   Lys Gly Arg Phe Thr Ile Ser Ser Asn Asn Ala Lys Asn Arg Ala Tyr
   65                  70                  75                  80
   Leu Gln Met Asn Ser Leu Lys Pro Glu Asp Thr Ala Val Tyr Tyr Cys
                   85                  90                  95
   Ala Val Arg Leu Phe Arg Gly Gly Cys Ala Val Val Ala Gly Thr Ser
               100                 105                 110
   Trp Ala Asp Phe Gly Ser Ser Gly Gln Gly Thr Gln Val Thr Val Ser
           115                 120                 125
   Ser
<210> 253
   <211> 124
   <212> PRT
   <213> Lama Glama
<400> 253
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
   35 40 45
   Ser Cys Ile Ser Ser Tyr Asp Gly Thr Thr Tyr Tyr Ala Asp Ser Val
   50 55 60
   Lys Gly Arg Phe Thr Ile Ser Ser Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Asp Pro Leu Val Cys Gly Tyr Asn Asp Pro Arg Leu Ala Asp
   100 105 110
   Tyr Trp Gly Gln Gly Thr Gln Val Thr Val Ser Ser
   115 120
<210> 254
   <211> 130
   <212> PRT
   <213> Lama Glama
<400> 254
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Leu Asp Tyr Tyr
   20 25 30
   Ala Ile Gly Trp Phe Arg Gln Ala Pro Gly Lys Glu Arg Glu Gly Val
   35 40 45
   Ser Cys Ile Ser Ser Arg Gly Gly Ser Thr Tyr Tyr Glu Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Asp Pro Ile His Asn Cys Tyr Ser Gly Arg Tyr Tyr Ala Ser
   100 105 110
   Pro Asp Ala Val Tyr Glu Tyr Trp Gly Gln Gly Thr Gln Val Thr Val
   115 120 125
   Ser Ser
   130
<210> 255
   <211> 130
   <212> PRT
   <213> Lama Glama
<400> 255
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Arg Gly Ser Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Ile His Asn Cys Tyr Ser Gly Asn Gly Tyr Asp Ser
               100                 105                 110
   Pro Glu Ala Val Tyr Asp Tyr Trp Gly Gln Gly Thr Gln Val Thr Val
           115                 120                 125
   Ser Ser
       130
<210> 256
   <211> 130
   <212> PRT
   <213> Lama Glama
<400> 256
   Glu Val Gln Leu Val Glu Ser Gly Gly Gly Leu Val Gln Ala Gly Gly
   1               5                   10                  15
   Ser Leu Arg Leu Ser Cys Thr Ala Ser Gly Phe Thr Leu Asp Tyr Tyr
               20                  25                  30
   Ala Ile Gly Trp Phe Arg Gln Ala Pro Gly Lys Glu Arg Glu Gly Val
   35 40 45
   Ser Cys Ile Ser Ser Ser Gly Gly Ser Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Asp Pro Phe His Asn Cys Tyr Ser Gly Ser Ala Tyr Ser Ser
   100 105 110
   Pro Glu Ala Val Tyr Glu Tyr Trp Gly Gln Gly Thr Gln Val Thr Val
   115 120 125
   Ser Ser
   130
<210> 257
   <211> 119
   <212> PRT
   <213> Lama Glama
<400> 257
   Glu Val Gln Leu Val Glu Ser Gly Gly Gly Leu Val Gln Ala Gly Gly
   1 5 10 15
   Ser Leu Arg Leu Ser Cys Ala Ala Ser Gly Ser Thr Phe Ser Thr Tyr
   20 25 30
   Ala Met Gly Trp Tyr Arg Gln Asp Pro Gly Asn Gln Arg Glu Leu Val
   35 40 45
   Ala Ala Ile Ser Ser Asp Gly Ser Thr His Tyr Ala Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr Tyr Cys Tyr
   85 90 95
   Ala Pro Val Lys Val Ala Gly Leu Glu Tyr Asp Tyr Trp Gly Gln Gly
   100 105 110
   Thr Gln Val Thr Val Ser Ser
   115
<210> 258
   <211> 120
   <212> PRT
   <213> Lama Glama
<400> 258
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Asn Tyr
   20 25 30
   Ala Ile Gly Trp Phe Arg Gln Ala Pro Gly Lys Glu Arg Glu Trp Val
   35 40 45
   Ser Cys Ile Ser Gly Phe Asp Gly Ser Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Ser Val Gly Ser Ser Trp Cys Ala Tyr Asp Tyr Trp Gly Gln
   100 105 110
   Gly Thr Gln Val Thr Val Ser Ser
   115 120
<210> 259
   <211> 120
   <212> PRT
   <213> Lama Glama
<400> 259
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Glu Asn Tyr
               20                  25                  30
   Ala Leu Gly Trp Phe Arg Gln Ala Pro Gly Lys Glu Arg Glu Trp Val
           35                  40                  45
   Ser Cys Ile Ser Ser Ser Asp Gly Thr Thr Tyr Tyr Ala Asp Ser Val
       50                  55                  60
   Arg Gly Arg Phe Thr Ile Ser Arg Asp Asn Ala Lys Asn Thr Val Tyr
   65                  70                  75                      80
   Leu Gln Met Asn Arg Leu Lys Pro Glu Asp Thr Ala Ile Tyr Tyr Cys
                   85                  90                  95
   Ala Leu Ser Leu Gly Ser Ser Trp Cys Ala Tyr Asp Tyr Trp Gly Gln
               100                 105                 110
   Gly Thr Gln Val Thr Val Ser Ser
           115                 120
<210> 260
   <211> 123
   <212> PRT
   <213> Lama Glama
<400> 260
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asn Tyr
               20                  25                  30
   Val Ile Gly Trp Phe Arg Gln Ala Pro Gly Lys Glu Arg Glu Glu Val
           35                  40                  45
   Ser Cys Ile Ser Ser Ser Gly Gly Ser Thr Asp Tyr Leu Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Ser Leu Pro Cys Tyr Tyr Asp Lys Met Val Tyr Asp Tyr
               100                 105                 110
   Trp Gly Gln Gly Thr Gln Val Thr Val Ser Ser
           115                 120
<210> 261
   <211> 123
   <212> PRT
   <213> Lama Glama
<400> 261
   Glu Val Gln Leu Val Glu Ser Gly Gly Gly Leu Val Gln Ala Gly Gly
   1               5                   10                  15
   Ser Leu Arg Leu Ser Cys Thr Ala Ser Gly Phe Lys Leu Asp Tyr Tyr
               20                  25                  30
   Val Ile Gly Trp Phe Arg Gln Ala Pro Gly Lys Glu Arg Glu Gly Val
           35                  40                  45
   Ser Cys Thr Ser Ser Ser Gly Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met His Ser Leu Lys Pro Glu Asp Thr Ala Val Tyr Tyr Cys
                   85                  90                  95
   Ala Ala Asp Ser Phe Ala Cys Asp Tyr Gly Lys Met Ile Tyr Asp Tyr
               100                 105                 110
   Trp Gly Gln Gly Thr Gln Val Thr Val Ser Ser
           115                 120
<210> 262
   <211> 120
   <212> PRT
   <213> Lama Glama
<400> 262
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Gly Phe Asp Asn Tyr
               20                  25                  30
   Ala Met Gly Trp Phe Arg Gln Ala Pro Gly Lys Glu Arg Glu Trp Val
           35                  40                  45
   Ser Cys Ile Ser Gly Ser Asp Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met His Ser Leu Lys Pro Glu Asp Thr Ala Val Tyr Tyr Cys
                   85                  90                  95
   Ala Ala Ser Leu Gly Ser Ser Trp Cys Ala Tyr Asp Tyr Trp Gly Gln
   100 105 110
   Gly Thr Gln Val Thr Val Ser Ser
   115 120
<210> 263
   <211> 124
   <212> PRT
   <213> Lama Glama
<400> 263
   Glu Val Gln Leu Val Glu Ser Gly Gly Gly Leu Val Gln Ala Gly Gly
   1 5 10 15
   Ser Leu Arg Leu Ser Cys Ser Ala Ser Gly Phe Thr Phe Asp Asp Tyr
   20 25 30
   Ala Ile Gly Trp Phe Arg Gln Ala Pro Gly Lys Glu Arg Glu Gly Val
   35 40 45
   Ser Cys Ile Ser Ser His Asp Gly Thr Thr Tyr Tyr Ala Asp Ser Val
   50 55 60
   Lys Gly Arg Phe Thr Ile Ser Ser Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Asp Pro Leu Val Cys Gly Tyr Asn Asp Pro Arg Leu Ala Asp
   100 105 110
   Tyr Trp Gly Gln Gly Thr Gln Val Thr Val Ser Ser
   115 120
<210> 264
   <211> 123
   <212> PRT
   <213> Lama Glama
<400> 264
   Glu Val Gln Leu Val Glu Ser Gly Gly Gly Leu Val Gln Ala Gly Gly
   1 5 10 15
   Ser Leu Arg Leu Ser Cys Ala Ala Ser Gly Ser Thr Phe Ser Ser Tyr
   20 25 30
   Ala Met Gly Trp Tyr Arg Gln Ala Pro Gly Lys Gln Arg Glu Leu Val
   35 40 45
   Ala Ala Ile Ser Asn Gly Gly Ser Thr Asn Tyr Val Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr Tyr Cys Phe
   85 90 95
   Tyr Ser Gly Ser Tyr Tyr Tyr Pro Thr Asp Val His Glu Tyr Asp Tyr
   100 105 110
   Trp Gly Gln Gly Thr Gln Val Thr Val Ser Ser
   115 120
<210> 265
   <211> 125
   <212> PRT
   <213> Lama Glama
<400> 265
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Tyr Tyr
   20 25 30
   Asn Ile Gly Trp Phe Arg Gln Ala Pro Gly Lys Glu Arg Glu Trp Val
   35 40 45
   Ser Cys Ile Ser Ser Ser Asp Gly Arg Thr Asn Tyr Val Asp Ser Val
   50 55 60
   Lys Gly Arg Phe Thr Met Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Asn Tyr Tyr Ser Asn Leu Cys Gly Val Asn Gly Val
   100 105 110
   Asp Tyr Trp Gly Gln Gly Thr Gln Val Thr Val Ser Ser
   115 120 125
<210> 266
   <211> 130
   <212> PRT
   <213> Lama Glama
<400> 266
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Ser Leu Asp Tyr Tyr
               20                  25                  30
   Ala Ile Gly Trp Phe Arg Gln Ala Pro Gly Lys Glu Arg Glu Gly Ile
           35                  40                  45
   Ser Cys Ile Ser Gly Arg Gly Gly Ser Thr Tyr Tyr Ile Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Ile His Asn Cys Tyr Ser Gly Ser His Tyr Tyr Ser
               100                 105                 110
   Pro Glu Ala Val Tyr Glu Tyr Trp Gly Gln Gly Thr Gln Val Thr Val
           115                 120                 125
   Ser Ser
       130
<210> 267
   <211> 130
   <212> PRT
   <213> Lama Glama
<400> 267
   Glu Val Gln Leu Val Glu Ser Gly Gly Gly Leu Val Gln Ala Gly Asp
   1               5                   10                  15
   Ser Leu Arg Leu Ala Cys Ala Ala Ser Gly Phe Ala Leu Asp Tyr Tyr
               20                  25                  30
   Ala Val Gly Trp Phe Arg Gln Ala Pro Gly Lys Glu Arg Glu Gly Val
           35                  40                  45
   Ser Cys Ile Ser Ser Arg Gly Gly Ser Thr Phe Tyr Ala Asp Ser Leu
   50 55 60
   Lys Gly Arg Phe Thr Thr Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Asp Pro Ile His Asn Cys Tyr Ser Gly Ser Asp Tyr Ala Ser
   100 105 110
   Pro Glu Ala Val Tyr Glu Tyr Trp Gly Gln Gly Thr Gln Val Thr Val
   115 120 125
   Ser Ser
   130
<210> 268
   <211> 125
   <212> PRT
   <213> Lama Glama
<400> 268
   Glu Val Gln Leu Val Glu Ser Gly Gly Gly Leu Val Gln Pro Gly Asp
   1 5 10 15
   Ser Leu Arg Leu Ser Cys Ala Ala Ser Gly Phe Thr Leu Asp Tyr Tyr
   20 25 30
   Asn Ile Gly Trp Phe Arg Gln Ala Pro Gly Lys Glu Arg Glu Trp Val
   35 40 45
   Ala Cys Ile Arg Ser Ser Asp Gly Ser Thr Tyr Tyr Thr Asp Ser Val
   50 55 60
   Lys Gly Arg Phe Thr Ile Ser Arg Asn Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Ile His Tyr Ser Asp Leu Cys Gly Val Asn Gly Asn
   100 105 110
   Asp Tyr Trp Gly Gln Gly Thr Gln Val Thr Val Ser Ser
   115 120 125
<210> 269
   <211> 123
   <212> PRT
   <213> Lama Glama
<400> 269
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Ser Thr Phe Asn Ser Tyr
               20                  25                  30
   Ala Met Gly Trp Tyr Arg Gln Ala Pro Gly Lys Gln Arg Glu Leu Val
           35                  40                  45
   Ala Val Ile Ser Ser Gly Ser Val Thr Asn Tyr Ala Asp Ser Val Lys
       50                  55                  60
   Gly Arg Phe Thr Ile Ser Arg Asp Asn Ala Lys Asn Thr Val Ser Leu
   65                  70                  75                  80
   Gln Met Asn Ser Leu Lys Pro Glu Asp Thr Ala Val Tyr Tyr Cys Phe
                   85                  90                  95
   Tyr Ser Gly Ser Tyr Tyr Tyr Pro Thr Asp Val His Glu Tyr Asp Tyr
               100                 105                 110
   Trp Gly Gln Gly Thr Gln Val Thr Val Ser Ser
           115                 120
<210> 270
   <211> 123
   <212> PRT
   <213> Lama Glama
<400> 270
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Arg Leu Asp Tyr Tyr
               20                  25                  30
   Ala Ile Gly Trp Phe Arg Gln Ala Pro Gly Lys Glu Arg Glu Trp Val
           35                  40                  45
   Ser Cys Met Gly Ser Ser Val Arg Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Ala Pro Ile Phe Glu Cys Pro Ser Gly Glu Ile Tyr Asp Tyr
               100                 105                 110
   Trp Gly Gln Gly Thr Gln Val Thr Val Ser Ser
           115                 120
<210> 271
   <211> 130
   <212> PRT
   <213> Lama Glama
<400> 271
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Arg Gly Gly Ser Thr Tyr Tyr Thr Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Gly Val Tyr Tyr Cys
                   85                  90                  95
   Ala Ala Asp Pro Ile His Asn Cys Tyr Ser Gly Thr Tyr Tyr Ala Ser
               100                 105                 110
   Pro Glu Ala Val Tyr Glu Tyr Trp Gly Gln Gly Thr Gln Val Thr Val
           115                 120                 125
   Ser Ser
       130
<210> 272
   <211> 130
   <212> PRT
   <213> Lama Glama
<400> 272
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Ala Leu Asp Tyr Tyr
               20                  25                  30
   Ala Val Gly Trp Phe Arg Gln Ala Pro Gly Lys Glu Arg Glu Gly Val
           35                  40                  45
   Ser Cys Ile Ser Ser Arg Gly Gly Ser Thr Tyr Tyr Val Asp Ser Val
       50                  55                  60
   Lys Gly Arg Phe Thr Thr Ser Arg Asp Asn Ala Lys Asn Thr Val Tyr
   65                  70                  75                  80
   Leu Glu Met Asn Ser Leu Lys Pro Glu Asp Thr Ala Val Tyr Tyr Cys
                   85                  90                  95
   Ala Ala Asp Pro Ile His Asn Cys Tyr Ser Gly Ser Tyr Tyr Ala Ser
               100                 105                 110
   Pro Glu Ala Val Tyr Asp Tyr Trp Gly Gln Gly Thr Gln Val Thr Val
           115                 120                 125
   Ser Ser
       130
<210> 273
   <211> 123
   <212> PRT
   <213> Lama Glama
<400> 273
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Ser Thr Phe Ser Ser Tyr
               20                  25                  30
   Ala Met Gly Trp Tyr Arg Gln Ala Pro Gly Lys Gln Arg Glu Leu Val
           35                  40                  45
   Ala Val Ile Ser Ser Gly Asp Ser Thr Asn Tyr Ser Asp Ser Val Lys
       50                  55                  60
   Gly Arg Phe Thr Ile Ser Arg Asp Asn Ala Lys Asn Thr Val Tyr Leu
   65                  70                  75                  80
   Gln Met Asn Ser Leu Lys Pro Glu Asp Thr Ala Val Tyr Tyr Cys Phe
   85 90 95
   Tyr Ser Gly Ser Tyr Tyr Tyr Pro Thr Asp Val His Glu Tyr Ala Tyr
   100 105 110
   Trp Gly Gln Gly Thr Gln Val Thr Val Ser Ser
   115 120
<210> 274
   <211> 123
   <212> PRT
   <213> Lama Glama
<400> 274
   Glu Val Gln Leu Val Glu Ser Gly Gly Gly Leu Val Gln Ala Gly Gly
   1 5 10 15
   Ser Leu Arg Leu Ser Cys Ala Ala Ser Gly Ser Ser Phe Ser Ser Tyr
   20 25 30
   Ala Met Gly Trp Tyr Arg Gln Ala Pro Gly Lys Gln Arg Glu Leu Val
   35 40 45
   Ala Val Ile Ser Ser Gly Asp Arg Thr Asn Tyr Leu Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr Tyr Cys Phe
   85 90 95
   Tyr Ser Gly Ser Tyr Tyr Tyr Pro Thr Asp Val His Glu Tyr Ala Tyr
   100 105 110
   Trp Gly Gln Gly Thr Gln Val Thr Val Ser Ser
   115 120
<210> 275
   <211> 125
   <212> PRT
   <213> Lama Glama
<400> 275
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
               20                  25                  30
   Asn Ile Gly Trp Phe Arg Gln Ala Pro Gly Lys Glu Arg Glu Trp Val
           35                  40                  45
   Ser Cys Ile Ser Gly Ser Asp Gly Ser Thr Gly Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Ala Tyr Tyr Ser Asp Leu Cys Gly Val Asn Glu Tyr
               100                 105                 110
   Asp Tyr Trp Gly Gln Gly Thr Gln Val Thr Val Ser Ser
           115                 120                 125
<210> 276
   <211> 125
   <212> PRT
   <213> Lama Glama
<400> 276
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ala Leu Asp Gly His
               20                  25                  30
   Asn Ile Gly Trp Phe Arg Gln Ala Pro Gly Lys Glu Arg Glu Trp Val
           35                  40                  45
   Ser Cys Ile Asn Ser Gly Asp Gly Ser Thr Gly Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Arg Leu Lys Pro Glu Asp Thr Ala Val Tyr Tyr Cys
                   85                  90                  95
   Ala Ala Pro Phe Asn His Tyr Ser Phe Leu Cys Gly Val Asn Glu Tyr
               100                 105                 110
   Asp Tyr Trp Gly Gln Gly Thr Gln Val Thr Val Ser Ser
   115 120 125
<210> 277
   <211> 123
   <212> PRT
   <213> Lama Glama
<400> 277
   Glu Val Gln Leu Val Glu Ser Gly Gly Gly Leu Val Gln Ala Gly Gly
   1 5 10 15
   Ser Leu Arg Leu Ser Cys Ala Ala Ser Gly Ser Thr Phe Ser Ser Tyr
   20 25 30
   Ala Met Gly Trp Tyr Arg Gln Ala Pro Gly Lys Gln Arg Glu Leu Ala
   35 40 45
   Ala Val Ile Ser Thr Gly Asp Asn Thr Asn Tyr Ala Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr His Cys Phe
   85 90 95
   Tyr Ser Gly Ser Tyr Tyr Tyr Pro Thr Glu Val Tyr Glu Tyr Asp Tyr
   100 105 110
   Trp Gly Gln Gly Thr Gln Val Thr Val Ser Ser
   115 120
<210> 278
   <211> 123
   <212> PRT
   <213> Lama Glama
<400> 278
   Glu Val Gln Leu Val Glu Ser Gly Gly Gly Leu Val Gln Ala Gly Gly
   1 5 10 15
   Ser Leu Arg Leu Ser Cys Ala Ala Ser Gly Ser Thr Phe Arg Ser Tyr
   20 25 30
   Ala Met Gly Trp Tyr Arg Gln Val Pro Gly Asn Gln Arg Glu Leu Val
   35 40 45
   Ala Val Ile Ser Ser Gly Asp Ser Ala Asn Tyr Ala Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr Tyr Cys Phe
   85 90 95
   Tyr Ser Gly Ser Tyr Tyr Tyr Pro Thr Asp Val His Glu Tyr Asp Tyr
   100 105 110
   Trp Gly Gln Gly Thr Gln Val Thr Val Ser Ser
   115 120
<210> 279
   <211> 125
   <212> PRT
   <213> Lama Glama
<400> 279
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Tyr Tyr
   20 25 30
   Asn Ile Gly Trp Phe Arg Gln Ala Pro Gly Lys Glu Arg Glu Trp Val
   35 40 45
   Ser Cys Ile Asn Ser Ser Asp Gly Thr Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Glu Tyr Tyr Ser Asp Leu Cys Gly Val Asn Gly Tyr
   100 105 110
   Asp Tyr Trp Gly Gln Gly Thr Gln Val Thr Val Ser Ser
   115 120 125
<210> 280
   <211> 130
   <212> PRT
   <213> Lama Glama
<400> 280
   Glu Val Gln Leu Val Glu Ser Gly Gly Gly Leu Val Gln Ala Gly Gly
   1               5                   10                  15
   Ser Leu Arg Leu Ser Cys Gly Ala Ser Gly Phe Ser Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Gly Arg Gly Ser Asn Thr Tyr Tyr Leu Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Ile His Asn Cys Tyr Gly Gly Ser Tyr Tyr Ala Ser
               100                 105                 110
   Pro Glu Ala Val Tyr Glu Tyr Trp Gly Gln Gly Thr Gln Val Thr Val
           115                 120                 125
   Ser Ser
       130
<210> 281
   <211> 120
   <212> PRT
   <213> Lama Glama
<400> 281
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Arg Glu Gly Val
   35 40 45
   Ser Cys Ile Ser Ser Ser Gly Ser Thr Tyr Tyr Ala Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr Tyr Cys Ala
   85 90 95
   Ile Ala Gly Ala Ser Ser Trp Cys Phe Pro Pro Gly Tyr Trp Gly Gln
   100 105 110
   Gly Thr Gln Val Thr Val Ser Ser
   115 120
<210> 282
   <211> 130
   <212> PRT
   <213> Lama Glama
<400> 282
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Ala Leu Asp Tyr Tyr
               20                  25                  30
   Ala Val Gly Trp Phe Arg Gln Ala Pro Gly Lys Glu Arg Glu Gly Val
           35                  40                  45
   Ser Cys Ile Ser Ser Arg Gly Gly Ser Thr Tyr Tyr Ala Asp Ser Val
       50                  55                  60
   Lys Gly Arg Phe Thr Thr Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Ile His Asn Cys Tyr Ser Gly Ile Tyr Tyr Ala Ser
               100                 105                 110
   Pro Glu Ala Val Tyr Asp Tyr Trp Gly Gln Gly Thr Gln Val Thr Val
           115                 120                 125
   Ser Ser
       130
<210> 283
   <211> 123
   <212> PRT
   <213> Lama Glama
<400> 283
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Ser Thr Phe Ser Ser Tyr
               20                  25                  30
   Ala Met Gly Trp Tyr Arg Gln Ala Pro Val Lys Gln Arg Glu Leu Val
           35                  40                  45
   Ala Val Ile Ser Asn Gly Gly Ser Thr Asn Tyr Ala Asp Ser Val Lys
       50                  55                  60
   Gly Arg Phe Thr Ile Ser Arg Asp Asn Ala Lys Asn Thr Val Tyr Leu
   65                  70                  75                  80
   Gln Met Asn Ser Leu Lys Pro Glu Asp Thr Ala Val Tyr Tyr Cys Phe
                   85                  90                  95
   Tyr Ser Gly Ser Tyr Tyr Tyr Pro Thr Asp Val His Glu Tyr Asp Tyr
               100                 105                 110
   Trp Gly Gln Gly Thr Gln Val Thr Val Ser Ser
           115                 120
<210> 284
   <211> 130
   <212> PRT
   <213> Lama Glama
<400> 284
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Arg Gly Gly Ser Thr Tyr Tyr Glu Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Ile His Asn Cys Tyr Ser Gly Arg Tyr Tyr Ala Ser
   100 105 110
   Pro Asp Ala Val Tyr Asp Tyr Trp Gly Gln Gly Thr Gln Val Thr Val
   115 120 125
   Ser Ser
   130
<210> 285
   <211> 125
   <212> PRT
   <213> Lama Glama
<400> 285
   Glu Val Gln Leu Val Glu Ser Gly Gly Gly Leu Val Gln Pro Gly Gly
   1 5 10 15
   Ser Leu Arg Leu Ser Cys Ala Ala Ser Glu Phe Thr Leu Asp His Tyr
   20 25 30
   Asn Ile Gly Trp Phe Arg Gln Ala Pro Gly Lys Glu Arg Glu Trp Val
   35 40 45
   Ser Cys Ile Ser Ser Ser Asp Gly Ser Thr Gly Tyr Ala Asp Ser Val
   50 55 60
   Lys Gly Arg Phe Thr Ile Ser Arg Asp Lys Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Ser Tyr Tyr Ser Asp Leu Cys Gly Val Asn Gly Tyr
   100 105 110
   Asp Tyr Trp Gly Gln Gly Thr Gln Val Thr Val Ser Ser
   115 120 125
<210> 286
   <211> 123
   <212> PRT
   <213> Lama Glama
<400> 286
   Glu Val Gln Leu Val Glu Ser Gly Gly Gly Leu Val Gln Ala Gly Gly
   1 5 10 15
   Ser Leu Arg Leu Ser Cys Ala Ala Ser Gly Ser Thr Phe Ser Ser Tyr
   20 25 30
   Ala Met Gly Trp Tyr Arg Gln Ala Pro Gly Lys Gln Arg Glu Leu Val
   35 40 45
   Ala Val Ile Ser Ser Gly Asp Ser Thr Asn Tyr Ala Asp Ser Val Lys
   50 55 60
   Gly Arg Phe Thr Met Ser Arg Asp Asn Ala Lys Asn Thr Val Tyr Leu
   65 70 75 80
   Gln Met Asn Ser Leu Arg Pro Glu Asp Thr Ala Val Tyr Tyr Cys Phe
   85 90 95
   Tyr Ser Gly Ser Tyr Tyr Tyr Pro Ser Asp Val His Glu Tyr Asp Tyr
   100 105 110
   Trp Gly Gln Gly Thr Gln Val Thr Val Ser Ser
   115 120
<210> 287
   <211> 125
   <212> PRT
   <213> Lama Glama
<400> 287
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Tyr Tyr
   20 25 30
   Asn Ile Gly Trp Phe Arg Gln Ala Pro Gly Lys Glu Arg Glu Trp Val
   35 40 45
   Ser Cys Ile Ser Ser Ser Asp Gly Ser Thr Asp Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Phe Ser Tyr Tyr Ser Gly Leu Cys Gly Val Asn Gly Val
   100 105 110
   Asp Tyr Trp Gly Gln Gly Thr Gln Val Thr Val Ser Ser
   115 120 125
<210> 288
   <211> 120
   <212> PRT
   <213> Lama Glama
<400> 288
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Gly Val Tyr
   20 25 30
   Ala Thr Gly Trp Phe Arg Gln Ala Pro Gly Lys Glu Arg Glu Trp Val
   35 40 45
   Ser Cys Ile Ser Gly Ser Asp Gly Ser Thr Trp Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Pro Lys Ser Glu Asp Thr Ala Val Tyr Tyr Cys
   85 90 95
   Ala Leu Ser Leu Gly Ser Ser Trp Cys Ala Tyr Asp Tyr Trp Gly Gln
   100 105 110
   Gly Thr Gln Val Thr Val Ser Ser
   115 120
<210> 289
   <211> 130
   <212> PRT
   <213> Lama Glama
<400> 289
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Leu Asp Tyr Tyr
   20 25 30
   Ala Ile Gly Trp Phe Arg Gln Ala Pro Gly Lys Glu Arg Glu Gly Val
   35 40 45
   Ser Cys Ile Ser Gly Arg Gly Gly Ser Thr Tyr Tyr Thr Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Gly Val Tyr Tyr Cys
   85 90 95
   Ala Ala Asp Pro Val His Asn Cys Tyr Ser Gly Arg Tyr Tyr Ala Ser
   100 105 110
   Pro Asp Ala Val Tyr Glu Tyr Trp Gly Gln Gly Thr Gln Val Thr Val
   115 120 125
   Ser Ser
   130
<210> 290
   <211> 123
   <212> PRT
   <213> Lama Glama
<400> 290
   Glu Val Gln Leu Val Glu Ser Gly Gly Gly Leu Val Gln Pro Gly Gly
   1 5 10 15
   Ser Leu Arg Leu Ser Cys Ala Ala Ser Gly Ser Thr Phe Ser Ser Tyr
   20 25 30
   Ala Met Gly Trp Tyr Arg Gln Ala Pro Gly Lys Gln Arg Glu Leu Val
   35 40 45
   Ala Val Ile Ser Asn Gly Gly Ser Thr Asn Tyr Ala Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr Ile Cys Phe
   85 90 95
   Tyr Ser Gly Ser Tyr Tyr Tyr Pro Thr Asp Val His Glu Tyr Ala Tyr
   100 105 110
   Trp Gly Gln Gly Thr Gln Val Thr Val Ser Ser
   115 120
<210> 291
   <211> 125
   <212> PRT
   <213> Lama Glama
<400> 291
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr His
               20                  25                  30
   Asn Ile Gly Trp Phe Arg Gln Ala Pro Gly Lys Glu Arg Glu Trp Val
           35                  40                  45
   Ser Cys Ile Ser Ser Ser Gly Gly Ser Thr Ala Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Ser His Tyr Asn Asp Leu Cys Gly Val Asn Ala Ile
               100                 105                 110
   Asp Tyr Trp Gly Gln Gly Thr Gln Val Thr Val Ser Ser
           115                 120                 125
<210> 292
   <211> 130
   <212> PRT
   <213> Lama Glama
<400> 292
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Arg Gly Ala Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Ile His Asn Cys Tyr Ser Gly Asn Gly Tyr Asp Ser
               100                 105                 110
   Pro Glu Ala Val Tyr Asp Tyr Trp Gly Gln Gly Thr Gln Val Thr Val
           115                 120                 125
   Ser Ser
       130
<210> 293
   <211> 125
   <212> PRT
   <213> Lama Glama
<400> 293
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu His Tyr Tyr
               20                  25                  30
   Asn Ile Gly Trp Phe Arg Gln Ala Pro Gly Lys Glu Arg Glu Trp Val
           35                  40                  45
   Ser Cys Ile Asn Ser Ser Asp Gly Ser Thr His Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Glu Tyr Tyr Ser Asp Leu Cys Gly Val Asn Gly Tyr
               100                 105                 110
   Asp Tyr Trp Gly Gln Gly Thr Gln Val Thr Val Ser Ser
           115                 120                 125
<210> 294
   <211> 125
   <212> PRT
   <213> Lama Glama
<400> 294
   Lys Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Asn Ile Gly Trp Phe Arg Gln Ala Pro Gly Lys Glu Arg Glu Trp Val
           35                  40                  45
   Ser Cys Ile Asn Ser Ser Asp Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Glu Tyr Tyr Ser Asn Leu Cys Gly Val Asn Gly Tyr
               100                 105                 110
   Asp Tyr Trp Gly Gln Gly Thr Gln Val Thr Val Ser Ser
           115                 120                 125
<210> 295
   <211> 130
   <212> PRT
   <213> Lama Glama
<400> 295
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Lys Tyr
               20                  25                  30
   Ser Ile Gly Trp Phe Arg Gln Ala Pro Gly Lys Glu Arg Glu Gly Val
           35                  40                  45
   Ser Cys Ile Ser Ser Ser Gly Gly Ser Thr Tyr Tyr Val Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Asn Leu Lys Pro Glu Asp Thr Ala Val Tyr Tyr Cys
                   85                  90                  95
   Ala Ala Asp Pro Leu His Asn Cys Tyr Ser Gly Arg Gly Tyr Tyr Ser
   100 105 110
   Pro Glu Ala Val Tyr Glu Tyr Trp Gly Gln Gly Thr Gln Val Thr Val
   115 120 125
   Ser Ser
   130
<210> 296
   <211> 130
   <212> PRT
   <213> Lama Glama
<400> 296
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Leu Asp Tyr Tyr
   20 25 30
   Ala Ile Gly Trp Phe Arg Gln Ala Pro Gly Lys Glu Arg Glu Gly Val
   35 40 45
   Ser Cys Ile Ser Ser Arg Gly Gly Ser Thr Tyr Tyr Thr Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Gly Val Tyr Tyr Cys
   85 90 95
   Ala Ala Asp Pro Ile His Asn Cys Tyr Ser Gly Ser Tyr Tyr Ala Ser
   100 105 110
   Pro Glu Ala Val Tyr Glu Tyr Trp Gly Gln Gly Thr Gln Val Thr Val
   115 120 125
   Ser Ser
   130
<210> 297
   <211> 125
   <212> PRT
   <213> Lama Glama
<400> 297
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Asn Ile Gly Trp Phe Arg Gln Ala Pro Gly Lys Glu Arg Glu Trp Val
           35                  40                  45
   Ser Cys Ile Asn Ser Ser Asp Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Pro Phe Glu Tyr Tyr Ser Asn Leu Cys Gly Val Asn Gly Tyr
               100                 105                 110
   Asp Tyr Trp Gly Gln Gly Thr Gln Val Thr Val Ser Ser
           115                 120                 125
<210> 298
   <211> 120
   <212> PRT
   <213> Lama Glama
<400> 298
   Glu Val Gln Leu Val Glu Ser Gly Gly Gly Leu Val Gln Pro Gly Gly
   1               5                   10                  15
   Ser Leu Arg Leu Ser Cys Thr Ala Ser Gly Phe Thr Phe Asp Asp Tyr
               20                  25                  30
   Ala Ile Gly Trp Phe Arg Gln Ala Pro Gly Lys Glu Pro Glu Gly Ile
           35                  40                  45
   Ser Cys Ile Ser Ser Ser Gly Ser Ile Thr Tyr Asp Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Pro Gly Ile Ala Ala Cys Arg Gly Ile His Tyr Trp Gly Gln
   100 105 110
   Gly Thr Gln Val Thr Val Ser Ser
   115 120
<210> 299
   <211> 120
   <212> PRT
   <213> Lama Glama
<400> 299
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Pro Glu Gly Ile
   35 40 45
   Ser Cys Ile Ser Ser Ser Gly Gly Ile Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Thr Pro Gly Ile Ala Ala Cys Arg Gly Ile His Tyr Thr Gly Gln
   100 105 110
   Gly Thr Gln Val Thr Val Ser Ser
   115 120
<210> 300
   <211> 120
   <212> PRT
   <213> Lama Glama
<400> 300
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Pro Glu Gly Ile
   35 40 45
   Ser Cys Ile Ser Ser Ser Gly Gly Ile Thr Tyr Tyr Ala Asp Ser Val
   50 55 60
   Lys Gly Arg Phe Thr Thr Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Thr Pro Gly Ile Ala Ala Cys Arg Gly Ile His Tyr Thr Gly Gln
   100 105 110
   Gly Thr Gln Val Thr Val Ser Ser
   115 120
<210> 301
   <211> 120
   <212> PRT
   <213> Lama Glama
<400> 301
   Glu Val Gln Leu Val Glu Ser Gly Gly Gly Leu Val Gln Pro Gly Gly
   1 5 10 15
   Ser Leu Arg Leu Ser Cys Thr Ala Ser Gly Phe Thr Phe Asp Val Tyr
   20 25 30
   Ala Ile Gly Trp Phe Arg Gln Ala Pro Gly Lys Glu Pro Glu Gly Ile
   35 40 45
   Ser Cys Ile Ser Ser Ser Gly Ser Ile Thr Tyr Tyr Ala Asp Ser Val
   50 55 60
   Lys Gly Arg Phe Thr Thr Ser Arg Asp Ser Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Thr Pro Gly Ile Ala Ala Cys Arg Gly Ile His Tyr Trp Gly Gln
   100 105 110
   Gly Thr Gln Val Thr Val Ser Ser
   115 120
<210> 302
   <211> 120
   <212> PRT
   <213> Lama Glama
<400> 302
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Pro Glu Glu Ile
           35                  40                  45
   Ser Cys Ile Ser Ser Ser Gly Gly Ile Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Pro Gly Ile Ala Ala Cys Arg Gly Ile His Tyr Thr Gly Gln
               100                 105                 110
   Gly Thr Gln Val Thr Val Ser Ser
           115                 120
<210> 303
   <211> 126
   <212> PRT
   <213> Lama Glama
<400> 303
   Glu Val Gln Leu Val Glu Ser Gly Gly Gly Leu Val Gln Ala Gly Gly
   1               5                   10                  15
   Ser Leu Arg Leu Ser Cys Thr Thr Ser Glu Arg Thr Val Ser Arg Tyr
               20                  25                  30
   Ser Met Gly Trp Phe Arg Gln Ala Pro Gly Lys Glu Arg Glu Ala Val
           35                  40                  45
   Ala Thr Ile Ser Trp Ser Gly Asp Ser Thr Tyr Tyr Ala Asp Ser Val
       50                  55                  60
   Lys Gly Arg Phe Thr Ile Ser Arg Asp Asn Thr Lys Asn Thr Leu Tyr
   65                  70                  75                  80
   Leu Gln Ile Asp Ser Leu Lys Pro Glu Asp Thr Ala Val Tyr Tyr Cys
                   85                  90                  95
   Val Ala Lys Pro Asn Leu Lys Tyr Gly Ser Tyr Trp Pro Pro Arg Gly
               100                 105                 110
   Tyr Asp Tyr Trp Gly Gln Gly Thr Gln Val Thr Val Ser Ser
           115                 120                 125
<210> 304
   <211> 126
   <212> PRT
   <213> Lama Glama
<400> 304
   Glu Val Gln Leu Val Glu Ser Gly Gly Gly Leu Val Gln Ala Gly Gly
   1               5                   10                  15
   Ser Leu Arg Leu Ser Cys Thr Thr Ser Glu Arg Thr Val Ser Arg Tyr
               20                  25                  30
   Ser Met Gly Trp Phe Arg Gln Ala Pro Gly Lys Glu Arg Glu Ala Val
           35                  40                  45
   Ala Thr Ile Ser Trp Ser Gly Asp Ser Thr Tyr Tyr Ala Asp Ser Val
       50                  55                  60
   Lys Gly Arg Phe Thr Ile Ser Arg Asp Asn Thr Lys Asn Met Leu Tyr
   65                  70                  75                  80
   Leu Gln Met Asn Ser Leu Lys Pro Glu Asp Thr Ala Val Tyr Tyr Cys
                   85                  90                  95
   Val Ala Lys Pro Asn Leu Lys Tyr Gly Ser Thr Trp Pro Pro Arg Gly
               100                 105                 110
   Tyr Asp Tyr Trp Gly Gln Gly Thr Gln Val Thr Val Ser Ser
           115                 120                 125
<210> 305
   <211> 126
   <212> PRT
   <213> Lama Glama
<400> 305
   Glu Val Gln Leu Val Glu Ser Gly Gly Gly Leu Val Gln Ala Gly Gly
   1               5                   10                  15
   Ser Leu Arg Leu Ser Cys Thr Thr Ser Glu Arg Ala Val Ser Arg Tyr
   20 25 30
   Thr Met Gly Trp Leu Arg Gln Ala Pro Gly Lys Glu Arg Glu Ala Val
   35 40 45
   Ala Thr Ile Ser Trp Ser Gly Asp Ser Thr Tyr Tyr Ala Asp Ser Val
   50 55 60
   Lys Gly Arg Phe Thr Ile Ser Arg Asp Asn Thr Lys Asn Thr Leu Tyr
   65 70 75 80
   Leu Gln Met Asn Ser Leu Lys Pro Glu Asp Thr Ala Asp Tyr Tyr Cys
   85 90 95
   Ala Ala Lys Pro Asn Leu Lys Tyr Gly Ser Tyr Trp Pro Pro Arg Gly
   100 105 110
   Tyr Asp Tyr Trp Gly Gln Gly Thr Gln Val Thr Val Ser Ser
   115 120 125
<210> 306
   <211> 126
   <212> PRT
   <213> Lama Glama
<400> 306
   Glu Val Gln Leu Val Lys Ser Gly Gly Gly Leu Val Gln Ala Gly Gly
   1 5 10 15
   Ser Leu Arg Leu Ser Cys Thr Thr Ser Glu Arg Thr Val Ser Arg Tyr
   20 25 30
   Gly Met Gly Trp Phe Arg Gln Ala Pro Gly Lys Glu Arg Glu Ala Val
   35 40 45
   Ala Thr Ile Ser Trp Ser Gly Asp Ser Thr Tyr Tyr Ala Asp Ser Val
   50 55 60
   Lys Gly Arg Phe Thr Ile Ser Arg Asp Asn Thr Lys Asn Thr Leu Tyr
   65 70 75 80
   Leu Gln Met Asn Ser Leu Lys Pro Glu Asp Thr Ala Val Tyr Tyr Cys
   85 90 95
   Ala Ala Lys Pro Asn Leu Lys Tyr Gly Ser Asp Trp Pro Pro Arg Gly
   100 105 110
   Tyr Asp Tyr Trp Gly Gln Gly Thr Gln Val Thr Val Ser Ser
   115 120 125
<210> 307
   <211> 117
   <212> PRT
   <213> Lama Glama
<400> 307
   Glu Val Gln Leu Val Glu Ser Gly Gly Gly Leu Val Gln Pro Gly Gly
   1 5 10 15
   Ser Leu Arg Leu Ser Cys Thr Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Phe Ile Asn Lys Asp Gly Ser Asp Thr Gly Tyr Ala Asp Ser Val
   50 55 60
   Lys Gly Arg Phe Thr Ile Ser Arg Asp Asn Ala Lys Asn Thr Met Tyr
   65 70 75 80
   Leu Gln Met Asn Ser Leu Lys Pro Glu Asp Thr Ala Val Tyr Phe Cys
   85 90 95
   Glu Thr Arg Thr Ser Arg Ser Pro Arg Pro Arg Gly Gln Gly Thr Gln
   100 105 110
   Val Thr Val Ser Ser
   115
<210> 308
   <211> 124
   <212> PRT
   <213> Lama Glama
<400> 308
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Phe Ser Arg Tyr
   20 25 30
   Ala Met Gly Trp Phe Arg Gln Ala Pro Gly Lys Glu Arg Glu Phe Val
   35 40 45
   Ala Ala Ile Asn Trp Ser Gly Gly Ser Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Asp Cys
   85 90 95
   Ala Ala Ser Asn Tyr Tyr Ser Val Tyr Asp Asp Arg Pro Val Met Asp
   100 105 110
   Tyr Trp Gly Gln Gly Thr Gln Val Thr Val Ser Ser
   115 120
<210> 309
   <211> 116
   <212> PRT
   <213> Lama Glama
<400> 309
   Glu Val Gln Leu Met Glu Ser Gly Gly Gly Leu Val Gln Pro Gly Gly
   1 5 10 15
   Ser Leu Arg Leu Ser Cys Val Ala Ala Gly Phe Thr Phe Ser Asn Tyr
   20 25 30
   Tyr Met Ser Trp Val Arg Gln Ala Pro Gly Lys Gly Leu Glu Trp Val
   35 40 45
   Ser Val Ile Ser Pro Asp Gly Ser Asn Thr Tyr Tyr Ala Asp Thr Val
   50 55 60
   Lys Gly Arg Phe Thr Ile Ser Arg Gly Asn Ala Lys Asn Thr Leu Phe
   65 70 75 80
   Leu Gln Met Thr Gly Leu Lys Ser Glu Asp Ala Ala Val Tyr Tyr Cys
   85 90 95
   Ala Arg Gly Ser Gly Ser Trp Gly Val His Gly Gln Gly Thr Gln Val
   100 105 110
   Thr Val Ser Ser
   115
<210> 310
   <211> 128
   <212> PRT
   <213> Lama Glama
<400> 310
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Asn Tyr
               20                  25                  30
   Ile Met Gly Trp Phe Arg Gln Ala Pro Gly Lys Glu Arg Glu Phe Val
           35                  40                  45
   Ala Gly Ile Ser Arg Tyr Gly Asp Tyr Thr Ala Tyr Ala Asp Ser Val
       50                  55                  60
   Lys Gly Arg Phe Thr Ile Ser Arg Asp Asn Val Lys Asn Thr Val Tyr
   65                  70                  75                  80
   Leu Arg Met Asn Ser Leu Lys Pro Asp Asp Thr Ala Val Tyr Tyr Cys
                   85                  90                  95
   Ala Ala Asn Glu Gly Tyr Cys Ser Gly Tyr Gly Cys Tyr Glu Asp Ser
               100                 105                 110
   Gly Gln Tyr Asp Tyr Trp Gly Gln Gly Thr Gln Val Thr Val Ser Ser
           115                 120                 125
<210> 311
   <211> 128
   <212> PRT
   <213> Lama Glama
<400> 311
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Asn Tyr
               20                  25                  30
   Ile Met Gly Trp Phe Arg Gln Ala Pro Gly Lys Glu Arg Glu Phe Val
           35                  40                  45
   Ala Gly Ile Ser Arg Tyr Gly Asp Tyr Thr Tyr Tyr Ala Asp Ser Val
       50                  55                  60
   Lys Gly Arg Phe Thr Ile Ser Arg Asp Asn Val Lys Asn Thr Val Tyr
   65                  70                  75                  80
   Leu Arg Met Asn Ser Leu Lys Pro Asp Asp Thr Ala Val Tyr Tyr Cys
   85 90 95
   Ala Ala Asn Glu Gly Tyr Cys Ser Gly Tyr Gly Cys Tyr Glu Asp Ser
   100 105 110
   Gly Gln Tyr Asp Tyr Trp Gly Gln Gly Thr Gln Val Thr Val Ser Ser
   115 120 125
<210> 312
   <211> 121
   <212> PRT
   <213> Lama Glama
<400> 312
   Glu Val Gln Leu Val Glu Ser Gly Gly Gly Leu Val Gln Ala Gly Gly
   1 5 10 15
   Ser Leu Thr Leu Ser Cys Ala Ala Ser Gly Gly Thr Phe Thr Thr Tyr
   20 25 30
   Ala Met Gly Trp Phe Arg Gln Ala Pro Gly Lys Glu Arg Glu Phe Val
   35 40 45
   Ala Ala Val Ser Arg Phe Gly Val Ser Trp Asp Tyr Ala Asp Ser Val
   50 55 60
   Lys Gly Arg Phe Thr Ile Ser Arg Asp Asn Thr Ala Asn Thr Leu Lys
   65 70 75 80
   Leu Arg Met Asn Ser Leu Lys Ala Asp Asp Thr Ala Val Tyr Tyr Cys
   85 90 95
   Ala Ala Gly Gly Arg Ser Phe Leu Pro Phe Val Pro Ala Tyr Trp Gly
   100 105 110
   Gln Gly Thr Gln Val Thr Val Ser Ser
   115 120
<210> 313
   <211> 128
   <212> PRT
   <213> Lama Glama
<400> 313
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Asn Tyr
               20                  25                  30
   Ile Met Gly Trp Phe Arg Gln Ala Pro Gly Lys Glu Arg Glu Phe Val
           35                  40                  45
   Ala Gly Ile Ser Arg Tyr Ala Asp Tyr Thr Gly Tyr Ala Asp Ser Val
       50                  55                  60
   Lys Gly Arg Phe Thr Ile Ser Arg Asp Asn Val Lys Asn Thr Val Tyr
   65                  70                  75                  80
   Leu Arg Met Asn Ser Leu Lys Pro Asp Asp Thr Ala Val Tyr Tyr Cys
                   85                  90                  95
   Ala Ala Asn Glu Gly Tyr Cys Ser Gly Tyr Gly Cys Tyr Glu Asp Ser
               100                 105                 110
   Gly Gln Tyr Asp Tyr Trp Gly Gln Gly Thr Gln Val Thr Val Ser Ser
           115                 120                 125
<210> 314
   <211> 126
   <212> PRT
   <213> Lama Glama
<400> 314
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Arg Thr Leu Tyr Ile Met
               20                  25                  30
   Gly Trp Tyr Arg Gln Ala Pro Gly Lys Glu Arg Glu Phe Val Ala Gly
           35                  40                  45
   Ile Ser Arg Tyr Gly Asp Ile Thr Tyr Ala Ala Asp Ser Val Lys Gly
       50                  55                  60
   Arg Phe Thr Ile Ser Arg Asp Ser Val Lys Asn Thr Val Tyr Leu Arg
   65                  70                  75                  80
   Met Asn Ser Leu Lys Pro Asp Asp Thr Ala Val Tyr Tyr Cys Ala Ala
                   85                  90                  95
   Asn Gly Gly Tyr Cys Ser Gly Tyr Gly Cys Tyr Glu Asp Ser Gly Gln
               100                 105                 110
   Tyr Asp Tyr Trp Gly Gln Gly Thr Gln Val Thr Val Ser Ser
             115                 120                 125
<210> 315
   <211> 120
   <212> PRT
   <213> Lama Glama
<400> 315
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Pro Glu Gly Ile
           35                  40                  45
   Ser Cys Ile Ser Ser Ser Gly Gly Ile Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Pro Gly Ile Ala Ala Cys Arg Gly Ile His Tyr Thr Gly Gln
               100                 105                 110
   Gly Thr Gln Val Thr Val Ser Ser
           115                 120
<210> 316
   <211> 120
   <212> PRT
   <213> Lama Glama
<400> 316
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Pro Glu Glu Ile
           35                  40                  45
   Ser Cys Ile Ser Ser Ser Gly Gly Ile Thr Tyr Tyr Ala Asp Ser Val
       50                  55                  60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Thr Pro Gly Ile Ala Ala Cys Arg Gly Ile His Tyr Thr Gly Gln
   100 105 110
   Gly Thr Gln Val Thr Val Ser Ser
   115 120
<210> 317
   <211> 120
   <212> PRT
   <213> Lama Glama
<400> 317
   Glu Val Gln Leu Val Glu Ser Gly Gly Gly Leu Val Gln Ala Gly Gly
   1 5 10 15
   Ser Leu Arg Leu Ser Cys Ala Val Ser Gly Phe Ser Phe Asp Asp Tyr
   20 25 30
   Ala Ile Gly Trp Phe Arg Gln Ala Pro Gly Lys Glu Pro Glu Gly Ile
   35 40 45
   Ser Cys Ile Ser Ser Ser Gly Gly Ile Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Thr Pro Gly Ile Ala Ala Cys Arg Gly Ile His Tyr Thr Gly Gln
   100 105 110
   Gly Thr Gln Val Thr Val Ser Ser
   115 120
<210> 318
   <211> 123
   <212> PRT
   <213> Lama Glama
<400> 318
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Ser Tyr
               20                  25                  30
   Asp Met Ser Trp Val Arg Gln Ala Pro Gly Lys Gly Pro Glu Trp Val
           35                  40                  45
   Ser Ala Ile Asn Ser Gly Gly Gly Ser Thr Tyr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ile Pro Arg Gly Trp Gly Pro Thr Gly Pro Ile Glu Tyr Ala Tyr
               100                 105                 110
   Trp Gly Gln Gly Thr Gln Val Thr Val Ser Ser
           115                 120
<210> 319
   <211> 123
   <212> PRT
   <213> Lama Glama
<400> 319
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Ser Tyr
               20                  25                  30
   Asp Met Ser Trp Val Arg Gln Ala Pro Gly Lys Gly Pro Glu Trp Val
           35                  40                  45
   Ser Ala Ile Asn Ser Gly Gly Gly Ser Thr Tyr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ile Pro Arg Gly Trp Gly Pro Thr Gly Pro Ile Glu Tyr Gly Tyr
   100 105 110
   Trp Gly Gln Gly Thr Gln Val Thr Val Ser Ser
   115 120
<210> 320
   <211> 123
   <212> PRT
   <213> Lama Glama
<400> 320
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Gly Ser Tyr
   20 25 30
   Asp Met Ser Trp Val Arg Gln Ala Pro Gly Lys Gly Pro Glu Trp Val
   35 40 45
   Ser Ala Ile Asn Ser Gly Gly Gly Ser Thr Tyr Tyr Ala Asp Ser Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Gly Val Tyr Ser Cys
   85 90 95
   Ala Ile Pro Arg Gly Trp Gly Pro Thr Gly Pro His Glu Tyr Gly Tyr
   100 105 110
   Trp Gly Gln Gly Thr Gln Val Thr Val Ser Ser
   115 120
<210> 321
   <211> 123
   <212> PRT
   <213> Lama Glama
<400> 321
   Glu Val Gln Leu Val Glu Ser Gly Gly Gly Leu Val Gln Ser Gly Gly
   1 5 10 15
   Ser Leu Arg Leu Ser Cys Ala Ala Ser Gly Phe Thr Phe Gly Ser Tyr
   20 25 30
   Asp Met Ser Trp Val Arg Gln Ala Pro Gly Lys Gly Pro Glu Trp Val
   35 40 45
   Ser Ala Ile Asn Ser Gly Gly Gly Ser Thr Tyr Tyr Ala Asp Ser Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Ile Pro Arg Gly Trp Gly Pro Thr Gly Pro His Glu Tyr Ala Tyr
   100 105 110
   Trp Gly Gln Gly Thr Gln Val Thr Val Ser Ser
   115 120
<210> 322
   <211> 121
   <212> PRT
   <213> Lama Glama
<400> 322
   Glu Val Gln Leu Val Glu Ser Gly Gly Gly Ser Val Gln Ala Gly Gly
   1 5 10 15
   Ser Leu Arg Leu Ser Cys Ala Ala Ser Gly Arg Thr Phe Ser Ser Tyr
   20 25 30
   Ala Met Gly Trp Phe Arg Gln Ala Pro Gly Lys Glu Arg Glu Phe Val
   35 40 45
   Ala Ala Ile Asn Trp Ser Gly Gly Tyr Thr Tyr Tyr Ala Asp Ser Val
   50 55 60
   Arg Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Pro Ala Pro Gly Ser Ser Gly Tyr Glu Tyr Asp Tyr Trp Gly
   100 105 110
   Gln Gly Thr Gln Val Thr Val Ser Ser
   115 120
<210> 323
   <211> 120
   <212> PRT
   <213> Lama Glama
<400> 323
   Glu Val Gln Leu Val Glu Ser Gly Gly Gly Leu Val Gln Pro Gly Gly
   1               5                   10                  15
   Ser Leu Arg Leu Ser Cys Thr Ala Ser Gly Phe Thr Phe Asp Val Tyr
               20                  25                  30
   Ala Ile Gly Trp Phe Arg Gln Ala Pro Gly Lys Glu Pro Glu Gly Ile
           35                  40                  45
   Ser Cys Ile Ser Ser Ser Gly Ser Ile Thr Tyr Tyr Ala Asp Ser Val
       50                  55                  60
   Lys Gly Arg Phe Thr Ile Ser Arg Asp Ser Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Pro Gly Ile Ala Ala Cys Arg Gly Ile His Tyr Trp Gly Gln
               100                 105                 110
   Gly Thr Gln Val Thr Val Ser Ser
           115                 120
<210> 324
   <211> 123
   <212> PRT
   <213> Lama Glama
<400> 324
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Asn Tyr
               20                  25                  30
   Asp Met Ser Trp Val Arg His Ala Pro Gly Lys Gly Pro Glu Trp Val
           35                  40                  45
   Ser Ala Ile Asn Ser Gly Gly Gly Ser Thr Tyr Tyr Thr Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ile Pro Arg Gly Trp Gly Pro Thr Gly Pro His Glu Tyr Ala Tyr
               100                 105                 110
   Trp Gly Gln Gly Thr Gln Val Thr Val Ser Ser
           115                 120
<210> 325
   <211> 123
   <212> PRT
   <213> Lama Glama
<400> 325
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Ser Tyr
               20                  25                  30
   Asp Met Ser Trp Val Arg Gln Ala Pro Gly Lys Gly Pro Glu Trp Val
           35                  40                  45
   Ser Ala Ile Asn Ser Gly Gly Gly Thr Thr Tyr Tyr Ala Asp Ser Val
       50                  55                  60
   Lys Gly Arg Phe Thr Ile Ser Arg Asp Asn Ala Lys Asn Thr Leu Phe
   65                  70                  75                  80
   Leu Gln Met Asn Ser Leu Lys Pro Glu Asp Thr Ala Val Tyr Tyr Cys
                   85                  90                  95
   Ala Ile Pro Arg Gly Trp Gly Pro Thr Gly Pro Leu Glu Tyr Gly Tyr
               100                 105                 110
   Trp Gly Gln Gly Thr Gln Val Thr Val Ser Ser
           115                 120
<210> 326
   <211> 123
   <212> PRT
   <213> Lama Glama
<400> 326
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Asn Tyr
   20 25 30
   Asp Met Ser Trp Val Arg Gln Ala Pro Gly Lys Gly Pro Glu Trp Val
   35 40 45
   Ser Ala Ile Asn Ser Gly Gly Gly Asp Thr Tyr Tyr Ala Asp Ser Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Thr Pro Arg Gly Trp Gly Pro Thr Gly Pro His Glu Tyr Gly Tyr
   100 105 110
   Trp Gly Gln Gly Thr Gln Val Thr Val Ser Ser
   115 120
<210> 327
   <211> 123
   <212> PRT
   <213> Lama Glama
<400> 327
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Gly Asn Tyr
   20 25 30
   Asp Met Ser Trp Val Arg Gln Ala Pro Gly Lys Gly Pro Glu Trp Val
   35 40 45
   Ser Ala Ile Asn Ser Gly Gly Gly Ile Thr Tyr Tyr Ala Asp Ser Val
   50 55 60
   Lys Gly Arg Phe Ala Ile Ser Arg Asp Asn Ala Lys Thr Thr Leu Tyr
   65 70 75 80
   Leu Gln Met Asn Asn Leu Gln Pro Glu Asp Thr Ala Val Tyr Tyr Cys
   85 90 95
   Ala Thr Pro Arg Gly Trp Gly Pro Thr Gly Pro His Glu Tyr Gly Tyr
   100 105 110
   Trp Gly Gln Gly Thr Gln Val Thr Val Ser Ser
   115 120
<210> 328
   <211> 123
   <212> PRT
   <213> Lama Glama
<400> 328
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Phe Gly Ser Tyr
   20 25 30
   Asp Met Ser Trp Val Arg Gln Ala Pro Gly Lys Gly Pro Glu Trp Val
   35 40 45
   Ser Ala Ile Asn Ser Gly Gly Gly Ile Thr Tyr Tyr Ala Asp Leu Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Ile Pro Arg Gly Trp Gly Pro Thr Gly Pro His Glu Tyr Gly Tyr
   100 105 110
   Trp Gly Gln Gly Thr Gln Val Thr Val Ser Ser
   115 120
<210> 329
   <211> 123
   <212> PRT
   <213> Lama Glama
<400> 329
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Gly Asn Tyr
   20 25 30
   Asp Met Ser Trp Val Arg Gln Ala Pro Gly Lys Gly Pro Glu Trp Val
   35 40 45
   Ser Ala Ile Asn Ser Gly Gly Gly Ile Thr Tyr Tyr Ala Asp Ser Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Thr Pro Arg Gly Trp Gly Pro Thr Gly Pro His Glu Tyr Gly Tyr
   100 105 110
   Trp Gly Gln Gly Thr Gln Val Thr Val Ser Ser
   115 120
<210> 330
   <211> 123
   <212> PRT
   <213> Lama Glama
<400> 330
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Gly Ser Tyr
   20 25 30
   Asp Met Ser Trp Val Arg Gln Ala Pro Gly Lys Gly Pro Glu Trp Val
   35 40 45
   Ser Ala Ile Asn Ser Gly Gly Gly Ser Thr Tyr Tyr Ala Asp Ser Val
   50 55 60
   Lys Gly Arg Phe Thr Ile Ser Arg Asp Asn Ala Lys Asn Thr Leu Tyr
   65 70 75 80
   Leu Gln Met Asn Ser Leu Thr Pro Glu Asp Thr Ala Val Tyr Tyr Cys
   85 90 95
   Ala Ile Pro Arg Gly Trp Gly Pro Thr Gly Pro His Glu Tyr Ala Tyr
   100 105 110
   Trp Gly Gln Gly Thr Gln Val Thr Val Ser Ser
   115 120
<210> 331
   <211> 123
   <212> PRT
   <213> Lama Glama
<400> 331
   Glu Val Gln Leu Val Glu Ser Gly Gly Gly Leu Val Gln Pro Gly Gly
   1               5                   10                  15
   Ser Leu Arg Leu Ser Cys Thr Ala Ser Gly Phe Thr Phe Gly Asn Tyr
               20                  25                  30
   Asp Met Ser Trp Val Arg Arg Pro Pro Gly Lys Gly Pro Glu Trp Val
           35                  40                  45
   Ser Ala Ile Asn Ser Gly Gly Gly Ser Thr Tyr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Thr Pro Arg Gly Trp Gly Pro Thr Gly Pro His Glu Tyr Gly Tyr
               100                 105                 110
   Trp Gly Gln Gly Thr Gln Val Thr Val Ser Ser
           115                 120
<210> 332
   <211> 123
   <212> PRT
   <213> Lama Glama
<400> 332
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Ser Tyr
               20                  25                  30
   Asp Met Ser Trp Val Arg Gln Ala Pro Gly Lys Gly Pro Glu Trp Val
           35                  40                  45
   Ser Ala Ile Asn Ser Gly Gly Gly Ser Thr Tyr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Asn Leu Lys Pro Glu Asp Thr Ala Val Tyr Ser Cys
   85 90 95
   Ala Ile Pro Arg Gly Trp Gly Pro Thr Gly Pro His Glu Tyr Ala Tyr
   100 105 110
   Trp Gly Gln Gly Thr Gln Val Thr Val Ser Ser
   115 120
<210> 333
   <211> 14
   <212> PRT
   <213> Artificial
<220>
   <223> Mutated Lama sequence
<400> 333
   Arg Ala Pro Asp Thr Arg Leu Arg Pro Tyr Leu Tyr Asp Tyr
   1 5 10
<210> 334
   <211> 14
   <212> PRT
   <213> Artificial
<220>
   <223> Mutated Lama sequence
<400> 334
   Arg Ala Pro Asp Thr Arg Leu Glu Pro Tyr Glu Tyr Asp His
   1 5 10
<210> 335
   <211> 14
   <212> PRT
   <213> Artificial
<220>
   <223> Mutated Lama sequence
<400> 335
   Arg Ala Pro Asp Thr Arg Leu Ala Pro Tyr Glu Tyr Asp His
   1 5 10
<210> 336
   <211> 14
   <212> PRT
   <213> Artificial
<220>
   <223> Mutated Lama sequence
<400> 336
   Arg Ala Pro Asp Thr Arg Leu Glu Pro Tyr Leu Tyr Asp His
   1               5                   10
<210> 337
   <211> 14
   <212> PRT
   <213> Artificial
<220>
   <223> Mutated Lama sequence
<400> 337
   Arg Ala Pro Asp Thr Arg Leu Glu Pro Tyr Leu Tyr Asp Tyr
   1               5                   10
<210> 338
   <211> 14
   <212> PRT
   <213> Artificial
<220>
   <223> Mutated Lama sequence
<400> 338
   Arg Ala Pro Asp Thr Arg Leu Ala Pro Tyr Leu Tyr Asp Tyr
   1               5                   10
<210> 339
   <211> 14
   <212> PRT
   <213> Artificial
<220>
   <223> Mutated Lama sequence
<400> 339
   Arg Ala Pro Asp Thr Arg Leu Ala Pro Tyr Glu Tyr Asp His
   1               5                   10
<210> 340
   <211> 14
   <212> PRT
   <213> Artificial
<220>
   <223> Mutated Lama sequence
<400> 340
   Arg Ala Pro Asp Thr Arg Leu Arg Pro Tyr Leu Tyr Asp Tyr
   1               5                   10
<210> 341
   <211> 14
   <212> PRT
   <213> Artificial
<220>
   <223> Mutated Lama sequence
<400> 341
   Arg Ala Pro Asp Thr Arg Leu Glu Pro Tyr Leu Tyr Asp His
   1               5                   10
<210> 342
   <211> 14
   <212> PRT
   <213> Artificial
<220>
   <223> Mutated Lama sequence
<400> 342
   Arg Ala Pro Asp Thr Arg Leu Glu Pro Tyr Glu Tyr Asp His
   1               5                   10
<210> 343
   <211> 14
   <212> PRT
   <213> Artificial
<220>
   <223> Mutated Lama sequence
<400> 343
   Arg Ala Pro Asp Thr Arg Leu Arg Pro Tyr Glu Tyr Asp Tyr
   1               5                   10
<210> 344
   <211> 14
   <212> PRT
   <213> Artificial
<220>
   <223> Mutated Lama sequence
<400> 344
   Arg Ala Pro Asp Thr Arg Leu Glu Pro Tyr Leu Tyr Asp His
   1               5                   10
<210> 345
   <211> 14
   <212> PRT
   <213> Artificial
<220>
   <223> Mutated Lama sequence
<400> 345
   Arg Ala Pro Asp Thr Arg Leu Gly Pro Tyr Leu Tyr Asp Tyr
   1 5 10
<210> 346
   <211> 14
   <212> PRT
   <213> Artificial
<220>
   <223> Mutated Lama sequence
<400> 346
   Arg Ala Pro Asp Thr Arg Leu Glu Pro Tyr Glu Tyr Asp Tyr
   1 5 10
<210> 347
   <211> 14
   <212> PRT
   <213> Artificial
<220>
   <223> Mutated Lama sequence
<400> 347
   Arg Ala Pro Asp Thr Arg Leu Gly Pro Tyr Leu Tyr Asp His
   1 5 10
<210> 348
   <211> 14
   <212> PRT
   <213> Artificial
<220>
   <223> Mutated Lama sequence
<400> 348
   Arg Ala Pro Asp Thr Arg Leu Ala Pro Tyr Leu Tyr Asp His
   1 5 10
<210> 349
   <211> 14
   <212> PRT
   <213> Artificial
<220>
   <223> Mutated Lama sequence
<400> 349
   Arg Ala Pro Asp Thr Arg Leu Ala Pro Tyr Leu Tyr Asp Tyr
   1 5 10
<210> 350
   <211> 14
   <212> PRT
   <213> Artificial
<220>
   <223> Mutated Lama sequence
<400> 350
   Arg Ala Pro Asp Thr Arg Leu Arg Pro Tyr Leu Tyr Asp His
   1 5 10
<210> 351
   <211> 14
   <212> PRT
   <213> Artificial
<220>
   <223> Mutated Lama sequence
<400> 351
   Arg Ala Pro Asp Thr Arg Leu Glu Pro Tyr Leu Tyr Asp His
   1 5 10
<210> 352
   <211> 14
   <212> PRT
   <213> Artificial
<220>
   <223> Mutated Lama sequence
<400> 352
   Arg Ala Pro Asp Thr Arg Leu Glu Pro Tyr Glu Tyr Asp His
   1 5 10
<210> 353
   <211> 14
   <212> PRT
   <213> Artificial
<220>
   <223> Mutated Lama sequence
<400> 353
   Arg Ala Pro Asp Thr Arg Leu Ala Pro Tyr Glu Tyr Asp Tyr
   1 5 10
<210> 354
   <211> 123
   <212> PRT
   <213> Artificial
<220>
   <223> Mutated Lama sequence
<400> 354
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Phe Arg Gln Ala Pro Gly Lys Glu Arg Glu Tyr Val
           35                  40                  45
   Ala Ala Ile Arg Trp Ser Gly Gly Thr Ala Tyr Tyr Ala Asp Ser Val
       50                  55                  60
   Gln Gly Arg Phe Thr Ile Ser Arg Asp Asn Ala Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Asn Arg Ala Pro Asp Thr Arg Leu Arg Pro Tyr Leu Tyr Asp Tyr
               100                 105                 110
   Trp Gly Gln Gly Thr Gln Val Thr Val Ser Ser
           115                 120
<210> 355
   <211> 123
   <212> PRT
   <213> Artificial
<220>
   <223> Mutated Lama sequence
<400> 355
   Glu Val Gln Leu Val Glu Ser Gly Gly Gly Leu Val Gln Ala Gly Gly
   1               5                   10                  15
   Ser Leu Ser Leu Ser Cys Ala Ala Ser Gly Arg Thr Phe Ser Ser Tyr
               20                  25                  30
   Ala Met Ala Trp Tyr Arg Gln Ala Pro Gly Lys Glu Arg Glu Tyr Val
           35                  40                  45
   Ala Ala Ile Arg Trp Ser Gly Gly Thr Ala Tyr Tyr Ala Asp Ser Val
       50                  55                  60
   Gln Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Asn Arg Ala Pro Asp Thr Arg Leu Glu Pro Tyr Glu Tyr Asp His
               100                 105                 110
   Trp Gly Gln Gly Thr Gln Val Thr Val Ser Ser
           115                 120
<210> 356
   <211> 123
   <212> PRT
   <213> Artificial
<220>
   <223> Mutated Lama sequence
<400> 356
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Tyr Arg Gln Ala Pro Gly Lys Glu Arg Glu Tyr Val
           35                  40                  45
   Ala Ala Ile Arg Trp Ser Gly Gly Thr Ala Tyr Tyr Ala Asp Ser Val
       50                  55                  60
   Gln Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Asn Arg Ala Pro Asp Thr Arg Leu Ala Pro Tyr Glu Tyr Asp His
               100                 105                 110
   Trp Gly Gln Gly Thr Gln Val Thr Val Ser Ser
           115                 120
<210> 357
   <211> 123
   <212> PRT
   <213> Artificial
<220>
   <223> Mutated Lama sequence
<400> 357
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Phe Ser Ser Tyr
   20 25 30
   Ala Met Ala Trp Phe Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Gln Ser Arg Phe Thr Ile Thr Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Asn Arg Ala Pro Asp Thr Arg Leu Glu Pro Tyr Leu Tyr Asp His
   100 105 110
   Trp Gly Gln Gly Thr Gln Val Thr Val Ser Ser
   115 120
<210> 358
   <211> 123
   <212> PRT
   <213> Artificial
<220>
   <223> Mutated Lama sequence
<400> 358
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Phe Ser Ser Tyr
   20 25 30
   Ala Met Ala Trp Tyr Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Gln Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Asn Arg Ala Pro Asp Thr Arg Leu Glu Pro Tyr Leu Tyr Asp Tyr
   100 105 110
   Trp Gly Gln Gly Thr Gln Val Thr Val Ser Ser
   115 120
<210> 359
   <211> 123
   <212> PRT
   <213> Artificial
<220>
   <223> Mutated Lama sequence
<400> 359
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Phe Ser Ser Tyr
   20 25 30
   Ala Met Ala Trp Phe Arg Gln Ala Pro Gly Lys Asp Arg Glu Tyr Val
   35 40 45
   Ala Ala Ile Arg Trp Ser Gly Gly Thr Ala Tyr Tyr Ala Asp Ser Val
   50 55 60
   Gln Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Asn Arg Ala Pro Asp Thr Arg Leu Ala Pro Tyr Leu Tyr Asp Tyr
   100 105 110
   Trp Gly Gln Gly Thr Gln Val Thr Val Ser Ser
   115 120
<210> 360
   <211> 123
   <212> PRT
   <213> Artificial
<220>
   <223> Mutated Lama sequence
<400> 360
   Glu Val Gln Leu Val Glu Ser Gly Gly Gly Leu Val Gln Ala Gly Gly
   1               5                   10                  15
   Ser Leu Arg Leu Ser Cys Ser Ala Ser Gly Arg Thr Phe Ser Ser Tyr
               20                  25                  30
   Ala Met Ala Trp Tyr Arg Gln Ala Pro Gly Lys Glu Arg Glu Tyr Val
           35                  40                  45
   Ala Ala Ile Arg Trp Ser Gly Gly Thr Ala Tyr Tyr Pro Asp Ser Val
       50                  55                  60
   Gln Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Asn Arg Ala Pro Asp Thr Arg Leu Ala Pro Tyr Glu Tyr Asp His
               100                 105                 110
   Trp Gly Gln Gly Thr Gln Val Thr Val Ser Ser
           115                 120
<210> 361
   <211> 123
   <212> PRT
   <213> Artificial
<220>
   <223> Mutated Lama sequence
<400> 361
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Tyr Arg Gln Ala Pro Gly Lys Glu Arg Glu Tyr Val
           35                  40                  45
   Ala Ala Ile Arg Trp Ser Gly Gly Thr Ala Tyr Tyr Ala Asp Ser Val
       50                  55                  60
   Gln Gly Arg Phe Thr Ile Ser Arg Asp Asn Ala Lys Asn Thr Val Tyr
   65                  70                  75                      80
   Leu Gln Met Asn Ser Leu Lys Pro Glu Asp Thr Ala Val Tyr Tyr Cys
                   85                  90                  95
   Ala Asn Arg Ala Pro Asp Thr Arg Leu Arg Pro Tyr Leu Tyr Asp Tyr
               100                 105                 110
   Trp Gly Gln Gly Thr Gln Val Thr Val Ser Ser
           115                 120
<210> 362
   <211> 123
   <212> PRT
   <213> Artificial
<220>
   <223> Mutated Lama sequence
<400> 362
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Tyr Arg Gln Ala Pro Gly Lys Glu Arg Glu Tyr Val
           35                  40                  45
   Ala Ala Ile Arg Trp Ser Gly Glu Thr Ala Tyr Tyr Ala Asp Ser Val
       50                  55                  60
   Gln Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Asn Arg Ala Pro Asp Thr Arg Leu Glu Pro Tyr Leu Tyr Asp His
               100                 105                 110
   Trp Gly Gln Gly Thr Gln Val Thr Val Ser Ser
           115                 120
<210> 363
   <211> 123
   <212> PRT
   <213> Artificial
<220>
   <223> Mutated Lama sequence
<400> 363
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Phe Ser Ser Tyr
   20 25 30
   Ala Met Ala Trp Tyr Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Gln Ser Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Asn Arg Ala Pro Asp Thr Arg Leu Glu Pro Tyr Glu Tyr Asp His
   100 105 110
   Trp Gly Gln Gly Thr Gln Val Thr Val Ser Ser
   115 120
<210> 364
   <211> 123
   <212> PRT
   <213> Artificial
<220>
   <223> Mutated Lama sequence
<400> 364
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Phe Ser Ser Tyr
   20 25 30
   Ala Met Ala Trp Tyr Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Gln Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Asn Arg Ala Pro Asp Thr Arg Leu Arg Pro Tyr Glu Tyr Asp Tyr
   100 105 110
   Trp Gly Gln Gly Thr Gln Val Thr Val Ser Ser
   115 120
<210> 365
   <211> 123
   <212> PRT
   <213> Artificial
<220>
   <223> Mutated Lama sequence
<400> 365
   Glu Val Gln Leu Val Glu Ser Arg Gly Gly Leu Val Gln Ala Gly Gly
   1 5 10 15
   Ser Leu Arg Leu Ser Cys Ala Ala Ser Gly Arg Thr Phe Ser Ser Tyr
   20 25 30
   Ala Met Ala Trp Phe Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Gln Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Asn Arg Ala Pro Asp Thr Arg Leu Glu Pro Tyr Leu Tyr Asp His
   100 105 110
   Trp Gly Gln Gly Thr Gln Val Thr Val Ser Ser
   115 120
<210> 366
   <211> 123
   <212> PRT
   <213> Artificial
<220>
   <223> Mutated Lama sequence
<400> 366
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Tyr Arg Leu Ala Pro Gly Lys Glu Arg Glu Tyr Val
           35                  40                  45
   Ala Ala Ile Arg Trp Ser Gly Gly Thr Ala Tyr Tyr Ala Asp Ser Val
       50                  55                  60
   Gln Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Asn Arg Ala Pro Asp Thr Arg Leu Gly Pro Tyr Leu Tyr Asp Tyr
               100                 105                 110
   Trp Gly Gln Gly Thr Gln Val Thr Val Ser Ser
           115                 120
<210> 367
   <211> 123
   <212> PRT
   <213> Artificial
<220>
   <223> Mutated Lama sequence
<400> 367
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Tyr Arg Gln Ala Pro Gly Lys Glu Arg Glu Tyr Val
           35                  40                  45
   Ala Ala Ile Arg Trp Ser Gly Gly Thr Ala Tyr Tyr Ala Asp Ser Val
       50                  55                  60
   Gln Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Asn Arg Ala Pro Asp Thr Arg Leu Glu Pro Tyr Glu Tyr Asp Tyr
               100                 105                 110
   Trp Gly Gln Gly Thr Gln Val Thr Val Ser Ser
           115                 120
<210> 368
   <211> 123
   <212> PRT
   <213> Artificial
<220>
   <223> Mutated Lama sequence
<400> 368
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Phe Ser Ser Tyr
   20 25 30
   Ala Met Ala Trp Tyr Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Gln Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Asn Arg Ala Pro Asp Thr Arg Leu Gly Pro Tyr Leu Tyr Asp His
   100 105 110
   Trp Gly Gln Gly Thr Gln Val Thr Val Ser Ser
   115 120
<210> 369
   <211> 123
   <212> PRT
   <213> Artificial
<220>
   <223> Mutated Lama sequence
<400> 369
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Phe Ser Ser Tyr
   20 25 30
   Ala Met Ala Trp Phe Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Gln Gly Arg Phe Thr Ile Ser Arg Asp Asn Ala Lys Asn Thr Val Tyr
   65 70 75 80
   Leu Gln Met Tyr Ser Leu Lys Pro Glu Asp Thr Ala Val Tyr Tyr Cys
   85 90 95
   Ala Asn Arg Ala Pro Asp Thr Arg Leu Ala Pro Tyr Leu Tyr Asp His
   100 105 110
   Trp Gly Gln Gly Thr Gln Val Thr Val Ser Ser
   115 120
<210> 370
   <211> 123
   <212> PRT
   <213> Artificial
<220>
   <223> Mutated Lama sequence
<400> 370
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Phe Ser Ser Tyr
   20 25 30
   Ala Met Ala Trp Tyr Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Gln Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
                   85                  90                  95
   Ala Asn Arg Ala Pro Asp Thr Arg Leu Ala Pro Tyr Leu Tyr Asp Tyr
               100                 105                 110
   Trp Gly Gln Gly Thr Gln Val Thr Val Ser Ser
           115                 120
<210> 371
   <211> 123
   <212> PRT
   <213> Artificial
<220>
   <223> Mutated Lama sequence
<400> 371
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Phe Arg Gln Ala Pro Gly Lys Glu Arg Glu Tyr Val
           35                  40                  45
   Ala Ala Ile Arg Trp Ser Gly Gly Thr Ala Tyr Tyr Ala Asp Ser Val
       50                  55                  60
   Gln Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Asn Arg Ala Pro Asp Thr Arg Leu Arg Pro Tyr Leu Tyr Asp His
               100                 105                 110
   Trp Gly Gln Gly Thr Gln Val Thr Val Ser Ser
           115                 120
<210> 372
   <211> 123
   <212> PRT
   <213> Artificial
<220>
   <223> Mutated Lama sequence
<400> 372
   Glu Val Gln Leu Val Glu Ser Gly Gly Gly Leu Val Gln Ala Gly Gly
   1               5                   10                  15
   Ser Leu Arg Leu Ser Cys Ser Ala Ser Gly Arg Thr Phe Ser Ser Tyr
               20                  25                  30
   Ala Met Ala Trp Tyr Arg Gln Ala Pro Gly Lys Glu Arg Glu Tyr Val
           35                  40                  45
   Ala Ala Ile Arg Trp Ser Gly Glu Thr Ala Tyr Tyr Ala Asp Ser Val
       50                  55                  60
   Gln Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Asn Arg Ala Pro Asp Thr Arg Leu Glu Pro Tyr Leu Tyr Asp His
               100                 105                 110
   Trp Gly Gln Gly Thr Gln Val Thr Val Ser Ser
           115                 120
<210> 373
   <211> 123
   <212> PRT
   <213> Artificial
<220>
   <223> Mutated Lama sequence
<400> 373
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Tyr Arg Gln Ala Pro Gly Lys Glu Arg Glu Tyr Val
           35                  40                  45
   Ala Ala Ile Arg Trp Ser Gly Gly Thr Ala Tyr Tyr Ala Asp Ser Val
       50                  55                  60
   Gln Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Asn Arg Ala Pro Asp Thr Arg Leu Glu Pro Tyr Glu Tyr Asp His
               100                 105                 110
   Trp Gly Gln Gly Thr Gln Val Thr Val Ser Ser
           115                 120
<210> 374
   <211> 123
   <212> PRT
   <213> Artificial
<220>
   <223> Mutated Lama sequence
<400> 374
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Phe Ser Ser Tyr
   20 25 30
   Ala Met Ala Trp Tyr Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Gln Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Asn Arg Ala Pro Asp Thr Arg Leu Ala Pro Tyr Glu Tyr Asp Tyr
   100 105 110
   Trp Gly Gln Gly Thr Gln Val Thr Val Ser Ser
   115 120
<210> 375
   <211> 19
   <212> PRT
   <213> Artificial
<220>
   <223> Mutated Lama sequence
<400> 375
   Asp Arg Tyr Ile Trp Ala Arg Gln Gly Glu Tyr Trp Gly Ala Tyr Gln
   1 5 10 15
   Tyr Asp Tyr
<210> 376
   <211> 19
   <212> PRT
   <213> Artificial
<220>
   <223> Mutated Lama sequence
<400> 376
   Asp Arg Tyr Ile Trp Ala Arg Gln Gly Glu Tyr Trp Gly Ala Tyr Ala
   1 5 10 15
   Tyr Asp Tyr
<210> 377
   <211> 19
   <212> PRT
   <213> Artificial
<220>
   <223> Mutated Lama sequence
<400> 377
   Asp Arg Tyr Ile Trp Ala Arg Gln Gly Glu Tyr Trp Gly Ala Tyr Val
   1 5 10 15
   Tyr Asp Tyr
<210> 378
   <211> 19
   <212> PRT
   <213> Artificial
<220>
   <223> Mutated Lama sequence
<400> 378
   Asp Arg Tyr Ile Trp Ala Arg Gln Gly Glu Tyr Trp Gly Ala Tyr Gln
   1 5 10 15
   Tyr Asp Tyr
<210> 379
   <211> 19
   <212> PRT
   <213> Artificial
<220>
   <223> Mutated Lama sequence
<400> 379
   Asp Arg Tyr Ile Trp Ala Arg Gln Gly Glu Tyr Trp Gly Ala Tyr Val
   1 5 10 15
   Tyr Asp Tyr
<210> 380
   <211> 19
   <212> PRT
   <213> Artificial
<220>
   <223> Mutated Lama sequence
<400> 380
   Asp Arg Tyr Ile Trp Ala Arg Gln Gly Glu Tyr Trp Gly Ala Tyr Gln
   1 5 10 15
   Tyr Asp Tyr
<210> 381
   <211> 19
   <212> PRT
   <213> Artificial
<220>
   <223> Mutated Lama sequence
<400> 381
   Asp Arg Tyr Ile Trp Ala Arg Gln Gly Glu Tyr Trp Gly Ala Tyr Ala
   1 5 10 15
   Tyr Asp Tyr
<210> 382
   <211> 19
   <212> PRT
   <213> Artificial
<220>
   <223> Mutated Lama sequence
<400> 382
   Asp Arg Tyr Ile Trp Ala Arg Gln Gly Glu Tyr Trp Gly Ala Tyr Ala
   1 5 10 15
   Tyr Asp Tyr
<210> 383
   <211> 19
   <212> PRT
   <213> Artificial
<220>
   <223> Mutated Lama sequence
<400> 383
   Asp Arg Tyr Ile Trp Ala Arg Gln Gly Glu Tyr Trp Gly Ala Tyr Ala
   1 5 10 15
   Tyr Asp Tyr
<210> 384
   <211> 19
   <212> PRT
   <213> Artificial
<220>
   <223> Mutated Lama sequence
<400> 384
   Asp Arg Tyr Ile Trp Ala Arg Gln Gly Glu Tyr Trp Gly Ala Tyr Ala
   1               5                   10                  15
   Tyr Asp Tyr
<210> 385
   <211> 19
   <212> PRT
   <213> Artificial
<220>
   <223> Mutated Lama sequence
<400> 385
   Asp Arg Tyr Ile Trp Ala Arg Gln Gly Glu Tyr Trp Gly Ala Tyr Gln
   1               5                   10                  15
   Tyr Asp Tyr
<210> 386
   <211> 19
   <212> PRT
   <213> Artificial
<220>
   <223> Mutated Lama sequence
<400> 386
   Asp Arg Tyr Ile Trp Ala Arg Gln Gly Glu Tyr Trp Gly Ala Tyr Glu
   1               5                   10                  15
   Tyr Asp Tyr
<210> 387
   <211> 19
   <212> PRT
   <213> Artificial
<220>
   <223> Mutated Lama sequence
<400> 387
   Asp Arg Tyr Ile Trp Ala Arg Gln Gly Glu Tyr Trp Gly Ala Tyr Ala
   1 5 10 15
   Tyr Asp Tyr
<210> 388
   <211> 19
   <212> PRT
   <213> Artificial
<220>
   <223> Mutated Lama sequence
<400> 388
   Asp Arg Tyr Ile Trp Ala Arg Gln Gly Asp Tyr Trp Gly Ala Tyr Val
   1 5 10 15
   Tyr Asp Tyr
<210> 389
   <211> 19
   <212> PRT
   <213> Artificial
<220>
   <223> Mutated Lama sequence
<400> 389
   Asp Arg Tyr Ile Trp Ala Arg Gln Gly Asp Tyr Trp Gly Ala Tyr Ala
   1 5 10 15
   Tyr Asp Tyr
<210> 390
   <211> 19
   <212> PRT
   <213> Artificial
<220>
   <223> Mutated Lama sequence
<400> 390
   Asp Arg Tyr Ile Arg Ala Arg Gln Gly Glu Tyr Trp Gly Ala Tyr Ala
   1 5 10 15
   Tyr Asp Tyr
<210> 391
   <211> 19
   <212> PRT
   <213> Artificial
<220>
   <223> Mutated Lama sequence
<400> 391
   Asp Arg Tyr Ile Trp Ala Arg Gln Gly Glu Tyr Trp Gly Ala Tyr Ala
   1               5                   10                  15
   Tyr Asp Tyr
<210> 392
   <211> 19
   <212> PRT
   <213> Artificial
<220>
   <223> Mutated Lama sequence
<400> 392
   Asp Arg Tyr Ile Trp Ala Arg Gln Gly Glu Tyr Trp Gly Ala Tyr Glu
   1               5                   10                  15
   Tyr Asp Tyr
<210> 393
   <211> 19
   <212> PRT
   <213> Artificial
<220>
   <223> Mutated Lama sequence
<400> 393
   Asp Arg Tyr Ile Trp Ala Arg Gln Gly Glu Tyr Trp Gly Ala Tyr Ala
   1               5                   10                  15
   Tyr Asp Tyr
<210> 394
   <211> 19
   <212> PRT
   <213> Artificial
<220>
   <223> Mutated Lama sequence
<400> 394
   Asp Arg Tyr Ile Trp Ala Arg Gln Gly Glu Tyr Trp Gly Ala Tyr Gln
   1               5                   10                  15
   Tyr Asp Tyr
<210> 395
   <211> 19
   <212> PRT
   <213> Artificial
<220>
   <223> Mutated Lama sequence
<400> 395
   Asp Arg Tyr Ile Trp Ala Arg Gln Gly Glu Tyr Trp Gly Ala Tyr Ala
   1 5 10 15
   Tyr Asp Tyr
<210> 396
   <211> 128
   <212> PRT
   <213> Artificial
<220>
   <223> Mutated Lama sequence
<400> 396
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Gly Ser Tyr
   20 25 30
   Asp Met Ser Trp Val Arg Arg Ser Pro Gly Lys Gly Pro Glu Trp Val
   35 40 45
   Ser Ala Ile Asn Ser Gly Gly Gly Ser Thr Phe Tyr Thr Asp Tyr Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Ala Asp Arg Tyr Ile Trp Ala Arg Gln Gly Glu Tyr Trp Gly Ala
   100 105 110
   Tyr Gln Tyr Asp Tyr Trp Gly Gln Gly Thr Gln Val Thr Val Ser Ser
   115 120 125
<210> 397
   <211> 128
   <212> PRT
   <213> Artificial
<220>
   <223> Mutated Lama sequence
<400> 397
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Ser Tyr
               20                  25                  30
   Asp Met Ser Trp Val Arg Arg Ser Pro Gly Lys Gly Pro Glu Trp Val
           35                  40                  45
   Ser Ala Ile Asn Ser Gly Gly Gly Ser Thr Tyr Tyr Ala Asp Tyr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Asp Arg Tyr Ile Trp Ala Arg Gln Gly Glu Tyr Trp Gly Ala
               100                 105                 110
   Tyr Ala Tyr Asp Tyr Trp Gly Gln Gly Thr Gln Val Thr Val Ser Ser
           115                 120                 125
<210> 398
   <211> 128
   <212> PRT
   <213> Artificial
<220>
   <223> Mutated Lama sequence
<400> 398
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Ser Tyr
               20                  25                  30
   Asp Met Ser Trp Val Arg Arg Ser Pro Gly Lys Gly Pro Glu Trp Val
           35                  40                  45
   Ser Ala Ile Asn Ser Gly Gly Gly Ser Thr Tyr Tyr Thr Asp Tyr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Asp Arg Tyr Ile Trp Ala Arg Gln Gly Glu Tyr Trp Gly Ala
               100                 105                 110
   Tyr Val Tyr Asp Tyr Trp Gly Gln Gly Thr Gln Val Thr Val Ser Ser
           115                 120                 125
<210> 399
   <211> 128
   <212> PRT
   <213> Artificial
<220>
   <223> Mutated Lama sequence
<400> 399
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Ile Gly Ser Tyr
   20 25 30
   Asp Met Ser Trp Val Arg Arg Ser Pro Gly Lys Gly Pro Glu Trp Val
   35 40 45
   Ser Ala Ile Asn Ser Gly Gly Gly Ser Thr Tyr Tyr Ala Asp Tyr Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Ala Asp Arg Tyr Ile Trp Ala Arg Gln Gly Glu Tyr Trp Gly Ala
   100 105 110
   Tyr Gln Tyr Asp Tyr Trp Gly Gln Gly Thr Gln Val Thr Val Ser Ser
   115 120 125
<210> 400
   <211> 128
   <212> PRT
   <213> Artificial
<220>
   <223> Mutated Lama sequence
<400> 400
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Ile Gly Ser Tyr
   20 25 30
   Asp Met Ser Trp Val Arg Arg Ser Pro Gly Lys Gly Pro Glu Trp Val
   35 40 45
   Ser Ala Ile Asn Ser Gly Gly Gly Ser Thr Tyr Tyr Thr Asp Tyr Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Ala Asp Arg Tyr Ile Trp Ala Arg Gln Gly Glu Tyr Trp Gly Ala
   100 105 110
   Tyr Val Tyr Asp Tyr Trp Gly Gln Gly Thr Gln Val Thr Val Ser Ser
   115 120 125
<210> 401
   <211> 128
   <212> PRT
   <213> Artificial
<220>
   <223> Mutated Lama sequence
<400> 401
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Gly Ser Tyr
   20 25 30
   Asp Met Ser Trp Val Arg Arg Ser Pro Gly Lys Gly Pro Glu Trp Val
   35 40 45
   Ser Ala Ile Asn Ser Gly Gly Gly Ser Thr Tyr Tyr Thr Asp Tyr Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Ala Asp Arg Tyr Ile Trp Ala Arg Gln Gly Glu Tyr Trp Gly Ala
   100 105 110
   Tyr Gln Tyr Asp Tyr Trp Gly Gln Gly Thr Gln Val Thr Val Ser Ser
   115 120 125
<210> 402
   <211> 128
   <212> PRT
   <213> Artificial
<220>
   <223> Mutated Lama sequence
<400> 402
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Ser Tyr
               20                  25                  30
   Asp Met Ser Trp Val Arg Arg Ser Pro Gly Lys Gly Pro Glu Trp Val
           35                  40                  45
   Ser Ala Ile Asn Ser Gly Gly Gly Ser Thr Tyr Tyr Thr Asp Tyr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Asp Arg Tyr Ile Trp Ala Arg Gln Gly Glu Tyr Trp Gly Ala
               100                 105                 110
   Tyr Ala Tyr Asp Tyr Trp Gly Gln Gly Thr Gln Val Thr Val Ser Ser
           115                 120                 125
<210> 403
   <211> 128
   <212> PRT
   <213> Artificial
<220>
   <223> Mutated Lama sequence
<400> 403
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Ile Gly Ser Tyr
               20                  25                  30
   Asp Met Ser Trp Val Arg Arg Ser Pro Gly Lys Gly Pro Glu Trp Val
           35                  40                  45
   Ser Ala Ile Asn Ser Gly Gly Gly Ser Thr Tyr Tyr Thr Asp Tyr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Asp Arg Tyr Ile Trp Ala Arg Gln Gly Glu Tyr Trp Gly Ala
               100                 105                 110
   Tyr Ala Tyr Asp Tyr Trp Gly Gln Gly Thr Gln Val Thr Val Ser Ser
           115                 120                 125
<210> 404
   <211> 128
   <212> PRT
   <213> Artificial
<220>
   <223> Mutated Lama sequence
<400> 404
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Ile Gly Ser Tyr
               20                  25                  30
   Asp Met Ser Trp Val Arg Arg Ser Pro Gly Lys Gly Pro Glu Trp Val
           35                  40                  45
   Ser Ala Ile Asn Ser Gly Gly Gly Ser Thr Tyr Tyr Thr Asp Phe Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Asp Arg Tyr Ile Trp Ala Arg Gln Gly Glu Tyr Trp Gly Ala
               100                 105                 110
   Tyr Ala Tyr Asp Tyr Trp Gly Gln Gly Thr Gln Val Thr Val Ser Ser
           115                 120                 125
<210> 405
   <211> 128
   <212> PRT
   <213> Artificial
<220>
   <223> Mutated Lama sequence
<400> 405
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Ile Gly Ser Tyr
               20                  25                  30
   Asp Met Ser Trp Val Arg Arg Ser Pro Gly Lys Gly Pro Glu Trp Val
           35                  40                  45
   Ser Ser Ile Asn Ser Gly Gly Gly Ser Thr Tyr Tyr Thr Asp Tyr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Asp Arg Tyr Ile Trp Ala Arg Gln Gly Glu Tyr Trp Gly Ala
               100                 105                 110
   Tyr Ala Tyr Asp Tyr Trp Gly Gln Gly Thr Gln Val Thr Val Ser Ser
           115                 120                 125
<210> 406
   <211> 128
   <212> PRT
   <213> Artificial
<220>
   <223> Mutated Lama sequence
<400> 406
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Ile Gly Ser Tyr
               20                  25                  30
   Asp Met Ser Trp Val Arg Arg Ser Pro Gly Lys Gly Pro Glu Trp Val
           35                  40                  45
   Ser Ala Ile Asn Ser Gly Gly Gly Ser Thr Tyr Tyr Ala Asp Tyr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
   85 90 95
   Ala Ala Asp Arg Tyr Ile Trp Ala Arg Gln Gly Glu Tyr Trp Gly Ala
   100 105 110
   Tyr Gln Tyr Asp Tyr Trp Gly Gln Gly Thr Gln Val Thr Val Ser Ser
   115 120 125
<210> 407
   <211> 128
   <212> PRT
   <213> Artificial
<220>
   <223> Mutated Lama sequence
<400> 407
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Gly Ser Tyr
   20 25 30
   Asp Met Ser Trp Val Arg Arg Ser Pro Gly Lys Gly Pro Glu Trp Val
   35 40 45
   Ser Ser Ile Asn Ser Gly Gly Gly Ser Thr Tyr Tyr Ala Asp Tyr Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Ala Asp Arg Tyr Ile Trp Ala Arg Gln Gly Glu Tyr Trp Gly Ala
   100 105 110
   Tyr Glu Tyr Asp Tyr Trp Gly Gln Gly Thr Gln Val Thr Val Ser Ser
   115 120 125
<210> 408
   <211> 128
   <212> PRT
   <213> Artificial
<220>
   <223> Mutated Lama sequence
<400> 408
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Ile Gly Ser Tyr
               20                  25                  30
   Asp Met Ser Trp Val Arg Arg Ser Pro Gly Lys Gly Pro Glu Trp Val
           35                  40                  45
   Ser Ala Ile Asn Ser Gly Gly Asp Ser Thr Phe Tyr Ala Asp Tyr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Asp Arg Tyr Ile Trp Ala Arg Gln Gly Glu Tyr Trp Gly Ala
               100                 105                 110
   Tyr Ala Tyr Asp Tyr Trp Gly Gln Gly Thr Gln Val Thr Val Ser Ser
           115                 120                 125
<210> 409
   <211> 128
   <212> PRT
   <213> Artificial
<220>
   <223> Mutated Lama sequence
<400> 409
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Ser Tyr
               20                  25                  30
   Asp Met Ser Trp Leu Arg Arg Ser Pro Gly Lys Gly Pro Glu Trp Val
           35                  40                  45
   Ser Ala Ile Asn Ser Gly Gly Gly Ser Thr Tyr Tyr Ala Asp Tyr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Asp Arg Tyr Ile Trp Ala Arg Gln Gly Asp Tyr Trp Gly Ala
               100                 105                 110
   Tyr Val Tyr Asp Tyr Trp Gly Gln Gly Thr Gln Val Thr Val Ser Ser
           115                 120                 125
<210> 410
   <211> 128
   <212> PRT
   <213> Artificial
<220>
   <223> Mutated Lama sequence
<400> 410
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Ser Tyr
               20                  25                  30
   Asp Met Ser Trp Val Arg Arg Ser Pro Gly Lys Gly Pro Glu Trp Val
           35                  40                  45
   Ser Ala Ile Asn Ser Gly Gly Gly Ser Thr Tyr Tyr Thr Asp Tyr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Asp Arg Tyr Ile Trp Ala Arg Gln Gly Asp Tyr Trp Gly Ala
               100                 105                 110
   Tyr Ala Tyr Asp Tyr Trp Gly Gln Gly Thr Gln Val Thr Val Ser Ser
           115                 120                 125
<210> 411
   <211> 128
   <212> PRT
   <213> Artificial
<220>
   <223> Mutated Lama sequence
<400> 411
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Gly Ser Tyr
               20                  25                  30
   Asp Met Ser Trp Val Arg Arg Ser Pro Gly Lys Gly Pro Glu Trp Val
           35                  40                  45
   Ser Ala Ile Asn Ser Gly Gly Gly Ser Thr Tyr Tyr Thr Asp Tyr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Asp Arg Tyr Ile Arg Ala Arg Gln Gly Glu Tyr Trp Gly Ala
               100                 105                 110
   Tyr Ala Tyr Asp Tyr Trp Gly Gln Gly Thr Gln Val Thr Val Ser Ser
           115                 120                 125
<210> 412
   <211> 128
   <212> PRT
   <213> Artificial
<220>
   <223> Mutated Lama sequence
<400> 412
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Ile Gly Ser Tyr
               20                  25                  30
   Asp Met Ser Trp Val Arg Arg Ser Pro Gly Lys Gly Pro Glu Trp Val
           35                  40                  45
   Ser Ser Ile Asn Ser Gly Gly Gly Ser Thr Tyr Tyr Thr Asp Phe Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
   85 90 95
   Ala Ala Asp Arg Tyr Ile Trp Ala Arg Gln Gly Glu Tyr Trp Gly Ala
   100 105 110
   Tyr Ala Tyr Asp Tyr Trp Gly Gln Gly Thr Gln Val Thr Val Ser Ser
   115 120 125
<210> 413
   <211> 128
   <212> PRT
   <213> Artificial
<220>
   <223> Mutated Lama sequence
<400> 413
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Ile Gly Ser Tyr
   20 25 30
   Asp Met Ser Trp Val Arg Arg Ser Pro Gly Lys Gly Pro Glu Trp Val
   35 40 45
   Ser Ala Ile Asn Ser Gly Gly Gly Ser Thr Tyr Tyr Thr Asp Tyr Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Ala Asp Arg Tyr Ile Trp Ala Arg Gln Gly Glu Tyr Trp Gly Ala
   100 105 110
   Tyr Glu Tyr Asp Tyr Trp Gly Gln Gly Thr Gln Val Thr Val Ser Ser
   115 120 125
<210> 414
   <211> 128
   <212> PRT
   <213> Artificial
<220>
   <223> Mutated Lama sequence
<400> 414
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Ile Gly Ser Tyr
               20                  25                  30
   Asp Met Ser Trp Val Arg Arg Ser Pro Gly Lys Gly Pro Glu Trp Val
           35                  40                  45
   Ser Ser Ile Asn Ser Gly Gly Gly Ser Thr Phe Tyr Thr Asp Phe Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Asp Arg Tyr Ile Trp Ala Arg Gln Gly Glu Tyr Trp Gly Ala
               100                 105                 110
   Tyr Ala Tyr Asp Tyr Trp Gly Gln Gly Thr Gln Val Thr Val Ser Ser
           115                 120                 125
<210> 415
   <211> 128
   <212> PRT
   <213> Artificial
<220>
   <223> Mutated Lama sequence
<400> 415
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Gly Ser Tyr
   20 25 30
   Asp Met Ser Trp Val Arg Arg Ser Pro Gly Lys Gly Pro Glu Trp Val
   35 40 45
   Ser Ala Ile Asn Ser Gly Gly Gly Ser Thr Tyr Tyr Thr Asp Tyr Val
   50 55 60
   Lys Gly Arg Phe Thr Ile Ser Arg Asn Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Ala Asp Arg Tyr Ile Trp Ala Arg Gln Gly Glu Tyr Trp Gly Ala
   100 105 110
   Tyr Gln Tyr Asp Tyr Trp Gly Gln Gly Thr Gln Val Thr Val Ser Ser
   115 120 125
<210> 416
   <211> 128
   <212> PRT
   <213> Artificial
<220>
   <223> Mutated Lama sequence
<400> 416
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Ile Gly Ser Tyr
   20 25 30
   Asp Met Ser Trp Val Arg Arg Ser Pro Gly Lys Gly Pro Glu Trp Val
   35 40 45
   Ser Ala Ile Asn Ser Gly Gly Gly Ser Thr Tyr Tyr Ala Asp Tyr Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Ala Asp Arg Tyr Ile Trp Ala Arg Gln Gly Glu Tyr Trp Gly Ala
   100 105 110
   Tyr Ala Tyr Asp Tyr Trp Gly Gln Gly Thr Gln Val Thr Val Ser Ser
   115 120 125
<210> 417
   <211> 687
   <212> PRT
   <213> Homo sapiens
<400> 417
   His Met Met Ala Ala Ala Ser Arg Ser Ala Ser Gly Trp Ala Leu Leu
   1 5 10 15
   Leu Leu Val Ala Leu Trp Gln Gln Arg Ala Ala Gly Ser Gly Val Phe
               20                  25                  30
   Gln Leu Gln Leu Gln Glu Phe Ile Asn Glu Arg Gly Val Leu Ala Ser
           35                  40                  45
   Gly Arg Pro Cys Glu Pro Gly Cys Arg Thr Phe Phe Arg Val Cys Leu
       50                  55                  60
   Lys His Phe Gln Ala Val Val Ser Pro Gly Pro Cys Thr Phe Gly Thr
   65                  70                  75                  80
   Val Ser Thr Pro Val Leu Gly Thr Asn Ser Phe Ala Val Arg Asp Asp
                   85                  90                  95
   Ser Ser Gly Gly Gly Arg Asn Pro Leu Gln Leu Pro Phe Asn Phe Thr
               100                 105                 110
   Trp Pro Gly Thr Phe Ser Leu Ile Ile Glu Ala Trp His Ala Pro Gly
           115                 120                 125
   Asp Asp Leu Arg Pro Glu Ala Leu Pro Pro Asp Ala Leu Ile Ser Lys
       130                 135                 140
   Ile Ala Ile Gln Gly Ser Leu Ala Val Gly Gln Asn Trp Leu Leu Asp
   145                 150                 155                 160
   Glu Gln Thr Ser Thr Leu Thr Arg Leu Arg Tyr Ser Tyr Arg Val Ile
                   165                 170                 175
   Cys Ser Asp Asn Tyr Tyr Gly Asp Asn Cys Ser Arg Leu Cys Lys Lys
               180                 185                 190
   Arg Asn Asp His Phe Gly His Tyr Val Cys Gln Pro Asp Gly Asn Leu
           195                 200                 205
   Ser Cys Leu Pro Gly Trp Thr Gly Glu Tyr Cys Gln Gln Pro Ile Cys
       210                 215                 220
   Leu Ser Gly Cys His Glu Gln Asn Gly Tyr Cys Ser Lys Pro Ala Glu
   225                 230                 235                 240
   Cys Leu Cys Arg Pro Gly Trp Gln Gly Arg Leu Cys Asn Glu Cys Ile
                   245                 250                 255
   Pro His Asn Gly Cys Arg His Gly Thr Cys Ser Thr Pro Trp Gln Cys
   260 265 270
   Thr Cys Asp Glu Gly Trp Gly Gly Leu Phe Cys Asp Gln Asp Leu Asn
   275 280 285
   Tyr Cys Thr His His Ser Pro Cys Lys Asn Gly Ala Thr Cys Ser Asn
   290 295 300
   Ser Gly Gln Arg Ser Tyr Thr Cys Thr Cys Arg Pro Gly Tyr Thr Gly
   305 310 315 320
   Val Asp Cys Glu Leu Glu Leu Ser Glu Cys Asp Ser Asn Pro Cys Arg
   325 330 335
   Asn Gly Gly Ser Cys Lys Asp Gln Glu Asp Gly Tyr His Cys Leu Cys
   340 345 350
   Pro Pro Gly Tyr Tyr Gly Leu His Cys Glu His Ser Thr Leu Ser Cys
   355 360 365
   Ala Asp Ser Pro Cys Phe Asn Gly Gly Ser Cys Arg Glu Arg Asn Gln
   370 375 380
   Gly Ala Asn Tyr Ala Cys Glu Cys Pro Pro Asn Phe Thr Gly Ser Asn
   385 390 395 400
   Cys Glu Lys Lys Val Asp Arg Cys Thr Ser Asn Pro Cys Ala Asn Gly
   405 410 415
   Gly Gln Cys Leu Asn Arg Gly Pro Ser Arg Met Cys Arg Cys Arg Pro
   420 425 430
   Gly Phe Thr Gly Thr Tyr Cys Glu Leu His Val Ser Asp Cys Ala Arg
   435 440 445
   Asn Pro Cys Ala His Gly Gly Thr Cys His Asp Leu Glu Asn Gly Leu
   450 455 460
   Met Cys Thr Cys Pro Ala Gly Phe Ser Gly Arg Arg Cys Glu Val Arg
   465 470 475 480
   Thr Ser Ile Asp Ala Cys Ala Ser Ser Pro Cys Phe Asn Arg Ala Thr
   485 490 495
   Cys Tyr Thr Asp Leu Ser Thr Asp Thr Phe Val Cys Asn Cys Pro Tyr
   500 505 510
   Gly Phe Val Gly Ser Arg Cys Glu Phe Pro Val Gly Leu Pro Pro Ser
   515 520 525
   Phe Pro Trp Val Ala Val Ser Leu Gly Val Gly Leu Ala Val Leu Leu
   530 535 540
   Val Leu Leu Gly Met Val Ala Val Ala Val Arg Gln Leu Arg Leu Arg
   545 550 555 560
   Arg Pro Asp Asp Gly Ser Arg Glu Ala Met Asn Asn Leu Ser Asp Phe
   565 570 575
   Gln Lys Asp Asn Leu Ile Pro Ala Ala Gln Leu Lys Asn Thr Asn Gln
   580 585 590
   Lys Lys Glu Leu Glu Val Asp Cys Gly Leu Asp Lys Ser Asn Cys Gly
   595 600 605
   Lys Gln Gln Asn His Thr Leu Asp Tyr Asn Leu Ala Pro Gly Pro Leu
   610 615 620
   Gly Arg Gly Thr Met Pro Gly Lys Phe Pro His Ser Asp Lys Ser Leu
   625 630 635 640
   Gly Glu Lys Ala Pro Leu Arg Leu His Ser Glu Lys Pro Glu Cys Arg
   645 650 655
   Ile Ser Ala Ile Cys Ser Pro Arg Asp Ser Met Tyr Gln Ser Val Cys
   660 665 670
   Leu Ile Ser Glu Glu Arg Asn Glu Cys Val Ile Ala Thr Glu Val
   675 680 685
<210> 418
   <211> 707
   <212> PRT
   <213> Macaca mulatta
<400> 418
   Met Ala Cys Ala Cys Ala Met Leu Ala Thr Thr Ala Arg His Glu Ser
   1 5 10 15
   Ser Met Asn Lys Glu Tyr Met Ala Ala Ala Ser Trp Ser Ala Ser Gly
   20 25 30
   Trp Ala Leu Leu Leu Leu Val Ala Leu Trp Gln Gln Arg Ala Ala Gly
   35 40 45
   Ser Gly Val Phe Gln Leu Gln Leu Gln Glu Phe Val Asn Glu Arg Gly
   50 55 60
   Val Leu Ala Ser Gly Arg Pro Cys Glu Pro Gly Cys Arg Thr Phe Phe
   65 70 75 80
   Arg Val Cys Leu Lys His Phe Gln Ala Val Val Ser Pro Gly Pro Cys
   85 90 95
   Thr Phe Gly Ser Val Ser Thr Pro Val Leu Gly Thr Asn Ser Phe Ala
   100 105 110
   Val Arg Asp Asp Ser Ser Gly Gly Gly Arg Asn Pro Leu Gln Leu Pro
   115 120 125
   Phe Asn Phe Thr Trp Pro Gly Thr Phe Ser Leu Ile Ile Glu Ala Trp
   130 135 140
   His Ala Pro Gly Asp Asp Leu Arg Pro Glu Ala Leu Pro Pro Asp Ala
   145 150 155 160
   Leu Ile Ser Lys Ile Ala Ile Gln Gly Ser Leu Ala Val Gly Gln Asn
   165 170 175
   Trp Leu Leu Asp Glu Gln Thr Ser Thr Leu Thr Arg Leu Arg Tyr Ser
   180 185 190
   Tyr Arg Val Ile Cys Ser Asp Asn Tyr Tyr Gly Asp Asn Cys Ser Arg
   195 200 205
   Leu Cys Lys Lys Arg Asn Asp His Phe Gly His Tyr Val Cys Gln Pro
   210 215 220
   Asp Gly Asn Leu Ser Cys Leu Pro Gly Trp Thr Gly Glu Tyr Cys Gln
   225 230 235 240
   Gln Pro Ile Cys Leu Ser Gly Cys His Glu Gln Asn Gly Tyr Cys Ser
   245 250 255
   Lys Pro Ala Glu Cys Leu Cys Arg Pro Gly Trp Gln Gly Arg Leu Cys
   260 265 270
   Asn Glu Cys Ile Pro His Asn Gly Cys Arg His Gly Thr Cys Ser Thr
   275 280 285
   Pro Trp Gln Cys Thr Cys Asp Glu Gly Trp Gly Gly Leu Phe Cys Asp
   290 295 300
   Gln Asp Leu Asn Tyr Cys Thr His His Ser Pro Cys Lys Asn Gly Ala
   305                 310                 315                 320
   Thr Cys Ser Asn Ser Gly Gln Arg Ser Tyr Thr Cys Thr Cys Arg Pro
                   325                 330                 335
   Gly Tyr Thr Gly Val Asp Cys Glu Leu Glu Leu Ser Glu Cys Asp Ser
               340                 345                 350
   Asn Pro Cys Arg Asn Gly Gly Ser Cys Lys Asp Gln Glu Asp Gly Tyr
           355                 360                 365
   His Cys Leu Cys Pro Pro Gly Tyr Tyr Gly Leu His Cys Glu His Ser
       370                 375                 380
   Thr Leu Ser Cys Ala Asp Ser Pro Cys Phe Asn Gly Gly Ser Cys Arg
   385                 390                 395                 400
   Glu Arg Asn Gln Gly Ala Ser Tyr Ala Cys Glu Cys Pro Pro Asn Phe
                   405                 410                 415
   Thr Gly Ser Asn Cys Glu Lys Lys Val Asp Arg Cys Thr Ser Asn Pro
               420                 425                 430
   Cys Ala Asn Gly Gly Gln Cys Leu Asn Arg Gly Pro Ser Arg Met Cys
           435                 440                 445
   Arg Cys Arg Pro Gly Phe Thr Gly Thr Tyr Cys Glu Arg His Val Ser
       450                 455                 460
   Asp Cys Ala Arg Asn Pro Cys Ala His Gly Gly Thr Cys His Asp Leu
   465                 470                 475                 480
   Glu Ser Gly Leu Met Cys Thr Cys Pro Ala Gly Phe Ser Gly Arg Arg
                   485                 490                 495
   Cys Glu Val Arg Thr Ser Ile Asp Ala Cys Ala Ser Ser Pro Cys Phe
               500                 505                 510
   Asn Arg Ala Thr Cys Tyr Thr Asp Leu Ser Thr Asp Thr Phe Val Cys
           515                 520                 525
   Asn Cys Pro Tyr Gly Phe Val Gly Ser Arg Cys Glu Phe Pro Val Gly
       530                 535                 540
   Leu Pro Pro Ser Phe Pro Trp Val Ala Val Ser Leu Gly Val Gly Leu
   545                 550                 555                 560
   Ala Val Leu Leu Val Leu Leu Gly Met Val Ala Val Ala Val Arg Gln
   565 570 575
   Leu Arg Leu Arg Arg Pro Asp Asp Gly Ser Arg Glu Ala Met Asn Asn
   580 585 590
   Leu Ser Asp Phe Gln Lys Asp Asn Leu Ile Pro Ala Ala Gln Leu Lys
   595 600 605
   Asn Thr Asn Gln Lys Lys Glu Leu Glu Val Asp Cys Gly Leu Asp Lys
   610 615 620
   Ser Asn Cys Gly Lys Gln Gln Asn His Thr Leu Asp Tyr Asn Leu Ala
   625 630 635 640
   Pro Gly Pro Leu Gly Arg Gly Thr Met Pro Gly Lys Phe Pro His Ser
   645 650 655
   Asp Lys Ser Leu Gly Glu Lys Ala Pro Leu Arg Leu His Ser Glu Lys
   660 665 670
   Pro Glu Cys Arg Ile Ser Ala Ile Cys Ser Pro Arg Asp Ser Met Tyr
   675 680 685
   Gln Ser Val Cys Leu Ile Ser Glu Glu Arg Asn Glu Cys Val Ile Ala
   690 695 700
   Thr Glu Val
   705
<210> 419
   <211> 472
   <212> PRT
   <213> Homo sapiens
<400> 419
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Thr Asp Asn
   20 25 30
   Trp Ile Ser Trp Val Arg Gln Ala Pro Gly Lys Gly Leu Glu Trp Val
   35 40 45
   Gly Tyr Ile Ser Pro Asn Ser Gly Phe Thr Tyr Tyr Ala Asp Ser Val
   50 55 60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Asp Asn Phe Gly Gly Tyr Phe Asp Tyr Trp Gly Gln Gly Thr
               100                 105                 110
   Leu Val Thr Val Ser Ser Gly Glu Trp Ile Leu Cys Ala Trp Ala Gln
           115                 120                 125
   Leu Cys Pro Thr Pro Arg Ser His Gly Thr Thr Ser Leu Ala Ala Ser
       130                 135                 140
   Thr Lys Gly Pro Ser Val Phe Pro Leu Ala Pro Ser Ser Lys Ser Thr
   145                 150                 155                 160
   Ser Gly Gly Thr Ala Ala Leu Gly Cys Leu Val Lys Asp Tyr Phe Pro
                   165                 170                 175
   Glu Pro Val Thr Val Ser Trp Asn Ser Gly Ala Leu Thr Ser Gly Val
               180                 185                 190
   His Thr Phe Pro Ala Val Leu Gln Ser Ser Gly Leu Tyr Ser Leu Ser
           195                 200                 205
   Ser Val Val Thr Val Pro Ser Ser Ser Leu Gly Thr Gln Thr Tyr Ile
       210                 215                 220
   Cys Asn Val Asn His Lys Pro Ser Asn Thr Lys Val Asp Lys Arg Val
   225                 230                 235                 240
   Glu Pro Lys Ser Cys Asp Lys Thr His Thr Cys Pro Pro Cys Pro Ala
                   245                 250                 255
   Pro Glu Leu Leu Gly Gly Pro Ser Val Phe Leu Phe Pro Pro Lys Pro
               260                 265                 270
   Lys Asp Thr Leu Met Ile Ser Arg Thr Pro Glu Val Thr Cys Val Val
           275                 280                 285
   Val Asp Val Ser His Glu Asp Pro Glu Val Lys Phe Asn Trp Tyr Val
       290                 295                 300
   Asp Gly Val Glu Val His Asn Ala Lys Thr Lys Pro Arg Glu Glu Gln
   305 310 315 320
   Tyr Asn Ser Thr Tyr Arg Val Val Ser Val Leu Thr Val Leu His Gln
   325 330 335
   Asp Trp Leu Asn Gly Lys Glu Tyr Lys Cys Lys Val Ser Asn Lys Ala
   340 345 350
   Leu Pro Ala Pro Ile Glu Lys Thr Ile Ser Lys Ala Lys Gly Gln Pro
   355 360 365
   Arg Glu Pro Gln Val Tyr Thr Leu Pro Pro Ser Arg Glu Glu Met Thr
   370 375 380
   Lys Asn Gln Val Ser Leu Thr Cys Leu Val Lys Gly Phe Tyr Pro Ser
   385 390 395 400
   Asp Ile Ala Val Glu Trp Glu Ser Asn Gly Gln Pro Glu Asn Asn Tyr
   405 410 415
   Lys Thr Thr Pro Pro Val Leu Asp Ser Asp Gly Ser Phe Phe Leu Tyr
   420 425 430
   Ser Lys Leu Thr Val Asp Lys Ser Arg Trp Gln Gln Gly Asn Val Phe
   435 440 445
   Ser Cys Ser Val Met His Glu Ala Leu His Asn His Tyr Thr Gln Lys
   450 455 460
   Ser Leu Ser Leu Ser Pro Gly Lys
   465 470
<210> 420
   <211> 115
   <212> PRT
   <213> Homo sapiens
<400> 420
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Thr Tyr Tyr Cys Gln Gln Ser Tyr Thr Gly Thr
                   85                  90                  95
   Val Thr Phe Gly Gln Gly Thr Lys Val Glu Ile Lys Arg Glu Trp Ile
               100                 105                 110
   His Leu Gly
           115
<210> 421
   <211> 21
   <212> DNA
   <213> Artificial
<220>
   <223> Primer
<400> 421
   gcgaacagag ccagattgag g 21
<210> 422
   <211> 21
   <212> DNA
   <213> Artificial
<220>
   <223> Primer
<400> 422
   ggatgtccag gtaggctcct g 21
<210> 423
   <211> 20
   <212> DNA
   <213> Artificial
<220>
   <223> Primer
<400> 423
   gagcgacatc cctaacaagc 20
<210> 424
   <211> 20
   <212> DNA
   <213> Artificial
<220>
   <223> Primer
<400> 424
   cctcaactct gttcccttgg 20
<210> 425
   <211> 21
   <212> DNA
   <213> Artificial
<220>
   <223> Primer
<400> 425
   gcgaacagag ccagattcag g 21
<210> 426
   <211> 20
   <212> DNA
   <213> Artificial
<220>
   <223> Primer
<400> 426
   ccagacagac acccaaaggt 20
<210> 427
   <211> 45
   <212> DNA
   <213> Artificial
<220>
   <223> Primer
<400> 427
   gaggtgcaat tggtggagtc tgggggtggt ctggttcagg ctggt 45
<210> 428
   <211> 45
   <212> DNA
   <213> Artificial
<220>
   <223> Primer
<400> 428
   tcctgcgcag cttctggtcg taccttctcc agctacgcga tggct 45
<210> 429
   <211> 45
   <212> DNA
   <213> Artificial
<220>
   <223> Primer
<400> 429
   ccaggcaaag aacgcgagtw cgtagccgca atccgttgga gcggt 45
<210> 430
   <211> 44
   <212> DNA
   <213> Artificial
<220>
   <223> Primer
<400> 430
   ctgattccgt tcagggtcgt ttcaccatct ctcgtgacaa cgcg 44
<210> 431
   <211> 45
   <212> DNA
   <213> Artificial
<220>
   <223> Primer
<400> 431
   ctgcagatga actctctgaa accggaagat acggcagtct actac 45
<210> 432
   <211> 46
   <212> DNA
   <213> Artificial
<220>
   <223> Primer
<400> 432
   gacactcgtc tgcgtccgta cctgtacgac yattggggtc agggta 46
<210> 433
   <211> 46
   <212> DNA
   <213> Artificial
<220>
   <223> Primer
<400> 433
   gacactcgtc tggvaccgta cctgtacgac yattggggtc agggta 46
<210> 434
   <211> 46
   <212> DNA
   <213> Artificial
<220>
   <223> Primer
<400> 434
   gacactcgtc tgcgtccgta cgagtacgac yattggggtc agggta 46
<210> 435
   <211> 46
   <212> DNA
   <213> Artificial
<220>
   <223> Primer
<400> 435
   gacactcgtc tggvaccgta cgagtacgac yattggggtc agggta 46
<210> 436
   <211> 45
   <212> DNA
   <213> Artificial
<220>
   <223> Primer
<400> 436
   cagacgagtg tccggcgcac ggtttgcaca gtagtagact gccgt 45
<210> 437
   <211> 45
   <212> DNA
   <213> Artificial
<220>
   <223> Primer
<400> 437
   cagacgagtg tctrccgcac ggtttgcaca gtagtagact gccgt 45
<210> 438
   <211> 45
   <212> DNA
   <213> Artificial
<220>
   <223> Primer
<400> 438
   agagttcatc tgcagataga cggtgttttt cgcgttgtca cgaga 45
<210> 439
   <211> 45
   <212> DNA
   <213> Artificial
<220>
   <223> Primer
<400> 439
   ctgaacggaa tcagsgtaat acgcagttyc accgctccaa cggat 45
<210> 440
   <211> 45
   <212> DNA
   <213> Artificial
<220>
   <223> Primer
<400> 440
   gcgttctttg cctggagcct gacgawacca agccatcgcg tagct 45
<210> 441
   <211> 45
   <212> DNA
   <213> Artificial
<220>
   <223> Primer
<400> 441
   agaagctgcg caggacagac ggagagagcc accagcctga accag 45
<210> 442
   <211> 35
   <212> DNA
   <213> Artificial
<220>
   <223> Primer
<400> 442
   tgaggagacg gtgacctggg tcccctgacc ccaat 35
<210> 443
   <211> 45
   <212> DNA
   <213> Artificial
<220>
   <223> Primer
<400> 443
   gaggtgcaat tggtggagtc tgggggtggt ctggttcagc caggt 45
<210> 444
   <211> 32
   <212> DNA
   <213> Artificial
<220>
   <223> Primer
<400> 444
   tgaggagacg gtgacctggg tcccctgacc cc 32
<210> 445
   <211> 45
   <212> DNA
   <213> Artificial
<220>
   <223> Primer
<400> 445
   gtgcagcttc cggctttacg wtcggctcct acgacatgtc ttggg 45
<210> 446
   <211> 49
   <212> DNA
   <213> Artificial
<220>
   <223> Primer
<400> 446
   acgcacccca gtattcaccc tgacgcgccc aaatgtagcg atctgcagc 49
<210> 447
   <211> 45
   <212> DNA
   <213> Artificial
<220>
   <223> Primer
<400> 447
   aggtccggaa tgggtgtcck ctatcaactc tggtggtggt agcac 45
<210> 448
   <211> 45
   <212> DNA
   <213> Artificial
<220>
   <223> Primer
<400> 448
   tcttccggtt tcaggctgtt catctgcagg tacagcgtgt ttttg 45
<210> 449
   <211> 45
   <212> DNA
   <213> Artificial
<220>
   <223> Primer
<400> 449
   aaaggtcgtt tcaccatctc tcgtgacaac gccaaaaaca cgctg 45
<210> 450
   <211> 45
   <212> DNA
   <213> Artificial
<220>
   <223> Primer
<400> 450
   tgaaacgacc ttttwcgwag tcggygtagw aggtgctacc accac 45
<210> 451
   <211> 45
   <212> DNA
   <213> Artificial
<220>
   <223> Primer
<400> 451
   tgaaaccgga agataccgcg gtatactact gcgctgcaga tcgct 45
<210> 452
   <211> 45
   <212> DNA
   <213> Artificial
<220>
   <223> Primer
<400> 452
   ccattccgga cctttacccg gagaacgacg aacccaagac atgtc 45
<210> 453
   <211> 41
   <212> DNA
   <213> Artificial
<220>
   <223> Primer
<400> 453
   tactggggtg cgtacghata cgactactgg ggtcagggta c 41
<210> 454
   <211> 41
   <212> DNA
   <213> Artificial
<220>
   <223> Primer
<400> 454
   tactggggtg cgtaccagta cgactactgg ggtcagggta c 41
<210> 455
   <211> 45
   <212> DNA
   <213> Artificial
<220>
   <223> Primer
<400> 455
   ccggaagctg cacagctcag acgcagagaa ccacctggct gaacc 45
<210> 456
   <211> 49
   <212> DNA
   <213> Artificial
<220>
   <223> Primer
<400> 456
   acgcacccca gtagtaaccc tgacgcgccc raatgtagcg atctgcagc 49
<210> 457
   <211> 49
   <212> DNA
   <213> Artificial
<220>
   <223> Primer
<400> 457
   acgcacccca gtaktcaccc tgacgcgccc raatgtagcg atctgcagc 49
<210> 458
   <211> 11
   <212> PRT
   <213> Lama glama
<400> 458
   Pro Gly Ile Ala Ala Cys Arg Gly Ile His Tyr
   1 5 10
<210> 459
   <211> 120
   <212> PRT
   <213> Lama glama
<400> 459
   Glu Val Gln Leu Val Glu Ser Gly Gly Gly Leu Val Gln Pro Gly Gly
   1               5                   10                  15
   Ser Leu Arg Leu Ser Cys Thr Ala Ser Gly Phe Thr Phe Asp Asp Tyr
               20                  25                  30
   Ala Ile Gly Trp Phe Arg Gln Ala Pro Gly Lys Glu Pro Glu Gly Ile
           35                  40                  45
   Ser Cys Ile Ser Ser Ser Gly Ser Ile Thr Tyr Asp Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Pro Gly Ile Ala Ala Cys Arg Gly Ile His Tyr Trp Gly Gln
               100                 105                 110
   Gly Thr Gln Val Thr Val Ser Ser
           115                 120
<210> 460
   <211> 513
   <212> PRT
   <213> Artificial
<220>
   <223> Mutated Lama sequence
<400> 460
   Asp Val Gln Leu Val Glu Ser Gly Gly Gly Leu Val Gln Pro Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Tyr Arg Gln Ala Pro Gly Lys Glu Arg Glu Tyr Val
           35                  40                  45
   Ala Ala Ile Arg Trp Ser Gly Gly Thr Ala Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Asn Arg Ala Pro Asp Thr Arg Leu Ala Pro Tyr Glu Tyr Asp His
               100                 105                 110
   Trp Gly Gln Gly Thr Leu Val Thr Val Ser Ser Gly Gly Gly Gly Ser
           115                 120                 125
   Gly Gly Gly Ser Glu Val Gln Leu Val Glu Ser Gly Gly Gly Leu Val
       130                 135                 140
   Gln Pro Gly Asn Ser Leu Arg Leu Ser Cys Ala Ala Ser Gly Phe Thr
   145                 150                 155                 160
   Phe Ser Ser Phe Gly Met Ser Trp Val Arg Gln Ala Pro Gly Lys Gly
                   165                 170                 175
   Leu Glu Trp Val Ser Ser Ile Ser Gly Ser Gly Ser Asp Thr Leu Tyr
               180                 185                 190
   Ala Asp Ser Val Lys Gly Arg Phe Thr Ile Ser Arg Asp Asn Ala Lys
           195                 200                 205
   Thr Thr Leu Tyr Leu Gln Met Asn Ser Leu Arg Pro Glu Asp Thr Ala
       210                 215                 220
   Val Tyr Tyr Cys Thr Ile Gly Gly Ser Leu Ser Arg Ser Ser Gln Gly
   225                 230                 235                 240
   Thr Leu Val Thr Val Ser Ser Gly Gly Gly Gly Ser Gly Gly Gly Ser
                   245                 250                 255
   Glu Val Gln Leu Val Glu Ser Gly Gly Gly Leu Val Gln Pro Gly Gly
               260                 265                 270
   Ser Leu Arg Leu Ser Cys Ala Ala Ser Gly Phe Thr Phe Asp Asp Tyr
           275                 280                 285
   Ala Leu Gly Trp Phe Arg Gln Ala Pro Gly Lys Glu Arg Glu Gly Val
       290                 295                 300
   Ser Cys Ile Arg Cys Ser Asp Gly Ser Thr Tyr Tyr Ala Asp Ser Val
   305                 310                 315                 320
   Lys Gly Arg Phe Thr Ile Ser Ser Asp Asn Ser Lys Asn Thr Val Tyr
                   325                 330                 335
   Leu Gln Met Asn Ser Leu Arg Pro Glu Asp Thr Ala Val Tyr Tyr Cys
               340                 345                 350
   Ala Ala Ser Ile Val Pro Arg Ser Lys Leu Glu Pro Tyr Glu Tyr Asp
           355                 360                 365
   Ala Trp Gly Gln Gly Thr Leu Val Thr Val Ser Ser Gly Gly Gly Gly
       370                 375                 380
   Ser Gly Gly Gly Ser Glu Val Gln Leu Val Glu Ser Gly Gly Gly Leu
   385                 390                 395                 400
   Val Gln Pro Gly Gly Ser Leu Arg Leu Ser Cys Ala Ala Ser Gly Phe
                   405                 410                 415
   Thr Phe Asp Asp Tyr Ala Leu Gly Trp Phe Arg Gln Ala Pro Gly Lys
               420                 425                 430
   Glu Arg Glu Gly Val Ser Cys Ile Arg Cys Ser Asp Gly Ser Thr Tyr
   435 440 445
   Tyr Ala Asp Ser Val Lys Gly Arg Phe Thr Ile Ser Ser Asp Asn Ser
   450 455 460
   Lys Asn Thr Val Tyr Leu Gln Met Asn Ser Leu Arg Pro Glu Asp Thr
   465 470 475 480
   Ala Val Tyr Tyr Cys Ala Ala Ser Ile Val Pro Arg Ser Lys Leu Glu
   485 490 495
   Pro Tyr Glu Tyr Asp Ala Trp Gly Gln Gly Thr Leu Val Thr Val Ser
   500 505 510
   Ser
<210> 461
   <211> 513
   <212> PRT
   <213> Artificial
<220>
   <223> Mutated Lama sequence
<400> 461
   Asp Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Leu Gly Trp Phe Arg Gln Ala Pro Gly Lys Glu Arg Glu Gly Val
   35 40 45
   Ser Cys Ile Arg Cys Ser Asp Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Ser Ile Val Pro Arg Ser Lys Leu Glu Pro Tyr Glu Tyr Asp
   100 105 110
   Ala Trp Gly Gln Gly Thr Leu Val Thr Val Ser Ser Gly Gly Gly Gly
           115                 120                 125
   Ser Gly Gly Gly Ser Glu Val Gln Leu Val Glu Ser Gly Gly Gly Leu
       130                 135                 140
   Val Gln Pro Gly Gly Ser Leu Arg Leu Ser Cys Ala Ala Ser Gly Phe
   145                 150                 155                 160
   Thr Phe Asp Asp Tyr Ala Leu Gly Trp Phe Arg Gln Ala Pro Gly Lys
                   165                 170                 175
   Glu Arg Glu Gly Val Ser Cys Ile Arg Cys Ser Asp Gly Ser Thr Tyr
               180                 185                 190
   Tyr Ala Asp Ser Val Lys Gly Arg Phe Thr Ile Ser Ser Asp Asn Ser
           195                 200                 205
   Lys Asn Thr Val Tyr Leu Gln Met Asn Ser Leu Arg Pro Glu Asp Thr
       210                 215                 220
   Ala Val Tyr Tyr Cys Ala Ala Ser Ile Val Pro Arg Ser Lys Leu Glu
   225                 230                 235                 240
   Pro Tyr Glu Tyr Asp Ala Trp Gly Gln Gly Thr Leu Val Thr Val Ser
                   245                 250                 255
   Ser Gly Gly Gly Gly Ser Gly Gly Gly Ser Glu Val Gln Leu Val Glu
               260                 265                 270
   Ser Gly Gly Gly Leu Val Gln Pro Gly Asn Ser Leu Arg Leu Ser Cys
           275                 280                 285
   Ala Ala Ser Gly Phe Thr Phe Ser Ser Phe Gly Met Ser Trp Val Arg
       290                 295                 300
   Gln Ala Pro Gly Lys Gly Leu Glu Trp Val Ser Ser Ile Ser Gly Ser
   305                 310                 315                 320
   Gly Ser Asp Thr Leu Tyr Ala Asp Ser Val Lys Gly Arg Phe Thr Ile
                   325                 330                 335
   Ser Arg Asp Asn Ala Lys Thr Thr Leu Tyr Leu Gln Met Asn Ser Leu
               340                 345                 350
   Arg Pro Glu Asp Thr Ala Val Tyr Tyr Cys Thr Ile Gly Gly Ser Leu
           355                 360                 365
   Ser Arg Ser Ser Gln Gly Thr Leu Val Thr Val Ser Ser Gly Gly Gly
   370 375 380
   Gly Ser Gly Gly Gly Ser Glu Val Gln Leu Val Glu Ser Gly Gly Gly
   385 390 395 400
   Leu Val Gln Pro Gly Gly Ser Leu Arg Leu Ser Cys Ala Ala Ser Gly
   405 410 415
   Arg Thr Phe Ser Ser Tyr Ala Met Ala Trp Tyr Arg Gln Ala Pro Gly
   420 425 430
   Lys Glu Arg Glu Tyr Val Ala Ala Ile Arg Trp Ser Gly Gly Thr Ala
   435 440 445
   Tyr Tyr Ala Asp Ser Val Lys Gly Arg Phe Thr Ile Ser Arg Asp Asn
   450 455 460
   Ala Lys Asn Thr Val Tyr Leu Gln Met Asn Ser Leu Arg Pro Glu Asp
   465 470 475 480
   Thr Ala Val Tyr Tyr Cys Ala Asn Arg Ala Pro Asp Thr Arg Leu Ala
   485 490 495
   Pro Tyr Glu Tyr Asp His Trp Gly Gln Gly Thr Leu Val Thr Val Ser
   500 505 510
   Ser
<210> 462
   <211> 385
   <212> PRT
   <213> Artificial
<220>
   <223> Mutated Lama sequence
<400> 462
   Asp Val Gln Leu Val Glu Ser Gly Gly Gly Leu Val Gln Pro Gly Gly
   1 5 10 15
   Ser Leu Arg Leu Ser Cys Ala Ala Ser Gly Arg Thr Phe Ser Ser Tyr
   20 25 30
   Ala Met Ala Trp Tyr Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Asn Arg Ala Pro Asp Thr Arg Leu Ala Pro Tyr Glu Tyr Asp His
               100                 105                 110
   Trp Gly Gln Gly Thr Leu Val Thr Val Ser Ser Gly Gly Gly Gly Ser
           115                 120                 125
   Gly Gly Gly Ser Glu Val Gln Leu Val Glu Ser Gly Gly Gly Leu Val
       130                 135                 140
   Gln Pro Gly Asn Ser Leu Arg Leu Ser Cys Ala Ala Ser Gly Phe Thr
   145                 150                 155                 160
   Phe Ser Ser Phe Gly Met Ser Trp Val Arg Gln Ala Pro Gly Lys Gly
                   165                 170                 175
   Leu Glu Trp Val Ser Ser Ile Ser Gly Ser Gly Ser Asp Thr Leu Tyr
               180                 185                 190
   Ala Asp Ser Val Lys Gly Arg Phe Thr Ile Ser Arg Asp Asn Ala Lys
           195                 200                 205
   Thr Thr Leu Tyr Leu Gln Met Asn Ser Leu Arg Pro Glu Asp Thr Ala
       210                 215                 220
   Val Tyr Tyr Cys Thr Ile Gly Gly Ser Leu Ser Arg Ser Ser Gln Gly
   225                 230                 235                 240
   Thr Leu Val Thr Val Ser Ser Gly Gly Gly Gly Ser Gly Gly Gly Ser
                   245                 250                 255
   Glu Val Gln Leu Val Glu Ser Gly Gly Gly Leu Val Gln Pro Gly Gly
               260                 265                 270
   Ser Leu Arg Leu Ser Cys Ala Ala Ser Gly Phe Thr Leu Asp Asp Tyr
           275                 280                 285
   Ala Ile Gly Trp Phe Arg Gln Ala Pro Gly Lys Glu Arg Glu Gly Val
       290                 295                 300
   Ser Ser Ile Arg Asp Asn Asp Gly Ser Thr Tyr Tyr Ala Asp Ser Val
   305 310 315 320
   Lys Gly Arg Phe Thr Ile Ser Ser Asp Asn Ser Lys Asn Thr Val Tyr
   325 330 335
   Leu Gln Met Asn Ser Leu Arg Pro Glu Asp Thr Ala Val Tyr Tyr Cys
   340 345 350
   Ala Ala Val Pro Ala Gly Arg Leu Arg Phe Gly Glu Gln Trp Tyr Pro
   355 360 365
   Leu Tyr Glu Tyr Asp Ala Trp Gly Gln Gly Thr Leu Val Thr Val Ser
   370 375 380
   Ser
   385
<210> 463
   <211> 385
   <212> PRT
   <213> Artificial
<220>
   <223> Mutated Lama sequence
<400> 463
   Asp Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
           115                 120                 125
   Ser Gly Gly Gly Gly Ser Gly Gly Gly Ser Glu Val Gln Leu Val Glu
       130                 135                 140
   Ser Gly Gly Gly Leu Val Gln Pro Gly Asn Ser Leu Arg Leu Ser Cys
   145                 150                 155                 160
   Ala Ala Ser Gly Phe Thr Phe Ser Ser Phe Gly Met Ser Trp Val Arg
                   165                 170                 175
   Gln Ala Pro Gly Lys Gly Leu Glu Trp Val Ser Ser Ile Ser Gly Ser
               180                 185                 190
   Gly Ser Asp Thr Leu Tyr Ala Asp Ser Val Lys Gly Arg Phe Thr Ile
           195                 200                 205
   Ser Arg Asp Asn Ala Lys Thr Thr Leu Tyr Leu Gln Met Asn Ser Leu
       210                 215                 220
   Arg Pro Glu Asp Thr Ala Val Tyr Tyr Cys Thr Ile Gly Gly Ser Leu
   225                 230                 235                 240
   Ser Arg Ser Ser Gln Gly Thr Leu Val Thr Val Ser Ser Gly Gly Gly
                   245                 250                 255
   Gly Ser Gly Gly Gly Ser Glu Val Gln Leu Val Glu Ser Gly Gly Gly
               260                 265                 270
   Leu Val Gln Pro Gly Gly Ser Leu Arg Leu Ser Cys Ala Ala Ser Gly
           275                 280                 285
   Arg Thr Phe Ser Ser Tyr Ala Met Ala Trp Tyr Arg Gln Ala Pro Gly
       290                 295                 300
   Lys Glu Arg Glu Tyr Val Ala Ala Ile Arg Trp Ser Gly Gly Thr Ala
   305                 310                 315                 320
   Tyr Tyr Ala Asp Ser Val Lys Gly Arg Phe Thr Ile Ser Arg Asp Asn
                   325                 330                 335
   Ala Lys Asn Thr Val Tyr Leu Gln Met Asn Ser Leu Arg Pro Glu Asp
               340                 345                 350
   Thr Ala Val Tyr Tyr Cys Ala Asn Arg Ala Pro Asp Thr Arg Leu Ala
           355                 360                 365
   Pro Tyr Glu Tyr Asp His Trp Gly Gln Gly Thr Leu Val Thr Val Ser
   370 375 380
   Ser
   385
<210> 464
   <211> 523
   <212> PRT
   <213> Artificial
<220>
   <223> Mutated Lama sequence
<400> 464
   Asp Val Gln Leu Val Glu Ser Gly Gly Gly Leu Val Gln Pro Gly Gly
   1 5 10 15
   Ser Leu Arg Leu Ser Cys Ala Ala Ser Gly Arg Thr Phe Ser Ser Tyr
   20 25 30
   Ala Met Ala Trp Tyr Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Arg Pro Glu Asp Thr Ala Val Tyr Tyr Cys
   85 90 95
   Ala Asn Arg Ala Pro Asp Thr Arg Leu Ala Pro Tyr Glu Tyr Asp His
   100 105 110
   Trp Gly Gln Gly Thr Leu Val Thr Val Ser Ser Gly Gly Gly Gly Ser
   115 120 125
   Gly Gly Gly Ser Glu Val Gln Leu Val Glu Ser Gly Gly Gly Leu Val
   130 135 140
   Gln Pro Gly Asn Ser Leu Arg Leu Ser Cys Ala Ala Ser Gly Phe Thr
   145 150 155 160
   Phe Ser Ser Phe Gly Met Ser Trp Val Arg Gln Ala Pro Gly Lys Gly
   165 170 175
   Leu Glu Trp Val Ser Ser Ile Ser Gly Ser Gly Ser Asp Thr Leu Tyr
               180                 185                 190
   Ala Asp Ser Val Lys Gly Arg Phe Thr Ile Ser Arg Asp Asn Ala Lys
           195                 200                 205
   Thr Thr Leu Tyr Leu Gln Met Asn Ser Leu Arg Pro Glu Asp Thr Ala
       210                 215                 220
   Val Tyr Tyr Cys Thr Ile Gly Gly Ser Leu Ser Arg Ser Ser Gln Gly
   225                 230                 235                 240
   Thr Leu Val Thr Val Ser Ser Gly Gly Gly Gly Ser Gly Gly Gly Ser
                   245                 250                 255
   Glu Val Gln Leu Val Glu Ser Gly Gly Gly Leu Val Gln Pro Gly Gly
               260                 265                 270
   Ser Leu Arg Leu Ser Cys Ala Ala Ser Gly Phe Thr Leu Asp Asp Tyr
           275                 280                 285
   Ala Ile Gly Trp Phe Arg Gln Ala Pro Gly Lys Glu Arg Glu Gly Val
       290                 295                 300
   Ser Ser Ile Arg Asp Asn Asp Gly Ser Thr Tyr Tyr Ala Asp Ser Val
   305                 310                 315                 320
   Lys Gly Arg Phe Thr Ile Ser Ser Asp Asn Ser Lys Asn Thr Val Tyr
                   325                 330                 335
   Leu Gln Met Asn Ser Leu Arg Pro Glu Asp Thr Ala Val Tyr Tyr Cys
               340                 345                 350
   Ala Ala Val Pro Ala Gly Arg Leu Arg Phe Gly Glu Gln Trp Tyr Pro
           355                 360                 365
   Leu Tyr Glu Tyr Asp Ala Trp Gly Gln Gly Thr Leu Val Thr Val Ser
       370                 375                 380
   Ser Gly Gly Gly Gly Ser Gly Gly Gly Ser Glu Val Gln Leu Val Glu
   385                 390                 395                 400
   Ser Gly Gly Gly Leu Val Gln Pro Gly Gly Ser Leu Arg Leu Ser Cys
                   405                 410                 415
   Ala Ala Ser Gly Phe Thr Leu Asp Asp Tyr Ala Ile Gly Trp Phe Arg
               420                 425                 430
   Gln Ala Pro Gly Lys Glu Arg Glu Gly Val Ser Ser Ile Arg Asp Asn
           435                 440                 445
   Asp Gly Ser Thr Tyr Tyr Ala Asp Ser Val Lys Gly Arg Phe Thr Ile
       450                 455                 460
   Ser Ser Asp Asn Ser Lys Asn Thr Val Tyr Leu Gln Met Asn Ser Leu
   465                 470                 475                 480
   Arg Pro Glu Asp Thr Ala Val Tyr Tyr Cys Ala Ala Val Pro Ala Gly
                   485                 490                 495
   Arg Leu Arg Phe Gly Glu Gln Trp Tyr Pro Leu Tyr Glu Tyr Asp Ala
               500                 505                 510
   Trp Gly Gln Gly Thr Leu Val Thr Val Ser Ser
           515                 520
<210> 465
   <211> 382
   <212> PRT
   <213> Artificial
<220>
   <223> Mutated Lama sequence
<400> 465
   Asp Val Gln Leu Val Glu Ser Gly Gly Gly Leu Val Gln Pro Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Tyr Arg Gln Ala Pro Gly Lys Glu Arg Glu Tyr Val
           35                  40                  45
   Ala Ala Ile Arg Trp Ser Gly Gly Thr Ala Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Asn Arg Ala Pro Asp Thr Arg Leu Ala Pro Tyr Glu Tyr Asp His
               100                 105                 110
   Trp Gly Gln Gly Thr Leu Val Thr Val Ser Ser Gly Gly Gly Gly Ser
               115                 120                 125
   Gly Gly Gly Ser Glu Val Gln Leu Val Glu Ser Gly Gly Gly Leu Val
       130                 135                 140
   Gln Pro Gly Asn Ser Leu Arg Leu Ser Cys Ala Ala Ser Gly Phe Thr
   145                 150                 155                 160
   Phe Ser Ser Phe Gly Met Ser Trp Val Arg Gln Ala Pro Gly Lys Gly
                   165                 170                 175
   Leu Glu Trp Val Ser Ser Ile Ser Gly Ser Gly Ser Asp Thr Leu Tyr
               180                 185                 190
   Ala Asp Ser Val Lys Gly Arg Phe Thr Ile Ser Arg Asp Asn Ala Lys
           195                 200                 205
   Thr Thr Leu Tyr Leu Gln Met Asn Ser Leu Arg Pro Glu Asp Thr Ala
       210                 215                 220
   Val Tyr Tyr Cys Thr Ile Gly Gly Ser Leu Ser Arg Ser Ser Gln Gly
   225                 230                 235                 240
   Thr Leu Val Thr Val Ser Ser Gly Gly Gly Gly Ser Gly Gly Gly Ser
                   245                 250                 255
   Glu Val Gln Leu Val Glu Ser Gly Gly Gly Leu Val Gln Pro Gly Gly
               260                 265                 270
   Ser Leu Arg Leu Ser Cys Ala Ala Ser Gly Phe Ala Leu Asp Tyr Tyr
           275                 280                 285
   Ala Ile Gly Trp Phe Arg Gln Ala Pro Gly Lys Glu Arg Glu Gly Val
       290                 295                 300
   Ser Cys Ile Ser Ser Ser Asp Gly Ile Thr Tyr Tyr Ala Asp Ser Val
   305                 310                 315                 320
   Lys Gly Arg Phe Thr Ile Ser Arg Asp Asn Ser Lys Asn Thr Val Tyr
                   325                 330                 335
   Leu Gln Met Asn Ser Leu Arg Pro Glu Asp Thr Ala Val Tyr Tyr Cys
               340                 345                 350
   Ala Thr Asp Ser Gly Gly Tyr Ile Asp Tyr Asp Cys Met Gly Leu Gly
           355                 360                 365
   Tyr Asp Tyr Trp Gly Gln Gly Thr Leu Val Thr Val Ser Ser
       370                 375                 380
<210> 466
   <211> 382
   <212> PRT
   <213> Artificial
<220>
   <223> Mutated Lama sequence
<400> 466
   Asp Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ala Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ile Thr Tyr Tyr Ala Asp Ser Val
       50                  55                  60
   Lys Gly Arg Phe Thr Ile Ser Arg Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Thr Asp Ser Gly Gly Tyr Ile Asp Tyr Asp Cys Met Gly Leu Gly
               100                 105                 110
   Tyr Asp Tyr Trp Gly Gln Gly Thr Leu Val Thr Val Ser Ser Gly Gly
           115                 120                 125
   Gly Gly Ser Gly Gly Gly Ser Glu Val Gln Leu Val Glu Ser Gly Gly
       130                 135                 140
   Gly Leu Val Gln Pro Gly Asn Ser Leu Arg Leu Ser Cys Ala Ala Ser
   145                 150                 155                 160
   Gly Phe Thr Phe Ser Ser Phe Gly Met Ser Trp Val Arg Gln Ala Pro
                   165                 170                 175
   Gly Lys Gly Leu Glu Trp Val Ser Ser Ile Ser Gly Ser Gly Ser Asp
               180                 185                 190
   Thr Leu Tyr Ala Asp Ser Val Lys Gly Arg Phe Thr Ile Ser Arg Asp
   195 200 205
   Asn Ala Lys Thr Thr Leu Tyr Leu Gln Met Asn Ser Leu Arg Pro Glu
   210 215 220
   Asp Thr Ala Val Tyr Tyr Cys Thr Ile Gly Gly Ser Leu Ser Arg Ser
   225 230 235 240
   Ser Gln Gly Thr Leu Val Thr Val Ser Ser Gly Gly Gly Gly Ser Gly
   245 250 255
   Gly Gly Ser Glu Val Gln Leu Val Glu Ser Gly Gly Gly Leu Val Gln
   260 265 270
   Pro Gly Gly Ser Leu Arg Leu Ser Cys Ala Ala Ser Gly Arg Thr Phe
   275 280 285
   Ser Ser Tyr Ala Met Ala Trp Tyr Arg Gln Ala Pro Gly Lys Glu Arg
   290 295 300
   Glu Tyr Val Ala Ala Ile Arg Trp Ser Gly Gly Thr Ala Tyr Tyr Ala
   305 310 315 320
   Asp Ser Val Lys Gly Arg Phe Thr Ile Ser Arg Asp Asn Ala Lys Asn
   325 330 335
   Thr Val Tyr Leu Gln Met Asn Ser Leu Arg Pro Glu Asp Thr Ala Val
   340 345 350
   Tyr Tyr Cys Ala Asn Arg Ala Pro Asp Thr Arg Leu Ala Pro Tyr Glu
   355 360 365
   Tyr Asp His Trp Gly Gln Gly Thr Leu Val Thr Val Ser Ser
   370 375 380
<210> 467
   <211> 523
   <212> PRT
   <213> Artificial
<220>
   <223> Mutated Lama sequence
<400> 467
   Asp Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser Gly Gly Gly Gly Ser Gly Gly Gly Ser Glu Val Gln Leu Val Glu
       130                 135                 140
   Ser Gly Gly Gly Leu Val Gln Pro Gly Gly Ser Leu Arg Leu Ser Cys
   145                 150                 155                 160
   Ala Ala Ser Gly Phe Thr Leu Asp Asp Tyr Ala Ile Gly Trp Phe Arg
                   165                 170                 175
   Gln Ala Pro Gly Lys Glu Arg Glu Gly Val Ser Ser Ile Arg Asp Asn
               180                 185                 190
   Asp Gly Ser Thr Tyr Tyr Ala Asp Ser Val Lys Gly Arg Phe Thr Ile
           195                 200                 205
   Ser Ser Asp Asn Ser Lys Asn Thr Val Tyr Leu Gln Met Asn Ser Leu
       210                 215                 220
   Arg Pro Glu Asp Thr Ala Val Tyr Tyr Cys Ala Ala Val Pro Ala Gly
   225                 230                 235                 240
   Arg Leu Arg Phe Gly Glu Gln Trp Tyr Pro Leu Tyr Glu Tyr Asp Ala
                   245                 250                 255
   Trp Gly Gln Gly Thr Leu Val Thr Val Ser Ser Gly Gly Gly Gly Ser
               260                 265                 270
   Gly Gly Gly Ser Glu Val Gln Leu Val Glu Ser Gly Gly Gly Leu Val
           275                 280                 285
   Gln Pro Gly Asn Ser Leu Arg Leu Ser Cys Ala Ala Ser Gly Phe Thr
       290                 295                 300
   Phe Ser Ser Phe Gly Met Ser Trp Val Arg Gln Ala Pro Gly Lys Gly
   305                 310                 315                 320
   Leu Glu Trp Val Ser Ser Ile Ser Gly Ser Gly Ser Asp Thr Leu Tyr
                   325                 330                 335
   Ala Asp Ser Val Lys Gly Arg Phe Thr Ile Ser Arg Asp Asn Ala Lys
               340                 345                 350
   Thr Thr Leu Tyr Leu Gln Met Asn Ser Leu Arg Pro Glu Asp Thr Ala
           355                 360                 365
   Val Tyr Tyr Cys Thr Ile Gly Gly Ser Leu Ser Arg Ser Ser Gln Gly
       370                 375                 380
   Thr Leu Val Thr Val Ser Ser Gly Gly Gly Gly Ser Gly Gly Gly Ser
   385                 390                 395                 400
   Glu Val Gln Leu Val Glu Ser Gly Gly Gly Leu Val Gln Pro Gly Gly
                   405                 410                 415
   Ser Leu Arg Leu Ser Cys Ala Ala Ser Gly Arg Thr Phe Ser Ser Tyr
               420                 425                 430
   Ala Met Ala Trp Tyr Arg Gln Ala Pro Gly Lys Glu Arg Glu Tyr Val
           435                 440                 445
   Ala Ala Ile Arg Trp Ser Gly Gly Thr Ala Tyr Tyr Ala Asp Ser Val
       450                 455                 460
   Lys Gly Arg Phe Thr Ile Ser Arg Asp Asn Ala Lys Asn Thr Val Tyr
   465                 470                 475                 480
   Leu Gln Met Asn Ser Leu Arg Pro Glu Asp Thr Ala Val Tyr Tyr Cys
                   485                 490                 495
   Ala Asn Arg Ala Pro Asp Thr Arg Leu Ala Pro Tyr Glu Tyr Asp His
               500                 505                 510
   Trp Gly Gln Gly Thr Leu Val Thr Val Ser Ser
           515                 520
<210> 468
   <211> 513
   <212> PRT
   <213> Artificial
<220>
   <223> Mutated Lama sequence
<400> 468
   Asp Val Gln Leu Val Glu Ser Gly Gly Gly Leu Val Gln Pro Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Tyr Arg Gln Ala Pro Gly Lys Glu Arg Glu Tyr Val
           35                  40                  45
   Ala Ala Ile Arg Trp Ser Gly Gly Thr Ala Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Asn Arg Ala Pro Asp Thr Arg Leu Ala Pro Tyr Glu Tyr Asp His
               100                 105                 110
   Trp Gly Gln Gly Thr Leu Val Thr Val Ser Ser Gly Gly Gly Gly Ser
           115                 120                 125
   Gly Gly Gly Ser Glu Val Gln Leu Val Glu Ser Gly Gly Gly Leu Val
       130                 135                 140
   Gln Pro Gly Gly Ser Leu Arg Leu Ser Cys Ala Ala Ser Gly Phe Thr
   145                 150                 155                 160
   Phe Asp Asp Tyr Ala Leu Gly Trp Phe Arg Gln Ala Pro Gly Lys Glu
                   165                 170                 175
   Arg Glu Gly Val Ser Cys Ile Arg Cys Ser Asp Gly Ser Thr Tyr Tyr
               180                 185                 190
   Ala Asp Ser Val Lys Gly Arg Phe Thr Ile Ser Ser Asp Asn Ser Lys
           195                 200                 205
   Asn Thr Val Tyr Leu Gln Met Asn Ser Leu Arg Pro Glu Asp Thr Ala
       210                 215                 220
   Val Tyr Tyr Cys Ala Ala Ser Ile Val Pro Arg Ser Lys Leu Glu Pro
   225                 230                 235                 240
   Tyr Glu Tyr Asp Ala Trp Gly Gln Gly Thr Leu Val Thr Val Ser Ser
                   245                 250                 255
   Gly Gly Gly Gly Ser Gly Gly Gly Ser Glu Val Gln Leu Val Glu Ser
               260                 265                 270
   Gly Gly Gly Leu Val Gln Pro Gly Gly Ser Leu Arg Leu Ser Cys Ala
           275                 280                 285
   Ala Ser Gly Phe Thr Phe Asp Asp Tyr Ala Leu Gly Trp Phe Arg Gln
       290                 295                 300
   Ala Pro Gly Lys Glu Arg Glu Gly Val Ser Cys Ile Arg Cys Ser Asp
   305                 310                 315                 320
   Gly Ser Thr Tyr Tyr Ala Asp Ser Val Lys Gly Arg Phe Thr Ile Ser
                   325                 330                 335
   Ser Asp Asn Ser Lys Asn Thr Val Tyr Leu Gln Met Asn Ser Leu Arg
               340                 345                 350
   Pro Glu Asp Thr Ala Val Tyr Tyr Cys Ala Ala Ser Ile Val Pro Arg
           355                 360                 365
   Ser Lys Leu Glu Pro Tyr Glu Tyr Asp Ala Trp Gly Gln Gly Thr Leu
       370                 375                 380
   Val Thr Val Ser Ser Gly Gly Gly Gly Ser Gly Gly Gly Ser Glu Val
   385                 390                 395                 400
   Gln Leu Val Glu Ser Gly Gly Gly Leu Val Gln Pro Gly Asn Ser Leu
                   405                 410                 415
   Arg Leu Ser Cys Ala Ala Ser Gly Phe Thr Phe Ser Ser Phe Gly Met
               420                 425                 430
   Ser Trp Val Arg Gln Ala Pro Gly Lys Gly Leu Glu Trp Val Ser Ser
           435                 440                 445
   Ile Ser Gly Ser Gly Ser Asp Thr Leu Tyr Ala Asp Ser Val Lys Gly
       450                 455                 460
   Arg Phe Thr Ile Ser Arg Asp Asn Ala Lys Thr Thr Leu Tyr Leu Gln
   465 470 475 480
   Met Asn Ser Leu Arg Pro Glu Asp Thr Ala Val Tyr Tyr Cys Thr Ile
   485 490 495
   Gly Gly Ser Leu Ser Arg Ser Ser Gln Gly Thr Leu Val Thr Val Ser
   500 505 510
   Ser
<210> 469
   <211> 513
   <212> PRT
   <213> Artificial
<220>
   <223> Mutated Lama sequence
<400> 469
   Asp Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Leu Gly Trp Phe Arg Gln Ala Pro Gly Lys Glu Arg Glu Gly Val
   35 40 45
   Ser Cys Ile Arg Cys Ser Asp Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Ser Ile Val Pro Arg Ser Lys Leu Glu Pro Tyr Glu Tyr Asp
   100 105 110
   Ala Trp Gly Gln Gly Thr Leu Val Thr Val Ser Ser Gly Gly Gly Gly
   115 120 125
   Ser Gly Gly Gly Ser Glu Val Gln Leu Val Glu Ser Gly Gly Gly Leu
   130 135 140
   Val Gln Pro Gly Gly Ser Leu Arg Leu Ser Cys Ala Ala Ser Gly Phe
   145 150 155 160
   Thr Phe Asp Asp Tyr Ala Leu Gly Trp Phe Arg Gln Ala Pro Gly Lys
   165 170 175
   Glu Arg Glu Gly Val Ser Cys Ile Arg Cys Ser Asp Gly Ser Thr Tyr
   180 185 190
   Tyr Ala Asp Ser Val Lys Gly Arg Phe Thr Ile Ser Ser Asp Asn Ser
   195 200 205
   Lys Asn Thr Val Tyr Leu Gln Met Asn Ser Leu Arg Pro Glu Asp Thr
   210 215 220
   Ala Val Tyr Tyr Cys Ala Ala Ser Ile Val Pro Arg Ser Lys Leu Glu
   225 230 235 240
   Pro Tyr Glu Tyr Asp Ala Trp Gly Gln Gly Thr Leu Val Thr Val Ser
   245 250 255
   Ser Gly Gly Gly Gly Ser Gly Gly Gly Ser Glu Val Gln Leu Val Glu
   260 265 270
   Ser Gly Gly Gly Leu Val Gln Pro Gly Gly Ser Leu Arg Leu Ser Cys
   275 280 285
   Ala Ala Ser Gly Arg Thr Phe Ser Ser Tyr Ala Met Ala Trp Tyr Arg
   290 295 300
   Gln Ala Pro Gly Lys Glu Arg Glu Tyr Val Ala Ala Ile Arg Trp Ser
   305 310 315 320
   Gly Gly Thr Ala Tyr Tyr Ala Asp Ser Val Lys Gly Arg Phe Thr Ile
   325 330 335
   Ser Arg Asp Asn Ala Lys Asn Thr Val Tyr Leu Gln Met Asn Ser Leu
   340 345 350
   Arg Pro Glu Asp Thr Ala Val Tyr Tyr Cys Ala Asn Arg Ala Pro Asp
   355 360 365
   Thr Arg Leu Ala Pro Tyr Glu Tyr Asp His Trp Gly Gln Gly Thr Leu
   370 375 380
   Val Thr Val Ser Ser Gly Gly Gly Gly Ser Gly Gly Gly Ser Glu Val
   385 390 395 400
   Gln Leu Val Glu Ser Gly Gly Gly Leu Val Gln Pro Gly Asn Ser Leu
   405 410 415
   Arg Leu Ser Cys Ala Ala Ser Gly Phe Thr Phe Ser Ser Phe Gly Met
   420 425 430
   Ser Trp Val Arg Gln Ala Pro Gly Lys Gly Leu Glu Trp Val Ser Ser
   435 440 445
   Ile Ser Gly Ser Gly Ser Asp Thr Leu Tyr Ala Asp Ser Val Lys Gly
   450 455 460
   Arg Phe Thr Ile Ser Arg Asp Asn Ala Lys Thr Thr Leu Tyr Leu Gln
   465 470 475 480
   Met Asn Ser Leu Arg Pro Glu Asp Thr Ala Val Tyr Tyr Cys Thr Ile
   485 490 495
   Gly Gly Ser Leu Ser Arg Ser Ser Gln Gly Thr Leu Val Thr Val Ser
   500 505 510
   Ser
<210> 470
   <211> 385
   <212> PRT
   <213> Artificial
<220>
   <223> Mutated Lama sequence
<400> 470
   Asp Val Gln Leu Val Glu Ser Gly Gly Gly Leu Val Gln Pro Gly Gly
   1 5 10 15
   Ser Leu Arg Leu Ser Cys Ala Ala Ser Gly Arg Thr Phe Ser Ser Tyr
   20 25 30
   Ala Met Ala Trp Tyr Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Arg Pro Glu Asp Thr Ala Val Tyr Tyr Cys
                   85                  90                  95
   Ala Asn Arg Ala Pro Asp Thr Arg Leu Ala Pro Tyr Glu Tyr Asp His
               100                 105                 110
   Trp Gly Gln Gly Thr Leu Val Thr Val Ser Ser Gly Gly Gly Gly Ser
           115                 120                 125
   Gly Gly Gly Ser Glu Val Gln Leu Val Glu Ser Gly Gly Gly Leu Val
       130                 135                 140
   Gln Pro Gly Gly Ser Leu Arg Leu Ser Cys Ala Ala Ser Gly Phe Thr
   145                 150                 155                 160
   Leu Asp Asp Tyr Ala Ile Gly Trp Phe Arg Gln Ala Pro Gly Lys Glu
                   165                 170                 175
   Arg Glu Gly Val Ser Ser Ile Arg Asp Asn Asp Gly Ser Thr Tyr Tyr
               180                 185                 190
   Ala Asp Ser Val Lys Gly Arg Phe Thr Ile Ser Ser Asp Asn Ser Lys
           195                 200                 205
   Asn Thr Val Tyr Leu Gln Met Asn Ser Leu Arg Pro Glu Asp Thr Ala
       210                 215                 220
   Val Tyr Tyr Cys Ala Ala Val Pro Ala Gly Arg Leu Arg Phe Gly Glu
   225                 230                 235                 240
   Gln Trp Tyr Pro Leu Tyr Glu Tyr Asp Ala Trp Gly Gln Gly Thr Leu
                   245                 250                 255
   Val Thr Val Ser Ser Gly Gly Gly Gly Ser Gly Gly Gly Ser Glu Val
               260                 265                 270
   Gln Leu Val Glu Ser Gly Gly Gly Leu Val Gln Pro Gly Asn Ser Leu
           275                 280                 285
   Arg Leu Ser Cys Ala Ala Ser Gly Phe Thr Phe Ser Ser Phe Gly Met
       290                 295                 300
   Ser Trp Val Arg Gln Ala Pro Gly Lys Gly Leu Glu Trp Val Ser Ser
   305                 310                 315                 320
   Ile Ser Gly Ser Gly Ser Asp Thr Leu Tyr Ala Asp Ser Val Lys Gly
                   325                 330                 335
   Arg Phe Thr Ile Ser Arg Asp Asn Ala Lys Thr Thr Leu Tyr Leu Gln
   340 345 350
   Met Asn Ser Leu Arg Pro Glu Asp Thr Ala Val Tyr Tyr Cys Thr Ile
   355 360 365
   Gly Gly Ser Leu Ser Arg Ser Ser Gln Gly Thr Leu Val Thr Val Ser
   370 375 380
   Ser
   385
<210> 471
   <211> 385
   <212> PRT
   <213> Artificial
<220>
   <223> Mutated Lama sequence
<400> 471
   Asp Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
   115 120 125
   Ser Gly Gly Gly Gly Ser Gly Gly Gly Ser Glu Val Gln Leu Val Glu
   130 135 140
   Ser Gly Gly Gly Leu Val Gln Pro Gly Gly Ser Leu Arg Leu Ser Cys
   145                 150                 155                 160
   Ala Ala Ser Gly Arg Thr Phe Ser Ser Tyr Ala Met Ala Trp Tyr Arg
                   165                 170                 175
   Gln Ala Pro Gly Lys Glu Arg Glu Tyr Val Ala Ala Ile Arg Trp Ser
               180                 185                 190
   Gly Gly Thr Ala Tyr Tyr Ala Asp Ser Val Lys Gly Arg Phe Thr Ile
           195                 200                 205
   Ser Arg Asp Asn Ala Lys Asn Thr Val Tyr Leu Gln Met Asn Ser Leu
       210                 215                 220
   Arg Pro Glu Asp Thr Ala Val Tyr Tyr Cys Ala Asn Arg Ala Pro Asp
   225                 230                 235                 240
   Thr Arg Leu Ala Pro Tyr Glu Tyr Asp His Trp Gly Gln Gly Thr Leu
                   245                 250                 255
   Val Thr Val Ser Ser Gly Gly Gly Gly Ser Gly Gly Gly Ser Glu Val
               260                 265                 270
   Gln Leu Val Glu Ser Gly Gly Gly Leu Val Gln Pro Gly Asn Ser Leu
           275                 280                 285
   Arg Leu Ser Cys Ala Ala Ser Gly Phe Thr Phe Ser Ser Phe Gly Met
       290                 295                 300
   Ser Trp Val Arg Gln Ala Pro Gly Lys Gly Leu Glu Trp Val Ser Ser
   305                 310                 315                 320
   Ile Ser Gly Ser Gly Ser Asp Thr Leu Tyr Ala Asp Ser Val Lys Gly
                   325                 330                 335
   Arg Phe Thr Ile Ser Arg Asp Asn Ala Lys Thr Thr Leu Tyr Leu Gln
               340                 345                 350
   Met Asn Ser Leu Arg Pro Glu Asp Thr Ala Val Tyr Tyr Cys Thr Ile
           355                 360                 365
   Gly Gly Ser Leu Ser Arg Ser Ser Gln Gly Thr Leu Val Thr Val Ser
       370                 375                 380
   Ser
   385
<210> 472
   <211> 523
   <212> PRT
   <213> Artificial
<220>
   <223> Mutated Lama sequence
<400> 472
   Asp Val Gln Leu Val Glu Ser Gly Gly Gly Leu Val Gln Pro Gly Gly
   1               5                   10                  15
   Ser Leu Arg Leu Ser Cys Ala Ala Ser Gly Arg Thr Phe Ser Ser Tyr
               20                  25                  30
   Ala Met Ala Trp Tyr Arg Gln Ala Pro Gly Lys Glu Arg Glu Tyr Val
           35                  40                  45
   Ala Ala Ile Arg Trp Ser Gly Gly Thr Ala Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Asn Arg Ala Pro Asp Thr Arg Leu Ala Pro Tyr Glu Tyr Asp His
               100                 105                 110
   Trp Gly Gln Gly Thr Leu Val Thr Val Ser Ser Gly Gly Gly Gly Ser
           115                 120                 125
   Gly Gly Gly Ser Glu Val Gln Leu Val Glu Ser Gly Gly Gly Leu Val
       130                 135                 140
   Gln Pro Gly Gly Ser Leu Arg Leu Ser Cys Ala Ala Ser Gly Phe Thr
   145                 150                 155                 160
   Leu Asp Asp Tyr Ala Ile Gly Trp Phe Arg Gln Ala Pro Gly Lys Glu
                   165                 170                 175
   Arg Glu Gly Val Ser Ser Ile Arg Asp Asn Asp Gly Ser Thr Tyr Tyr
               180                 185                 190
   Ala Asp Ser Val Lys Gly Arg Phe Thr Ile Ser Ser Asp Asn Ser Lys
           195                 200                 205
   Asn Thr Val Tyr Leu Gln Met Asn Ser Leu Arg Pro Glu Asp Thr Ala
       210                 215                 220
   Val Tyr Tyr Cys Ala Ala Val Pro Ala Gly Arg Leu Arg Phe Gly Glu
   225                 230                 235                 240
   Gln Trp Tyr Pro Leu Tyr Glu Tyr Asp Ala Trp Gly Gln Gly Thr Leu
                   245                 250                 255
   Val Thr Val Ser Ser Gly Gly Gly Gly Ser Gly Gly Gly Ser Glu Val
               260                 265                 270
   Gln Leu Val Glu Ser Gly Gly Gly Leu Val Gln Pro Gly Gly Ser Leu
           275                 280                 285
   Arg Leu Ser Cys Ala Ala Ser Gly Phe Thr Leu Asp Asp Tyr Ala Ile
       290                 295                 300
   Gly Trp Phe Arg Gln Ala Pro Gly Lys Glu Arg Glu Gly Val Ser Ser
   305                 310                 315                 320
   Ile Arg Asp Asn Asp Gly Ser Thr Tyr Tyr Ala Asp Ser Val Lys Gly
                   325                 330                 335
   Arg Phe Thr Ile Ser Ser Asp Asn Ser Lys Asn Thr Val Tyr Leu Gln
               340                 345                 350
   Met Asn Ser Leu Arg Pro Glu Asp Thr Ala Val Tyr Tyr Cys Ala Ala
           355                 360                 365
   Val Pro Ala Gly Arg Leu Arg Phe Gly Glu Gln Trp Tyr Pro Leu Tyr
       370                 375                 380
   Glu Tyr Asp Ala Trp Gly Gln Gly Thr Leu Val Thr Val Ser Ser Gly
   385                 390                 395                 400
   Gly Gly Gly Ser Gly Gly Gly Ser Glu Val Gln Leu Val Glu Ser Gly
                   405                 410                 415
   Gly Gly Leu Val Gln Pro Gly Asn Ser Leu Arg Leu Ser Cys Ala Ala
               420                 425                 430
   Ser Gly Phe Thr Phe Ser Ser Phe Gly Met Ser Trp Val Arg Gln Ala
           435                 440                 445
   Pro Gly Lys Gly Leu Glu Trp Val Ser Ser Ile Ser Gly Ser Gly Ser
       450                 455                 460
   Asp Thr Leu Tyr Ala Asp Ser Val Lys Gly Arg Phe Thr Ile Ser Arg
   465 470 475 480
   Asp Asn Ala Lys Thr Thr Leu Tyr Leu Gln Met Asn Ser Leu Arg Pro
   485 490 495
   Glu Asp Thr Ala Val Tyr Tyr Cys Thr Ile Gly Gly Ser Leu Ser Arg
   500 505 510
   Ser Ser Gln Gly Thr Leu Val Thr Val Ser Ser
   515 520
<210> 473
   <211> 523
   <212> PRT
   <213> Artificial
<220>
   <223> Mutated Lama sequence
<400> 473
   Asp Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
   115 120 125
   Ser Gly Gly Gly Gly Ser Gly Gly Gly Ser Glu Val Gln Leu Val Glu
   130 135 140
   Ser Gly Gly Gly Leu Val Gln Pro Gly Gly Ser Leu Arg Leu Ser Cys
   145                 150                 155                 160
   Ala Ala Ser Gly Phe Thr Leu Asp Asp Tyr Ala Ile Gly Trp Phe Arg
                   165                 170                 175
   Gln Ala Pro Gly Lys Glu Arg Glu Gly Val Ser Ser Ile Arg Asp Asn
               180                 185                 190
   Asp Gly Ser Thr Tyr Tyr Ala Asp Ser Val Lys Gly Arg Phe Thr Ile
           195                 200                 205
   Ser Ser Asp Asn Ser Lys Asn Thr Val Tyr Leu Gln Met Asn Ser Leu
       210                 215                 220
   Arg Pro Glu Asp Thr Ala Val Tyr Tyr Cys Ala Ala Val Pro Ala Gly
   225                 230                 235                 240
   Arg Leu Arg Phe Gly Glu Gln Trp Tyr Pro Leu Tyr Glu Tyr Asp Ala
                   245                 250                 255
   Trp Gly Gln Gly Thr Leu Val Thr Val Ser Ser Gly Gly Gly Gly Ser
               260                 265                 270
   Gly Gly Gly Ser Glu Val Gln Leu Val Glu Ser Gly Gly Gly Leu Val
           275                 280                 285
   Gln Pro Gly Gly Ser Leu Arg Leu Ser Cys Ala Ala Ser Gly Arg Thr
       290                 295                 300
   Phe Ser Ser Tyr Ala Met Ala Trp Tyr Arg Gln Ala Pro Gly Lys Glu
   305                 310                 315                 320
   Arg Glu Tyr Val Ala Ala Ile Arg Trp Ser Gly Gly Thr Ala Tyr Tyr
                   325                 330                 335
   Ala Asp Ser Val Lys Gly Arg Phe Thr Ile Ser Arg Asp Asn Ala Lys
               340                 345                 350
   Asn Thr Val Tyr Leu Gln Met Asn Ser Leu Arg Pro Glu Asp Thr Ala
           355                 360                 365
   Val Tyr Tyr Cys Ala Asn Arg Ala Pro Asp Thr Arg Leu Ala Pro Tyr
       370                 375                 380
   Glu Tyr Asp His Trp Gly Gln Gly Thr Leu Val Thr Val Ser Ser Gly
   385                 390                 395                 400
   Gly Gly Gly Ser Gly Gly Gly Ser Glu Val Gln Leu Val Glu Ser Gly
   405 410 415
   Gly Gly Leu Val Gln Pro Gly Asn Ser Leu Arg Leu Ser Cys Ala Ala
   420 425 430
   Ser Gly Phe Thr Phe Ser Ser Phe Gly Met Ser Trp Val Arg Gln Ala
   435 440 445
   Pro Gly Lys Gly Leu Glu Trp Val Ser Ser Ile Ser Gly Ser Gly Ser
   450 455 460
   Asp Thr Leu Tyr Ala Asp Ser Val Lys Gly Arg Phe Thr Ile Ser Arg
   465 470 475 480
   Asp Asn Ala Lys Thr Thr Leu Tyr Leu Gln Met Asn Ser Leu Arg Pro
   485 490 495
   Glu Asp Thr Ala Val Tyr Tyr Cys Thr Ile Gly Gly Ser Leu Ser Arg
   500 505 510
   Ser Ser Gln Gly Thr Leu Val Thr Val Ser Ser
   515 520
<210> 474
   <211> 382
   <212> PRT
   <213> Artificial
<220>
   <223> Mutated Lama sequence
<400> 474
   Asp Val Gln Leu Val Glu Ser Gly Gly Gly Leu Val Gln Pro Gly Gly
   1 5 10 15
   Ser Leu Arg Leu Ser Cys Ala Ala Ser Gly Arg Thr Phe Ser Ser Tyr
   20 25 30
   Ala Met Ala Trp Tyr Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Arg Pro Glu Asp Thr Ala Val Tyr Tyr Cys
                   85                  90                  95
   Ala Asn Arg Ala Pro Asp Thr Arg Leu Ala Pro Tyr Glu Tyr Asp His
               100                 105                 110
   Trp Gly Gln Gly Thr Leu Val Thr Val Ser Ser Gly Gly Gly Gly Ser
           115                 120                 125
   Gly Gly Gly Ser Glu Val Gln Leu Val Glu Ser Gly Gly Gly Leu Val
       130                 135                 140
   Gln Pro Gly Gly Ser Leu Arg Leu Ser Cys Ala Ala Ser Gly Phe Ala
   145                 150                 155                 160
   Leu Asp Tyr Tyr Ala Ile Gly Trp Phe Arg Gln Ala Pro Gly Lys Glu
                   165                 170                 175
   Arg Glu Gly Val Ser Cys Ile Ser Ser Ser Asp Gly Ile Thr Tyr Tyr
               180                 185                 190
   Ala Asp Ser Val Lys Gly Arg Phe Thr Ile Ser Arg Asp Asn Ser Lys
           195                 200                 205
   Asn Thr Val Tyr Leu Gln Met Asn Ser Leu Arg Pro Glu Asp Thr Ala
       210                 215                 220
   Val Tyr Tyr Cys Ala Thr Asp Ser Gly Gly Tyr Ile Asp Tyr Asp Cys
   225                 230                 235                 240
   Met Gly Leu Gly Tyr Asp Tyr Trp Gly Gln Gly Thr Leu Val Thr Val
                   245                 250                 255
   Ser Ser Gly Gly Gly Gly Ser Gly Gly Gly Ser Glu Val Gln Leu Val
               260                 265                 270
   Glu Ser Gly Gly Gly Leu Val Gln Pro Gly Asn Ser Leu Arg Leu Ser
           275                 280                 285
   Cys Ala Ala Ser Gly Phe Thr Phe Ser Ser Phe Gly Met Ser Trp Val
       290                 295                 300
   Arg Gln Ala Pro Gly Lys Gly Leu Glu Trp Val Ser Ser Ile Ser Gly
   305                 310                 315                 320
   Ser Gly Ser Asp Thr Leu Tyr Ala Asp Ser Val Lys Gly Arg Phe Thr
                   325                 330                 335
   Ile Ser Arg Asp Asn Ala Lys Thr Thr Leu Tyr Leu Gln Met Asn Ser
   340 345 350
   Leu Arg Pro Glu Asp Thr Ala Val Tyr Tyr Cys Thr Ile Gly Gly Ser
   355 360 365
   Leu Ser Arg Ser Ser Gln Gly Thr Leu Val Thr Val Ser Ser
   370 375 380
<210> 475
   <211> 382
   <212> PRT
   <213> Artificial
<220>
   <223> Mutated Lama sequence
<400> 475
   Asp Val Gln Leu Val Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ile Thr Tyr Tyr Ala Asp Ser Val
   50 55 60
   Lys Gly Arg Phe Thr Ile Ser Arg Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Thr Asp Ser Gly Gly Tyr Ile Asp Tyr Asp Cys Met Gly Leu Gly
   100 105 110
   Tyr Asp Tyr Trp Gly Gln Gly Thr Leu Val Thr Val Ser Ser Gly Gly
   115 120 125
   Gly Gly Ser Gly Gly Gly Ser Glu Val Gln Leu Val Glu Ser Gly Gly
   130 135 140
   Gly Leu Val Gln Pro Gly Gly Ser Leu Arg Leu Ser Cys Ala Ala Ser
   145 150 155 160
   Gly Arg Thr Phe Ser Ser Tyr Ala Met Ala Trp Tyr Arg Gln Ala Pro
                   165                 170                 175
   Gly Lys Glu Arg Glu Tyr Val Ala Ala Ile Arg Trp Ser Gly Gly Thr
               180                 185                 190
   Ala Tyr Tyr Ala Asp Ser Val Lys Gly Arg Phe Thr Ile Ser Arg Asp
           195                 200                 205
   Asn Ala Lys Asn Thr Val Tyr Leu Gln Met Asn Ser Leu Arg Pro Glu
       210                 215                 220
   Asp Thr Ala Val Tyr Tyr Cys Ala Asn Arg Ala Pro Asp Thr Arg Leu
   225                 230                 235                 240
   Ala Pro Tyr Glu Tyr Asp His Trp Gly Gln Gly Thr Leu Val Thr Val
                   245                 250                 255
   Ser Ser Gly Gly Gly Gly Ser Gly Gly Gly Ser Glu Val Gln Leu Val
               260                 265                 270
   Glu Ser Gly Gly Gly Leu Val Gln Pro Gly Asn Ser Leu Arg Leu Ser
           275                 280                 285
   Cys Ala Ala Ser Gly Phe Thr Phe Ser Ser Phe Gly Met Ser Trp Val
       290                 295                 300
   Arg Gln Ala Pro Gly Lys Gly Leu Glu Trp Val Ser Ser Ile Ser Gly
   305                 310                 315                 320
   Ser Gly Ser Asp Thr Leu Tyr Ala Asp Ser Val Lys Gly Arg Phe Thr
                   325                 330                 335
   Ile Ser Arg Asp Asn Ala Lys Thr Thr Leu Tyr Leu Gln Met Asn Ser
               340                 345                 350
   Leu Arg Pro Glu Asp Thr Ala Val Tyr Tyr Cys Thr Ile Gly Gly Ser
           355                 360                 365
   Leu Ser Arg Ser Ser Gln Gly Thr Leu Val Thr Val Ser Ser
       370                 375                 380
<210> 476
   <211> 513
   <212> PRT
   <213> Artificial
<220>
   <223> Mutated Lama sequence
<400> 476
   Asp Val Gln Leu Val Glu Ser Gly Gly Gly Leu Val Gln Pro Gly Gly
   1 5 10 15
   Ser Leu Arg Leu Ser Cys Ala Ala Ser Gly Arg Thr Phe Ser Ser Tyr
   20 25 30
   Ala Met Ala Trp Tyr Arg Gln Ala Pro Gly Lys Glu Arg Glu Tyr Val
   35 40 45
   Ala Ala Ile Arg Trp Ser Gly Gly Thr Ala Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Arg Pro Glu Asp Thr Ala Val Tyr Tyr Cys
   85 90 95
   Ala Asn Arg Ala Pro Asp Thr Arg Leu Ala Pro Tyr Glu Tyr Asp His
   100 105 110
   Trp Gly Gln Gly Thr Leu Val Thr Val Ser Ser Gly Gly Gly Gly Ser
   115 120 125
   Gly Gly Gly Ser Glu Val Gln Leu Val Glu Ser Gly Gly Gly Leu Val
   130 135 140
   Gln Pro Gly Asn Ser Leu Arg Leu Ser Cys Ala Ala Ser Gly Phe Thr
   145 150 155 160
   Phe Ser Ser Phe Gly Met Ser Trp Val Arg Gln Ala Pro Gly Lys Gly
   165 170 175
   Leu Glu Trp Val Ser Ser Ile Ser Gly Ser Gly Ser Asp Thr Leu Tyr
   180 185 190
   Ala Asp Ser Val Lys Gly Arg Phe Thr Ile Ser Arg Asp Asn Ala Lys
   195 200 205
   Thr Thr Leu Tyr Leu Gln Met Asn Ser Leu Arg Pro Glu Asp Thr Ala
   210 215 220
   Val Tyr Tyr Cys Thr Ile Gly Gly Ser Leu Ser Arg Ser Ser Gln Gly
   225 230 235 240
   Thr Leu Val Thr Val Ser Ser Gly Gly Gly Gly Ser Gly Gly Gly Ser
                   245                 250                 255
   Glu Val Gln Leu Val Glu Ser Gly Gly Gly Leu Val Gln Pro Gly Gly
               260                 265                 270
   Ser Leu Arg Leu Ser Cys Ala Ala Ser Gly Phe Thr Phe Asp Asp Tyr
           275                 280                 285
   Ala Leu Gly Trp Phe Arg Gln Ala Pro Gly Lys Glu Arg Glu Gly Val
       290                 295                 300
   Ser Cys Ile Arg Cys Ser Gly Gly Ser Thr Tyr Tyr Ala Asp Ser Val
   305                 310                 315                 320
   Lys Gly Arg Phe Thr Ile Ser Ser Asp Asn Ser Lys Asn Thr Val Tyr
                   325                 330                 335
   Leu Gln Met Asn Ser Leu Arg Pro Glu Asp Thr Ala Val Tyr Tyr Cys
               340                 345                 350
   Ala Ala Ser Ile Val Pro Arg Ser Lys Leu Glu Pro Tyr Glu Tyr Asp
           355                 360                 365
   Ala Trp Gly Gln Gly Thr Leu Val Thr Val Ser Ser Gly Gly Gly Gly
       370                 375                 380
   Ser Gly Gly Gly Ser Glu Val Gln Leu Val Glu Ser Gly Gly Gly Leu
   385                 390                 395                 400
   Val Gln Pro Gly Gly Ser Leu Arg Leu Ser Cys Ala Ala Ser Gly Phe
                   405                 410                 415
   Thr Phe Asp Asp Tyr Ala Leu Gly Trp Phe Arg Gln Ala Pro Gly Lys
               420                 425                 430
   Glu Arg Glu Gly Val Ser Cys Ile Arg Cys Ser Gly Gly Ser Thr Tyr
           435                 440                 445
   Tyr Ala Asp Ser Val Lys Gly Arg Phe Thr Ile Ser Ser Asp Asn Ser
       450                 455                 460
   Lys Asn Thr Val Tyr Leu Gln Met Asn Ser Leu Arg Pro Glu Asp Thr
   465                 470                 475                 480
   Ala Val Tyr Tyr Cys Ala Ala Ser Ile Val Pro Arg Ser Lys Leu Glu
                   485                 490                 495
   Pro Tyr Glu Tyr Asp Ala Trp Gly Gln Gly Thr Leu Val Thr Val Ser
   500 505 510
   Ser
<210> 477
   <211> 385
   <212> PRT
   <213> Artificial
<220>
   <223> Mutated Lama sequence
<400> 477
   Asp Val Gln Leu Val Glu Ser Gly Gly Gly Leu Val Gln Pro Gly Gly
   1 5 10 15
   Ser Leu Arg Leu Ser Cys Ala Val Ser Gly Ile Thr Leu Asp Asp Tyr
   20 25 30
   Ala Ile Gly Trp Phe Arg Gln Ala Pro Gly Lys Glu Arg Glu Gly Val
   35 40 45
   Ser Ala Ile Arg Ser Ser Gly Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Tyr Gly Glu Gln Trp Tyr Pro
   100 105 110
   Ile Tyr Glu Tyr Asp Ala Trp Gly Gln Gly Thr Leu Val Thr Val Ser
   115 120 125
   Ser Gly Gly Gly Gly Ser Gly Gly Gly Ser Glu Val Gln Leu Val Glu
   130 135 140
   Ser Gly Gly Gly Leu Val Gln Pro Gly Gly Ser Leu Arg Leu Ser Cys
   145 150 155 160
   Ala Ala Ser Gly Arg Thr Phe Ser Ser Tyr Ala Met Ala Trp Tyr Arg
   165 170 175
   Gln Ala Pro Gly Lys Glu Arg Glu Tyr Val Ala Ala Ile Arg Trp Ser
               180                 185                 190
   Gly Gly Thr Ala Tyr Tyr Ala Asp Ser Val Lys Gly Arg Phe Thr Ile
           195                 200                 205
   Ser Arg Asp Asn Ala Lys Asn Thr Val Tyr Leu Gln Met Asn Ser Leu
       210                 215                 220
   Arg Pro Glu Asp Thr Ala Val Tyr Tyr Cys Ala Asn Arg Ala Pro Asp
   225                 230                 235                 240
   Thr Arg Leu Ala Pro Tyr Glu Tyr Asp His Trp Gly Gln Gly Thr Leu
                   245                 250                 255
   Val Thr Val Ser Ser Gly Gly Gly Gly Ser Gly Gly Gly Ser Glu Val
               260                 265                 270
   Gln Leu Val Glu Ser Gly Gly Gly Leu Val Gln Pro Gly Asn Ser Leu
           275                 280                 285
   Arg Leu Ser Cys Ala Ala Ser Gly Phe Thr Phe Ser Ser Phe Gly Met
       290                 295                 300
   Ser Trp Val Arg Gln Ala Pro Gly Lys Gly Leu Glu Trp Val Ser Ser
   305                 310                 315                 320
   Ile Ser Gly Ser Gly Ser Asp Thr Leu Tyr Ala Asp Ser Val Lys Gly
                   325                 330                 335
   Arg Phe Thr Ile Ser Arg Asp Asn Ala Lys Thr Thr Leu Tyr Leu Gln
               340                 345                 350
   Met Asn Ser Leu Arg Pro Glu Asp Thr Ala Val Tyr Tyr Cys Thr Ile
           355                 360                 365
   Gly Gly Ser Leu Ser Arg Ser Ser Gln Gly Thr Leu Val Thr Val Ser
       370                 375                 380
   Ser
   385
<210> 478
   <211> 523
   <212> PRT
   <213> Artificial
<220>
   <223> Mutated Lama sequence
<400> 478
   Asp Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ala Ile Arg Ser Ser Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser Gly Gly Gly Gly Ser Gly Gly Gly Ser Glu Val Gln Leu Val Glu
       130                 135                 140
   Ser Gly Gly Gly Leu Val Gln Pro Gly Gly Ser Leu Arg Leu Ser Cys
   145                 150                 155                 160
   Ala Ala Ser Gly Phe Thr Leu Asp Asp Tyr Ala Ile Gly Trp Phe Arg
                   165                 170                 175
   Gln Ala Pro Gly Lys Glu Arg Glu Gly Val Ser Ala Ile Arg Ser Ser
               180                 185                 190
   Gly Gly Ser Thr Tyr Tyr Ala Asp Ser Val Lys Gly Arg Phe Thr Ile
           195                 200                 205
   Ser Ser Asp Asn Ser Lys Asn Thr Val Tyr Leu Gln Met Asn Ser Leu
       210                 215                 220
   Arg Pro Glu Asp Thr Ala Val Tyr Tyr Cys Ala Ala Val Pro Ala Gly
   225                 230                 235                 240
   Arg Leu Arg Phe Gly Glu Gln Trp Tyr Pro Leu Tyr Glu Tyr Asp Ala
   245 250 255
   Trp Gly Gln Gly Thr Leu Val Thr Val Ser Ser Gly Gly Gly Gly Ser
   260 265 270
   Gly Gly Gly Ser Glu Val Gln Leu Val Glu Ser Gly Gly Gly Leu Val
   275 280 285
   Gln Pro Gly Gly Ser Leu Arg Leu Ser Cys Ala Ala Ser Gly Arg Thr
   290 295 300
   Phe Ser Ser Tyr Ala Met Ala Trp Tyr Arg Gln Ala Pro Gly Lys Glu
   305 310 315 320
   Arg Glu Tyr Val Ala Ala Ile Arg Trp Ser Gly Gly Thr Ala Tyr Tyr
   325 330 335
   Ala Asp Ser Val Lys Gly Arg Phe Thr Ile Ser Arg Asp Asn Ala Lys
   340 345 350
   Asn Thr Val Tyr Leu Gln Met Asn Ser Leu Arg Pro Glu Asp Thr Ala
   355 360 365
   Val Tyr Tyr Cys Ala Asn Arg Ala Pro Asp Thr Arg Leu Ala Pro Tyr
   370 375 380
   Glu Tyr Asp His Trp Gly Gln Gly Thr Leu Val Thr Val Ser Ser Gly
   385 390 395 400
   Gly Gly Gly Ser Gly Gly Gly Ser Glu Val Gln Leu Val Glu Ser Gly
   405 410 415
   Gly Gly Leu Val Gln Pro Gly Asn Ser Leu Arg Leu Ser Cys Ala Ala
   420 425 430
   Ser Gly Phe Thr Phe Ser Ser Phe Gly Met Ser Trp Val Arg Gln Ala
   435 440 445
   Pro Gly Lys Gly Leu Glu Trp Val Ser Ser Ile Ser Gly Ser Gly Ser
   450 455 460
   Asp Thr Leu Tyr Ala Asp Ser Val Lys Gly Arg Phe Thr Ile Ser Arg
   465 470 475 480
   Asp Asn Ala Lys Thr Thr Leu Tyr Leu Gln Met Asn Ser Leu Arg Pro
   485 490 495
   Glu Asp Thr Ala Val Tyr Tyr Cys Thr Ile Gly Gly Ser Leu Ser Arg
   500 505 510
   Ser Ser Gln Gly Thr Leu Val Thr Val Ser Ser
   515 520
<210> 479
   <211> 124
   <212> PRT
   <213> Lama glama
<400> 479
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Phe Asp Asp Tyr
   20 25 30
   Ala Leu Gly Trp Phe Arg Gln Ala Ala Gly Lys Glu Arg Glu Gly Val
   35 40 45
   Ser Cys Ile Arg Cys Ser Asp Gly Ser Thr Tyr Tyr Ala Asp Ser Val
   50 55 60
   Lys Gly Arg Phe Thr Ile Ser Ser Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Ser Ile Val Pro Arg Ser Lys Leu Glu Pro Tyr Glu Tyr Asp
   100 105 110
   Ala Trp Gly Gln Gly Thr Gln Val Thr Val Ser Ser
   115 120
<210> 480
   <211> 126
   <212> PRT
   <213> Lama glama
<400> 480
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
   20 25 30
   Ala Ile Gly Trp Phe Arg Gln Val Pro Gly Lys Glu Arg Glu Gly Val
   35 40 45
   Ser Cys Ile Ser Ser Ser Asp Gly Ile Thr Tyr Tyr Val Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Thr Asp Ser Gly Gly Tyr Ile Asp Tyr Asp Cys Met Gly Leu Gly
   100 105 110
   Tyr Asp Tyr Trp Gly Gln Gly Thr Gln Val Thr Val Ser Ser
   115 120 125
<210> 481
   <211> 129
   <212> PRT
   <213> Lama glama
<400> 481
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Cys Ile Arg Asp Ser Asp Gly Ser Thr Tyr Tyr Ala Asp Ser Val
   50 55 60
   Lys Gly Arg Phe Thr Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Gln Val Thr Val Ser
   115 120 125
   Ser
<210> 482
   <211> 129
   <212> PRT
   <213> Artificial
<220>
   <223> Mutated lama sequence
<400> 482
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser
<210> 483
   <211> 124
   <212> PRT
   <213> Artificial
<220>
   <223> Mutated lama sequence
<400> 483
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asp Asp Tyr
   20 25 30
   Ala Leu Gly Trp Phe Arg Gln Ala Pro Gly Lys Glu Arg Glu Gly Val
   35 40 45
   Ser Cys Ile Arg Cys Ser Asp Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Ser Ile Val Pro Arg Ser Lys Leu Glu Pro Tyr Glu Tyr Asp
   100 105 110
   Ala Trp Gly Gln Gly Thr Leu Val Thr Val Ser Ser
   115 120
<210> 484
   <211> 126
   <212> PRT
   <213> Artificial
<220>
   <223> Mutated lama sequence
<400> 484
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ile Thr Tyr Tyr Ala Asp Ser Val
   50 55 60
   Lys Gly Arg Phe Thr Ile Ser Arg Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Thr Asp Ser Gly Gly Tyr Ile Asp Tyr Asp Cys Met Gly Leu Gly
   100 105 110
   Tyr Asp Tyr Trp Gly Gln Gly Thr Leu Val Thr Val Ser Ser
   115 120 125
<210> 485
   <211> 124
   <212> PRT
   <213> Artificial
<220>
   <223> Mutated lama sequence
<400> 485
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Leu Gly Trp Phe Arg Gln Ala Pro Gly Lys Glu Arg Glu Gly Val
   35 40 45
   Ser Cys Ile Arg Cys Ser Gly Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Ser Ile Val Pro Arg Ser Lys Leu Glu Pro Tyr Glu Tyr Asp
   100 105 110
   Ala Trp Gly Gln Gly Thr Leu Val Thr Val Ser Ser
   115 120
<210> 486
   <211> 126
   <212> PRT
   <213> Artificial
<220>
   <223> Mutated lama sequence
<400> 486
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ala Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Gly Gly Ile Thr Tyr Tyr Ala Asp Ser Val
       50                  55                  60
   Lys Gly Arg Phe Thr Ile Ser Arg Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Thr Asp Ser Gly Gly Tyr Ile Asp Tyr Asp Cys Ser Gly Leu Gly
               100                 105                 110
   Tyr Asp Tyr Trp Gly Gln Gly Thr Leu Val Thr Val Ser Ser
           115                 120                 125
<210> 487
   <211> 129
   <212> PRT
   <213> Artificial
<220>
   <223> Mutated lama sequence
<400> 487
   Glu Val Gln Leu Val Glu Ser Gly Gly Gly Leu Val Gln Pro Gly Gly
   1 5 10 15
   Ser Leu Arg Leu Ser Cys Ala Val Ser Gly Ile Thr Leu Asp Asp Tyr
   20 25 30
   Ala Ile Gly Trp Phe Arg Gln Ala Pro Gly Lys Glu Arg Glu Gly Val
   35 40 45
   Ser Ala Ile Arg Ser Ser Gly Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Tyr Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 488
   <211> 129
   <212> PRT
   <213> Artificial
<220>
   <223> Mutated lama sequence
<400> 488
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ala Ile Arg Ser Ser Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser
<210> 489
   <211> 5
   <212> PRT
   <213> Lama glama
<400> 489
   Asp Tyr Ala Leu Gly
   1               5
<210> 490
   <211> 17
   <212> PRT
   <213> Lama glama
<400> 490
   Cys Ile Arg Cys Ser Asp Gly Ser Thr Tyr Tyr Ala Asp Ser Val Lys
   1               5                   10                  15
   Gly
<210> 491
   <211> 15
   <212> PRT
   <213> Lama glama
<400> 491
   Ser Ile Val Pro Arg Ser Lys Leu Glu Pro Tyr Glu Tyr Asp Ala
   1 5 10 15
<210> 492
   <211> 5
   <212> PRT
   <213> Lama glama
<400> 492
   Tyr Tyr Ala Ile Gly
   1 5
<210> 493
   <211> 17
   <212> PRT
   <213> Lama glama
<400> 493
   Cys Ile Ser Ser Ser Asp Gly Ile Thr Tyr Tyr Val Asp Ser Val Lys
   1 5 10 15
   Gly
<210> 494
   <211> 17
   <212> PRT
   <213> Lama glama
<400> 494
   Asp Ser Gly Gly Tyr Ile Asp Tyr Asp Cys Met Gly Leu Gly Tyr Asp
   1 5 10 15
   Tyr
<210> 495
   <211> 5
   <212> PRT
   <213> Lama glama
<400> 495
   Asp Tyr Ala Ile Gly
   1 5
<210> 496
   <211> 17
   <212> PRT
   <213> Lama glama
<400> 496
   Cys Ile Arg Asp Ser Asp Gly Ser Thr Tyr Tyr Ala Asp Ser Val Lys
   1               5                   10                  15
   Gly
<210> 497
   <211> 20
   <212> PRT
   <213> Lama glama
<400> 497
   Val Pro Ala Gly Arg Leu Arg Phe Gly Glu Gln Trp Tyr Pro Leu Tyr
   1               5                   10                  15
   Glu Tyr Asp Ala
               20
<210> 498
   <211> 5
   <212> PRT
   <213> Lama glama
<400> 498
   Asp Tyr Ala Ile Gly
   1               5
<210> 499
   <211> 17
   <212> PRT
   <213> Lama glama
<400> 499
   Ser Ile Arg Asp Asn Asp Gly Ser Thr Tyr Tyr Ala Asp Ser Val Lys
   1               5                   10                  15
   Gly
<210> 500
   <211> 20
   <212> PRT
   <213> Lama glama
<400> 500
   Val Pro Ala Gly Arg Leu Arg Phe Gly Glu Gln Trp Tyr Pro Leu Tyr
   1               5                   10                  15
   Glu Tyr Asp Ala
               20
<210> 501
   <211> 5
   <212> PRT
   <213> Lama glama
<400> 501
   Asp Tyr Ala Leu Gly
   1 5
<210> 502
   <211> 17
   <212> PRT
   <213> Lama glama
<400> 502
   Cys Ile Arg Cys Ser Asp Gly Ser Thr Tyr Tyr Ala Asp Ser Val Lys
   1 5 10 15
   Gly
<210> 503
   <211> 15
   <212> PRT
   <213> Lama glama
<400> 503
   Ser Ile Val Pro Arg Ser Lys Leu Glu Pro Tyr Glu Tyr Asp Ala
<210> 504
   <211> 5
   <212> PRT
   <213> Lama glama
<400> 504
   Tyr Tyr Ala Ile Gly
   1 5
<210> 505
   <211> 17
   <212> PRT
   <213> Lama glama
<400> 505
   Cys Ile Ser Ser Ser Asp Gly Ile Thr Tyr Tyr Ala Asp Ser Val Lys
   1 5 10 15
   Gly
<210> 506
   <211> 17
   <212> PRT
   <213> Lama glama
<400> 506
   Asp Ser Gly Gly Tyr Ile Asp Tyr Asp Cys Met Gly Leu Gly Tyr Asp
   1 5 10 15
   Tyr
<210> 507
   <211> 5
   <212> PRT
   <213> Lama glama
<400> 507
   Asp Tyr Ala Leu Gly
   1 5
<210> 508
   <211> 17
   <212> PRT
   <213> Lama glama
<400> 508
   Cys Ile Arg Cys Ser Gly Gly Ser Thr Tyr Tyr Ala Asp Ser Val Lys
   1 5 10 15
   Gly
<210> 509
   <211> 15
   <212> PRT
   <213> Lama glama
<400> 509
   Ser Ile Val Pro Arg Ser Lys Leu Glu Pro Tyr Glu Tyr Asp Ala
   1 5 10 15
<210> 510
   <211> 5
   <212> PRT
   <213> Lama glama
<400> 510
   Tyr Tyr Ala Ile Gly
   1 5
<210> 511
   <211> 17
   <212> PRT
   <213> Lama glama
<400> 511
   Cys Ile Ser Ser Ser Gly Gly Ile Thr Tyr Tyr Ala Asp Ser Val Lys
   1               5                   10                  15
   Gly
<210> 512
   <211> 17
   <212> PRT
   <213> Artificial
<220>
   <223> Mutated lama sequence
<400> 512
   Asp Ser Gly Gly Tyr Ile Asp Tyr Asp Cys Ser Gly Leu Gly Tyr Asp
   1               5                   10                  15
   Tyr
<210> 513
   <211> 5
   <212> PRT
   <213> Lama glama
<400> 513
   Asp Tyr Ala Ile Gly
   1               5
<210> 514
   <211> 17
   <212> PRT
   <213> Artificial
<220>
   <223> Mutated lama sequence
<400> 514
   Ala Ile Arg Ser Ser Gly Gly Ser Thr Tyr Tyr Ala Asp Ser Val Lys
   1               5                   10                  15
   Gly
<210> 515
   <211> 20
   <212> PRT
   <213> Artificial
<220>
   <223> Mutated lama sequence
<400> 515
   Val Pro Ala Gly Arg Leu Arg Tyr Gly Glu Gln Trp Tyr Pro Ile Tyr
   1 5 10 15
   Glu Tyr Asp Ala
   20
<210> 516
   <211> 5
   <212> PRT
   <213> Lama glama
<400> 516
   Asp Tyr Ala Ile Gly
   1 5
<210> 517
   <211> 17
   <212> PRT
   <213> Artificial
<220>
   <223> Mutated lama sequence
<400> 517
   Ala Ile Arg Ser Ser Gly Gly Ser Thr Tyr Tyr Ala Asp Ser Val Lys
   1 5 10 15
   Gly
<210> 518
   <211> 20
   <212> PRT
   <213> Artificial
<220>
   <223> Mutated lama sequence
<400> 518
   Val Pro Ala Gly Arg Leu Arg Phe Gly Glu Gln Trp Tyr Pro Leu Tyr
   1 5 10 15
   Glu Tyr Asp Ala
   20
<210> 519
   <211> 115
   <212> PRT
   <213> Artificial
<220>
   <223> Mutated lama sequence
<400> 519
<210> 520
   <211> 5
   <212> PRT
   <213> Lama glama
<400> 520
   Ser Phe Gly Met Ser
   1               5
<210> 521
   <211> 17
   <212> PRT
   <213> Lama glama
<400> 521
   Ser Ile Ser Gly Ser Gly Ser Asp Thr Leu Tyr Ala Asp Ser Val Lys
   1               5                   10                  15
   Gly
<210> 522
   <211> 6
   <212> PRT
   <213> Lama glama
<400> 522
   Gly Gly Ser Leu Ser Arg
   1 5
<210> 523
   <211> 5
   <212> PRT
   <213> Lama glama
<400> 523
   Leu Asn Leu Met Gly
   1 5
<210> 524
   <211> 16
   <212> PRT
   <213> Lama glama
<400> 524
   Thr Ile Thr Val Gly Asp Ser Thr Asn Tyr Ala Asp Ser Val Lys Gly
   1 5 10 15
<210> 525
   <211> 8
   <212> PRT
   <213> Lama glama
<400> 525
   Arg Arg Thr Trp His Ser Glu Leu
   1 5
<210> 526
   <211> 5
   <212> PRT
   <213> Lama glama
<400> 526
   Ile Asn Leu Leu Gly
   1 5
<210> 527
   <211> 16
   <212> PRT
   <213> Lama glama
<400> 527
   Thr Ile Thr Val Gly Asp Ser Thr Ser Tyr Ala Asp Ser Val Lys Gly
   1 5 10 15
<210> 528
   <211> 8
   <212> PRT
   <213> Lama glama
<400> 528
   Arg Arg Thr Trp His Ser Glu Leu
   1 5
<210> 529
   <211> 5
   <212> PRT
   <213> Lama glama
<400> 529
   Ser Phe Gly Met Ser
   1 5
<210> 530
   <211> 17
   <212> PRT
   <213> Lama glama
<400> 530
   Ser Ile Asn Gly Arg Gly Asp Asp Thr Arg Tyr Ala Asp Ser Val Lys
   1 5 10 15
   Gly
<210> 531
   <211> 7
   <212> PRT
   <213> Lama glama
<400> 531
   Gly Arg Ser Val Ser Arg Ser
   1 5
<210> 532
   <211> 5
   <212> PRT
   <213> Lama glama
<400> 532
   Ser Phe Gly Met Ser
   1 5
<210> 533
   <211> 17
   <212> PRT
   <213> Lama glama
<400> 533
   Ala Ile Ser Ala Asp Ser Ser Asp Lys Arg Tyr Ala Asp Ser Val Lys
   1 5 10 15
   Gly
<210> 534
   <211> 5
   <212> PRT
   <213> Lama glama
<400> 534
   Gly Arg Gly Ser Pro
   1 5
<210> 535
   <211> 5
   <212> PRT
   <213> Lama glama
<400> 535
   Ser Phe Gly Met Ser
   1 5
<210> 536
   <211> 17
   <212> PRT
   <213> Lama glama
<400> 536
   Ala Ile Ser Ala Asp Ser Ser Asp Lys Arg Tyr Ala Asp Ser Val Lys
   1 5 10 15
   Gly
<210> 537
   <211> 5
   <212> PRT
   <213> Lama glama
<400> 537
   Gly Arg Gly Ser Pro
   1 5
<210> 538
   <211> 5
   <212> PRT
   <213> Lama glama
<400> 538
   Asn Tyr Trp Met Tyr
   1 5
<210> 539
   <211> 17
   <212> PRT
   <213> Lama glama
<400> 539
   Arg Ile Ser Thr Gly Gly Gly Tyr Ser Tyr Tyr Ala Asp Ser Val Lys
   1 5 10 15
   Gly
<210> 540
   <211> 13
   <212> PRT
   <213> Lama glama
<400> 540
   Asp Arg Glu Ala Gln Val Asp Thr Leu Asp Phe Asp Tyr
   1 5 10